# EUROPEAN PATENT SPECIFICATION

(11) **EP 0 910 406 B1**
(45) Date of publication and mention of the grant of the patent: **06.12.2006**
(21) Application number: 97915994.4
(22) Date of filing: 14.02.1997
(51) Int. Cl.: A61K 39/245, C12N 7/01, C12N 5/00, C07K 1/00, C07K 16/00, C07K 14/03, C12N 15/38

(54) **RECOMBINANT CANINE HERPESVIRUSES**
REKOMBINANTE HUNDE-HERPESVIREN
HERPESVIRUS CANINS RECOMBINES

(30) Priority: 15.02.1996 US 602010
(43) Date of publication of application: 28.04.1999
(73) Proprietor: Heska Corporation, Fort Collins, CO 80525 (US)
(72) Inventor: HAANES, Elizabeth, J., Boulder, CO 80306 (US); FRANK, Rexann, A., Wellington, CO 80549 (US)
(74) Representative: Evens, Paul Jonathan
(86) International application number: PCT/US1997/004115
(87) International publication number: WO 1997/029772

(56) References cited:
- WO-A-95/26751
- US-A- 5 223 424
- REMOND M ET AL: "GENE ORGANIZATION IN THE UL REGION AND INVERTED REPEATS OF THE CANINE HERPESVIRUS GENOME" JOURNAL OF GENERAL VIROLOGY,GB,SOCIETY FOR GENERAL MICROBIOLOGY, READING, vol. 77, no. PART 01, 1996, page 37-48 XP000543180 ISSN: 0022-1317
- REMOND M ET AL: "SEQUENCE OF THE CANINE HERPESVIRUS THYMIDINE KINASE GENE: TAXON-PREFERRED AMINO ACID RESIDUES IN THE ALPHAHERPESVIRAL THYMIDINE KINASES" VIRUS RESEARCH,NL,AMSTERDAM, vol. 39, 1995, page 341-354 XP000646056 ISSN: 0168-1702
- TYACK S G ET AL: "Nucleotide sequence of canine herpesvirus homologues of herpes simplex virus type 1 US2, US3, glycoproteins I and E, US8.5 and US9 genes." DNA SEQUENCE, (1997) 7 (6) 365-8. , XP002122947
- HAANES E J ET AL: "Genomic organization of the canine herpesvirus US region." VIRUS RESEARCH, (1998 FEB) 53 (2) 151-62. , XP002122948
- XUAN X ET AL: "Construction of canine herpesvirus vector expressing foreign genes using a lacZ-TK gene cassette as a double selectional marker." VIRUS GENES, (1998) 17 (1) 25-32. , XP002122949
- VIROLOGY, July 1993, Vol. 195, No. 1, LIANG et al., "Identification and Deletion Mutagenesis of the Bovine Herpesvirus 1 dUTPase Gene and Gene Homologous to Herpes Simplex Virus/JL49.5", pages 42-50.
- ARCH. VIROL., 1993, Vol. 133, No. 1-2, RIGGIO et al., "DNA Sequence of a Gene Cluster in the Equine Herpesvirus-4 Genome Which Contains a Newly Identified Herpesvirus Gene Encoding a Membrane Protein", pages 171-178.
- J. VIROL., November 1992, Vol. 66, No. 11, PYLES et al., "Herpes Simplex Virus Type 1 dUTPase Mutants are Attenuated for Neurovirulence, Neuroinvasiveness and Reactivation from Latency", pages 6706-6713.
- J. VIROL., July 1994, Vol. 68, No. 7, PYLES et al., "Mutations in Accessory DNA Replicating Functions Alter the Relative Mutation Frequency of Herpes Simplex Virus Type 1 Strains in Cultured Murine Cells", pages 4514-4524.

## Description

### FIELD OF THE INVENTION

The present invention relates to novel isolated CHV nucleic acid molecules, to proteins encoded by such nucleic acid molecules, and to use of such CHV nucleic acid molecules to insert heterologous nucleic acid molecules into CHV genomes.

The present invention also relates to isolated canine herpesvirus (CHV) nucleic acid molecules comprising recombinant CHV genomes, including those that contain heterologous nucleic acid molecules. The present invention also relates to the use of such genomes and viruses in a variety of applications, including therapeutic compositions to protect animals from disease.

### BACKGROUND OF THE INVENTION

Dogs and other canids are affected by a number of diseases against which it would be desirable to develop protective vaccines. Live vaccines, and particularly live viral vector vaccines, are attractive vaccine vector candidates as they appear to be associated with longer-lasting immunity than inactivated virus vaccines or subunit vaccines. One disadvantage of live vaccines, however, has been that attenuated virus strains often revert to virulence. Another disadvantage has been the host range of a number of viral vaccines. In an attempt to deliver genes to an animal, several viral and bacterial systems, such as poxviruses, adenoviruses, *Salmonella,* and BCG (*Bacillus Calmette-Guerin*), have been genetically manipulated to generate vectors containing heterologous antigen genes in order to immunize a host with a vaccine in which the antigens are presented in a "live" configuration. See, for example, the following two review articles: Esposito et al., pp. 195-247, 1989, *Advances in Veterinary Science and Comparative Medicine,* Vol. 33; Dougan et al., pp. 271-300, 1989, *Advances in Veterinary science and Comparative Medicine,* Vol. 33.

Several herpes virus vaccines, such as those based on bovine herpes virus (BHV), cytomegalovirus (CMV), Epstein Barr virus (EBV), equine herpes virus (EHV), feline herpes virus (FHV) , herpes simplex virus (HSV) , Marek' s disease virus (MDV) , pseudorabies virus (PRV), turkey herpes virus (HVT), and varicella zoster virus (VZV) have been developed and several have shown at least some efficacy as vaccines against the virus per se or as vectors carrying other genes in certain indications. The listed herpes viruses, however, also have the drawback that even if attenuated, they are subject to reversion.

Canine herpes virus (CHV) infection is a relatively benign infection except in newborn puppies. A few vaccines to protect against CHV infection have been reported including a small-plaque variant CHV vaccine disclosed in U.S. Patent No. 4,213,965, by Carmichael, issued July 22, 1980. The nucleotide sequences of CHV genes encoding gB, gC, gD and UL45 homologs have been reported by Limbach et al., 1994, *J*. *Gen. Virol. 75,* 2029-2039, but these proteins, while proposed as vaccine candidates against CHV, were not tested as such by Limbach et al., *ibid*. In addition, the complete nucleotide sequence of the CHV thymidine kinase (tk) gene was reported by Remond et al., 1995, *Virus Res., 39*, 341-354. More recently, a restriction map of the CHV genome, as well as a number of small, incomplete sequences in from the ends of various restriction fragments were reported by Remond, et al., 1996, *J*. *Gen. Virol., 77,* 37-48. These small sequences were used to infer the placement of certain genes within the CHV genome.

The inventors are not aware of any reports which describe the use of CHV as a vaccine vector, either with respect to inactivating genes in the CHV genome using recombinant DNA techniques, and/or to delivering protective compounds to a canid, in spite of the need to develop safe and efficacious delivery systems to protect canids, and especially dogs, from disease. Two U.S. patents (i.e., U.S. Patent No. 5,266,489, by Rey-Senelonge et al., issued November 30, 1993; and U.S. Patent No. 5,223,424, by Cochran et al., issued June 29, 1993) at best speculate on the insertion of genes into certain CHV loci, but neither claims CHV vectors or vaccines, nor provides data supporting such speculations. US 5,266,489, *ibid*., claimed HVT having a foreign gene inserted into the ribonucleotide reductase (RR) small subunit gene of the HVT genome, but also disclosed without support the insertion of foreign genes into the RR small subunit genes of BHV, CHV, CMV, duck herpes virus, EBV, EHV, FHV, HSV, PRV and VZV. The inventors, however, have demonstrated the inaccuracy of this disclosure in that the inventors have found, and disclosed in the present application, that the CHV genome lacks the RR small subunit gene. That is, the CHV RR small subunit gene does not exist to provide a target for the insertion of foreign genes. This result was similarly verified by Remond, et al., 1996, *ibid*.

US 5,223,424, *ibid*., claimed specific hybrid PRV constructs having deletions in the TK, repeat, or and/or gX regions and heterologous sequences inserted into the repeat and/or gX regions, but also proposed without data the ability to insert foreign genes into the repeat region of the CHV genome, even though that genome had not yet been mapped. Also disclosed were certain BHV and HVT constructs and proposals, without data, to delete and insert genes in other herpesviruses, such as EHV and FHV, HSV and MDV. It is also of note that a patent application (now U.S. 5,273,876, by Hock et al., issued December 28, 1993) that was filed significantly later than US 5,223,424, *ibid*., and shares two co-inventors with US 5,223,424, *ibid*., states in column 2, lines 57-61, "Among the herpesviruses, only four herpesviruses (herpes simplex of humans, herpes saimiri of monkeys, pseudorabies virus and varicella-zoster virus) have been engineered to contain foreign DNA sequences previous to this disclosure," thereby indicating the lack of CHV, or a number of other, herpes virus vectors.

Thus, there remains a need for new and improved methods to vaccinate canids to protect them from diseases, such as those caused by genetic or metabolic disorders as well as those caused by infectious agents such as protozoan parasites, helminth parasites, ectoparasites, fungi, bacteria, and viruses.

### SUMMARY OF THE INVENTION

The present invention relates to an isolated CHV nucleic acid molecule or protein as defined in the claims and compositions thereof for protecting animals from disease using a recombinant virus or virus genome. When a recombinant CHV of the present invention is administered to an animal, the virus is able to infect cells within the animal. Infected cells are able to express nucleic acid sequences present on the recombinant CHV genome to produce protective compounds, such as proteins and RNAs, capable of protecting the animal from a variety of diseases. Using methods taught in the present invention, vaccines can be generated that are capable of protecting an animal from any disease for which a protective compound can be produced. As such, the present invention is of extremely broad scope and includes a wide variety of vaccines that have a variety of applications.

The present invention includes an isolated CHV nucleic acid molecule that comprises a recombinant CHV genome. The invention also includes recombinant CHV genomes. In one embodiment, the recombinant CHV has an inactive gene within its genome, with a preferred recombinant CHV in this embodiment being a CdUTPase negative CHV, a CgC negative CHV, a CgE negative CHV, a CgG negative CHV, a CgI negative CHV, a CPK negative CHV, a CTK negative CHV, a CIR6 negative CHV, a CUS2 negative CHV, a CUS9 negative CHV, a CUL49 negative CHV, a CUL51 negative CHV, a CUL45 negative CHV, a CgD negative CHV, a CgB negative CHV, a CUL48 negative CHV, a CUL52 negative CHV, a CgL negative CHV, a CUL49.5 negative CHV, a CICPO negative CHV, a CICP4 negative CHV, and/or a CUS8.5 negative CHV. In the case of the CUS8.5 negative CHV, a CUS8.5 negative CHV refers to a CHV in which the CUS8.5 open reading frame is disrupted.

The present invention also includes an isolated CHV nucleic acid molecule comprising a recombinant CHV genome that comprises a heterologous nucleic acid molecule, which preferably encodes a protective compound that protects a canid from disease. Such a heterologous nucleic acid molecule can be located in an essential gene, a nonessential gene, and/or in an intergenic region. Insertion of a heterologous nucleic acid into a CHV genome can, but need not, inactivate a gene within that genome. Also included in the present invention are methods to produce a recombinant CHV and a recombinant CHV genome, as well as a canine cell line that complements a defect in a gene essential to CHV reproduction *in vitro*.

One embodiment of the present invention is a method to increase recombinant CHV plaque forming efficiency. Such a method can include the steps of introducing a recombinant CHV genome into a canine cell expressing CHV alpha trans-inducing factor and culturing the cell to produce recombinant CHV. In another embodiment, the method includes the steps of co-introducing a recombinant CHV genome and a CHV alpha trans-inducing factor gene into a canine cell and culturing the cell to produce recombinant CHV. The present invention also includes a canine cell line comprising a CHV alpha trans-inducing factor gene.

One embodiment of the present invention is a therapeutic composition comprising an isolated CHV nucleic and molecule as described herein. Animals may be protected from disease by administering such a therapeutic composition to the animal.

The present invention also includes an isolated CHV nucleic acid molecule consisting of:
(a) a nucleic acid sequence that is any of

| | |
|---|---|
| a CdUTPase gene | SEQ ID NO: 13, 15, 77 or 79, |
| a CgE gene | SEQ ID NO: 51 or 57, |
| a CgG gene | SEQ ID NO: 1, 9 or 51, |
| a CgI gene | SEQ ID NO: 29, 32, 51 or 55, |
| a CPK gene | SEQ ID NO: 1, 7 or 51, |
| a CUS2 gene | SEQ ID NO: 2, 5 or 52, |
| a CUS9 gene | SEQ ID NO: 17, 20, 51 or 61, |
| a CUL49 gene | SEQ ID NO: 65, 78 or 83, |
| a CUL51 gene | SEQ ID NO: 14, 33 or 78, |
| a CgL gene | SEQ ID NO: 74 or 75, |
| a CUL48.5 gene | SEQ ID NO: 78 or 81, |
| a CUS8.5 open reading frame | SEQ ID NO: 51 or 59, |

SEQ ID NO:3, SEQ ID NO:19, SEQ ID NO:31 and SEQ ID NO:73; or
(b) a nucleic acid sequence that is fully complementary to a nucleic acid sequence of (a); or a nucleic acid sequence that is at least 80% identical to a nucleic acid sequence of any of SEQ ID NO:'s 1 to 3, 51, 52, 63, 64, 67, 69 to 71 and 85 to 87, and complements of such nucleic acid sequences; or a nucleic acid sequence that is at least 80% identical to a nucleic acid sequence of any of SEQ ID NO:'s 5, 7, 9, 13 to 15, 17, 19, 20, 29, 31 to 33, 55, 57, 59, 61, 65, 73 to 75, 77 to 79, 81 and 83, and complements of such nucleic acid sequences; or a nucleic acid sequence of any of SEQ ID NOs: 1 to 3, 5, 7, 9, 13 to 15, 17, 19, 20, 29, 31 to 33, 51, 52, 55, 57, 59, 61, 65, 73 to 75, 77 to 79, 81 and 83, and complements of such nucleic acid sequences; or that is an allelic variant of a CHV nucleic acid molecule consisting of any such nucleic acid sequence; or
which encodes either:
(a) a protein consisting of an amino acid sequence of any of SEQ ID NOs: 4, 6, 8, 10, 16, 18, 30, 34, 56, 58, 60, 62, 66, 76, 80, 82 and 84; or
(b) a protein encoded by an allelic variant of a CHV nucleic acid molecule encoding a protein according to (a). The present invention also includes CHV proteins encoded by such CHV nucleic acid molecules and antibodies that selectively bind to such CHV proteins, as well as methods to produce such CHV nucleic acid molecules, recombinant molecules, recombinant viruses, recombinant vectors, recombinant cells, CHV proteins and antibodies. Also included in the present invention are therapeutic compositions including such CHV nucleic acid molecules, CHV proteins and antibodies, which compositions may be used to protect an animal from disease.

### BRIEF DESCRIPTION OF THE FIGURES

Fig. 1 depicts a schematic map of two contiguous *Hin*dIII fragments that include at least portions of CHV UL48 UL49, dUTPase, UL51 and UL52 genes.
Fig. 2 depicts a schematic map of the *Asc*I fragment that includes the unique short region of CHV.
Fig. 3 depicts a schematic map of the *Asc*I/blunt fragment that includes the terminal inverted repeat region of CHV.
Fig. 4 depicts a schematic map of a region of the CHV genome spanning at least from the CUS9 gene through the CIR6 gene.
Fig. 5 depicts a schematic map of a region of the CHV genome spanning at least from a portion of the CUL52 gene through at least a portion of the CUL48 gene.

### DETAILED DESCRIPTION OF THE INVENTION

The present invention relates to canine herpesviruses (CHV), and particularly to isolated CHV nucleic acid molecules including those comprising recombinant CHV genomes, as well as to methods to produce and use such viruses and genomes. The present invention also includes identification of regions of the CHV genome that are preferred targets for deletion and/or for insertion of heterologous nucleic acid molecules. As used herein, the term "canine" refers to "of the family Canidae" and, as such, includes reference to dogs (including wild and domesticated dogs), foxes, wolves, jackals, coyotes, and other members of the family Canidae. Similarly, a "canid" is any member of the family Canidae. As used herein, the term CHV refers to any herpesvirus endogenous to the family Canidae. A particularly preferred CHV of the present invention is a recombinant CHV that infects dogs.

One embodiment of the present invention is the use of an isolated CHV nucleic acid molecule, and particularly of one comprising a recombinant CHV genome that includes a (i.e., one or more than one) heterologous nucleic acid molecule, as a live CHV-based therapeutic composition, or vaccine, to protect an animal from a disease against which the heterologous nucleic acid molecule is targeted. Live vaccines are advantageous because they are believed to confer more vigorous and longer-lasting immunity than subunit or killed vaccines. While not being bound by theory, it is believed that such advantages are due to the ability of the genetic information carried by the virus to enter cells of the treated animal, replicate itself, and direct the expression of a protective compound, such as a protective protein or a protective RNA, for extended periods of time. Thus, the therapeutic composition need not be administered frequently and, at least in some embodiments, the virus vaccine can function as a self-booster.

CHV is particularly useful as a delivery vehicle for a heterologous nucleic acid molecule to protect canids from a disease for a variety of reasons, including, but not limited to, CHV's limited host range and pathogenicity. CHV, to the inventors' knowledge, has the most limited host range of any herpesvirus, including FHV, in that CHV's host range appears to be substantially limited to canine cells. As far as the inventors are aware, the only report of CHV infection of a cell type other than canine cells is limited infection of mink lung cells; see, for example, Peterson et al., 1988, *Comp. Immunol. Microbiol. Infect. Diseases 11,* 93-98. This limited host range illustrates the safety of CHV in that the virus apparently cannot be transmitted to a variety of species unrelated to canids.

Furthermore, CHV exhibits only limited pathogenicity. Although the virus has been shown to cause a fatal hemorrhagic disease in hypothermic neonatal pups (i.e., essentially all pups experimentally infected with CHV and maintained at room temperature (i.e., from about 25°C to 27°C ) within a week of birth die from the infection), CHV causes insignificant respiratory infection in adult dogs; see, for example, Carmichael, 1970, *J. Am. Vet. Med. Assn. 156,* 1714-1721. Moreover, prolonged survival or recovery of experimentally infected neonatal pups maintained at 38.4°C to 39.5°C was observed. Adult dogs exposed to CHV do, however, become infected since virus shedding has been shown to occur for at least two weeks post-inoculation; and latency is postulated to occur, since CHV has been isolated from primary cultured cells of normal healthy dogs; see, for example, Carmichael, *ibid.* Furthermore, maternal antibody, or passive transfer of antibody from seropositive dogs has been shown to protect puppies from an otherwise fatal CHV challenge; see, for example, Carmichael, *ibid*. Due to its limited pathogenicity, CHV apparently need not be attenuated to the extent required for other viruses used as live vaccine vectors. In addition, vaccination of a dam with CHV can lead to passive protection in her pups.

Another advantage of CHV is its limited temperature range. CHV grows well at temperatures ranging from about 34°C to about 36°C, with optimal growth occurring at about 35°C. CHV, however, does not grow well at temperatures less than or equal to about 33°C or at temperatures greater than or equal to about 37°C. As such CHV is significantly more temperature sensitive than any other known wild type herpesvirus, including FHV.

Yet another advantage of CHV is its potential for use as a single, multivalent therapeutic composition against a variety of canine pathogens. That is, the CHV genome can be manipulated to incorporate multiple heterologous nucleic acid molecules without disrupting the ability of the genome to be packaged (i.e., assembled) into a live virus. Examples of multivalent therapeutic compositions are described below.

As far as the inventors are aware, this application is the first report of the genetic engineering of a CHV genome, particularly for the development of efficacious canid vaccines, in spite of a long felt need for efficacious vaccines against canine pathogens. The inventors have developed methods to identify CHV genes and intergenic regions, particularly those having utility as targets for the insertion of heterologous nucleic acid molecules, despite the difficulty of using known herpesvirus sequences to identify such regions due to the AT-rich nature of the CHV genome. The CHV genome contains about 70% adenosine and thymidine residues, compared to other known herpesvirus genomes which, on the average, contain from about 30% to about 58% adenosine and thymidine residues (e.g., HSV, BHV, and PRV contain about 30%, EHV about 54%, and FHV about 58%, adenosine and thymidine residues). As such, it is very difficult to design primers or probes using known herpesvirus sequences to identify CHV analogs.

One embodiment of the present invention comprises a recombinant CHV. As used herein, a recombinant CHV is a CHV that comprises (i.e., has or includes) a genome that has been genetically engineered (i.e., subjected to recombinant nucleic acid (i.e., DNA or RNA) techniques, such as those disclosed in Sambrook et al., 1989, *Molecular Cloning: A Laboratory Manual,* Cold Spring Harbor Labs Press) to differ from the genome of a natural CHV isolate (i.e., a herpesvirus endogenous to the family canidae). Such a genetically engineered genome is referred to herein as a recombinant CHV genome and is described in more detail below.

An isolated CHV nucleic acid molecule of the present invention may comprise not only a recombinant CHV genome but also an envelope and capsid in which the genome is packaged. The viral envelope and capsid are preferably a CHV envelope and a CHV capsid, encoded at least in part by CHV genes, thereby imparting to the recombinant CHV the host range of a natural CHV isolate. It is to be noted, however, that the present invention also includes recombinant CHV having envelopes and/or capsids that have been modified to, for example, alter (e.g., broaden, narrow, or completely change) the host range of the recombinant CHV genome. Such modifications can be accomplished by one skilled in the art by, for example, modifying CHV envelope and/or capsid genes and/or replacing such genes with those of another virus. Altered genes can be located on the CHV genome itself and/or in the genome of the cell in which the recombinant virus is produced.

A recombinant CHV genome comprised by the present invention is a CHV genome in which nucleotides have been deleted, inserted, substituted or inverted using recombinant techniques known to those skilled in the art such that the recombinant CHV genome is no longer the same as a natural CHV genome. A recombinant CHV genome comprised by the present invention is capable of effecting expression (e.g., transcription, translation) of coding regions that are operatively linked to regulatory sequences within the genome. As used herein, a coding region is a stretch of nucleotides that encodes an RNA molecule and/or a protein. Coding regions can be endogenous to CHV or can be heterologous nucleic acid molecules of the present invention, which are described in more detail below. The phrase operatively linked refers to the positioning of a coding region in the CHV genome such that the coding region is able to be expressed when the genome is inside a cell. Regulatory sequences include transcription control sequences, translation control sequences, and other regulatory sequences that control the expression of coding regions. Transcription control sequences are sequences which control the initiation, elongation, and termination of transcription. Particularly important transcription control sequences are those which control transcription initiation, such as promoter, enhancer, operator and repressor sequences. Suitable regulatory sequences include any regulatory sequence that can function in the present invention. Preferred regulatory sequences are disclosed herein.

A recombinant CHV genome comprised by the present invention can include a gene that has been inactivated. As used herein, a gene includes a coding region as well as the regulatory sequences involved in expression of that coding region. An inactive gene refers to a gene that no longer exhibits the function of its natural counterpart. Methods to inactivate a gene include, but are not limited to, deletion of one or more nucleotides within the gene, insertion of one or more nucleotides into the gene, replacement of one or more nucleotides within the gene by other nucleotides (i.e., nucleotide substitution), and/or inversion of nucleotides within the gene such that the resulting gene no longer has the function of the corresponding natural gene. Such alterations can be effected anywhere within the gene, such as within the coding region, within the regulatory sequences and/or in regions surrounding the coding region or regulatory sequences such that the alteration(s) cause gene inactivation. In one embodiment, an entire gene or the coding region and/or regulatory sequences thereof can be deleted or replaced.

One embodiment of the present invention may comprise an attenuated recombinant CHV. As used herein, an attenuated CHV is a CHV that does not cause 100% mortality if used to infect canid neonates less than 1 week old that are maintained in room temperature. A preferred attenuated CHV comprised by the present invention causes less than about 90% and preferably less than about 70% mortality when used to infect canid neonates less than 1 week of age maintained at room temperature.

An attenuated recombinant CHV can be produced by inactivating a CHV gene that, due to that gene's inactivation, results in an attenuated virus. Methods to inactivate a gene are disclosed above. An attenuated CHV can be identified by exposing pups less than 1 week old to the recombinant virus to be tested and determining the percentage of exposed pups that die; such an exposure method is disclosed, for example, in Carmichael, *ibid.* If less than 100% percent of the pups die, the virus being tested is attenuated in accordance with the present invention. Suitable CHV genes to inactivate in order to produce an attenuated CHV include any gene that when inactivated leads to an attenuated virus, as determined using an assay as disclosed above. A preferred attenuated recombinant CHV of the present invention is a CHV having a recombinant genome in which a heterologous nucleic acid molecule is inserted into a gene, the insertion resulting in an attenuated virus. An attenuated recombinant CHV has utility, for example, as a therapeutic composition to protect an animal from CHV infection and/or as a live CHV-based vaccine carrying a heterologous nucleic acid molecule. It is to be noted, however, that, as disclosed above, it is believed that CHV need not be attenuated for use as a live vaccine vector due to the low pathogenicity of natural CHV, particularly as compared to that of other herpesviruses.

One embodiment of the present invention comprises a recombinant CHV that can reproduce (i.e., grow) in tissue culture; that is, the virus is a reproduction competent CHV. A reproduction competent CHV is a CHV that upon *in vitro* infection of an appropriate host cell is able to use host cell machinery, as well as its own regulatory control regions and/or encoded enzymes, to effect self-reproduction, i.e., to form infectious virus.

Reproduction competent recombinant CHV genomes can have gene alterations in one or more genes non-essential for growth *in vitro*. Suitable gene targets (i.e., genes to alter) include any non-essential CHV gene. A non-essential CHV gene can be identified by altering a CHV gene within a CHV genome (e.g., by genetic engineering or classical mutagenesis) and demonstrating that the altered genome is capable of effecting self-reproduction in tissue culture. Preferred non-essential CHV genes to target include, but are not limited to, a CHV deoxyuridine triphosphate pyrophosphatase (CdUTPase) gene, a CHV glycoprotein C (CgC) gene, a CHV glycoprotein E (CgE) gene, a CHV glycoprotein G (CgG) gene, a CHV glycoprotein I (CgI) gene, a CHV serine-threonine protein kinase US3 (CPK) gene, a CHV thymidine kinase (CTK) gene, a CHV IR6 (CIR6) gene, a CHV US2 (CUS2) gene, a CHV tegument phosphoprotein US9 (CUS9) gene, a CHV membrane protein UL49 (CUL49) gene, a CHV membrane protein CHV UL49.5 (CUL49.5) gene, a CHV regulatory protein ICP0 (cICP0) gene, a CHV membrane protein UL51 (CUL51) gene, and a CHV membrane protein UL45 (CUL45) gene. Particularly preferred non-essential genes to target include a CdUTPase gene, a CgC gene, a CgE gene, a CgG gene, a CgI gene, a CIR6 gene, a CUS2 gene and a CUS9 gene. It is to be noted that CHV regions and genes disclosed herein are named in accordance with herpesvirus nomenclature in that the names include "C" for canine and the rest of the name indicating the corresponding herpes simplex virus type-1 (HSV-1) homolog, when one exists (e.g., "dUTPase"). For example, the CHV unique short region (CUS) is the shorter region of the CHV genome that has unique sequences, analogous to the US region of HSV-1; and the CHV unique long region (CUL) is the longer region of CHV that has unique sequences, analogous to the UL region of HSV-1. While the CHV inverted repeat regions (CIRs) are analogous to the HSV-1 IR regions, they appear to be most analogous to the equine herpesvirus type-1 (EHV-1) IR regions. CUS genes are CHV genes that are homologs of (i.e., genes that share some degree of similarity with) HSV-1 genes located in the US region of the HSV-1 genome. CUL genes are CHV genes that are homologs of HSV-1 genes located in the UL region of the HSV genome. CIR genes are CHV genes that are homologs of HSV-1 or EHV-1 genes located in the IR regions of the HSV-1 or EHV-1 genome. It is also to be noted that although the UL, US, and IR designations refer to the respective genes' locations in the HSV-1 or EHV-1 genome, they do not necessarily refer to the respective genes' locations in the CHV genome. For example, while CUS2 is partially in the IR region of CHV, HSV US2 is entirely in the US region of HSV.

Another embodiment of the present invention comprises a recombinant CHV that is defective for reproduction in tissue culture. A reproduction defective CHV is a CHV that when inserted into an appropriate host cell is unable to form infectious virus. Such a defective CHV has at least one inactive gene that encodes a protein essential for reproduction, including, but not limited to proteins essential for viral entry, immediate early or early gene expression, DNA replication, capsid assembly, and viral egress. Suitable gene targets include any essential CHV gene, with preferred targets being genes encoding proteins involved in viral entry and/or egress. CHV defective in viral entry and/or egress are easy to complement and are advantageous over most other reproduction defective mutants in that such virus are able to undergo one round of viral replication. An essential CHV gene can be identified by altering a CHV gene within a CHV genome and demonstrating (a) that the altered genome is not capable of effecting self-reproduction in tissue culture under wild type conditions or, if a temperature sensitive mutant, at a non-permissive temperature; and (b) that the altered genome can reproduce in a complementing cell line that expresses an active protein corresponding to the essential gene defect on the CHV genome (assuming the defect can be complemented in trans), or at a permissive temperature. Preferred essential CHV genes to target include, but are not limited to, a CHV glycoprotein D (CgD) gene, a CHV glycoprotein B (CgB) gene, a CHV alpha trans-inducing factor UL48 (CUL48) gene, a CHV helicase/primase UL52 (CUL52) gene, a CHV glycoprotein L (CgL) gene and a CHV ICP4 (cICP4) gene. Particularly preferred essential genes to target include a CgD gene and a CgB gene.

The present invention also includes cell lines that complement replication defective CHVs and use of such cell lines to produce replication defective viruses. As such, the present invention includes canine cell lines that complement, or supplement, a CHV defect in a gene encoding CgD, CgB, CUL48, or CUL52, CICP4 and/or CgL. Such cell lines can be produced by a variety of means known to those skilled in the art. For example, a cell capable of complementing a CgD negative, or CgD-, CHV (i.e., a virus with a CHV genome having an inactive CgD gene), can be produced by stable integration of an active CgD gene into the cellular genome or by co-transfection of the CgD- CHV with a nucleic acid molecule capable of complementing the defective CgD gene. Such a nucleic acid molecule can be a nucleic acid containing an active CgD gene operatively linked to regulatory sequences to enable expression of the CgD gene in the transfected cell. In another embodiment, such a nucleic acid molecule can be incorporated into a virus that is co-infected with the CgD-CHV. Such methods can also be used to produce cell lines complementing other replication defective CHVs of the present invention. Any canine cell line that CHV can infect and that expresses the complementary active protein can be used in the production of reproduction-defective CHV. Examples include, but are not limited to, the following cell lines available from American Type Culture Collection (ATCC), Rockville, MD: ATCC CRL-1542 A-72 (Tumor, canine), ATCC CRL-1430 Cf2Th (Thymus, canine, Canis familiaris), ATCC CRL-10389 DH82 (Monocyte-macrophage, canine), ATCC CRL-8468 D17 (Osteogenic sarcoma, canine), ATCC CCL-183 D-17 (Primary osteogenic sarcoma, canine, Canis familiaris), ATCC CCL-34.1 DoC11 (S+L-) (Kidney, canine, Canis familiaris), ATCC CCL-34 MDCK (NBL-2) (Kidney, canine, Canis familiaris), and ATCC CCL-34.2 MDCK/SF (Kidney, canine, Canis familiaris), such cell lines expressing, preferably in a stable manner, the desired essential gene(s) for complementation. Particularly preferred complementing cell lines include MDCK cells that stably express CgD, CgB, CUL48, CUL52 and/or CgL.

An additional preferred CHV open reading frame to inactivate, or disrupt, includes a CUS8.5 open reading frame.

While not being bound by theory, it is believed that a reproductive defective virus-based vaccine may be safer than a reproduction competent virus-based vaccine. On the other hand, a reproduction competent virus-based vaccine may be more efficacious than a reproduction defective virus-based vaccine. Since CHV, as disclosed above, exhibits low pathogenicity, a reproduction competent recombinant CHV is a preferred embodiment of the present invention.

As heretofore disclosed, one embodiment of the present invention is an isolated CHV nucleic acid molecule comprising a recombinant CHV genome having one or more inactive genes. Preferred recombinant CHV of the present invention include genomes in which one or more of the following CHV genes have been inactivated, preferably using recombinant techniques: a CdUTPase gene, a CgC gene, a CgE gene, a CgG gene, a CgI gene, a CPK gene, a CTK gene, a CIR6 gene, a CUS2 gene, a CUS9 gene, a CUL49 gene, a CUL51 gene, a CUL45 gene, a CgD gene, a CgB gene, a CUL48 gene, and a CUL52 gene, a CgL gene, a CUL49.5 gene, a CICP0 gene, a CICP4 gene, and a CUS8.5 open reading frame. Other than the genes encoding CgD, CgB, CUL48, CUL52, CgL and CICP4, the preferred genes to inactivate are nonessential genes. Each of the preferred genes to inactivate is a preferred target for the insertion of heterologous nucleic acid molecules; such insertion is a preferred method to inactivate these genes. More preferred recombinant CHV include genomes in which one or more of the following CHV genes have been inactivated, preferably using recombinant techniques: a CdUTPase gene, a CgC gene, a CgE gene, a CgG gene, a CgI gene, a CUS2 gene, a CUS9 gene, a CgD gene, and/or a CgB gene being more preferred. Also preferred are the corresponding recombinant CHV genomes.

Particularly preferred recombinant CHV of this embodiment include a CdUTPase negative CHV, a CgC negative CHV, a CgE negative CHV, a CgG negative CHV, a CgI negative CHV, a CPK negative CHV, a CTK negative CHV, a CIR6 negative CHV, a CUS2 negative CHV, a CUS9 negative CHV, a CUL49 negative CHV, a CUL51 negative CHV, a CUL45 negative CHV, a CgD negative CHV, a CgB negative CHV, a CUL48 negative CHV, a CUL52 negative CHV, a CgL negative CHV, a CUL49.5 negative CHV, a CICP0 negative CHV, a CICP4 negative CHV, and a CUS8.5 negative CHV. Particularly preferred recombinant CHV include a CdUTPase negative CHV, a CgC negative CHV, a CgE negative CHV, a CgG negative CHV, a CgI negative CHV, a CUS2 negative CHV, a CUS9 negative CHV, a CgD negative CHV, and a CgB negative CHV. Also preferred are the corresponding recombinant CHV genomes, as well as recombinant CHV and CHV genomes having more than one of these preferred genes inactivated. Examples of such CHV include, but are not limited to: a CUS2 negative, CdUTPase negative CHV; a CUS2 negative, CdUTPase negative, CgG negative CHV; and a CUS2 negative, CdUTPase negative, CgG negative, CgC negative CHV, a CUL49.5, CdUTPase negative CHV.

One embodiment of the present invention includes an isolated CHV nucleic acid molecule comprising a recombinant CHV genome comprising a heterologous nucleic acid molecule; i.e., the recombinant CHV genome includes one or more heterologous nucleic acid molecule(s) located, or positioned, in the CHV genome. The present invention also includes a recombinant CHV genome having a heterologous nucleic acid molecule in the genome. Also included is the use of such a CHV and/or CHV genome in a therapeutic composition as well as in the production of a compound encoded by the heterologous nucleic acid molecule(s).

As used herein, a heterologous nucleic acid molecule is a nucleic acid molecule that is not derived from CHV; that is, a heterologous nucleic acid molecule is isolated from a source other than CHV. An isolated nucleic acid molecule of the present invention can be obtained from its natural source, can be produced using recombinant DNA technology (e.g., by amplification, such as by polymerase chain reaction (PCR) amplification and/or cloning) or can be produced by chemical synthesis.

In accordance with the present invention, a heterologous nucleic acid molecule can be inserted into a CHV genome simply to inactivate a CHV gene. A heterologous nucleic acid molecule can also be inserted into a CHV genome to serve as a target for the insertion of a second heterologous nucleic acid molecule, such as to introduce a restriction enzyme site or a recombination site otherwise not present in the CHV genome. For example, CHV strain D 004 (available from ATCC), which does not contain a *Sse*83871, I-*Sce*-I, or *Not*I restriction enzyme site, can be genetically engineered to include one or more sites by inserting into the genome a heterologous nucleic acid molecule containing a *Sse*83871, I-*Sce*-I, and/or *Not*I site(s). Such a heterologous nucleic acid molecule is a good target for the insertion of another heterologous nucleic acid molecule. Other restriction enzyme sites lacking in CHV can be identified using techniques known to those skilled in the art. Without being bound by theory, it is believed that CHV is more likely to lack GC-rich restriction enzyme sites than AT-rich sites, since the CHV genome is AT-rich, as disclosed above.

Another example of a heterologous nucleic acid molecule to insert into a CHV genome is a gene encoding a selectable marker, such as, but not limited to, an *E. coli lac*Z gene, a green fluorescent protein gene, a chloramphenicol transacetylase gene, a xanthine-guanine phosphoribosyl transferase gene, a β-glucuronidase gene, a neomycin resistance gene, an *E. coli* hygromycin resistance gene, and a heterologous thymidine kinase gene (e.g., HSV, FHV; assuming the CHV genome is a CTK negative genome). The presence of such a gene in a CHV genome allows for the selection of recombinant CHV having such a marker gene, and includes the ability to distinguish recombinant CHV from natural CHV isolates. In addition, a second heterologous nucleic acid molecule can be inserted into such a selectable marker gene, thereby inactivating the protein encoded by the marker gene, allowing for yet another method to select for CHV having a desirable heterologous nucleic acid molecule. Methods to select CHV having selectable marker genes, as well as for the inactivation of such markers, is known to those skilled in the art.

A preferred embodiment of the present invention is an isolated CHV nucleic acid molecule comprising a recombinant CHV genome, and corresponding virus, in which the genome contains a heterologous nucleic acid molecule operatively linked to a transcription control sequence. As such, the heterologous nucleic acid molecule can be transcribed when transfected into a cell. A heterologous nucleic acid molecule can be joined to CHV transcription control sequences, can be joined to its own or other homologous transcription control sequences, and/or can be joined to transcription control sequences heterologous to both the heterologous nucleic acid molecule and CHV. The heterologous nucleic acid molecule can also be operatively linked to other regulatory sequences. Suitable regulatory sequences include any regulatory sequence that can function in the present invention. Preferred transcription control sequences include those sequences that can function in canine cells, including, but not limited to: mammalian, preferably canine; viral; or natural (i.e., endogenous to the heterologous nucleic acid molecule) transcription control sequences. Examples of transcription control sequences include antibiotic resistance gene, baculovirus, *Heliothis zea* insect virus, vaccinia virus, herpesvirus, poxvirus, adenovirus, simian virus 40, retrovirus, actin, Rous sarcoma virus, heat shock, and mammalian hormone transcription control sequences. Additional suitable transcription control sequences include tissue-specific promoters and enhancers as well as lymphokine-inducible promoters (e.g., promoters inducible by interferons or interleukins). In one embodiment, expression of a heterologous nucleic acid molecule inserted into a CHV genome is mediated, at least in part, by a human cytomegalovirus (CMV) immediate early promoter and a bovine growth hormone polyadenylation site.

A heterologous nucleic acid molecule comprised in the present invention can be located in any region of the CHV genome (i.e., in the UL, US, and/or IR regions), including, but not limited to, in an essential gene, in a non-essential gene, or in an intergenic region. As such, a heterologous nucleic acid molecule can be located in a coding region, a regulatory region, an intron, an untranslated region, or a non-transcribed region of a gene. A heterologous nucleic acid molecule can also be located in a direct or inverted repeat, including direct and/or inverted repeats within the IR, US or UL regions of CHV. For example, a heterologous nucleic acid molecule can be located in one or more CHV origins of replication (Cori), such as in CoriS.

In one embodiment, a heterologous nucleic acid molecule is located in a CHV genome such that a gene is inactivated. Suitable and preferred gene targets are as disclosed above, with non-essential gene targets being preferred.

In another embodiment, a heterologous nucleic acid molecule is located in a region of the CHV genome spanning from about the 3' end of the coding region of the CUL41 gene through about the 3' end of the coding region of the CUL38 gene. CUL41 gene refers to the CHV homolog of the HSV virion host shutoff protein UL41 gene. CUL38 gene refers to the CHV homolog of the HSV capsid protein VP19C UL38 gene. In most herpesviruses analyzed by cloning and sequencing techniques to date, the region between UL41 and UL38 contains genes encoding the large and small subunits of ribonucleotide reductase. The inventors have found, however, that the ribonucleotide reductase genes appear to be at least partially deleted in CHV in such a manner that there is an intergenic target in that region for heterologous nucleic acid molecule insertion.

A preferred embodiment comprises a heterologous nucleic acid molecule located in a region of the genome such that the heterologous nucleic acid molecule-containing CHV genome can be easily distinguished from a CHV genome not containing the heterologous nucleic acid molecule; that is, the heterologous nucleic acid molecule is inserted into a selectable region of the genome. suitable selectable regions include any region of the CHV genome that, upon introduction of a heterologous nucleic acid molecule, leads to a detectable (e.g., growth-related, biochemical, or molecular) change in the CHV genome or CHV containing the genome. Examples of such selectable regions include, but are not limited to, a CTK gene and a CdUTPase gene. CHV genomes in which a heterologous nucleic acid molecule is inserted into a CTK gene or a CdUTPase gene can be selected using methods known to those skilled in the art; see for example, Kit et al., 1983, *Virology 130,* 381-389; and Holliday et al., 1991, *Antiviral Research 16,* 197-203.

Additional examples of selectable regions include restriction endonuclease sites, such as the *Hin*dIII site or *Xba*I site in the CdUTPase gene and the *Asc*I site in the CUS2 gene. This diploid *Asc*I site is particularly preferred as there are no other *Asc*I sites in the CHV genome. As such, a particularly preferred CHV of the present invention is a recombinant CHV having a CHV genome including a heterologous nucleic acid molecule in a naturally occurring *Asc*I site in the CHV genome. The term "naturally occurring" denotes an AscI restriction site that is present in the wild-type CHV genome. Even more preferred is recombinant CHV strain D 004 having a heterologous nucleic acid molecule in a naturally occurring *Asc*I site in the CHV genome. Also preferred are the corresponding genomes. Examples of methods to insert a heterologous nucleic acid molecule into a genome, including into restriction endonuclease site(s) in the genome, are disclosed herein. Such methods are known to those skilled in the art.

One embodiment of the present invention is an isolated CHV nucleic acid molecule comprising a recombinant CHV genome having a heterologous nucleic acid molecule in one of the following regions of the CHV genome: a region spanning the 9,300 nucleotide *Asc*I restriction endonuclease fragment, denoted herein as nCAsc₉₃₀₀, that apparently includes the entire US region; a region spanning the 10,000 nucleotide fragment from *Asc*I to the end of the genome, denoted herein as nCAsc₁₀₀₀₀, that apparently essentially comprises an IR region, including the CIR6 gene; a region spanning the 3,000 nucleotide *Hin*dIII fragment, denoted herein as nCHin₃₀₀₀, that spans from a portion of CUL48 through a portion of the CdUTPase gene; a region spanning the 1,900 nucleotide *Hin*dIII fragment, denoted herein as nCHin₁₉₀₀, that includes the remainder of the CdUTPase gene through a portion of CUL52; a region spanning the 5,500 nucleotide *Hin*dIII fragment, denoted herein as nCHin₅₅₀₀, that includes at least a portion of CgL, CICPO, and CICP4; and/or a region spanning the 8,500 nucleotide *Hin*dIII fragment, denoted herein as nCHin₈₅₀₀, that includes a portion of CUL48, CUL45 and CgC. Details regarding the production of these and certain other nucleic acid molecules of the present invention are provided in the Examples section.

Also included in the present invention is an isolated CHV nucleic acid molecule comprising a recombinant CHV genome having a heterologous nucleic acid molecule in a region of the genome spanning at least one of the following: a CHV US region comprising nCUS₅₄₉₅, a CHV UL region comprising nCgC/CUL45₂₁₀₀, a CgE gene comprising nCgE₇₅₀, a CgI gene comprising ncgI₁₆₁, a CUS9 gene comprising nCUS9₅₇₉, a CHV UL region comprising nCdUTP/CUL51₇₄₃, a CTK gene comprising nCTK₂₈₀, a CUL48 gene comprising nCUL48₂₉₄, a CUL49 gene included in nCHin₃₀₀₀, a CUL52 gene comprising nCUL52₁₄₆, a CHV UL region comprising nCUL₁₈₂₃, a CHV UL region comprising nCUL49/CUL48₂₀₄₄, a CHV IR region comprising nCICP4₆₂₆, a CHV UL region comprising nCgL₆₅₅, a CHV UL region comprising nCUL52₇₄₉, a CHV UL region comprising ncdUTP₃₂₀₀, as well as allelic variants of such (i.e., said, any of these) regions. As such, the present invention also includes an isolated CHV nucleic acid molecule comprising a recombinant CHV genome having a heterologous nucleic acid molecule in a region of the genome spanning at least one of the following: a CIR6 gene including nCIR6₅₅₂, a CUS2 gene including nCUS2₁₁₇₆, a CPK gene including nCPK₁₂₀₃, a CgG gene including nCgG₁₂₄₈, a CgD gene including nCgD₃₅₇, a CdUTPase gene including nCdUTP₄₅₉, a CUL51 gene including nCUL51₂₆₁, a CgD gene including nCgD₁₀₃₈, a CgI gene including nCgI₁₀₉₅, a CgE gene including nCgE₁₅₆₉, a CUS8.5 open reading frame including nCUS8.5₂₃₇, a CUS9 gene including nCUS9₃₆₀, a CUL49 gene including nCUL49₄₂₀, a CUL48 gene including nCUL48₁₂₆₉, a CICP4 gene including nCICP4₆₂₆, a CgL gene including nCgL₆₅₅, a CdUTPase gene including nCdUTP₉₁₈, a CUL49 gene including nCUL49₂₅₅, a CUL49.5 gene including nCUL49.5₂₆₁, and a CUL52 gene including nCUL52₇₄₉, as well as allelic variants of such regions.

As used herein, an allelic variant of a nucleic acid molecule, or region, for which nucleic acid sequence is provided herein is a nucleic acid molecule, or region, that occurs at essentially the same locus in another CHV genome as the nucleic acid molecule in CHV strain D 004, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions. Allelic variants are well known to those skilled in the art and would be expected to be found among the family of canine herpesviruses.

The present invention also includes an isolated CHV nucleic acid molecule comprising a recombinant CHV genome having a heterologous nucleic acid molecule in a region of the genome spanning at least one of the following nucleic acid molecules: nCUS₅₄₉₅ (and, as such, nCIR6₅₅₂, nCUS2₁₁₇₆, nCPK₁₂₀₃, nCgG₁₂₄₈, and/or nCgD₃₅₇), nCgC/CUL45₂₁₀₀, nCgE₇₅₀, nCgI₁₆₁, nCUS9₅₇₉, nCdUTP/CUL51₇₄₃ (and, as such, nCdUTP₄₅₉ and nCUL51₂₆₁), nCTK₂₈₀, nCUL48₂₉₄, nCUL52₁₄₆, nCUS₁₀₅₉₂, nCgD₁₀₃₈, nCgI₁₀₉₅, nCgE₁₅₆₉, nCUS8.5₂₃₇, nCUS9₃₆₀, nCUL49/CUL48₂₀₄₄, nCUL49₄₂₀, nCUL48₁₂₆₉, nCICP4₆₂₆, nCgL₆₅₅, nCUL₁₈₂₃, nCdUTP₉₁₈, nCUL49.5₂₆₁, nCUL49₂₅₅, nCUL52₇₄₉, and/or nCdUTP₃₂₀₀, as well as allelic variants of such regions.

A particularly preferred CHV genome comprised in the present invention includes a heterologous nucleic acid molecule located in a region of the CHV genome comprising (e.g., including, represented by, or identified by) at least one of the following nucleic acid sequences: SEQ ID NO:1, SEQ ID NO:2, SEQ ID NO:3, SEQ ID NO:5, SEQ ID NO:7, SEQ ID NO:9, SEQ ID NO:11, SEQ ID NO:13, SEQ ID NO:14, SEQ ID NO:15, SEQ ID NO:17, SEQ ID NO:19, SEQ ID NO:20, SEQ ID NO:21, SEQ ID NO:23, SEQ ID NO:24, SEQ ID NO:25, SEQ ID NO:27, SEQ ID NO:28, SEQ ID NO:29, SEQ ID NO:31, SEQ ID NO:32, SEQ ID NO:33, SEQ ID NO:35, SEQ ID NO:37, SEQ ID NO:38, SEQ ID NO:51, SEQ ID NO:52, SEQ ID NO:53, SEQ ID NO:55, SEQ ID NO:57, SEQ ID NO:59, SEQ ID NO:61, SEQ ID NO:63, SEQ ID NO:64, SEQ ID NO:65, SEQ ID NO:67, SEQ ID NO:69, SEQ ID NO:70, SEQ ID NO:71, SEQ ID NO:73, SEQ ID NO:74, SEQ ID NO:75, SEQ ID NO:77, SEQ ID NO:78, SEQ ID NO:79, SEQ ID NO:81, SEQ ID NO:83, SEQ ID NO:85, SEQ ID NO:86, and/or SEQ ID NO:87, as well as complements of such regions and allelic variants of such regions. The relationships between certain nucleic acid molecules and nucleic acid sequences of the present invention are detailed below. It should be noted that since nucleic acid sequencing technology is not entirely error-free, the sequences represented by the SEQ ID NOs in the present invention at best represent apparent nucleic acid or amino acid sequences of CHV nucleic acid molecules or proteins of the present invention. It is also to be noted that a double-stranded nucleic acid molecule of the present invention for which a nucleic acid sequence has been determined for one strand that is represented by a SEQ ID NO also comprises a complementary strand having a sequence that is a complement of that SEQ ID NO. As such, nucleic acid molecules of the present invention, which can be either double-stranded or single-stranded, include those nucleic acid molecules that form stable hybrids under stringent hybridization conditions with either a given SEQ ID NO denoted herein and/or with the complement of that SEQ ID NO, which may or may not be denoted herein. Methods to deduce a complementary sequence are known to those skilled, in the art.

The present invention includes an isolated CHV nucleic acid molecule comprising a recombinant CHV genome that includes a heterologous nucleic acid molecule that encodes a protective compound; the present invention also includes a recombinant CHV containing such a genome. As used herein, a protective compound is a compound that when administered to an animal protects that animal from a disease corresponding to that compound. For example, a compound derived from *Dirofilaria immitis* protects an animal from heartworm and other related infections, whereas a compound derived from a virus protects an animal from disease caused by that and related viruses. As used herein, the ability of a compound to protect an animal from a disease refers to the ability of that protective compound to treat, ameliorate and/or prevent the disease.

A protective compound of the present invention includes, but is not limited to, a protective protein and a protective RNA species. Essentially any heterologous nucleic acid molecule that encodes a protective protein or RNA can be used in the present invention. A protective protein of the present invention can be, for example, an immunogen that elicits an immune response which will protect an animal from the corresponding disease or some other compound (e.g., an immunomodulator, a toxin, an enzyme, an antibody, or other binding protein) that neutralizes and/or reduces the disease. A protective RNA of the present invention can be, for example, an RNA-based drug, a ribozyme, a molecule capable of triple helix formation, or an antisense RNA that effectively prevents the expression of a detrimental protein, thereby protecting an animal from disease.

It is within the scope of the present invention to produce therapeutic compositions against a variety of diseases, including infectious diseases, genetic diseases, and other metabolic diseases, including diseases that lead to abnormal cell growth, degenerative processes, and/or immunological defects. Therapeutic compositions of the present invention can protect animals from a variety of diseases including, but not limited to, allergies, autoimmune diseases, cancers, cardiovascular diseases, graft rejection, hematopoietic disorders, immunodeficiency diseases, immunoproliferative diseases, immunosuppressive disorders, infectious diseases, inflammatory diseases, jaundice, septic shock, other immunological defects, as well as other genetic or metabolic defects.

One preferred embodiment of the present invention is an isolated CHV nucleic acid molecule comprising a recombinant CHV genome comprising a heterologous nucleic acid molecule that encodes a compound that protects a canid, or other animal susceptible to CHV infection, from infectious disease. Such disease can be caused by a variety of infectious agents, including, but not limited to, helminth parasites, protozoan parasites, ectoparasites, fungi (including yeast), bacteria and/or viruses. It should also be noted that although some infectious agents have not been definitively classified into one of these groups, such infectious agents are also included in the present invention. A preferred protective compound is derived from (e.g., obtained from natural source or produced using recombinant or synthetic chemistry techniques) an infectious agent.

Preferred helminth infectious agents to target include nematodes, cestodes and trematodes, with filariid, ascarid, capillarid, strongylid, strongyloides, trichostrongyle, and trichurid, parasitic helminths being more preferred, and filariid nematodes being even more preferred. More preferred parasitic helminths to target include the following: *Aelurostrongylus, Ancylostoma, Angiostrongylus, Ascaris, Brugia, Bunostomum, Capillaria, Chabertia, Cooperia, Crenosoma, Dictyocaulus, Dioctophyme, Dipetalonema, Diphyllobothrium, Diplydium, Dirofilaria* (e.g., *D. immitis), Dracunculus, Echinococcus, Enterobius, Filaroides, Haemonchus, Loa, Mansonella, Muellerius, Nanophyetus, Necator, Nematodirus, Oesophagostomum, Onchocerca, Opisthorchis, Ostertagia, Parafilaria, Paragonimus, Parascaris, Physaloptera, Protostrongylus, Setaria, Spirometra, Spirocera, Stephanofilaria, Strongyloides, Strongylus, Taenia, Thelazia, Toxascaris, Toxocara, Trichinella, Trichostrongylus, Trichuris. Uncinaria,* and *Wuchereria .* More preferred helminths to target, particularly with respect to being canine pathogens include *Ancylostoma, Angiostrongylus, Brugia, Capillaria, Crenosoma, Dioctophyme, Dipetalonema, Diphyllobothrium, Diplydium, Dirofilaria, Echinococcus, Filaroides, Nanophyetus, Opisthorchis, Paragonimus, Physaloptera, Spirometra, Spirocera, Strongyloides, Taenia, Toxascaris, Toxocara, Trichinella, Trichuris,* and *Uncinaria.*

Preferred protozoal infectious agents to target include Acetospora, Apicoplexa, Ciliophora, Labyrinthomorphorpha, Microspora, Myxozoa and Sarcomastigophora, with *Babesia, Balantidium, Besnoitia, Cryptosporidium, Eimeria, Encephalitozoon, Entamoeba*, *Giardia, Hammondia, Hepatozoon, Isospora, Leishmania,* Microsporidia, *Neospora, Nosema, Pentatrichomonas, Plasmodium, Pneumocystis, Sarcocystis, Schistosoma, Theileria, Toxoplasma,* and *Trypanosoma,* being more preferred. More preferred protozoans to target, particularly with respect to being canine pathogens, include *Babesia*, *Balantidium, Besnoitia*, *Cryptosporidium, Encephalitozoon, Entamoeba, Giardia, Hammondia, Hepatozoon, Isospora, Leishmania, Microsporidia, Neospora, Pentatrichomonas, Pneumocystis, Sarcocystis, Schistosoma, Toxoplasma,* and *Trypanosoma.*

Preferred ectoparasite infectious agents to target include fleas, flies, mosquitoes, ticks, mites, lice, spiders, ants and true bugs, with fleas being more preferred. More preferred are fleas of the genera *Ctenocephalides, Ctopsyllus, Diamanus, Echidnophaga Nosopsyllus, Pulex, Tunga,* and Xenopsylla, with *Ctenocephalides canis* and *Ctenocephalides felis* fleas being particularly preferred.

Preferred fungal agents include *Absidia, Acremonium*, *Alternaria, Aspergillus, Basidiobolus, Bipolaris, Blastomyces, Candida, Chlamydia, Coccidioides, Conidiobolus, Cryptococcus, Curvalaria, Epidermophyton, Exophiala, Geotrichum, Histoplasma, Madurella, Malassezia, Microsporum, Moniliella, Mortierella, Mucor, Paecilomyces, Penicillium, Phialemonium, Phialophora, Prototheca, Pseudallescheria, Pseudomicrodochium, Pythium*, *Rhinosporidium, Rhizopus, Scolecobasidium, Sporothrix, Stemphylium, Trichophyton, Trichosporon,* and *Xylohypha.*

Preferred bacterial infectious agents to target include *Actinomyces, Bacillus, Bacteroides, Bordetella, Bartonella* (e.g., *B. henselae), Borrelia* (e.g., *B. burgdorferi*), *Brucella, Campylobacter, Capnocytophaga, Clostridium, Corynebacterium, Coxiella, Dermatophilus, Enterococcus, Ehrlichia* (e.g., *E. canis), Escherichia, Francisella, Fusobacterium, Haemobartonella, Helicobacter, Klebsiella,* L-form bacteria, *Leptospira, Listeria, Mycobacteria, Mycoplasma, Neorickettsia, Nocardia, Pasteurella, Peptococcus, Peptostreptococcus*, *Proteus*, *Pseudomonas, Rickettsia, Rochalimaea, Salmonella, Shigella, Staphylococcus, Streptococcus,* and *Yersinia* (e.g., *Y*. *pestis).*

Preferred virus infectious agents to target include adenoviruses, caliciviruses, coronaviruses, distemper viruses, hepatitis viruses, herpesviruses, immunodeficiency viruses, infectious peritonitis viruses, leukemia viruses, oncogenic viruses, panleukopenia viruses, papilloma viruses, parainfluenza viruses, parvoviruses, rabies viruses, and reoviruses, as well as other cancer-causing or cancer-related viruses.

A preferred isolated CHV nucleic acid molecule of the present invention includes one comprising a recombinant CHV genome having a heterologous nucleic acid molecule encoding a protective compound that elicits an immune response. As used herein, a protective compound that elicits an immune response refers to a compound that when administered to an animal in an appropriate manner, known to those skilled in the art, leads to the production of an immune response in that animal against the protective compound. The immune response, which can include humoral and/or cell-mediated components, preferably protects the immunized animal against the disease(s) targeted by the protective compound. As such, preferred protective compounds include, but are not limited to, antigens associated with diseases, as disclosed herein. Particularly preferred protective compounds of the present invention include *Dirofilaria immitis* antigens, *Toxoplasma gondii* antigens, *Sarcocystis* antigens, *Bartonella henselae* antigens, *Yersinia pestis* antigens, flea antigens, flea allergens, viral antigens, and cancer antigens. Even more preferred protective compounds are those disclosed in the following patent applications and patent publications: U.S. Patent No. 5,356,622, issued October 18, 1994; U.S. Patent No. 5,399,485, issued March 21, 1995; U.S. Patent No. 5,418,137, issued May 23, 1995; PCT Publication No. WO 92/13560, published August 20, 1992; PCT Publication No. WO 93/10225, published May 27, 1993; PCT Publication No. WO 93/23077, published November 25, 1993; PCT Publication No. WO 94/15593, published July 21, 1994; PCT Publication No. WO 94/17813, published August 18, 1994; PCT Publication No. WO 94/17824, published August 18, 1994; PCT Publication No. WO 95/24198, published September 14, 1995; PCT Publication No. WO 95/32988, published December 7, 1995; U.S. Serial No. 08/401,509, filed March 9, 1995; U.S. Serial No. 08/415,365, filed March 30, 1995; U.S. Serial No. 08/450,944, filed May 23, 1995; U.S. Serial No. 08/473,034, filed June 6, 1995; U.S. Serial No. 08/482,304, filed June 7, 1995; U.S. Serial No. 08/485,434, filed June 7, 1995; U.S. Serial No. 08/486,036, filed June 7, 1995; U.S. Serial No. 08/558,735 filed November 16, 1995; PCT Serial No. PCT/US95/13200, filed October 6, 1995; PCT Serial No. PCT/US95/14442, filed October 18, 1995; U.S. Serial No. 08/630,822, filed April 10, 1996; U.S. Serial No. 08/602,262, filed February 15, 1996; PCT Serial No.: PCT/US96/03133, filed March filed April 24, 1996; PCT Serial No. PCT/US96/07709, filed May 23, 1996; and PCT Serial No. PCT/US96/09848, filed June 7, 1996, and related filings.

Another preferred protective compound of the present invention is an immunomodulator. Suitable immunomodulators include compounds that enhance the immune response as well as compounds that suppress the immune response. Compounds that enhance the immune response include compounds that preferentially enhance humoral immunity as well as compounds that preferentially enhance cell-mediated immunity. Suitable compounds can be selected depending on the disease being targeted. Suitable immunomodulators include, but are not limited to, cytokines, chemokines, superantigens, and other immunomodulators as well as compounds that induce the production of cytokines, chemokines and other immunomodulators. Examples of such protective compounds include, but are not limited to, granulocyte macrophage colony stimulating factor (GM-CSF), granulocyte colony stimulating factor (G-CSF), macrophage colony stimulating factor (M-CSF), colony stimulating factor (CSF), erythropoietin (EPO), interleukin 2 (IL-2), interleukin-3 (IL-3), interleukin 4 (IL-4), interleukin 5 (IL-5), interleukin 6 (IL-6), interleukin 7 (IL-7), interleukin 8 (IL-8), interleukin 10 (IL-10), interleukin 12 (IL-12), interferon gamma, interferon gamma inducing factor I (IGIF), transforming growth factor beta (TGF-β), RANTES (regulated upon activation, normal T-cell expressed and presumably secreted), macrophage inflammatory proteins (e.g., MIP-1 alpha and MIP-1 beta), and Leishmania elongation initiating factor (LEIF).

One preferred embodiment of the present invention is an isolated CHV nucleic acid molecule comprising a recombinant CHV genome comprising more than one heterologous nucleic acid molecule. Such a CHV can include two or more heterologous nucleic acid molecules encoding two or more protective compounds to protect an animal from a given disease (e.g., two or more heartworm antigens), or can include two or more heterologous nucleic acid molecules encoding protective compounds each targeted against a different disease (e.g., a compound to protect an animal against heartworm and a compound to protect an animal against a viral infection). A preferred multivalent CHV can also include an heterologous nucleic acid molecule encoding a protective compound that elicits an immune response as well as an heterologous nucleic acid molecule encoding an immunomodulator to enhance the desired immune response. Also included in the present invention are protective compounds that are fusion, or multivalent, proteins comprising more than one functional domain.

Also included in the present invention are cells comprising recombinant CHV genomes of the present invention. As used herein, a cell comprising a recombinant CHV genome is a cell into which a recombinant CHV genome has been introduced. Such introduction can be accomplished by any method by which a nucleic acid molecule can be inserted into a cell. Such methods, known to those skilled in the art, include, but are not limited to, infection (i.e., with a virus comprising the genome), transfection, transformation, electroporation, microinjection, lipofection, adsorption, and protoplast fusion. A preferred cell comprises a recombinant CHV having a heterologous nucleic acid molecule, which preferably is operatively linked to a transcription control sequence. Cells containing CHV genomes are useful in the production of recombinant CHV. Methods to produce recombinant CHV are disclosed herein.

The present invention also includes isolated CHV nucleic acid molecules. As used herein, a CHV nucleic acid molecule is a nucleic acid molecule that is derived from CHV. As such, the nucleic acid molecule can be produced, for example, by recovery of such a nucleic acid molecule directly from a CHV genome, by recombinant DNA techniques, or by chemical synthesis. That the CHV nucleic acid molecule is isolated indicates that the molecule is removed from its natural milieu. An isolated CHV nucleic acid molecule can include DNA, RNA, or derivatives of either DNA or RNA.

A preferred CHV nucleic acid molecule of the present invention is a CHV nucleic acid molecule that hybridizes under stringent hybridization conditions with at least one of the following: with a CdUTPase gene, a CgC gene, a CgE gene, a CgG gene, a CgI gene, a CPK gene, a CTK gene, a CIR6 gene, a CUS2 gene, a CUS9 gene, a CUL49 gene, a CUL51 gene, a CUL45 gene, a CgD gene, a CUL48 gene, a CgL gene, a CUL49.5 gene, a CICP4 gene, a CUS8.5 open reading frame, and/or a CUL52 gene; with other regions of a CUS; and/or with a region of the CHV genome spanning from about the 3' end of the coding region of the CUL41 gene through about the 3' end of the coding region of the CUL38 gene. The identifying characteristics of such regions, including the CHV genes listed, are heretofore described.

As used herein, stringent hybridization conditions refer to standard hybridization conditions under which nucleic acid molecules, including oligonucleotides, are used to identify molecules having similar nucleic acid sequences. Stringent hybridization conditions typically permit isolation of nucleic acid molecules having at least about 70% nucleic acid sequence identity with the nucleic acid molecule being used as a probe in the hybridization reaction. Formulae to calculate the appropriate hybridization and wash conditions to achieve hybridization permitting 30% or less mis-match between two nucleic acid molecules are disclosed, for example, in Meinkoth et al., 1984, *Anal. Biochem 138,* 267-284. An example of such conditions includes, but is not limited to, the following: Oligonucleotide probes of about 18-25 nucleotides in length with Tₘ's ranging from about 50°C to about 65°C, for example, can be hybridized to nucleic acid molecules typically immobilized on a filter (e.g., nitrocellulose filter) in a solution containing 2X SSPE, 1% Sarkosyl, 5X Denhardts and 0.1 mg/ml denatured salmon sperm DNA at a temperature as calculated using the formulae of Meinkoth et al., *ibid*. for about 2 to about 12 hours. The filters are then washed 3 times in a wash solution containing 2X SSPE, 1% Sarkosyl at about 55°C for about 15 minutes each. The filters can be further washed in a wash solution containing 2X SSPE, 1% Sarkosyl at about 55°C for about 15 minutes per wash.

A CHV nucleic acid molecule of the present invention can include an isolated natural CHV gene or a homolog thereof, the latter of which is described in more detail below. A CHV nucleic acid molecule of the present invention can include one or more regulatory regions, full-length or partial coding regions, or combinations thereof. The minimal size of a CHV nucleic acid molecule of the present invention is the minimal size that can form a stable hybrid with one of the aforementioned CHV genes and other regions under stringent hybridization conditions.

Isolated CHV nucleic acid molecules include natural nucleic acid molecules and homologs thereof, including, but not limited to, natural allelic variants and modified nucleic acid molecules in which nucleotides have been inserted, deleted, substituted, and/or inverted in such a manner that such modifications do not substantially interfere with the nucleic acid molecule's ability to encode a CHV protein of the present invention or to form stable hybrids under stringent conditions with natural gene isolates.

A CHV nucleic acid molecule homolog can be produced using a number of methods known to those skilled in the art (see, for example, Sambrook et al., *ibid*.). CHV nucleic acid molecule homologs can be selected from a mixture of modified nucleic acids by screening for the function of the protein encoded by the nucleic acid molecule and/or by hybridization with a CHV region as defined above.

An isolated CHV nucleic acid molecule of the present invention can include a nucleic acid sequence that encodes at least one CHV protein of the present invention; such proteins are discussed in further detail below. Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding a CHV protein.

One embodiment of the present invention is a CHV nucleic acid molecule that, when administered to an animal, is capable of protecting that animal from CHV infection. Such a CHV nucleic acid molecule can be, or encode, an antisense RNA, a molecule capable of triple helix formation, a ribozyme, or other nucleic acid-based drug compound. In an additional embodiment, a CHV nucleic acid molecule of the present invention can encode a protective protein, the nucleic acid molecule being delivered to the animal by direct injection (i.e, as a naked nucleic acid) or in a vehicle such as a recombinant virus vaccine or a recombinant cell vaccine.

One embodiment of the present invention is a CHV nucleic acid molecule that hybridizes under stringent hybridization conditions with nCUS₅₄₉₅, ncgC/CUL45₂₁₀₀, nCgE₇₅₀, nCgI₁₆₁, nCUS9₅₇₉, nCdUTP/CUL51₇₄₃, nCTK₂₈₀, nCUL48₂₉₄, a nCUL49 included in nCHin₃₀₀₀, nCUL52₁₄₆, nCgI₁₀₉₅, nCgE₁₅₆₉, nCUS8.5₂₃₇, nCUS9₃₆₀, nCUL49/CUL48₂₀₄₄, nCUL49₄₂₀, nCUL48₁₂₆₉, nCICP4₆₂₆, nCgL₆₅₅, nCUL₁₈₂₃, nCdUTP₉₁₈, nCUL49.5₂₆₁, nCUL49₂₅₅, and/or nCUL52₇₄₉. Such a CHV nucleic acid molecule can also hybridize under stringent hybridization conditions with nCAsc₉₃₀₀, nCAsc₁₀₀₀₀, nCHin₃₀₀₀, nCHin₁₉₀₀, nCHin₅₅₀₀, nCHin₈₅₀₀, nCIR6₅₅₂, nCUS2₁₁₇₆, nCPK₁₂₀₃, nCgG₁₂₄₈, nCgD₃₅₇, nCdUTP₄₅₉, nCTK₂₇₉, nCUS9₄₅₀, nCUL48₂₉₁, nCUL51₂₆₁, nCUL52₁₄₄, nCgI₁₅₉, nCUS₁₀₅₉₂, nCICP4₆₂₄, nCgL₅₁₆, nCUL52₇₄₇, nCdUTP₈₅₈, and/or nCdUTP₃₂₀₀. At least some of such CHV nucleic acid molecules can hybridize under stringent hybridization conditions with a nucleic acid molecule having nucleic acid sequence SEQ ID NO:1, SEQ ID NO:2, SEQ ID NO:13, SEQ ID NO:14, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:35, SEQ ID NO:37, SEQ ID NO:51, SEQ ID NO:52, SEQ ID NO:63, SEQ ID NO:64, SEQ ID NO:69, SEQ ID NO:70, SEQ ID NO:71, SEQ ID NO:73, SEQ ID NO:74, SEQ ID NO:77, SEQ ID NO:78, SEQ ID NO:85, SEQ ID NO:86, and/or SEQ ID NO:87, as well as complements of such sequences.

SEQ ID NO:1 and SEQ ID NO:2 represent the deduced nucleic acid sequences of the two complementary strands of nCUS₅₄₉₅. Translation of SEQ ID NO:1 and SEQ ID NO:2 indicates that nucleic acid molecule nCUS₅₄₉₅ encodes CIR6, CUS2, CPK and CgG proteins as well as a portion of a CgD protein. Specifically, SEQ ID NO:2 includes: a coding region for a CIR6 protein of about 183 amino acids, denoted herein as nCIR6₅₅₂ and represented by SEQ ID NO:3, assuming a start codon spanning about nucleotides 4566-4568 and a stop codon spanning about nucleotides 5115-5117 of SEQ ID NO:2; and a coding region for a CUS2 protein of about 391 amino acids, denoted herein as nCUS2₁₁₇₆ and represented by SEQ ID NO:5, assuming a start codon spanning about nucleotides 3232-3234 and a stop codon spanning about nucleotides 4405-4407 of SEQ ID NO:2. The amino acid sequences of the respective encoded proteins PCIR6₁₈₃ and PCUS2₃₉₁ are represented by SEQ ID NO:4 and SEQ ID NO:6. SEQ ID NO:1 includes: a coding region for a CPK protein of about 400 amino acids, denoted herein as nCPK₁₂₀₃ and represented by SEQ ID NO:7, assuming a start codon spanning about nucleotides 2384-2386 and a stop codon spanning about nucleotides 3584-3586 of SEQ ID NO:1; a coding region for a CgG protein of about 415 amino acids, denoted herein as nCgG₁₂₄₈ and represented by SEQ ID NO:9, assuming a start codon spanning about nucleotides 3698-3700 and a stop codon spanning about nucleotides 4943-4945 of SEQ ID NO:1; and a partial coding region for a CgD protein of about 119 amino acids, denoted herein as nCgD₃₅₇ and represented by SEQ ID NO:11, assuming a start codon spanning about nucleotides 5137-5139. The amino acid sequences of the respective encoded proteins PCPK₄₀₀, PCgG₄₁₅, and PCgD₁₁₉ are represented by SEQ ID NO:8, SEQ ID NO:10 and SEQ ID NO:12.

SEQ ID NO:13 and SEQ ID NO:14 represent the deduced nucleic acid sequences of the two complementary strands of nCdUTP/CUL51₇₄₃. SEQ ID NO:13 includes a partial coding region for a CdUTPase protein of about 152 amino acids, denoted herein as nCdUTP₄₅₉ and represented by SEQ ID NO:15, assuming a first in-frame codon spanning about nucleotides 3-5, and a stop codon spanning about nucleotides 459-461 of SEQ ID NO:13. The amino acid sequence of the encoded protein PCdUTP₁₅₂ is represented by SEQ ID NO:16. SEQ ID NO:14 includes a partial coding region for a CUL51 protein of about 86 amino acids, denoted herein as nCUL51₂₆₁ and represented by SEQ ID NO:33, assuming a first in-frame codon spanning about nucleotides 1-3, and a stop codon spanning about nucleotides 259-261 of SEQ ID NO:14. The amino acid sequence of the encoded protein PCUL51₈₆ is represented by SEQ ID NO:34.

SEQ ID NO:17 and SEQ ID NO:19 represent the deduced nucleic acid sequences of the two complementary strands of nCUS9₅₇₉. SEQ ID NO:17 includes a coding region for a CUS9 protein of about 149 amino acids, denoted herein as nCUS9₄₅₀ and represented by SEQ ID NO:20, assuming a start codon spanning about nucleotides 54-56 and a stop codon spanning about nucleotides 501-503 of SEQ ID NO:17. The amino acid sequence of the encoded protein PCUS9₁₄₉ is represented by SEQ ID NO:18.

SEQ ID NO:21 and SEQ ID NO:23 represent the deduced nucleic acid sequences of the two complementary strands of nCUL48₂₉₄. SEQ ID NO:21 includes a partial coding region for a CUL48 protein of about 97 amino acids, denoted herein as nCUL48₂₉₁ and represented by SEQ ID NO:24, assuming a first in-frame codon spanning about nucleotides 3-5 of SEQ ID NO:21. The amino acid sequence of the encoded protein PCUL48₉₇ is represented by SEQ ID NO:22.

SEQ ID NO:25 and SEQ ID NO:27 represent the deduced nucleic acid sequences of the two complementary strands of nCUL52₁₄₆. SEQ ID NO:25 includes a partial coding region for a CUL52 protein of about 48 amino acids, denoted herein as nCUL52₁₄₄ and represented by SEQ ID NO:28, assuming a first in-frame codon spanning about nucleotides 1-3 of SEQ ID NO:25. The amino acid sequence of the encoded protein PCUL52₄₈ is represented by SEQ ID NO:26.

SEQ ID NO:29 and SEQ ID NO:31 represent the deduced nucleic acid sequences of the two complementary strands of nCgI₁₆₁. SEQ ID NO:29 includes a partial coding region for a CgI protein of about 53 amino acids, denoted herein as ncgI₁₅₉ and represented by SEQ ID NO:32, assuming a first in-frame codon spanning about nucleotides 3-5 of SEQ ID NO:29. The amino acid sequence of the encoded protein PCgI₅₃ is represented by SEQ ID NO:30.

SEQ ID NO:35 and SEQ ID NO:37 represent the deduced nucleic acid sequences of the two complementary strands of nCTK₂₈₀. SEQ ID NO:35 includes a partial coding region for a CTK protein of about 93 amino acids, denoted herein as nCTK₂₇₉ and represented by SEQ ID NO:38, assuming a first in-frame codon spanning about nucleotides 2-4 of SEQ ID NO:35. The amino acid sequence of the encoded protein PCTK₉₃ is represented by SEQ ID NO:36.

The identities of additional nucleic acid molecules, nucleic acid sequences, proteins, and amino acid sequences are presented in the Examples.

Comparison of the CHV nucleic acid sequences SEQ ID NO:1, SEQ ID NO:2, SEQ ID NO:3, SEQ ID NO:5, SEQ ID NO:7, SEQ ID NO:9, SEQ ID NO:13, SEQ ID NO:14, SEQ ID NO:15, SEQ ID NO:17, SEQ ID NO:19, SEQ ID NO:20, SEQ ID NO:21, SEQ ID NO:23, SEQ ID NO:24, SEQ ID NO:25, SEQ ID NO:27, SEQ ID NO:28, SEQ ID NO:29, SEQ ID NO:31, SEQ ID NO:32, SEQ ID NO:33, SEQ ID NO:35, SEQ ID NO:37, SEQ ID NO:38, SEQ ID NO:51, SEQ ID NO:52, SEQ ID NO:55, SEQ ID NO:57, SEQ ID NO:59, SEQ ID NO:61, SEQ ID NO:63, SEQ ID NO:64, SEQ ID NO:65, SEQ ID NO:67, SEQ ID NO:69, SEQ ID NO:70, SEQ ID NO:71, SEQ ID NO:73, SEQ ID NO:74, SEQ ID NO:75, SEQ ID NO:77, SEQ ID NO:78, SEQ ID NO:79, SEQ ID NO:81, SEQ ID NO:83, SEQ ID NO:85, SEQ ID NO:86, and SEQ ID NO:87 with known sequences indicates that none of these CHV nucleic acid sequences share more than about 70% identity (many, if not all, sharing significantly less identity) with a known nucleic acid sequence. As such, a preferred CHV nucleic acid molecule has a nucleic acid sequence that is at least about 80%, preferably at least about 85%, more preferably at least about 90%, even more preferably at least about 95%, and even more preferably at least about 99% identical to nucleic acid sequence SEQ ID NO:1, SEQ ID NO:2, SEQ ID NO:3, SEQ ID NO:5, SEQ ID NO:7, SEQ ID NO:9, SEQ ID NO:13, SEQ ID NO:14, SEQ ID NO:15, SEQ ID NO:17, SEQ ID NO:19, SEQ ID NO:20, SEQ ID NO:21, SEQ ID NO:23, SEQ ID NO:24, SEQ ID NO:25, SEQ ID NO:27, SEQ ID NO:28, SEQ ID NO:29, SEQ ID NO:31, SEQ ID NO:32, SEQ ID NO:33, SEQ ID NO:35, SEQ ID NO:37, SEQ ID NO:38, SEQ ID NO:51, SEQ ID NO:52, SEQ ID NO:55, SEQ ID NO:57, SEQ ID NO:59, SEQ ID NO:61, SEQ ID NO:63, SEQ ID NO:64, SEQ ID NO:65, SEQ ID NO:67, SEQ ID NO:69, SEQ ID NO:70, SEQ ID NO:71, SEQ ID NO:73, SEQ ID NO:74, SEQ ID NO:75, SEQ ID NO:77, SEQ ID NO:78, SEQ ID NO:79, SEQ ID NO:81, SEQ ID NO:83, SEQ ID NO:85, SEQ ID NO:86, and/or SEQ ID NO:87, as well as complements of such sequences.

A more preferred CHV nucleic acid molecule of the present invention includes at least a portion of CHV nucleic acid molecule nCAsc₉₃₀₀, nCAsc₁₀₀₀₀, nCHin₃₀₀₀, nCHin₁₉₀₀, nCHin₅₅₀₀, nCHin₈₅₀₀, nCUS₅₄₉₅, nCIR6₅₅₂, nCUS2₁₁₇₆, nCPK₁₂₀₃, nCgG₁₂₄₈, nCdUTP/CUL51₇₄₃, nCdUTP₄₅₉, nCUS9₅₇₉, nCUS9₄₅₀, nCUL48₂₉₄, nCUL48₂₉₁, nCUL52₁₄₆, nCUL52₁₄₄, nCgI₁₆₁, nCgI₁₅₉, nCgE₇₅₀, nCTK₂₈₀, nCTK₂₇₉, nCUL51₂₆₁, nCUS₁₀₅₉₂, nCgI₁₀₉₅, nCgE₁₅₆₉, nCUS8.5₂₃₇, nCUS9₃₆₀, nCUL49/CUL48₂₀₄₄, nCUL49₄₂₀, nCUL48₁₂₆₉, nCICP4₆₂₆, nCICP4₆₂₄, nCgL₆₅₅, nCgL₅₁₆, nCUL₁₈₂₃, nCdUTP₉₁₈, nCUL49.5₂₆₁, nCUL49₂₅₅, nCUL52₇₄₉, nCUL52₇₄₇, nCdUTP₈₅₈, and/or nCdUTP₃₂₀₀, as well as allelic variants of those CHV nucleic acid molecules. Such CHV nucleic acid molecules can include nucleotides in addition to those included in the defined fragments; examples of such CHV nucleic acid molecules include full-length genes, full-length coding regions, or nucleic acid molecules encoding multivalent proteins. Particularly preferred CHV nucleic acid molecules are nCAsc₉₃₀₀, nCAsc₁₀₀₀₀, nCHin₃₀₀₀, nCHin₁₉₀₀, nCHin₅₅₀₀, nCHin₈₅₀₀, nCUS₅₄₉₅, nCIR6₅₅₂, nCUS2₁₁₇₆, nCPK₁₂₀₃, nCgG₁₂₄₈, nCdUTP/CUL51₇₄₃, nCdUTP₄₅₉, nCUS9₅₇₉, nCUS9₄₅₀, nCUL48₂₉₄, nCUL48₂₉₁, nCUL52₁₄₆, nCUL52₁₄₄, nCgI₁₆₁, nCgI₁₅₉, nCgE₇₅₀, nCTK₂₈₀, nCTK₂₇₉, nCUL51₂₆₁, nCUS₁₀₅₉₂, nCgI₁₀₉₅, nCgE₁₅₆₉, nCUS8.5₂₃₇, nCUS9₃₆₀, nCUL49/CUL48₂₀₄₄, nCUL49₄₂₀, nCUL48₁₂₆₉, nCICP4₆₂₆, nCICP4₆₂₄, nCgL₆₅₅, nCgL₅₁₆, nCUL₁₈₂₃, nCdUTP₉₁₈, nCUL49.5₂₆₁, nCUL49₂₅₅, nCUL52₇₄₉, nCUL52₇₄₇, nCdUTP₈₅₈, and/or nCdUTP₃₂₀₀,

Similarly, a preferred CHV nucleic acid molecule of the present invention includes at least a portion of nucleic acid sequence SEQ ID NO:1, SEQ ID NO:2, SEQ ID NO:3, SEQ ID NO:5, SEQ ID NO:7, SEQ ID NO:9, SEQ ID NO:13, SEQ ID NO:14, SEQ ID NO:15, SEQ ID NO:17, SEQ ID NO:19, SEQ ID NO:20, SEQ ID NO:21, SEQ ID NO:23, SEQ ID NO:24, SEQ ID NO:25, SEQ ID NO:27, SEQ ID NO:28, SEQ ID NO:29, SEQ ID NO:31, SEQ ID NO:32, SEQ ID NO:33, SEQ ID NO:35, SEQ ID NO:37, SEQ ID NO:38, SEQ ID NO:51, SEQ ID NO:52, SEQ ID NO:55, SEQ ID NO:57, SEQ ID NO:59, SEQ ID NO:61, SEQ ID NO:63, SEQ ID NO:64, SEQ ID NO:65, SEQ ID NO:67, SEQ ID NO:69, SEQ ID NO:70, SEQ ID NO:71, SEQ ID NO:73, SEQ ID NO:74, SEQ ID NO:75, SEQ ID NO:77, SEQ ID NO:78, SEQ ID NO:79, SEQ ID NO:81, SEQ ID NO:83, SEQ ID NO:85, SEQ ID NO:86, and/or SEQ ID NO:87; or a complement of SEQ ID NO:3, SEQ ID NO:5, SEQ ID NO:7, SEQ ID NO:9, SEQ ID NO:15, SEQ ID NO:20, SEQ ID NO:24, SEQ ID NO:28, SEQ ID NO:32, SEQ ID NO:33, SEQ ID NO:38, SEQ ID NO:55, SEQ ID NO:57, SEQ ID NO:59, SEQ ID NO:61, SEQ ID NO:65, SEQ ID NO:67, SEQ ID NO:71, SEQ ID NO:75, SEQ ID NO:79, SEQ ID NO:81, SEQ ID NO:83, and/or SEQ ID NO:87; as well as allelic variants of such nucleic acid molecules. More preferred is a nucleic acid molecule that includes at least one of the following nucleic acid sequences: SEQ ID NO:1, SEQ ID NO:2, SEQ ID NO:3, SEQ ID NO:5, SEQ ID NO:7, SEQ ID NO:9, SEQ ID NO:13, SEQ ID NO:14, SEQ ID NO:15, SEQ ID NO:17, SEQ ID NO:19, SEQ ID NO:20, SEQ ID NO:21, SEQ ID NO:23, SEQ ID NO:24, SEQ ID NO:25, SEQ ID NO:27, SEQ ID NO:28, SEQ ID NO:29, SEQ ID NO:31, SEQ ID NO:32, SEQ ID NO:33, SEQ ID NO:35, SEQ ID NO:37, SEQ ID NO:38, SEQ ID NO:51, SEQ ID NO:52, SEQ ID NO:55, SEQ ID NO:57, SEQ ID NO:59, SEQ ID NO:61, SEQ ID NO:63, SEQ ID NO:64, SEQ ID NO:65, SEQ ID NO:67, SEQ ID NO:69, SEQ ID NO:70, SEQ ID NO:71, SEQ ID N0:73, SEQ ID NO:74, SEQ ID NO:75, SEQ ID NO:77, SEQ ID NO:78, SEQ ID NO:79, SEQ ID NO:81, SEQ ID NO:83, SEQ ID NO:85, SEQ ID NO:86, and/or SEQ ID NO:87; or a complement SEQ ID NO:5, SEQ ID NO:7, SEQ ID NO:9, SEQ ID NO:15, SEQ ID NO:20, SEQ ID NO:24, SEQ ID NO:28, SEQ ID NO:32, SEQ ID NO:33, SEQ ID NO:38, SEQ ID NO:55, SEQ ID NO:57, SEQ ID NO:59, SEQ ID NO:61, SEQ ID NO:65, SEQ ID NO:67, SEQ ID NO:71, SEQ ID NO:75, SEQ ID NO:79, SEQ ID NO:81, SEQ ID NO:83, and/or SEQ ID NO:87; also included are nucleic acid molecules that are allelic variants of nucleic acid molecules having those nucleic acid sequences. Such nucleic acid molecules can include nucleotides in addition to those included in the SEQ ID NOs.

The present invention also includes CHV nucleic acid molecules encoding a protein, including nucleic acid molecules that have been modified to accommodate codon usage properties of the cells in which such nucleic acid molecules are to be expressed. CHV proteins of the present invention are described in more detail below. Particularly preferred nucleic acid molecules are those that encode a protein having at least one of the following amino acid sequences: SEQ ID NO:4, SEQ ID NO:6, SEQ ID NO:8, SEQ ID NO:10, SEQ ID NO:16, SEQ ID NO:18, SEQ ID NO:22, SEQ ID NO:26, SEQ ID NO:30, SEQ ID NO:34, SEQ ID NO:36, SEQ ID NO:56, SEQ ID NO:58, SEQ ID NO:60, SEQ ID NO:62, SEQ ID NO:66, SEQ ID NO:68, SEQ ID NO:72, SEQ ID NO:76, SEQ ID NO:80, SEQ ID NO:82, SEQ ID NO:84, and/or SEQ ID NO:88.

The present invention also includes CHV nucleic acid molecules that are oligonucleotides capable of hybridizing, under stringent hybridization conditions, with complementary regions of other, preferably longer, CHV nucleic acid molecules of the present invention. Oligonucleotides of the present invention can be RNA, DNA, or derivatives of either. The minimal size of such oligonucleotides is the size required to form a stable hybrid between a given oligonucleotide and the complementary sequence on another nucleic acid molecule of the present invention. Minimal size characteristics are disclosed herein. The size of the oligonucleotide must also be sufficient for the use of the oligonucleotide in accordance with the present invention. Oligonucleotides of the present invention can be used in a variety of applications including, but not limited to, as probes to identify additional nucleic acid molecules, as primers to amplify or extend nucleic acid molecules or in therapeutic applications to inhibit CHV infection as disclosed herein.

The present invention also includes an isolated CHV protein encoded by a CHV nucleic acid molecule of the present invention. As such, the present invention includes a CHV protein encoded by a CHV nucleic acid molecule that hybridizes under stringent hybridization conditions with at least one of the following: a CdUTPase gene, a CgC gene, a CgE gene, a CgG gene, a CgI gene, a CPK gene, a CTK gene, a CIR6 gene, a CUS2 gene, a CUS9 gene, a CUL49 gene, a CUL51 gene, a CUL45 gene, a CgD gene, a CUL48 gene, and/or a CUL52 gene; and/or with other portions of a CUS region; and/or with a CgL gene, a CUL49.5 gene, a CICP4 gene, and/or a CUS8.5 open reading frame.

According to the present invention, an isolated, or biologically pure, protein, is a protein that has been removed from its natural milieu. As such, "isolated" and "biologically pure" do not necessarily reflect the extent to which the protein has been purified. An isolated protein of the present invention can be obtained from its natural source, can be produced using recombinant DNA technology or can be produced by chemical synthesis. As used herein, a CHV protein can be a full-length protein or any homolog of such a protein. Examples of CHV homologs include CHV proteins in which amino acids have been deleted (e.g., a truncated version of the protein, such as a peptide), inserted, inverted, substituted and/or derivatized (e.g., by glycosylation, phosphorylation, acetylation, myristoylation, prenylation, palmitoylation, amidation and/or addition of glycerophosphatidyl inositol) such that the homolog retains a desired activity of the natural protein, such as, but not limited to, enzymatic activity, activity important for viral growth, and/or ability to elicit an immune response. These activities can be measured using techniques known to those skilled in the art.

CHV protein homologs can be the result of natural allelic variation or natural mutation. CHV protein homologs of the present invention can also be produced using techniques known in the art including, but not limited to, direct modifications to the protein or modifications to the gene encoding the protein using, for example, classic or recombinant DNA techniques to effect random or targeted mutagenesis.

The minimal size of a CHV protein homolog of the present invention is a size sufficient to be encoded by a nucleic acid molecule capable of forming a stable hybrid (i.e., hybridize under stringent hybridization conditions) with the complementary sequence of a nucleic acid molecule encoding the corresponding natural protein. As such, the size of the nucleic acid molecule encoding such a protein homolog is dependent on nucleic acid composition and percent homology between the nucleic acid molecule and complementary sequence. It should also be noted that the extent of homology required to form a stable hybrid can vary depending on whether the homologous sequences are interspersed throughout the nucleic acid molecules or are clustered (i.e., localized) in distinct regions on the nucleic acid molecules. The minimal size of such nucleic acid molecules is typically at least about 12 to about 15 nucleotides in length if the nucleic acid molecules are GC-rich and at least about 15 to about 17 bases in length if they are AT-rich. As such, the minimal size of a nucleic acid molecule used to encode a CHV protein homolog of the present invention is from about 12 to about 18 nucleotides in length. There is no limit, other than a practical limit, on the maximal size of such a nucleic acid molecule in that the nucleic acid molecule can include a portion of a gene, an entire gene, or multiple genes, or portions thereof. Similarly, the minimal size of a CHV protein homolog of the present invention is from about 4 to about 6 amino acids in length, with preferred sizes depending on whether full-length, fusion, or other functional portions of such proteins are desired.

One embodiment of the present invention is a CHV protein that can protect an animal from disease, preferably by eliciting an immune response against CHV, and/or can detect CHV infection in an animal. The minimum size of such a protein is a minimum size sufficient to form an epitope, a size that typically is at least from about 5 to about 9 amino acids. As is appreciated by those skilled in the art, an epitope can include amino acids that naturally are contiguous to each other as well as amino acids that, due to the tertiary structure of the natural protein, are in sufficiently close proximity to form an epitope.

The present invention also includes mimetopes of CHV proteins that can be used in accordance with methods as disclosed for CHV proteins of the present invention. As used herein, a mimetope of a CHV protein of the present invention refers to any compound that is able to mimic the activity of such a CHV protein, often because the mimetope has a structure that mimics the CHV protein. Mimetopes can be, but are not limited to: peptides that have been modified to decrease their susceptibility to degradation; anti-idiotypic and/or catalytic antibodies, or fragments thereof; non-proteinaceous immunogenic portions of an isolated protein (e.g., carbohydrate structures); and synthetic or natural organic molecules, including nucleic acids. Such mimetopes can be designed using computer-generated structures of proteins of the present invention. Mimetopes can also be obtained by generating random samples of molecules, such as oligonucleotides, peptides or other organic molecules, and screening such samples by affinity chromatography techniques using the corresponding binding partner.

One embodiment of the present invention is a fusion, or multivalent, protein that includes a CHV protein-containing domain attached to another functional domain. Such a domain can be an entire protein, or function portion thereof. Examples of such domains include not only protective compounds as disclosed above, but also domains that enhance a protein's stability (e.g., during production, storage and/or use) or that aid in protein purification.

A preferred isolated protein of the present invention is a protein encoded by a nucleic acid molecule that hybridizes under stringent hybridization conditions with nCIR6₅₅₂, nCUS2₁₁₇₆, nCPK₁₂₀₃, ncgG₁₂₄₈, nCgD₃₅₇, nCgC/CUL45₂₁₀₀, nCdUTP₄₅₉, nCUS9₄₅₀, nCUL48₂₉₁, nCUL52₁₄₄, nCgI₁₅₉, nCgE₇₅₀, nCTK₂₇₉, nCUL51₂₆₁, nCgI₁₀₉₅, nCgE₁₅₆₉, nCUS8.5₂₃₇, nCUS9₃₆₀, nCUL49/CUL48₂₀₄₄, nCUL49₄₂₀, nCUL48₁₂₆₉, nCICP4₆₂₆, nCgL₆₅₅, nCUL₁₈₂₃, nCdUTP₉₁₈, nCUL49.5₂₆₁, nCUL49₂₅₅, and/or nCUL52₇₄₉. Also included is a protein that hybridizes under stringent hybridization conditions with nCAsc₉₃₀₀, nCAsc₁₀₀₀₀, nCHin₃₀₀₀, nCHin₁₉₀₀, nCHin₅₅₀₀, and/or nCHin₈₅₀₀. A further preferred isolated protein is encoded by a nucleic acid molecule that hybridizes under stringent hybridization conditions with a nucleic acid molecule having the complement of nucleic acid sequence SEQ ID NO:3, SEQ ID NO:5, SEQ ID NO:7, SEQ ID NO:9, SEQ ID NO:11, SEQ ID NO:15, SEQ ID NO:20, SEQ ID NO:24, SEQ ID NO:28, SEQ ID NO:32, SEQ ID NO:33, SEQ ID NO:38, SEQ ID NO:55, SEQ ID NO:57, SEQ ID NO:59, SEQ ID NO:61, SEQ ID NO:65, SEQ ID NO:67, SEQ ID NO:71, SEQ ID NO:75, SEQ ID NO:79, SEQ ID NO:81, SEQ ID NO:83, and/or SEQ ID.NO:87, or by an allelic variant of any of such nucleic acid molecules. Proteins encoded by nucleic acid sequences SEQ ID NO:3, SEQ ID NO:5, SEQ ID NO:7, SEQ ID NO:9, SEQ ID NO:11, SEQ ID NO:15, SEQ ID NO:20, SEQ ID NO:24, SEQ ID NO:28, SEQ ID NO:32, SEQ ID NO:33, SEQ ID NO:38, SEQ ID NO:55, SEQ ID NO:57, SEQ ID NO:59, SEQ ID NO:61, SEQ ID NO:65, SEQ ID NO:67, SEQ ID NO:71, SEQ ID NO:75, SEQ ID NO:79, SEQ ID NO:81, SEQ ID NO:83, and SEQ ID NO:87 have amino acid sequences SEQ ID NO:4, SEQ ID NO:6, SEQ ID NO:8, SEQ ID NO:10, SEQ ID NO:12, SEQ ID NO:16, SEQ ID NO:18, SEQ ID NO:22, SEQ ID NO:26, SEQ ID NO:30, SEQ ID NO:34, SEQ ID NO:36, SEQ IDSEQ ID NO:88, respectively.

Comparison of the CHV amino acid sequences SEQ ID NO:4, SEQ ID NO:6, SEQ ID NO:8, SEQ ID NO:10, SEQ ID NO:16, SEQ ID NO:18, SEQ ID NO:22, SEQ ID NO:26, SEQ ID NO:30, SEQ ID NO:34, SEQ ID NO:36, SEQ ID NO:56, SEQ ID NO:58, SEQ ID NO:60, SEQ ID NO:62, SEQ ID NO:66, SEQ ID NO:68, SEQ ID NO:72, SEQ ID NO:76, SEQ ID NO:80, SEQ ID NO:82, SEQ ID NO:84, and SEQ ID NO:88 with known sequences indicates that none of these CHV amino acid sequences share more than about 75% identity (many, if not all, sharing significantly less identity) with a known amino acid sequence.

A preferred CHV protein has an amino acid sequence that is at least about 80%, preferably at least about 85%, more preferably at least about 90%, even more preferably at least about 95%, and even more preferably at least about 99% identical to amino acid sequence SEQ ID NO:4, SEQ ID NO:6, SEQ ID NO:8, SEQ ID NO:10, SEQ ID NO:16, SEQ ID NO:18, SEQ ID NO:22, SEQ ID NO:26, SEQ ID NO:30, SEQ ID NO:34, SEQ ID NO:36, SEQ ID NO:56, SEQ ID NO:58, SEQ ID NO:60, SEQ ID NO:62, SEQ ID NO:66, SEQ ID NO:68, SEQ ID NO:72, SEQ ID NO:76, SEQ ID NO:80, SEQ ID NO:82, SEQ ID NO:84, and/or SEQ ID NO:88.

A more preferred CHV protein includes at least a portion of PCIR6₁₈₃, PCUS2₃₉₁, PCPK₄₀₀, PCgG₄₁₅, PCdUTP₁₅₂, PCUS9₁₄₉, PCUL48₉₇, PCUL52₄₈, PCUL51₈₆, PCgI₅₃, PCTK₉₃, PCgI₃₆₄, PCgE₅₂₂, PCUS8.5₇₈, PCUS9₁₁₉, PCUL49₁₃₉, PCUL48₄₂₂, PCICP4₂₀₈, PCgL₁₇₁, PCdUTP₃₀₅, PCUL49.5₈₆, PCUL49₈₅, and/or PCUL52₂₄₉, as well as proteins encoded by allelic variants of the nucleic acid molecules encoding such proteins. Also preferred are proteins including at least a portion of CgE and/or CUL49 proteins. A particularly preferred CHV protein includes PCIR6₁₈₃, PCUS2₃₉₁, PCPK₄₀₀, PCgG₄₁₅, PCDUTP₁₅₂, PCUS9₁₄₉, PCUL48₉₇, PCUL52₄₈, PCUL51₈₆, PCgI₅₃, PCTK₉₃, PCgI₃₆₄, PCgE₅₂₂, PCUS8.5₇₈, PCUS9₁₁₉, PCUL49₁₃₉, PCUL48₄₂₂, PCICP4₂₀₈, PCgL₁₇₁, PCdUTP₃₀₅, PCUL49.5₈₆, PCUL49₈₅, and/or PCUL52₂₄₉ (including, but not limited to the encoded proteins, full-length proteins, processed proteins, multivalent proteins).

Similarly, a preferred CHV protein of the present invention includes at least a portion of SEQ ID NO:4, SEQ ID NO:6, SEQ ID NO:8, SEQ ID NO:10, SEQ ID NO:16, SEQ ID NO:18, SEQ ID NO:22, SEQ ID NO:26, SEQ ID NO:30, SEQ ID NO:34, SEQ ID NO:36, SEQ ID NO:56, SEQ ID NO:58, SEQ ID NO:60, SEQ ID NO:62, SEQ ID NO:66, SEQ ID NO:68, SEQ ID NO:72, SEQ ID NO:76, SEQ ID NO:80, SEQ ID NO:82, SEQ ID NO:84, and/or SEQ ID NO:88. A particularly preferred CHV protein includes SEQ ID NO:4, SEQ ID NO:6, SEQ ID NO:8, SEQ ID NO:10, SEQ ID NO:16, SEQ ID NO:18, SEQ ID NO:22, SEQ ID NO:26, SEQ ID NO:30, SEQ ID NO:34, SEQ ID NO:36, SEQ ID NO:56, SEQ ID NO:58, SEQ ID NO:60, SEQ ID NO:62, SEQ ID NO:66, SEQ ID NO:68, SEQ ID NO:72, SEQ ID NO:76, SEQ ID NO:80, SEQ ID NO:82, SEQ ID NO:84, and/or SEQ ID NO:88. Also included are proteins encoded by allelic variants of the nucleic acid molecules encoding such proteins.

The present invention also includes a recombinant vector, which includes at least one isolated CHV nucleic acid molecule inserted into any vector capable of delivering the CHV nucleic acid molecule into a host cell. The vector can be either RNA or DNA, either prokaryotic or eukaryotic, and typically is a virus or a plasmid. Recombinant vectors can be used in the cloning, sequencing, and/or otherwise manipulating of CHV nucleic acid molecules of the present invention. Suitable and preferred CHV nucleic acid molecules to include in a recombinant vector are disclosed herein.

One embodiment of the present invention is a recombinant vector comprising an inactive CHV gene. Such a recombinant vector, also referred to as a transfer vector, can be used to produce a CHV comprising a CHV genome having an inactive gene by, for example, co-transfecting such a transfer vector with a CHV genome into a host cell and selecting for a CHV comprising a recombinant CHV genome having an inactive gene. Such a recombinant CHV genome is produced in the host cell by homologous recombination between the inactive gene on the transfer vector and the corresponding active gene on the transfected CHV genome. Transfection, culturing and purification methods to obtain recombinant CHV and CHV genomes are known to the art; see, for example, Graham et al., 1973, *Virology* 52:456-467

Another embodiment of the present invention is a recombinant vector comprising a CHV nucleic acid molecule that includes a heterologous nucleic acid molecule (i.e., a heterologous nucleic acid molecule is located within a CHV nucleic acid molecule). Suitable and preferred heterologous nucleic acid molecules are disclosed herein. Such a heterologous nucleic acid molecule can be operatively linked to a transcription control sequence, as disclosed above. A recombinant vector comprising a CHV nucleic acid molecule into which a heterologous nucleic acid molecule is inserted is also a transfer vector. Such a transfer vector can be co-transfected with a CHV genome into a host cell to produce a recombinant CHV having a CHV genome including a heterologous nucleic acid molecule, using methods as described above. Recombinant CHV can be selected by identifying those CHV that have the heterologous nucleic acid molecule. If the recombinant vector comprises a selectable marker into which the heterologous nucleic acid molecule is inserted, selection methods as disclosed herein can also be used to identify recombinant CHV. A preferred embodiment is a recombinant vector comprising a CHV nucleic acid molecule having a heterologous nucleic acid molecule in which a majority of the CHV nucleic acid molecule is deleted; a sufficient size of the CHV nucleic acid molecule is retained to allow homologous recombination to occur with the corresponding target gene on the CHV genome. Examples of insertion of a heterologous nucleic acid molecule into a CHV genomic restriction site and into a CHV gene, as well as use of a selectable marker are provided in the Examples section.

Transfer vectors of the present invention are preferably able to replicate in bacterial, and particularly *E*. *coli,* hosts, thereby enabling easy manipulation of the CHV nucleic acid molecules, and, if included, heterologous nucleic acid molecules, prior to insertion of such CHV nucleic acid molecules into a CHV genome. Such manipulations, including culturing of *E. coli* comprising such vectors, is described, for example, in Sambrook et al., *ibid*.

In one embodiment, recombinant CHV are produced by co-transfection of a set of overlapping cosmid clones comprising the entire viral CHV genome, at least one of the cosmid clones having been genetically engineered to, for example, contain an inactive CHV gene and/or a heterologous nucleic acid molecule. Details of such a method are presented in the Examples.

Any canid host cell is suitable for recombinant CHV production. Examples of suitable and preferred host cells are provided herein. After transfection, transfected cells are cultured in an effective medium, using techniques such as those described in Graham et al., *ibid*. As used herein, an effective medium refers to any medium in which the transfected cells can produce recombinant CHV. An effective medium is typically an aqueous medium comprising assimilable carbohydrate, nitrogen and phosphate sources, as well as appropriate salts, minerals, metals and other nutrients, such as vitamins, growth factors and hormones. Culturing is carried out at a temperature, pH and oxygen content appropriate for the transfected cell. Such culturing conditions are well within the expertise of one of ordinary skill in the art.

Recombinant CHV can be recovered from the cultured transfected cells using a combination of standard techniques such as, but not limited to, freeze/thaw cycles, sonication, sucrose gradient centrifugation, and/or high speed centrifugation. Recombinant CHV genomes can be recovered from the cultured transfected cells using a combination of standard techniques such as, but not limited to, those described by Walboomers et al., 1976, *Virology 74*, 256-258.

Preferably, a recombinant CHV or recombinant CHV genome comprised in the present invention is recovered in "substantially pure" form. As used herein, "substantially pure" refers to a purity that allows for the effective use of the recombinant CHV or CHV genome as a vaccine without substantial negative side effects.

The present invention also includes a method to increase recombinant CHV plaque forming efficiency, thereby facilitating the production of recombinant CHV and CHV genomes. The phrase to increase recombinant CHV plaque forming efficiency refers to the ability to increase, or enhance, the production of CHV above the frequency of plaque forming units produced when a CHV genome is transfected into a cell in the absence of additional factors. The method includes the step of expressing the CHV alpha trans-inducing factor gene (i.e., CUL48 gene, also called the CHV alpha-tif gene) when a CHV genome is introduced into a canine cell.

Herpesviruses studied to date carry into infected cells a transcriptional activator called alpha trans-inducing factor (also called VP16 or Vmw65); see, for example, Batterson et al., 1983, *J*. *Virol.,* 46:371-377. This transactivator protein interacts, along with cellular proteins, with a cis-acting sequence element present in the upstream regulatory regions of herpesvirus immediate early genes. Since purified herpesvirus DNA is infectious in the absence of alpha-tif, it is not absolutely required for expression of immediate early genes, but its presence in stably transfected cell lines has been shown to enhance infectivity of herpes simplex virus type-1 viral DNA; see, for example, Werstuck et al., 1990, *J*. *Virol.,* 64:984-991.

Expression of the CHV alpha-tif gene can be accomplished in a variety of ways, including, but not limited to, the following. In one embodiment, a recombinant CHV genome is introduced into a canine cell expressing CHV alpha trans-inducing factor and the cells are cultured to produce recombinant CHV. A canine cell expressing CHV alpha-tif is a canine cell that has been genetically engineered to produce CHV alpha-tif in either a stable or transient manner. Such a canine cell can be produced by introducing a CUL48 nucleic acid molecule of the present invention into a canine cell line in such a manner that the CUL48 nucleic acid molecule can direct the expression of active alpha-tif. An example of a useful CUL48 nucleic acid molecule is such nCUL48₁₂₆₉. Details regarding the production of an alpha-tif-expressing canine cell and the use such a cell in the production of recombinant CHV and CHV genomes is present in the Examples.

In another embodiment, recombinant CHV plaque forming efficiency is increased by co-introducing a recombinant CHV genome and a CHV alpha trans-inducing factor gene into a canine cell and culturing the cell to produce recombinant CHV. The CHV alpha trans-inducing factor gene can be introduced as part of a recombinant molecule of the present invention. Details regarding this method are also presented in the Examples.

The present invention also includes canine cell lines that include a CHV alpha-tif gene; such cell lines are able to express alpha-tif, thereby facilitating CHV production. One example of such a cell line is a canine cell line transfected with CHV nucleic acid molecule nCUL48₁₂₆₉.

One embodiment of the present invention is a recombinant molecule that includes a CHV nucleic acid molecule operatively linked to a transcription control sequence. Such a recombinant molecule, when introduced into a host cell, can direct the expression of the CHV nucleic acid molecule(s), thereby leading to the production of one or more CHV protein of the present invention. Such a recombinant molecule preferably is replication competent. Suitable and preferred CHV nucleic acid molecules to include in such a recombinant molecule are as disclosed herein for suitable and preferred CHV nucleic acid molecules per se.

Isolated CHV proteins of the present invention can be produced in a variety of ways, including production and recovery of natural proteins, production and recovery of recombinant proteins, and chemical synthesis of the proteins. In one embodiment, an isolated protein of the present invention is produced by culturing a cell capable of expressing the protein under conditions effective to produce the protein, and recovering the protein. A preferred cell to culture is a recombinant cell that is capable of expressing one or more CHV proteins, the recombinant cell being produced by transforming a host cell with one or more nucleic acid molecules of the present invention. Transformation of a nucleic acid molecule into a cell can be accomplished by any method by which a nucleic acid molecule can be inserted into the cell. Transformation techniques include, but are not limited to, transfection, electroporation, microinjection, lipofection, adsorption, and protoplast fusion. A recombinant cell may remain unicellular or may grow into a tissue, organ or a multicellular organism. Transformed nucleic acid molecules of the present invention can remain extrachromosomal or can integrate into one or more sites within a chromosome of the transformed (i.e., recombinant) cell in such a manner that their ability to be expressed is retained. Suitable and preferred CHV nucleic acid molecules with which to transform a cell are as disclosed herein for suitable and preferred CHV nucleic acid molecules per se.

Suitable host cells to transform include any cell that can be transformed with a CHV nucleic acid molecule of the present invention. Host cells can be either untransformed cells or cells that are already transformed with at least one nucleic acid molecule. Host cells of the present invention either can be endogenously (i.e., naturally) capable of producing CHV proteins of the present invention or can be capable of producing such proteins after being transformed with at least one CHV nucleic acid molecule of the present invention. Host cells of the present invention can be any cell capable of producing at least one protein of the present invention, and include bacterial, fungal (including yeast), parasite (including helminth, protozoa and ectoparasite), insect, other animal and plant cells. Preferred host cells include bacterial, yeast, insect and mammalian cells. Suitable transcription control sequences include any transcription control sequence that can function in at least one of the recombinant cells of the present invention. A variety of such transcription control sequences are known to those skilled in the art. Preferred transcription control sequences include those which function in bacterial, yeast, insect and mammalian cells, such as, but not limited to, *tac*, *lac, trp, trc*, oxy-pro, omp/lpp, rrnB, bacteriophage lambda (such as lambda P_{L} and lambda P_{R} and fusions that include such promoters), bacteriophage T7, T7*lac,* bacteriophage T3, bacteriophage SP6, bacteriophage SP01, metallothionein, alpha-mating factor, *Pichia* alcohol oxidase, alphavirus, phosphate-regulated and nitrate-regulated transcription control sequences as well as other sequences capable of controlling gene expression in prokaryotic or eukaryotic cells, including those disclosed herein for expression of heterologous nucleic acid molecules, including endogenous CHV transcription control regions.

Recombinant cells of the present invention can be used to produce one or more proteins by culturing such cells under conditions effective to produce such a protein, and recovering the protein. Effective conditions to produce a protein include, but are not limited to, appropriate media, bioreactor, temperature, pH and oxygen conditions that permit protein production. Such culturing conditions are well within the expertise of one of ordinary skill in the art.

Depending on the vector and host system used for production, resultant CHV proteins may either remain within the recombinant cell; be secreted into the fermentation medium; be secreted into a space between two cellular membranes, such as the periplasmic space in *E*. *coli*; or be retained on the outer surface of a cell or viral membrane.

The phrase "recovering the protein" refers simply to collecting the whole fermentation medium containing the protein and need not imply additional steps of separation or purification. Proteins of the present invention can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization. Proteins of the present invention are preferably retrieved in "substantially pure" form. As used herein, "substantially pure" refers to a purity that allows for the effective use of the protein as a therapeutic composition or diagnostic. A therapeutic composition for animals, for example, should exhibit no substantial toxicity and should be capable of stimulating the production of antibodies in a treated animal.

The present invention also includes isolated antibodies capable of selectively binding to a CHV protein of the present invention or to a mimetope thereof. Such antibodies are also referred to herein as anti-CHV antibodies. Isolated antibodies are antibodies that have been removed from their natural milieu. The term "isolated" does not refer to the state of purity of such antibodies. As such, isolated antibodies can include anti-sera containing such antibodies, or antibodies that have been purified to varying degrees.

As used herein, the term "selectively binds to" refers to the ability of antibodies of the present invention to preferentially bind to specified proteins and mimetopes thereof of the present invention. Binding can be measured using a variety of methods known to those skilled in the art.

Antibodies of the present invention can be either polyclonal or monoclonal antibodies. Antibodies of the present invention include functional equivalents such as antibody fragments and genetically-engineered antibodies, including single chain antibodies, that are capable of selectively binding to at least one of the epitopes of the protein or mimetope used to obtain the antibodies. Antibodies of the present invention also include chimeric antibodies that can bind to more than one epitope. Preferred antibodies are raised in response to proteins, or mimetopes thereof, that are encoded, at least in part, by a nucleic acid molecule of the present invention.

A preferred method to produce antibodies of the present invention includes (a) administering to an animal an effective amount of a protein or mimetope thereof of the present invention to produce the antibodies and (b) recovering the antibodies. In another method, antibodies of the present invention are produced recombinantly using techniques as heretofore disclosed to produce CHV proteins of the present invention. Antibodies raised against defined proteins or mimetopes can be advantageous because such antibodies are not substantially contaminated with antibodies against other substances that might otherwise cause interference in a diagnostic assay or an assay to monitor recombinant CHV administration, or side effects if used in a therapeutic composition.

Antibodies of the present invention have a variety of potential uses that are within the scope of the present invention. For example, such antibodies can be used (a) as compounds to monitor recombinant CHV or recombinant CHV genome administration, (b) as therapeutic compounds to passively immunize an animal in order to protect the animal from CHV infection, and/or (c) as reagents in assays to detect CHV infection.

One embodiment of the present invention is a therapeutic composition comprising an isolated CHV nucleic acid molecule of the present invention. As used herein, a therapeutic composition, or vaccine, is a formulation that, when administered to an animal in an effective manner, is capable of protecting that animal from a disease against which the therapeutic composition is targeted. Some therapeutic compositions of the present invention can modulate (i.e., either elicit or suppress) an immune response to protect an animal from disease, whereas other therapeutic compositions can protect an animal in other ways, as disclosed herein. A therapeutic composition of the present invention can be used to prevent and/or treat a disease depending on whether the composition is administered as a prophylactic or after the animal has the disease.

A therapeutic composition of the present invention, when administered to an animal in an effective manner, can infect the cells of the animal (in a manner essentially harmless to the animal) and direct the production of a protective compound able to protect the animal from a disease targeted by the therapeutic composition. If a recombinant CHV includes a protective compound in its envelope and/or capsid, the CHV can function as an immunogen per se.

As disclosed herein, therapeutic compositions can be designed to target a variety of diseases, depending on the nature of the heterologous nucleic acid molecule(s) included in such recombinant CHV and recombinant CHV genomes. Examples of such diseases, and the nature of the corresponding heterologous nucleic acid molecules, are disclosed herein. Furthermore, therapeutic compositions of the present invention can comprise multivalent vaccines. For example, a CHV genome can encode a variety of protective compounds, and/or more than one recombinant CHV genome and/or recombinant CHV can be administered. The present invention also includes the use of a recombinant CHV or recombinant CHV genome to protect an animal against CHV infection. Such a therapeutic composition can, but need not, include a heterologous nucleic acid molecule.

A therapeutic composition of the present invention is preferably administered to a canid, due to the host range specificity of CHV. Suitable canids include dogs (including domesticated and wild dogs), foxes, wolves, jackals, coyotes, and other members of the family Canidae. Particularly preferred canids to treat include domesticated dogs.

It is, however, also within the scope of the present invention to administer therapeutic compositions to other animals. Recombinant CHV can be administered to any animal susceptible to such therapy. Without being bound by theory, it is believed that sufficiently high doses of a recombinant CHV composition of the present invention may be infectious in other animals, particularly other mammals. Mink and other mink-like mammals (e.g., those of the family Mustelidae, such as ermines, ferrets, fishers, martens, otters, and weasels), in particular, may be susceptible to CHV infection, as suggested by the ability of CHV to infect mink lung cells. The host range of recombinant CHV of the present invention can also be altered, as disclosed herein, to infect other animal cells. It is also to be noted that CHV genomes of the present invention can be administered as naked DNA vaccines or in association with other carriers (e.g., liposomes). In accordance with these embodiments, any animal, including, but not limited to, mammals, birds, amphibians, and arthropods (including arachnids and insects) can be administered a therapeutic composition of the present invention. Preferred animals to treat include dogs, cats, humans, ferrets, prairie dogs, other rodents, horses, cattle, sheep, pigs, and poultry, as well as other pets, work animals, economic food animals and zoo animals.

Therapeutic compositions of the present invention can be formulated in an excipient that the animal to be treated can tolerate. Examples of such excipients include water, saline, Ringer's solution, dextrose solution, Hank's solution, and other aqueous physiologically balanced salt solutions. Other useful formulations include suspensions containing viscosity enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer and Tris buffer. Standard formulations can either be liquid injectables or solids which can be taken up in a suitable liquid as a suspension or solution for injection. Thus, in a non-liquid formulation, the excipient can comprise dextrose, human serum albumin, preservatives, etc., to which sterile water or saline can be added prior to administration.

In one embodiment of the present invention, the vaccine can also include an adjuvant and/or a carrier. One advantage of live virus-based vaccines, such as the recombinant CHVs of the present invention, is that adjuvants and carriers are not required to produce an efficacious vaccine, and in some cases, the advantages of recombinant CHV vaccines of the present invention would be precluded by the use of some adjuvants. However, it should be noted that use of adjuvants or carriers is not precluded by the present invention. Adjuvants are typically substances that generally enhance the immune response of an animal to a specific antigen. Suitable adjuvants include, but are not limited to, other bacterial cell wall components; aluminum-based salts; calcium-based salts; silica; polynucleotides; toxoids; serum proteins; other viral coat proteins; other bacterial-derived preparations; block copolymer adjuvants, such as Hunter's Titermax^{™} adjuvant (Vaxcel^{™}, Inc. Norcross, GA); Ribi adjuvants (available from Ribi ImmunoChem Research, Inc., Hamilton, MT); and saponins and their derivatives, such as Quil A (available from Superfos Biosector A/S, Denmark). Carriers are typically compounds that increase the half-life of a therapeutic composition in the treated animal. Suitable carriers include, but are not limited to, polymeric controlled release formulations, biodegradable implants, liposomes, bacteria, viruses, oils, esters, and glycols.

In one embodiment, a therapeutic composition of the present invention is administered to an animal in an effective manner to enable the animal to produce sufficient protective compound(s) and/or to directly mount a sufficient immune response to protect the animal from disease. Acceptable protocols to administer therapeutic compositions in an effective manner include enumeration of individual dose size, number of doses, frequency of dose administration, and mode of administration. Determination of such protocols can be accomplished by those skilled in the art. A suitable single dose is a dose that is capable of protecting an animal from disease when administered one or more times over a suitable time period.

A preferred single dose of a recombinant CHV of the present invention is from about 1 x 10⁴ to about 1 x 10⁷ virus plaque forming units (pfu) per kilogram body weight of the animal. Booster vaccinations can be administered from about 2 weeks to several years after the original vaccination. Booster vaccinations preferably are administered when the immune response of the animal becomes insufficient to protect the animal from disease. A preferred administration schedule is one in which from about 1 x 10⁴ to about 1 x 10⁷ virus plaque forming units per kilogram (kg) body weight of the animal are administered from about 1 to about 2 times over a time period of from about 12 to about 18 months. Modes of administration can include, but are not limited to, subcutaneous, intradermal, intravenous, intranasal, oral, transdermal and intramuscular routes.

A recombinant CHV genome can be administered in a variety of ways, with intramuscular, subcutaneous, intradermal, transdermal, intranasal and oral routes of administration being preferred. A preferred single dose of a recombinant CHV genome ranges from about 1 nanogram (ng) to about 100 micrograms (µg), depending on the route of administration and/or method of delivery, as can be determined by those skilled in the art; see, for example, Wolff et al., 1990, *Science 247,* 1465-1468. Suitable delivery methods include, for example, injection, as drops, aerosolized and/or topical administration. Suitable excipients include, for example, physiologically acceptable aqueous solutions (e.g., phosphate buffered saline as well as others disclosed above), liposomes (including neutral or cationic liposomes), and other lipid membrane-based vehicles (e.g., micelles or cellular membranes).

In one embodiment, a therapeutic composition of the present invention is administered to a dam to protect her offspring from disease. In this method, the dam is administered the therapeutic composition at such a time as to be able to develop an immune response such that she can passively transfer antibodies produced against a protective compound of the present invention to her offspring. Such a method can also be used to protect offspring from CHV infection and is particularly useful since neonates are most affected by CHV infection.

The efficacy of a therapeutic composition of the present invention to protect an animal from disease can be tested in a variety of ways including, but not limited to, detection of protective protein or RNA within the treated animal, detection of protective antibodies within the treated animal, detection of cellular immunity within the treated animal, or challenge of the treated animal with an appropriate infectious agent, or other disease component, to determine whether the animal is now protected from the disease caused by such an agent or other component. Such techniques are known to those skilled in the art. In one embodiment, anti-CHV antibodies of the present invention are used to monitor recombinant CHV infection and can be used to distinguish wild type infections from infections using recombinant CHV of the present invention (i.e., by using antibodies that specifically recognize either recombinant CHV of the present invention or wild type virus).

In one embodiment, the efficacy of a therapeutic composition of the present invention may be improved by coadministering (a) a recombinant CHV or recombinant CHV genome and (b) a protective compound (e.g., subunit vaccine) encoded by a CHV nucleic acid molecule or heterologous nucleic acid molecule present in the CHV genome. While not being bound by theory, it is believed that administration of a protective compound in conjunction with the recombinant CHV or CHV genome can boost the immune response, particularly the antibody titer. The protective compound can be administered prior to, concomitant with, and/or following administration of the recombinant CHV or CHV genome. The protective compound can be either produced naturally, recombinantly, or synthetically. The protective compound should be sufficiently pure to allow for effective use of the compound as a vaccine: i.e., it should not cause substantial side effects. The protective compound can be joined (i.e., conjugated) to a carrier or other material that enhances the immunogenicity of the compound.

The present invention also includes the use of CHV nucleic acid molecules, CHV proteins, and anti-CHV antibodies in the preparation of a therapeutic composition for protecting animals from CHV infection. Methods to administer such compositions to canids are known to those skilled in the art. For example, a preferred single dose of a protein, mimetope or antibody therapeutic composition is from about 1 microgram (µg) to about 10 milligrams (mg) of the therapeutic composition per kilogram body weight of the animal. Booster vaccinations can be administered from about 2 weeks to several years after the original administration. Booster vaccinations preferably are administered when the immune response of the animal becomes insufficient to protect the animal from disease. A preferred administration schedule is one in which from about 10 µg to about 1 mg of the therapeutic composition per kg body weight of the animal is administered from about one to about two times over a time period of from about 2 weeks to about 12 months. Modes of administration can include, but are not limited to, subcutaneous, intradermal, intravenous, intranasal, oral, transdermal and intramuscular routes. A CHV nucleic acid molecule, including recombinant molecules, can be administered as described herein for administration of CHV genomes or CHV of the present invention. Recombinant molecules including heterologous nucleic acid molecules can also be used as therapeutic compositions to protect an animal from disease, using methods as disclosed herein.

It is also within the scope of the present invention to use isolated CHV proteins, mimetopes, CHV nucleic acid molecules and anti-CHV antibodies of the present invention as diagnostic reagents to detect CHV infection. Methods to use such diagnostic reagents to diagnose infection are well known to those skilled in the art.

The following examples are provided for the purposes of illustration and are not intended to limit the scope of the present invention.

### EXAMPLES

It is to be noted that the Examples include a number of molecular biology, virology, microbiology, immunology and biochemistry techniques considered to be known to those skilled in the art. Disclosure of such techniques can be found, for example, in Sambrook et al., *ibid*.; Ausubel et al., 1993, *Current Protocols in Molecular Biology,* Greene/Wiley Interscience, New York, NY; Graham et al., *ibid*.; and related references. Nucleic acid and amino acid sequences of the present invention were compared to known sequences using BLAST (NCBI) and DNAsis (Hitachi Software, San Bruno, CA).

### Example 1

This Example demonstrates the isolation of certain CHV nucleic acid molecules of the present invention.

The disclosed GHV-nucleic acid molecules were amplified from a CHV genome by PCR amplification using a variety of primers designed in view of published herpesvirus sequences. The following PCR conditions were used: 0.2 millimolar (mM) dNTPs, 1 µM of each primer, 1X PCR buffer (available from Perkin Elmer Cetus, Emeryville, CA), 50 ng of CHV DNA (isolated from CHV strain D 004 as described in Example 2) and 0.5 µl of a thermostable DNA polymerase, all in an about 100 µl total volume. The PCR reactions included an initial denaturation for 3 minutes at 95°C, five cycles of 1 minute each at 95°C, 35°C for 1 minute, 72°C for 1 minute, 35 cycles of 1 minute each at 95°C, 37°C for 1 minute, 72°C for 1 minute, and finally 10 minutes at 72°C. The resultant PCR products were directly cloned into the pCRII TA cloning vector (available from Invitrogen Corp., San Diego, CA) according to the manufacturer's specifications. Primers which were successful in amplifying fragments from a CHV genome, which was determined to be very AT-rich compared to other herpesvirus genomes, are described below.

### A. Isolation of a nucleic acid molecule including a partial CHV dUTPase gene and a partial CHV UL51 gene

The following primers were designed using dUTPase protein sequence derived from HSV-1 (McGeoch et al., 1988, J. *Gen. Virol. 69,* 1531-1574), EHV-4 (Riggio et al., 1993, Arch. *Virol. 133,* 171-178), BHV-1 (Liang et al., 1993, *Virology 195,* 42-50), and EBV (Lees et al., 1993, *Virology 195,* 578-586: Primer 212S (dUTPase forward) having nucleic acid sequence 5' GG CGA ATT CCI AAR MGI GAI GAR GAY G 3', denoted herein as SEQ ID NO:39; and Primer 365A (dUTPase reverse) having nucleic acid sequence 5' C GCG GAT CCI GTI SWI CCY AAI CC 3', denoted herein as SEQ ID NO:40. These primers led to the amplification of an about 743 nucleotide fragment, which was significantly larger than expected. Nucleic acid sequence analysis, described in Example 3, indicated that the 743 nucleotide fragment contained part of the CUL51 gene as well as part of the CdUTPase gene; as such, the fragment was denoted nCdUTP/CUL51₇₄₃. Nucleic acid sequence analysis also indicated that the dUTPase reverse primer actually hybridized to a region of the CHV DNA genome within the CUL51 gene rather than within the CdUTPase gene. It is believed that the mispriming was due to nucleotide position 19 of SEQ ID NO:40 being a Y instead of an R; the latter sequence would have matched more closely to the targeted priming region, about 290 nucleotides upstream from the 3' end of nucleic acid molecule nCdUTP/CUL51₇₄₃. This result demonstrates the sensitivity of PCR amplification to primer design.

### B. Isolation of a CHV gE nucleic acid molecule

The following primers were designed using gE protein sequence derived from FHV-1 (Spatz et al., 1994, *J Gen. Virol. 75,* 1235-1244 ), EHV-1 (Elton et al., 1991, *Gene 101,* 203-208), and BHV-1 (Leung-Tack et al., 1994, *Virology 199,* 409-421): Primer 197S (gE forward) having nucleic acid sequence 5' GGC GAA TTC TAY CAY WSI CAY GTI TA 3', denoted herein as SEQ ID NO:41; and Primer 441A (gE reverse) having nucleic acid sequence 5' CGC GGA TCC RTC RTT ISW IGG DAI ISW IGT 3', denoted herein as SEQ ID NO:42. These primers led to the amplification of an about 750 nucleotide fragment, referred to herein as nCgE₇₅₀.

### C. Isolation of a CHV TK nucleic acid molecule

The following primers were designed using TK protein sequence derived from HSV-1 (McGeoch et al., 1988, ibid.), HSV-2 (Kit et al., 1987, *Antimicrob*. *Agents Chemother.* 31, 1483-1490, BHV-1 (Kit et al., U.S. Patent No. 4703011, issued October 27, 1987), FHV-1 (Nunberg et al., 1989, *J. Virol. 63,* 3240-3249), EHV-1 (Robertson et al., 1988, *Nuc. Acids Res. 16*, 11303-11317), and PRV (Prieto et al., 1991, *J. Gen. Virol. 72*, 1435-1439): Primer EJH 002 (TK forward) having nucleic acid sequence 5' GGC GAA TTC GGI AAR WSI ACI RC 3', denoted herein as SEQ ID NO:43; and Primer EJH004 (TK reverse) having nucleic acid sequence 5' GGC GGA TCC GGT TGI CKR TC 3', denoted herein as SEQ ID NO:44. These primers led to the amplification of an about 280 nucleotide fragment, referred to herein as nCTK₂₈₀.

### D. Isolation of a nucleic acid molecule including CHV gC and CHV UL45 genes

The following primers were designed using gC and UL45 sequences derived from Limbach et al., *ibid*.: Primer gC sense, having nucleic acid sequence 5' CGCGGATCCAAGGTAATAAGTCAAAATGAG 3' , denoted herein as SEQ ID NO:45; and Primer gC ant, having nucleic acid sequence 5' CGCGGATCCGACAAAAACAAAAAGTAATG 3', denoted herein as SEQ ID NO:46. These primers led to the amplification of an about 2100 nucleotide fragment, referred to herein as nCgC/CUL45₂₁₀₀.

### E. Attempt to isolate a CHV ribonucleotide reductase gene

The following primers were designed using ribonucleotide subunit small subunit protein sequence derived from HSV-1 (McGeoch et al., 1988, *ibid*.), PRV (Dewind et al., 1993, J. *Gen. Virol. 74*, 351-359), EHV-1 (Telford et al., 1992, *Virology 189*, 04-316) and BHV-1 (Simard et al., 1992, *Virology 190,* 689-701): Primer EJH 021 (RR forward) having nucleic acid sequence 5' CCG AAT TCY TIA TGA THY TIA THG ARG G 3', denoted herein as SEQ ID NO:47; and Primer EJH022 (RR reverse) having nucleic acid sequence 5' CCG GAT CCY TCR AAR AAR TTI GTR TGY TT 3' , denoted herein as SEQ ID NO:48. These primers did not lead to the amplification of a fragment under a variety of magnesium and amplification conditions, suggesting the lack of a coding region for a ribonucleotide reductase small subunit in CHV. As a control, these primers were shown to be able to easily amplify a ribonucleotide reductase small subunit fragment from an FHV genome.

### Example 2

This Example describes the production of CHV genomic libraries.

Canine herpesvirus strain D 004 (Binn, et al., 1967, *Proc. Soc. Exp. Biol. Med. 126,* 140) was obtained from ATCC. Virus were propagated on Madin-Darby Canine Kidney (MDCK) cells according to standard virological procedures. Viral DNA was prepare from CHV-infected MDCK cells by previously described methods; see, for example, Walboomers et al., *ibid*. The viral DNA was digested with restriction endonucleases *Hind*III, *Pst*I*, Eco*RI or *Xba*I, and the resultant digests were cloned into either vector pSP72 (available from Promega Corp., Madison, WI), or pLitmus 28 or 38 (available from New England Biolabs, Beverly, MA). DNA was prepared from the resultant recombinant plasmids and the inserts were sorted according to size.

### Example 3

This Example describes the isolation of genomic *Hin*dIII restriction fragment nucleic acid molecules containing CdUTPase nucleic acid sequences and the nucleic acid sequencing of at least regions of these nucleic acid molecules.

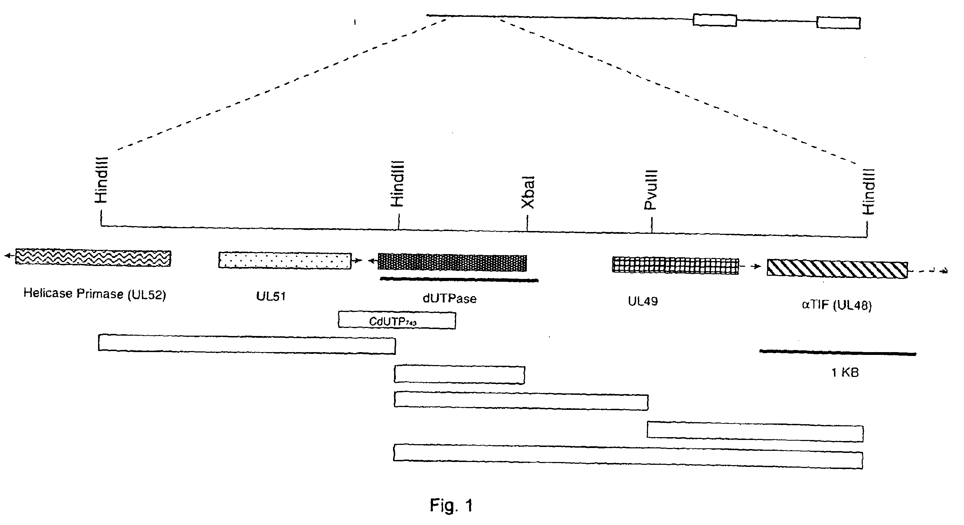

Nucleic acid molecule nCdUTP/CUL51₇₄₃, produced as described in Example 1, was labelled with either ³²P or digoxigenin (available from Boehringer Mannheim Biochemicals (BMB), Indianapolis, IN) and used to screen the CHV *Hin*dIII fragment-containing library, either by colony hybridization or Southern blotting. Two clones hybridized with nCdUTP/CUL51₇₄₃, one containing a *Hin*dIII fragment of about 3000 nucleotides, referred to herein as nCHin₃₀₀₀, and another containing a *Hin*dIII fragment of about 1,900 nucleotides, referred to herein as nCHin₁₉₀₀. The *Hin*dIII fragments were further characterized by restriction mapping, as shown in Fig. 1. Also shown is the relative location of nCdUTP/CUL51₇₄₃ (referred to therein as nCdUTP₇₄₃) with respect to the *Hin*dIII fragments.

Nucleic acid molecules nCdUTP/CUL51₇₄₃, nCHin₃₀₀₀, and nCHin₁₉₀₀ were submitted to DNA sequence analysis, using a sequencing strategy as depicted in Fig. 1. Among sequences obtained were SEQ ID NO:13 and SEQ ID NO:14, which include SEQ ID NO:15 and SEQ ID NO:33, as well as SEQ ID NO:21, SEQ ID NO:23, SEQ ID NO:25 and SEQ ID NO:27. The relative locations of genes included in these sequences (i.e., the CUL52, CUL51, CdUTPase and CUL48 genes) are shown in Fig. 1. Also shown is the apparent location of the CUL49 gene.

SEQ ID NO:13 and SEQ ID NO:14 represent the deduced nucleic acid sequences of the two complementary strands of nCdUTP/CUL51₇₄₃. SEQ ID NO:13 includes a partial coding region for a CdUTPase protein of about 152 amino acids, denoted herein as nCdUTP₄₅₉ and represented by SEQ ID NO:15, assuming a first in-frame codon spanning about nucleotides 3-5, and a stop codon spanning about nucleotides 459-461 of SEQ ID NO:13. The amino acid sequence of the encoded protein PCdUTP₁₅₂ is represented by SEQ ID NO:16. SEQ ID NO:14 includes a partial coding region for a CUL51 protein of about 86 amino acids, denoted herein as nCUL51₂₆₁ and represented by SEQ ID NO:33, assuming a first in-frame codon spanning about nucleotides 1-3, and a stop codon spanning about nucleotides 259-261 of SEQ ID NO:14. The amino acid sequence of the encoded protein PCUL5186 is represented by SEQ ID NO:34.

SEQ ID NO:21 and SEQ ID NO:23 represent the deduced nucleic acid sequences of the two complementary strands of nCUL48₂₉₄. SEQ ID NO:21 includes a partial coding region for a CUL48 protein of about 97 amino acids, denoted herein as nCUL48₂₉₁ and represented by SEQ ID NO:24, assuming a first in-frame codon spanning about nucleotides 3-5 of SEQ ID NO:21. The amino acid sequence of the encoded protein PCUL48₉₇ is represented by SEQ ID NO:22.

SEQ ID NO:25 and SEQ ID NO:27 represent the deduced nucleic acid sequences of the two complementary strands of nCUL52₁₄₆. SEQ ID NO:25 includes a partial coding region for a CUL52 protein of about 48 amino acids, denoted herein as nCUL52₁₄₄ and represented by SEQ ID NO:28, assuming a first in-frame codon spanning about nucleotides 1-3 of SEQ ID NO:25. The amino acid sequence of the encoded protein PCUL52₄₈ is represented by SEQ ID NO:26.

### Example 4

This Example describes a method to identify restriction enzymes that effect limited cleavage of the CHV genome. Also described is the isolation and sequencing of an *Asc*I fragment containing the entire US region of CHV.

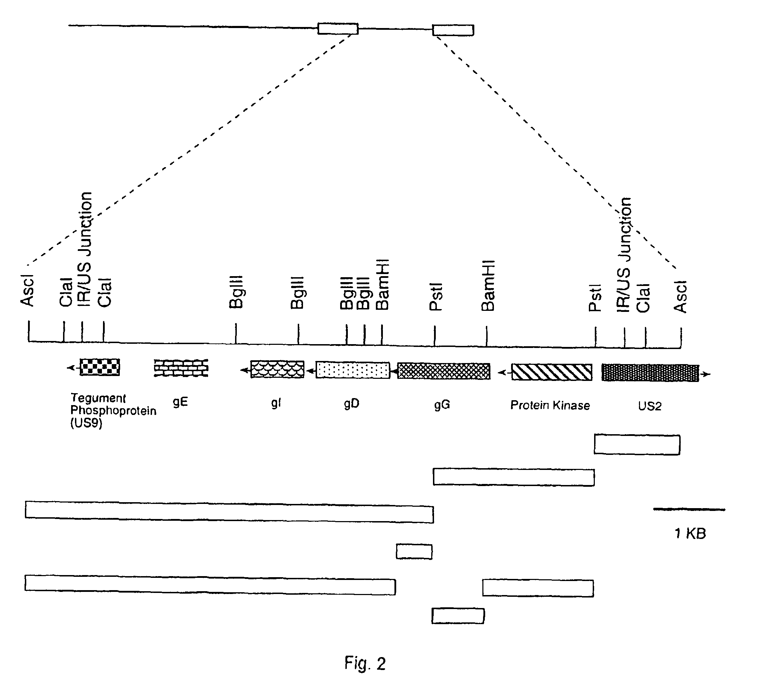

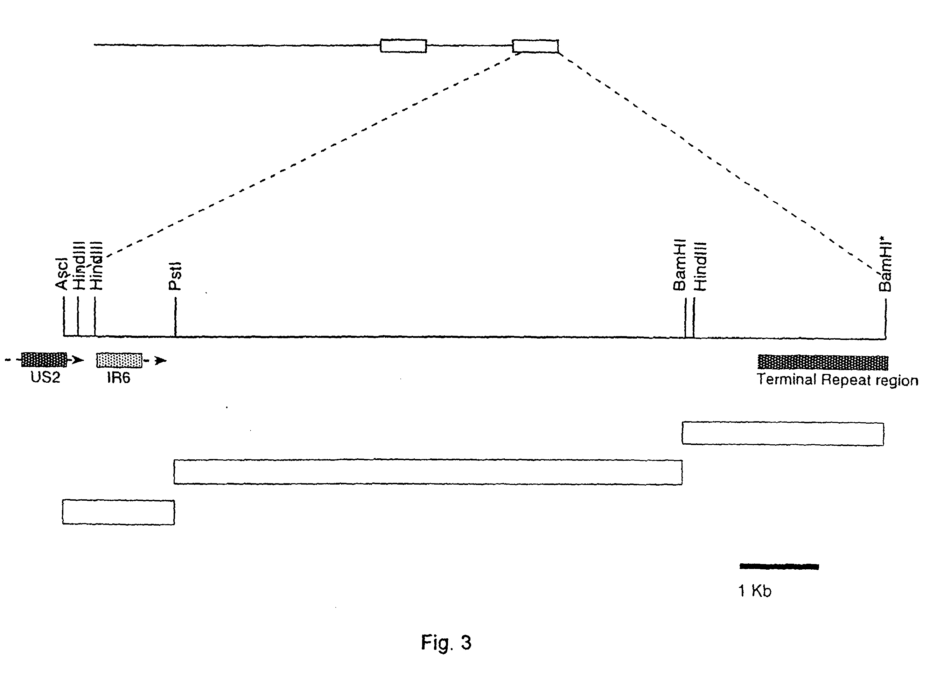

CHV DNA was evaluated for recognition sites for several restriction enzymes with 8-base, or larger, recognition sites. No sites were found for enzymes *Sse*8387I, I-*Sce*-I, or *Not*I. Two *Asc*I sites were found, which were mapped to sites within the inverted repeats surrounding the US region, specifically within CHV gene US2 (see Fig. 2). The *Asc*I fragments, of 9.3, 10.0 and approximately 100-120 kilobases (kb), were cloned into pLitmus 38 and submitted to restriction enzyme mapping analysis. The restriction map of the 9.3-kb AscI fragment, denoted herein as nCAsc9300 is shown in Fig. 2; this fragment apparently contains the entire CUS region, as well as small portions of the internal and terminal IR regions. The 10.0-kb *Asc*I-blunt fragment, denoted herein as nCAsc10000, contains most of the terminal IR region and is shown in Fig. 3. The about 100-120-kb AscI-blunt fragment contains the remainder of the CHV genome. Both AscI sites are located just 5' of the diploid copies of the CHV homolog of the EHV-1 IR6 gene (Breeden et al., 1992, *Virology 191,* 649-660), and the orfl of BHV-1 (Leung-Tack et al., *ibid*.). The AscI sites appear to be located within an open reading frame that is the homolog of the EHV US1 gene (Breeden et al *ibid*.) and the HSV-1 US2 gene (McGeoch et al., 1985, *J. Mol. Biol 181*, 1-13), although the size is somewhat different from these other genes. No function has been ascribed to the HSV-1 US2 gene product, but it has been shown to be dispensable for growth in tissue culture and to attenuate the virus (Meignier et al., 1988, *Virology 162,* 251-254).

Nucleic acid molecules nCAsc₉₃₀₀ and nCAsc₁₀₀₀₀ were submitted to DNA sequence analysis using a strategy as depicted in Fig. 2 and Fig. 3. Among sequences obtained were SEQ ID NO:1, SEQ ID NO:2, SEQ ID NO:17, SEQ ID NO:19, SEQ ID NO:29, and SEQ ID NO:31 The relative locations of genes included in these sequences (i.e., the CUS9, CgI, CgD, CgG, CPK, CUS2, and C!R6 genes) are shown in Fig. 2 and Fig.3. Also shown is the putative location of the CgE gene.

SEQ ID NO:1 and SEQ ID NO:2 represent the deduced nucleic acid sequences of the two complementary strands of nCUS₅₄₉₅. Translation of SEQ ID NO:1 and SEQ ID NO:2 indicates that nucleic acid molecule nCUS₅₄₉₅ encodes CIR6, CUS2, CPK and CgG proteins as well as a portion of a CgD protein. Specifically, SEQ ID NO:2 includes: a coding region for a CIR6 protein of about 183 amino acids, denoted herein as nCIR6₅₅₂ and represented by SEQ ID NO:3, assuming a start codon spanning about nucleotides 4566-4568 and a stop codon spanning about nucleotides 5115-5117 of SEQ ID NO:2; and a coding region for a CUS2 protein of about 391 amino acids, denoted herein as nCUS2₁₁₇₆ and represented by SEQ ID NO:5, assuming a start codon spanning about nucleotides 3232-3234 and a stop codon spanning about nucleotides 4405-4407 of SEQ ID NO:2. The amino acid sequences of the respective encoded proteins PCIR6₁₈₃ and PCUS2₃₉₁ are represented by SEQ ID NO:4 and SEQ ID NO:6. A portion of SEQ ID NO:3, from about nucleotide 232 through about nucleotide 548, was found to be similar to GenBank Accession No. X90423, as disclosed by Remond, et al., 1996, *ibid*. The published partial sequence, however, appears to contain sequencing errors, in that a G-C inversion and two frame-shifts were found in this sequence relative to SEQ ID NO:3. SEQ ID NO:1 includes: a coding region for a CPK protein of about 400 amino acids, denoted herein as nCPK₁₂₀₃ and represented by SEQ ID NO:7, assuming a start codon spanning about nucleotides 2384-2386 and a stop codon spanning about nucleotides 3584-3586 of SEQ ID NO:1; a coding region for a CgG protein of about 415 amino acids, denoted herein as nCgG₁₂₄₈ and represented by SEQ ID NO:9, assuming a start codon spanning about nucleotides 3698-3700 and a stop codon spanning about nucleotides 4943-4945 of SEQ ID NO:1; and a partial coding region for a CgD protein of about 119 amino acids, denoted herein as nCgD₃₅₇ and represented by SEQ ID NO:11, assuming a start codon spanning about nucleotides 5137-5139. The amino acid sequences of the respective encoded proteins PCPK₄₀₀, PCGG₄₁₅, and PCgD₁₁₉ are represented by SEQ ID NO:8, SEQ ID NO:10 and SEQ ID NO:12.

SEQ ID NO:17 and SEQ ID NO:19 represent the deduced nucleic acid sequences of the two complementary strands of nCUS9₅₇₉. SEQ ID NO:17 includes a coding region for a CUS9 protein of about 149 amino acids, denoted herein as nCUS9₄₅₀ and represented by SEQ ID NO:20, assuming a start codon spanning about nucleotides 54-56 and a stop codon spanning about nucleotides 501-503 of SEQ ID NO:17. The amino acid sequence of the encoded protein PCUS9₁₄₉ is represented by SEQ ID NO:18.

SEQ ID NO:29 and SEQ ID NO:31 represent the deduced nucleic acid sequences of the two complementary strands of nCgI₁₆₁. SEQ ID NO:29 includes a partial coding region for a CgI protein of about 53 amino acids, denoted herein as nCgI₁₅₉ and represented by SEQ ID NO:32, assuming a first in-frame codon spanning about nucleotides 3-5 of SEQ ID NO:29. The amino acid sequence of the encoded protein PCgI₅₃ is represented by SEQ ID NO:30.

### Example 5

This Example describes the nucleic acid sequencing of additional nucleic acid molecules of the present invention.

Nucleic acid molecule nCTK₂₈₀, produced as described in Example 1, was submitted to DNA sequence analysis to obtain the following sequences. SEQ ID NO:35 and SEQ ID NO:37 represent the deduced nucleic acid sequences of the two complementary strands of nCTK₂₈₀. SEQ ID NO:35 includes a partial coding region for a CTK protein of about 93 amino acids, denoted herein as nCTK₂₇₉ and represented by SEQ ID NO:38, assuming a first in-frame codon spanning about nucleotides 2-4 of SEQ ID NO:35. The amino acid sequence of the encoded protein PCTK₉₃ is represented by SEQ ID NO:36.

### Example 6

This Example discloses the production of a recombinant CHV genome and recombinant CHV of the present invention.

A cassette including the human CMV immediate early promoter and the poly-adenylation signal from bovine growth hormone separated by a polylinker was PCR amplified from plasmid pcDNA3 (available from Invitrogen) using forward primer EJH058 having nucleic acid sequence 5' TCCCCCGGGGGCGCGCCTTGACATTGATTATTGAC 3', denoted SEQ ID NO:49, and reverse primer EJH059 having nucleic acid sequence 5' GCCCTTAAGGGGCGCGCCAATGCGATGCAATTTCC 3', denoted SEQ ID NO:50. EJH058 has *Sma*I and AscI sites attached to the 5' end of pcDNA3 homologous sequences, and EJH059 has *Afl*II and *Asc*I sites attached to the 5' end of pcDNA3 homologous sequences. The resultant PCR amplified fragment of about 930 nucleotides was digested with *Sma*I and *Afl*II and ligated into the *Sna*BI and *Afl*II sites of plasmid pLitmus 38. This cloning procedure eliminated the entire polylinker region of pLitmus38. The resulting recombinant plasmid, denoted herein as pAscCMV/BGH, contains the CMV promoter - BGH polyadenylation signal cassette, with its original polylinker, in a plasmid such that the entire cassette can be excised with enzyme *Asc*I. This cassette plasmid can also be prepared with other rare-cutting enzyme sites flanking the cassette.

Plasmid pAscCMV/BGH allows for the insertion of heterologous nucleic acid molecules between the CMV promoter and the BGH polyadenylation signal. The resulting heterologous nucleic acid molecule-containing cassette was excised from the plasmid for insertion into a CHV genome. For example, a heterologous nucleic acid molecule containing a *lac*Z gene was inserted into the polylinker region of pAscCMV/BGH such that the *lac*Z gene is expressed by the CMV promoter in a eukaryotic system. The cassette containing the *lac*Z gene, referred to herein as AscCMV/lacZ/BGH is then excised from the plasmid by *Asc*I digestion and gel purified by standard methods.

About 5-10 µg of CHV DNA is digested with *Asc*I, resulting in three fragments as disclosed herein. The DNA fragments are dephosphorylated with calf intestine alkaline phosphatase (available from BMB) for 10 minutes at 37°C; the enzyme is then inactivated for by incubation for 10 minutes at 65°C. The phosphatase-treated digested CHV DNA is then subjected to extraction with phenol and phenol/chloroform and precipitated with ethanol.

The phosphatase-treated digested CHV genomic DNA is mixed with the gel-purified AscCMV/lacZ/BGH cassette at a molar ratio of approximately 1:2 under standard ligation conditions. Since the viral DNA is dephosphorylated, it should not be able to self ligate; thus all resultant ligated viral molecules should contain two copies of the inserted cassette. The ligated DNA is then subjected to phenol extraction and ethanol precipitated.

The precipitated ligated DNA is resuspended in hepes-buffered saline and submitted to standard viral transfection conditions, such as that described by Graham et al., *ibid*., along with appropriate controls (e.g., undigested viral DNA, digested and dephosphorylated viral DNA that was self-ligated, and no DNA). Resultant viral plaques are screened under an X-gal overlay for expression of β-galactosidase.

### Example 7

This Example discloses the production of another recombinant CHV genome and recombinant CHV of the present invention.

A recombinant, or transfer, vector to be used in the production of a recombinant CHV having a heterologous nucleic acid molecule in a TK gene of the CHV genome is constructed as follows. A CHV TK nucleic acid molecule of the present invention (e.g., nCTK₂₈₀) is ligated into a pLitmus plasmid to produce a pCTK-Litmus plasmid. An expression cassette including a heterologous nucleic acid molecule (e.g., a *lac*Z gene or a nucleic acid molecule encoding an antigen isolated from a pathogenic organism) ligated to a CMV immediate early promoter and a BHV polyadenylation site in such a manner that the heterologous nucleic acid molecule is expressed in a eukaryotic cell is inserted into the CTK nucleic acid molecule within pCTK-Litmus such that there are CTK flanking sequences on either side of the expression cassette (e.g., into a restriction site internal to nCTK₂₈₀).

A recombinant CHV is produced by co-transfecting the recombinant vector and CHV DNA into canine MDCK cells using previously described methods; see, for example, Graham et al., *ibid*. Recombinant TK negative CHV are selected for by passage in bromodeoxyuridine; see, for example, Kit et al., 1983, *Virology 130,* 381-389. If the heterologous nucleic acid molecule is the *lac*Z gene, such recombinant CHV can also be selected as described in Example 6.

### Example 8

This Example discloses the production of another recombinant CHV genome and recombinant CHV of the present invention.

A recombinant, or transfer, vector to be used in the production of a recombinant CHV having a heterologous nucleic acid molecule in a dUTPase gene of the CHV genome is constructed as follows. A CHV dUTPase nucleic acid molecule of the present invention (e.g., nCdUTP₄₅₉) is ligated into a pLitmus plasmid to produce a pCdUTP-Litmus plasmid. An expression cassette including a heterologous nucleic acid molecule (e.g., a *lac*Z gene or a nucleic acid molecule encoding an antigen isolated from a pathogenic organism) ligated to a CMV immediate early promoter and a BHV polyadenylation site in such a manner that the heterologous nucleic acid molecule is expressed in a eukaryotic cell is inserted into the CdUTP nucleic acid molecule within pCdUTP-Litmus such that there are CdUTPase flanking sequences on either side of the expression cassette (e.g., into a restriction site internal to nCdUTP₄₅₉).

A recombinant CHV is produced by co-transfecting the recombinant vector and CHV DNA into canine MDCK cells as described in Example 7. Recombinant dUTPase negative CHV are selected for by passage in mercurithio analogs of deoxyuridine; see, for example, Holliday et al., 1991, *Antiviral Research 16,* 197-203. If the heterologous nucleic acid molecule is the *lacZ* gene, such recombinant CHV can also be selected as described in Example 6. Recombinant virus carrying the foreign DNA of interest can also be selected by either by plaque hybridizations, or by dot-blot hybridizations of infected cell cultures. Verification of the proper insert within the CHV genome is conducted by Southern hybridization analysis.

### Example 9

This Example discloses the production of a recombinant CHV by co-transfection of a set of overlapping cosmid clones comprising the entire viral CHV genome.

A library of CHV cosmid clones is created in the cosmid vector SuperCos (available from Stratagene Cloning Systems, La Jolla, CA) according to manufacturer's specifications, except that the vector is modified to contain one or more restriction sites not present in CHV genomic DNA (e.g., *Sse*83871, I-*Sce*-I, or *Not*I) so that the cosmid inserts can be excised prior to cotransfection. A heterologous nucleic acid molecule in an expression cassette, such as one of those described in Examples 6-8, is inserted into a cosmid clone using standard procedures. A recombinant CHV is produced by co-transfection of a set of overlapping cosmid clones comprising the entire viral CHV genome, including the cosmid comprising a heterologous nucleic acid molecule, using techniques as described in van Zihl et al., 1988, *J. Virol. 62*, 2191-2195.

### Example 10

This Example describes the nucleic acid sequencing of additional nucleic acid molecules of the present invention.

Nucleic acid molecules nCAsc₉₃₀₀ and nCAsc₁₀₀₀₀, produced as described in Example 4, were submitted to additional DNA sequence analysis. The resultant nucleic acid sequences were compiled to produce SEQ ID NO:51 and SEQ ID NO:52. SEQ ID NO:51 and SEQ ID NO:52 represent the deduced nucleic acid sequences of the two complementary strands of a nucleic acid molecule referred to herein as nCUS₁₀₅₉₂. Nucleic acid molecule nCUs₁₀₅₉₂ includes the entire US region of CHV plus terminal repeat and internal repeat sequences. Analysis of SEQ ID NO:51 indicates that the US region spans from nucleotides about 2047 through about 9724 of SEQ ID NO:51, whereas the terminal repeat sequences and inverted repeat sequences span from nucleotides about 1 through about 2046 and from nucleotides about 9725 through about 10592, respectively, of SEQ ID NO:51.

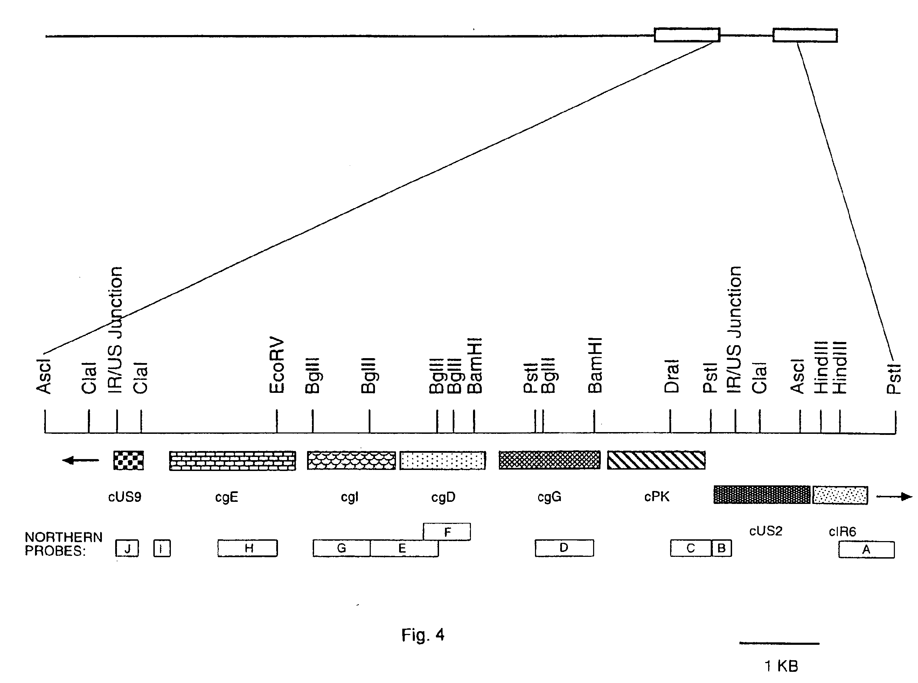

Translation of SEQ ID NO:51 and SEQ ID NO:52 indicates that nucleic acid molecule nCUS₁₀₅₉₂ contains at least the following open reading frames: CIR6, CUS2, CPK, CgG, CgD, CgI, CgE, CUS8.5 and CUS9. The relative locations of these open reading frames is shown in Fig. 4.

Specifically, SEQ ID NO:52 includes: the coding strand of nCIR6₅₅₂ (having SEQ ID NO:3) which encodes a CIR6 protein of about 183 amino acids, assuming a start codon spanning about nucleotides 9672-9674 and a stop codon spanning about nucleotides 10221-10223 of SEQ ID NO:52; and the coding strand of nCUS2₁₁₇₆ (having SEQ ID NO:5) which encodes a CUS2 protein of about 391 amino acids, assuming a start codon spanning about nucleotides 8338-8340 and a stop codon spanning about nucleotides 9511-9513 of SEQ ID NO:52. Nucleic acid molecules nCIR6₅₅₂ and nCUS2₁₁₇₆ are also described in Example 4 in relation to nCUS₅₄₉₅, as are the corresponding nucleic acid sequences and amino acid sequences they encode.

SEQ ID NO:51 includes: the coding strand of nCPK₁₂₀₃ (having SEQ ID NO:7) which encodes a CPK protein of about 400 amino acids, assuming a start codon spanning about nucleotides 2375-2377 and a stop codon spanning about nucleotides 3575-3577 of SEQ ID NO:51; the coding strand of nCgG₁₂₄₈ (having SEQ ID NO:9) which encodes a CgG protein of about 415 amino acids, assuming a start codon spanning about nucleotides 3689-3691 and a stop codon spanning about nucleotides 4934-4936 of SEQ ID NO:51; the coding strand of nCgD₁₀₃₈ (having SEQ ID NO:53, and including SEQ ID NO:11) which encodes a CgD protein of about 345 amino acids, assuming a start codon spanning about nucleotides 5128-5130 and a stop codon spanning about nucleotides 6163-6165 of SEQ ID NO:51; the coding strand of ncgI₁₀₉₅ (having SEQ ID NO:55, and including SEQ ID NO:29) which encodes a CgI protein of about 364 amino acids, assuming a start codon spanning about nucleotides 6225-6227 and a stop codon spanning about nucleotides 7317-7319 of SEQ ID NO:51; the coding strand of nCgE₁₅₆₉ (having SEQ ID NO:57) which encodes a CgE protein of about 522 amino acids, assuming a start codon spanning about nucleotides 7467-7469 and a stop codon spanning about nucleotides 9033-9035 of SEQ ID NO:51; the coding strand of nCUS8.5₂₃₇ (having SEQ ID NO:59) which encodes a CUS8.5 protein of about 78 amino acids, assuming a start codon spanning about nucleotides 9028-9030 and a stop codon spanning about nucleotides 9262-9264 of SEQ ID NO:51; and the coding strand of nCUS9₃₆₀ (having SEQ ID NO:61) which encodes a CUS9 protein of about 119 amino acids, assuming a start codon spanning about nucleotides 9376-9378 and a stop codon spanning about nucleotides 9733-9735 of SEQ ID NO:51. SEQ ID NO:61 differs from the coding region reported for CUS9 in Example 4 (e.g., SEQ ID NO:20), in that additional sequence analysis indicated that SEQ ID NO:17 included a sequencing error resulting in a frameshift, leading to a longer deduced open reading frame. Nucleic acid molecules nCPK₁₂₀₃ and nCgG₁₂₄₈ are also described in Example 4 in relation to nCUS₅₄₉₅, as are the corresponding nucleic acid sequences and amino acid sequences they encode. The amino acid sequences of the respective encoded proteins PCgD₃₄₅, PCgI₃₆₄, PCgE₅₂₂, PCUS8.5₇₈, and PCUS9₁₁₉ are represented by SEQ ID NO:54, SEQ ID NO:56, SEQ ID NO:58, SEQ ID NO:60 and SEQ ID NO:62, respectively.

### Example 11

This Example describes the nucleic acid sequencing of additional nucleic acid molecules of the present invention.

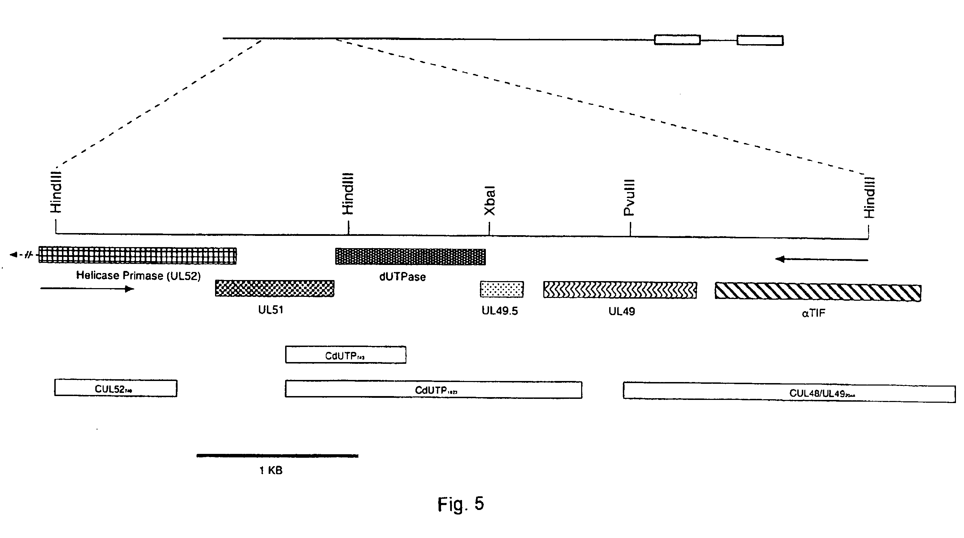

Nucleic acid molecules nCHin₃₀₀₀, and nCHin₁₉₀₀, produced as described in Example 3, were submitted to additional DNA sequence analysis. Also submitted to additional nucleic acid sequence analysis was nucleic acid molecule nCHin₈₅₀₀, a *Hin*dIII fragment shown to include the 3' end of CUL48 as well as CgC and CUL45. The resultant nucleic acid sequences were compiled to produce SEQ ID NO:63, SEQ ID NO:64, SEQ ID NO:77, SEQ ID NO:78, SEQ ID NO:85, and SEQ ID NO:86. SEQ ID NO:63 and SEQ ID NO:63 represent the deduced nucleic acid sequences of the two complementary strands of a nucleic acid molecule referred to herein as nCUL49/CUL48₂₀₄₄. Translation of SEQ ID NO:63 and SEQ ID NO:64 indicates that nucleic acid molecule nCUL49/CUL48₂₀₄₄ contains at least the following open reading frames: CUL48 and the 3' end of CUL49. SEQ ID NO:77 and SEQ ID NO:78 represent the deduced nucleic acid sequences of the two complementary strands of a nucleic acid molecule referred to herein as nCUL₁₈₂₃. Translation of SEQ ID NO:77 and SEQ ID NO:78 indicates that nucleic acid molecule nCUL₁₈₂₃ contains at least the following open reading frames: CUL51, CdUTPase, CUL49.5, and the 5' end of CUL49. SEQ ID NO:85 and SEQ ID NO:86 represent the deduced nucleic acid sequences of the two complementary strands of a nucleic acid molecule referred to herein as nCUL52₇₄₉. Translation of SEQ ID NO:85 and SEQ ID NO:86 indicates that nucleic acid molecule nCUL52₇₄₉ contains a partial open reading frame for CUL52, the 3' end of which is included in SEQ ID NO:28. A portion of SEQ ID NO:86, from about nucleotide 539 through about nucleotide 749, was found to be identical to GenBank Accession No. X90456, as disclosed by Remond, et al., 1996, *ibid*. The relative location of each of these open reading frames is shown in Fig. 5.

Specifically, SEQ ID NO:63 includes: the coding strand of nCUL49₄₂₀ (having SEQ ID NO:65) which encodes a non-full length CUL49 protein of about 139 amino acids, assuming a stop codon spanning about nucleotides 419-421 of SEQ ID NO:63; and the coding strand of nCUL48₁₂₆₉ (having SEQ ID NO:67, and including SEQ ID NO:24) which encodes an apparently full-length CUL48 protein of about 422 amino acids, assuming a start codon spanning about nucleotides 541-543 and a stop codon spanning about nucleotides 1807-1809 of SEQ ID NO:63. A portion of SEQ ID NO:63, from about nucleotide 1252 through about nucleotide 1647, was found to be about 98% identical to GenBank Accession No. X89373, as disclosed by Remond, et al., 1996, *ibid*. Although this published sequence is contained within the coding region for the CUL48 protein, it does not disclose a full-length coding region. The amino acid sequences of the respective encoded proteins PCUL49₁₃₉ and PCUL48₄₂₂ are represented by SEQ ID NO:66 and SEQ ID NO:68, respectively.

SEQ ID NO:77 includes the coding strand of nCdUTP₉₁₈ (having SEQ ID NO:79 and including SEQ ID NO:15) which encodes a CdUTPase protein of about 305 amino acids, assuming a start codon spanning about nucleotides 624-626 and a stop codon spanning about nucleotides 1539-1541 of SEQ ID NO:77. The amino acid sequence of the respective encoded protein PCdUTP₃₀₅ is represented by SEQ ID NO:80.

SEQ ID NO:78 includes: the coding strand of nCUL51₂₆₁ (having SEQ ID NO:33) which encodes a non-full length CUL51 protein of about 86 amino acids, assuming translation begins at about nucleotide 1 and a stop codon spanning about nucleotides 259-261 of SEQ ID NO:78; the coding strand of nCUL49.5₂₆₁ (having SEQ ID NO:81) which encodes a CUL49.5 protein of about 86 amino acids, assuming a start codon spanning about nucleotides 1175-1177 and a stop codon spanning about nucleotides 1433-1435 of SEQ ID NO:78; and the coding strand of nCUL49₂₅₅ (having SEQ ID NO:83) which encodes a non-full length CUL49 protein of about 85 amino acids, assuming a start codon spanning about nucleotides 1586-1588 of SEQ ID NO:78. The amino acid sequence of the respective encoded proteins PCUL51₈₆, PCUL49.5₈₆, and PCUL49₈₅ are represented by SEQ ID NO:34, SEQ ID NO:82 and SEQ ID NO:84, respectively.

SEQ ID NO:85 includes the coding strand of nCUL52₇₄₇ (having SEQ ID NO:87) which encodes a non-full length CUL52 protein of about 249 amino acids, assuming translation begins at about nucleotide 1 of SEQ ID NO:85. The amino acid sequence of the respective encoded protein PCUL52₂₄₉ is represented by SEQ ID NO:88.

### Example 12

This Example discloses the production of another recombinant CHV genome and recombinant CHV of the present invention.

A 3.2 kb fragment containing the entire CdUTPase sequence was amplified from CHV genomic DNA using forward primer RSF009 having nucleic acid sequence 5' GCCGGTACCAGGCTTTGGACGAGATTTAGG 3', denoted SEQ ID NO:89, and reverse primer RSF008 having nucleic acid sequence 5' GCCGAATTCAATATAATTAATAAACTCTC3', denoted SEQ ID NO:90. RSF009 has an Asp718 site attached to the 5' end of CHV homologous sequences, and RSF008 has an *Eco*RI site attached to the 5' end of CHV homologous sequences. The resultant PCR-amplified fragment of about 3.2 kb, referred to herein as nCdUTP₃₂₀₀, was digested with *Asp*718 and *Eco*RI and ligated into plitmus28 (available from New England Biolabs). The resultant recombinant plasmid, denoted herein as p28CdUTP₃₂₀₀, was verified by end-sequencing and restriction mapping. Plasmid p28CdUTP₃₂₀₀ was then digested with *Hin*dIII and *Xba*I, releasing an 858 base-pair fragment, referred to herein as nCdUTP₈₅₈, containing all of the dUTPase open reading frame except 108 bp at the 3' end. The *Xba*I site was found to be 49 nucleotides upstream of the start codon of the dUTPase ORF (open reading frame), and is also 70 nucleotides into the UL49.5 ORF on the opposite strand, which overlaps the dUTPase ORF. Previous studies have shown that the UL49.5 ORF in other herpesviruses encodes a membrane protein, and is nonessential for growth in tissue culture (e.g., Liang et al., *ibid*.).

The cohesive ends on the remaining 2.3 kb fragment of p28CdUTP₃₂₀₀, minus the 858 bp *Hind*III/*Xba*I fragment, were filled in using Klenow fragment and dNTPs according to standard methods. The resulting blunt-ended fragment was gel purified by standard methods. A heterologous nucleic acid sequence operatively linked to transcription control regions, in this case an AscCMV/lacZ/BGH cassette described in Example 6, was isolated from its plasmid by digestion with *Asc*I, and the cohesive ends were filled in by Klenow fragment and dNTPs. This cassette was ligated to the *Hind*III and *Xba*I-digested p28CdUTP₃₂₀₀ fragment described above by standard methods resulting in a plasmid containing flanking regions to the CHV dUTPase gene and with the *lac*Z gene, operatively linked to transcription control regions, inserted into a deletion of the dUTPase gene, herein denoted as pdUTP/*lac*Z.

A recombinant CHV is produced by co-transfecting the plasmid with the deleted dUTPase gene and the inserted heterologous nucleic acid molecule, in this case pdUTP/*lac*Z, and CHV DNA into canine cells using previously described methods; see, for example, Graham et al., *ibid*.). Alternatively, a recombinant CHV is produced by transfecting canine cells with the aforementioned plasmid as described, and then infecting the cells with CHV. Recombinant dUTPase-negative CHV are selected for by passage in mercurithio analogs of deoxyuridine as described in Example 8. If the heterologous nucleic acid molecule is the *lac*Z gene, such a recombinant CHV can be selected as described in Example 6.

### Example 13

This example discloses a method for obtaining higher plaque forming efficiencies to facilitate the production of recombinant CHV genomes and recombinant CHV of the present invention. The method involves the expression of the CHV alpha-tif gene in the presence of CHV genomes introduced into canine cells during CHV production.

The alpha-tif gene of CHV (denoted herein as nCUL48₁₂₆₉) was identified to lie on two CHV clones described herein: namely, nCHin₃₀₀₀ and nCHin_{8500.} Nucleotide sequencing from the ends of these two fragments using universal primers and internal primers designed based on previous sequences was used to determine the entire sequence. A single fragment including the complete alpha-tif gene was PCR amplified from CHV viral DNA using forward primer RSF014 having nucleic acid sequence 5' CCGGAATTCGCTTAGTGAGAGTATAAAC 3', denoted SEQ ID NO 91, and reverse primer RSF016 having nucleic acid sequence 5' CCGGAATTCCCTCATATTATATACTAAC 3', denoted SEQ ID NO:92. Both primers have *Eco*RI sites attached to the 5' end of the CHV homologous sequences. The resultant PCR amplified fragment of about 1351 base pairs was digested with EcoRI and ligated into *Eco*RI-digested pCDNA3 (available from Invitrogen, described in Example 6). This plasmid contains a neomycin-resistance gene for selection of stably transformed cell lines by techniques well known to those skilled in the art. Resulting plasmids were checked for the proper orientation by digestion with *Hin*dIII. A plasmid consisting of pcDNA3 with the alpha-tif gene inserted in the proper orientation for expression was found, and is designated herein as pATIF₁₃₅₁. Expression of CHV alpha-tif from pATIF₁₃₅₁ was verified by cell-free transcription/translation using T7 RNA polymerase and a rabbit reticulocyte lysate system. These techniques are readily apparent to those skilled in the art, and are available in kit form, for example, from Amersham. Improved CHV DNA infectivity is then achieved by cotransfection of pATIF₁₃₅₁ with from about 1 to about 5 µg of CHV DNA by methods such as described in Examples 6-9 and Example 12.

Alternatively, cell lines that stably express CHV alpha-tif are derived. Three canine cell lines, A72, D17 and CF2Th (all available from ATCC) were transfected with pATIF₁₃₅₁ by standard methods, see, for example, Graham et al., *ibid*. The cells are then subjected to selection using varying amounts of the antibiotic G418 (available from BMB), determined empirically for each cell line. Cloned G418-resistant cell lines are then derived by standard dilution methods. Cell lines stably expressing CHV alpha-tif are then used for standard CHV DNA transfections or co-transfections such as those described in Examples 6-9 and Example 12.

### Example 14

This Example describes the identification and sequencing of additional CHV nucleic acid molecules of the present invention.

CHV nucleic acid molecule nCHin₅₅₀₀, a CHV *Hin*dIII fragment derived as described in Example 2, was submitted to nucleic acid sequence analysis. Sequences derived from the sequence analysis include SEQ ID NO:69, SEQ ID NO:70, SEQ ID NO:73 and SEQ ID NO:74. Translation of these sequences indicated that nCHin₅₅₀₀ includes at least a portion of the CgL and CICP4 coding regions. The nucleic acid molecule also apparently contains the CICP0 coding region.

SEQ ID NO:69 and SEQ ID NO:70 represent the deduced nucleic acid sequences of the two complementary strands of a CHV ICP4 nucleic acid molecule referred to herein as nCICP4₆₂₆. Translation of SEQ ID NO:69 indicates that nCICP4₆₂₆ encodes a non-full length CICP4 protein of about 208 amino acids, denoted herein as PCICP4₂₀₈, having an amino acid sequence represented by SEQ ID NO:72. The coding region for PCICP4₂₀₈ is denoted herein as nCICP4₆₂₄, having nucleic acid sequence SEQ D NO:71. A portion of SEQ ID NO:69, from about nucleotide 1 through about nucleotide 307, was found to be identical to GenBank Accession No. X90422, as disclosed by Remond, et al., 1996, *ibid*.

SEQ ID NO:73 and SEQ ID NO:74 represent the deduced nucleic acid sequences of the two complementary strands of a CHV gL nucleic acid molecule referred to herein as nCgL₆₅₅. Translation of SEQ ID NO:74 indicates that nCgL₆₅₅ encodes a non-full length CgL protein of about 171 amino acids, denoted herein as PCgL₁₇₁, having an amino acid sequence represented by SEQ ID NO:76. The coding region for PCgL₁₇₁ is denoted herein as nCgL₅₁₆, having nucleic acid sequence SEQ D NO:75, assuming that the open reading frame spans about nucleotides 1 through 516 of SEQ ID NO:74, with a stop codon spanning about nucleotides 514-516.

### Example 15

This example describes the mapping of messenger RNA molecules transcribed from nucleic acid molecules of the present invention. These transcripts are useful for verifying expression (or lack of expression) of various genes.

Total RNA was prepared from CHV-infected cells using a phenol/guanidine extraction method familiar to those skilled in the art. See, for example, Chomczynski et al., 1987, *Anal. Biochem. 162,* 156-159. Multiple samples of RNA were separated on formaldehyde/agarose gels and transferred to nitrocellulose by standard methods. Based on the sequences of nCUS₁₀₅₉₂, designated SEQ ID NO:51 and SEQ ID NO:52, nucleic acid fragments were isolated for use as probes, such that they would hybridize to sequences homologous to the open reading frames nCPK₁₂₀₃ (SEQ ID NO:7), nCgG₁₂₄₈ (SEQ ID NO:9), nCgD₁₀₃₈ (SEQ ID NO:53), nCgI₁₀₉₅ (SEQ ID NO:55), nCgE₁₅₆₉ (SEQ ID NO:57), nCUS8.5237 (SEQ ID NO:59), nCUS9₃₆₀ (SEQ ID NO:61), nCIR6₅₅₂ (SEQ ID NO:3), and nCUS2₁₁₇₆ (SEQ ID NO:5). The probes were as follows: to hybridize with an nCIR6₅₅₂ (SEQ ID NO:3) transcript, a *Pst*I-*Hin*dIII restriction fragment extending from about nucleotide 1 to about nucleotide 685 in nCUS₁₀₅₉₂ herein denoted as probe A; to hybridize with an nCUS2₁₁₇₆ (SEQ ID NO:5) transcript, a PCR -amplified fragment extending from about nucleotide 2056 to about nucleotide 2273 in nCUS₁₀₅₉₂ herein denoted as probe B; to hybridize with an nCPK₁₂₀₃ (SEQ ID NO:7) transcript, a *Pst*I-*Dra*I restriction fragment extending from about nucleotide 2326 to about nucleotide 2788 in nCUS₁₀₅₉₂ herein denoted as probe C; to hybridize with an nCgG₁₂₄₈ (SEQ ID NO:9) transcript, a *Bam*HI-*Pst*I restriction fragment extending from about nucleotide 3766 to about nucleotide 4494 in nCUS₁₀₅₉₂ herein denoted as probe D; to hybridize with an nCgD₁₀₃₈ (SEQ ID NO:53) transcript, either a *Bgl*II-*Bgl*II restriction fragment extending from about nucleotide 5728 to about nucleotide 6561 in nCUS₁₀₅₉₂ (this piece slightly overlaps ncgI₁₀₉₅) herein denoted as probe E, or a PCR-amplified fragment that will extend from about nucleotide 5295 to about nucleotide 5885 in nCUS₁₀₅₉₂ herein denoted as probe F; to hybridize with an nCgI₁₀₉₅ (SEQ ID NO:55) transcript, a *Bgl*II-*Bgl*II restriction fragment extending from about nucleotide 6561 to about nucleotide 7263 in nCUS₁₀₅₉₂ herein denoted as probe G; to hybridize with an nCgE₁₅₆₉ (SEQ ID NO:57) transcript, a deletion subclone extending from about nucleotide 7667 to about nucleotide 8425 in nCUS₁₀₅₉₂ herein denoted as probe H; to hybridize with a putative ncus85237 (SEQ ID NO:59) transcript, a PCR-amplified fragment extending from about nucleotide 9023 to about nucleotide 9242 in nCUS₁₀₅₉₂ herein denoted as probe I; to hybridize to an nCUS9₃₆₀ (SEQ ID NO:61) transcript, a PCR-amplified fragment extending from about nucleotide 9447 to about nucleotide 9715 in nCUS₁₀₅₉₂ herein denoted as probe J. These probes are shown graphically in Fig 4. The probes were labelled with ³²P dATP using random priming, a technique well known to those skilled in the art. Hybridization of the probes to the RNA samples on nitrocellulose and washing at stringent temperatures were done according to well known methods.

The main transcript sizes that hybridized to the various probes were as follows: probe A hybridized strongly to a transcript of about 0.7 kb, but also hybridized weakly to transcripts of about 1.7, 2.2 and 2.6 kb; probe B hybridized with a transcript of about 2.2 kb; probe C hybridized to a transcript of about 2.6 kb; probe D hybridized to two transcripts of about 1.9 kb and 2.6 kb; probe E hybridized to two transcripts of about 4.2 kb and 3.2 kb; probe G hybridized to two transcripts of about 4.2 kb and 3.2 kb; probe H hybridized to three transcripts of about 1.9 kb, 3.2 kb, and 4.2 kb; probe I hybridized to the same three transcripts as probe H; probe J hybridized to a transcript of about 2.1 kb. These data suggest that the transcripts of US2, US9, and IR6, although starting at different points, terminated in a common general area in the inverted repeats, since probe A hybridized to transcripts that were generally the same size as the US2 and US9 transcripts. Furthermore, these data suggest that the PK and gG transcripts overlapped, probably terminating at a common 3' terminus, since probe C hybridized to only a 2.6 kb transcript but probe D hybridized with both the 2.6 kb transcript and the 1.4 kb transcript. This result was even more likely because only one polyadenylation consensus sequence (AATAAA) was found downstream of the gG ORF, but before the gD ORF. This polyadenylation signal was found from nucleotide 4935-4940 of nCUS₁₀₅₉₂. Similarly, these data indicate the gI and gE transcripts overlapped, probably terminating at a common 3' terminus since probe G hybridized to a transcript of 2.6 kb, while probe H hybridized both to the 2.6 kb transcript and the 1.9 kb transcript. Probes E, G, and H also all hybridized to a 4.2 kb transcript, suggesting that the gD transcript also overlapped the gI and gE transcripts, probably terminating at a common 3' terminus. Verification of this result is if probe F hybridizes only to the 4.2 kb transcript. Probe I hybridized to only the 4.2, 2.6 and 2.9 kb transcripts suggesting two things; first, that these three transcripts probably terminated at polyadenylation signals at about 9186-9181, 9256-9261 or 9260-9265 of nCUS₁₀₅₉₂, also that the putative US8.5 open reading frame is not transcribed to levels detectable by Northern analysis.

### Example 16

This example discloses the production of recombinant canine cell lines that stably express the CHV alpha-trans-inducing factor (α-TIF), and demonstrates that such recombinant cell lines have greatly improved plaque forming efficiencies upon transfection with CHV DNA.

Six recombinant canine cell lines that stably express the CHV α-TIF gene were produced as follows. D17 canine tumor cells (available from ATCC) were plated at 4 X 10⁶ cells per 100 mm petri dish. The cells were transfected with 2 µg of pATIF₁₃₅₁ plasmid DNA, produced as described in Example 13, using the transfection reagent Lipofectamine^{™} (available from Life Technologies, Inc., (LTI) Gaithersburg, MD) according to the manufacturer's instructions. Twenty-four hours following the transfection, the antibiotic G418 (Geneticin^{™}, available from BMB) was added to the media at a concentration of 350 µg/ml, to select for cells expressing the neomycin resistance gene contained on plasmid pATIF₁₃₅₁. Forty-eight hours later, the cells were diluted to 10 cells per well in a 96-well tissue culture plate. Thirteen G418-resistant cell colonies were cloned out and expanded under antibiotic selection by standard methods well-known to those skilled in the art.

Six of these cloned cell lines, denoted herein as D17:pATIF₁₃₅₁-A through D17:pATIF₁₃₅₁-F, were tested for their ability to support improved plaque-forming efficiency upon transfection with CHV DNA, as follows. The six cell lines, as well as wild-type D17 canine tumor cells, were each plated, in duplicate, at 4 x 10⁶ cells per 100mm petri dish just prior to transfection. Each cell line was transfected with 1 µg of CHV DNA using the CaPO₄ method of Graham, et al., ibid. Six hours post-transfection, the cells were treated for one minute with 20% glycerol in PBS as a hypertonic shock, washed with PBS, and grown at 35°C in DMEM containing G418 at 350 µg/ml. Seventy-two hours post-transfection the medium was removed and replaced with medium containing 0.7% agarose, the total number of virus plaques from each transfection were counted, and the duplicate transfections for each cell line were averaged. The results of these transfections, shown in Table 1, indicate that at least four of the recombinant canine cell lines, D17:pATIF₁₃₅₁-B, D17:pATIF₁₃₅₁-C, D17:pATIF₁₃₅₁-D, and D17:pATIF₁₃₅₁-F, promoted significantly higher plaque forming efficiency upon transfection with CHV DNA than did wild-type D17 cells.

**TABLE 1**

| Transfection Efficiencies of Recombinant Cell Lines | |
|---|---|
| **Cell Line** | **Average Number of Plaques (PFU)** |
| D17:pATIF₁₃₅₁-A | 5 |
| D17:pATIF₁₃₅₁-B | 29 |
| D17:pATIF₁₃₅₁-C | 30 |
| D17:pATIF₁₃₅₁-D | 166 |
| D17:pATIF₁₃₅₁-E | 0 |
| D17:pATIF₁₃₅₁-F | 275 |
| Wild-Type D17 | 0 |

### Example 17

This example discloses the production of another recombinant CHV genome and recombinant CHV of the present invention.

A specific deletion in a plasmid containing the CdUTPase open reading frame was produced as follows. Plasmid p28CdUTP₃₂₀₀, produced as described in Example 12, was digested with *Hind*III and *Xmn*I, releasing a 727-bp fragment, referred to herein as nCdUTP₇₂₇. This fragment includes most of the CdUTPase ORF, extending from about nucleotide 84 to about nucleotide 810 of SEQ ID NO:79. Release of this *Xmn*I/*Hind*III fragment does not delete any of the CUL49.5 ORF contained on the opposite strand of p28CdUTP₃₂₀₀, as did *Xba*I/*Hind*III digestion as disclosed in Example 12, but *Xmn*I digestion could delete a portion of the CUL49.5 transcription control region, since the restriction site is only about 58 nucleotides upstream of the start codon for the CUL49.5 ORF, i.e., at about nucleotide 1117 of SEQ ID NO:78. Following *Xmn*I/*Hind*III digestion of p28CdUTP₃₂₀₀, the cohesive *Hind*III end on the about 4.9-kb fragment (the remaining CHV insert of about 2.5 kb, plus the 2.4-kb pLitmus28 vector) was filled in using Klenow enzyme and dNTPs according to standard methods.

A recombinant transfer vector containing a reporter gene encoding green fluorescent protein (GFP) operatively linked to transcription control regions, inserted into a deletion in the CHV dUTPase gene, was constructed as follows. The pGreen Lantern™-1 vector (available from LTI) was digested with *Not*I to release a 750-bp fragment containing the coding region of a genetically engineered GFP gene. This 750-bp fragment was cloned into plasmid vector pAscCMV/BGH, prepared as described in Example 6, which had been digested with *Not*I and dephosphorylated with calf intestinal alkaline phosphatase. The proper orientation of the insert was verified by a diagnostic restriction digest. The resulting recombinant plasmid, denoted herein as pAscCMV/GFP/BGH, contained a genetically engineered GFP gene operatively linked to the CMV immediate-early promoter and the BGH polyadenylation signal, such that the entire transcriptional unit can be excised from the plasmid by digestion with *Asc*I. Plasmid pAscCMV/GFP/BGH was digested with *Asc*I, releasing a 1.8-kB transcription unit. The cohesive ends of the fragment were filled in with Klenow enzyme and dNTPs and the fragment was gel purified by standard methods. The 1.8-kB transcription unit containing the GFP gene was then ligated into the 4.9-kb p28CdUTP₃₂₀₀ *Xmn*I/*Hin*dIII restriction fragment, produced as described in the previous paragraph, using T4 DNA ligase. Resulting recombinant plasmids were analyzed and verified by restriction digestion and end-sequencing. The resulting recombinant plasmid, containing flanking regions to the CHV dUTPase gene into which had been inserted a genetically-engineered GFP gene operatively linked to transcription control regions, is denoted herein as pdUTP/GFP.

A recombinant CHV genome and a recombinant dUTPase-negative CHV expressing green fluorescent protein was produced as follows. Recombinant cell line D17:pATIF_{1351[F]}, produced as described in Example 16, was plated at 4 x 10⁶ cells/100mm petri dish just prior to transfection. The cells were transfected with 0.5 µg of CHV DNA, produced as described in Example 2, and 0.5 µg of pdUTP/GFP DNA, produced as described in the preceding paragraph, by the CaPO₄ method of Graham et al., *ibid.* Six hours post-transfection, the cells were treated for one minute with 20% glycerol in PBS as a hypertonic shock, washed with PBS, and grown at 35°C. in DMEM containing G418 at 350 µg/ml. Seventy-two hours post-transfection the medium was removed and replaced with medium containing 0.7% agarose. Resulting virus plaques were screened for expression of GFP by fluorescent microscopy using a standard fluorescein filter on an inverted fluorescent microscope, for example an Olympus 1X70 microscope, available from Olympus Optical Company, Japan. Out of forty-two plaques screened, four were positive for expression of GFP, indicating that the GFP gene contained on pdUTP/GFP had recombined into the dUTPase locus of the CHV genome. One of these recombinant CHV, denoted herein as CHVΔdUTP:GFP-1, is isolated and expanded for further analysis.

### SEQUENCE LISTING

The following Sequence Listing is submitted pursuant to 37 CFR §1.821. A copy in computer readable form is also submitted herewith.

Applicants assert pursuant to 37 CFR §1.821(f) that the content of the paper and computer readable copies of SEQ ID NO:1 through SEQ ID NO:92 submitted herewith are the same.
(1) GENERAL INFORMATION:
   (i) APPLICANT: Haanes, Elizabeth J. Frank, Rexann S.
   (ii) TITLE OF INVENTION: RECOMBINANT CANINE HERPESVIRUSES
   (iii) NUMBER OF SEQUENCES: 92
   (iv) CORRESPONDENCE ADDRESS:
      (A) ADDRESSEE: Sheridan Ross
      (B) STREET: 1700 Lincoln Street, Suite 3500
      (C) CITY: Denver
      (D) STATE: Colorado
      (E) COUNTRY: U.S.A.
      (F) ZIP: 80203
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Connell, Gary J.
      (B) REGISTRATION NUMBER: 32,020
      (C) REFERENCE/DOCKET NUMBER: 2618-46-PCT
   (ix) TELECOMMUNICATION INFORMATION:
      (A) TELEPHONE: (303) 863-9700
      (B) TELEFAX: (303) 863-0223
(2) INFORMATION FOR SEQ ID NO:1:
   (i) SEQUENCE CHARACTERISTICS:
      (A) LENGTH: 5495 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   CTGCAGTGTA TTTAAAAAAT AAAATCTATG AATGAAATCT ATGAATGAAA TCTATGAATG 60
   AAATCTATGA ATGAAATCTA TGAATGAAAT CTATGAATGA AATCTATGAA TGAAATCTAT 120
   GAATGAAATC TATGAATGAA ATCTATGAAT GAAATCTATG AATGAAATCT ATGAATGAAA 180
   TCTATGAATG AAATCTATGA ATGAAATCTA TGAATGAAAT CTATGAATGA AATCTATGAA 240
   TGAAATCTAT GAATGAAATC TATGAATGAA ATCTATGAAT GAAATCTATG AATGAAATCT 300
   ATGAGACAAG TATTTTAAAA ATATTTTAAA TTTATAAGGT TAAGTACAGT AGGCGGTTGG 360
   GTAAACATTT TTAGTTTTTC AAGTTTTTAG TTTTTCTGGT ATCCTACCCA ACACAAATGC 420
   ATCTTCGGAT ACATTTATTT TAAGAGAGTA ATCACTTTTT AGAATATATC TTATTGGTGG 480
   TACATTTATA AATTTTGGAC CATCCCAATA ACACTTCGAT TCCACAAGCG AAGAAGGTAC 540
   TTCCATAAGC TGAGAAGCGT TTACTTGATT GTAGGGAGAA CTTGGCGTTT CAAAATCCTT 600
   TAGAACGTAT AGTCTGCAAT ACATAGGTTC AATATCATCT TCATACCTCT CATCAGGATA 660
   TGAAAATGGA AGTTTCACAA AGGTTCCATC ACGAAGCTTT TTGAAGAATC GTACATCCGT 720
   AGGTGGTGTA GGAACAATAG TGAAGGCGTG CGGTTCACCC TGGTTTTTCA CACGTGCAAG 780
   CGCTGGTGTG GTTTTAGGGC GAACTGGAAA ATAACCAGGC GGAACTTGTG CGTAGAATCC 840
   TTCATTAAGT TCTCCACGAC AGCGTCTGAA GTATGATGGC ATATTAGCTT GATCGGAGTT 900
   GTTATCAAAT AGAGCAAATG AAGCACTCAT TTTAAAACTT TTTAGTTAAG CTTTAAAAAC 960
   AAGTGAAGAT TTAAAAATGT AGGATAAAAT GCCAGTTTAT ATACAGTAAG AATATGGGAG 1020
   TGGTTCACAT AAAAAACCAG AATTTCAGGT TTACATCTAC TGTTTATTCA CAACAAATAT 1080
   AAACAAACTT AGTTTCCACA TAAACATGAA CTAAATAGAG ATGAACGTTG AGCGTTGGTA 1140
   GGTTGTGTAG AAGACATACC ATCGTTTTCA TTTTTGGTTA TTGTTTTGGC GCGCCTTGAA 1200
   AATAATCGTT TAAAAATATT TGGTTTGGAT AGCCTTTTCA TAGGTTTCAC CCCATGCAAG 1260
   TCATCCTCTT CTGGTTCAGG AATTTCTTCA TAACCATTAT GGGATATTAT TGCACACATA 1320
   AATGATTCGA TTACCGGGGG GGCAGAACGT GTCTCATTTA TATAAAGAGA ATCACATACA 1380
   TCGCTTATAG AACATGTAGA ACTGTCAGAA TCCTCTTTAA AACTATTTTT AATTTCACAA 1440
   TTAGTTTCTT CTAGTTCATT ATCCACCATC GCATTAGCGT ATTTCCAAAT ATCATTCTCT 1500
   GAGGAATAAT GAGATGCAGA GCATGAAGAA GAGGATGAGG AGGAGGAGGA TGAAGATGAG 1560
   GATATAGAGG GACATCTTGG AGAGCTTTCA AAGTTGAATG GAGTATTAAA TGTTGTACCA 1620
   TAAAAAATGT CACTTAACAT AGGGGGTACT TTAAAGGAGG ACAGAAAGGT GTCTAATACA 1680
   GGTACCCATA TAAACGAGGG GCAATAAACA CTCCCAGAAT CATCGATATG TTTTACATTA 1740
   TTTTTGGAAA TCTCAAGACA CTCAGGTTTC CAGGATGGTT CCGGCCATTC ACATGATACA 1800
   TATGCATAAA TTAGTCGCTT TGGTCCTGGG ATATTAGAAA TGACTGGCTC ACATAAATCC 1860
   GCTGCACCGA AAACCCATAG ATTAAGAGGA TAGTTTCCAA ATATACCAGA GTTTAGATAG 1920
   TTATACCCCG AAACAGCCGA TTTCCATTCG ATGCTAGCCC CAGGTTTATC CTCATAAAAT 1980
   AAAAAGTCCT CCTCCTCCCC CTCCGTTGGT TTTAAAAATT TACTATTAGA GGTTGATGTT 2040
   CTTACTATAG GCCTTGAAAC TCTAGGTAGA TGTTTTATAG AGTCCATAAA ATAACATAAG 2100
   TTTGCAGATC GTAATATTAT AGGCATAGCC AATCGTGTGA GAGAAAGGAT ATAGCATTGT 2160
   CTAGCCATAA AACACCAAAG ATCAGGATGA ACATCTTGGG AGTTTCCTGG TAACGCCCCA 2220
   TTTTTGTCAA TAAACGTAAC AATATTAACT TCAACCACAC CCATAATTAA ATTTTATGTA 2280
   TGAATCCAAT AAAGGTTAAT ACACACCTAA TTTATGTTAT AATTTTAGAA GAAGCTGCAG 2340
   TTGATGAGTT GATATTAACA TAACAATTTC ACAATTACCT GATATGGCAA AGTGTACCAC 2400
   CGAAAAGTTT TGTTGTATCA GCGTGAATAG AGAATCTTCT GTCGATCCAG AAGACTTCTA 2460
   TAAACCGGTT CCTCTAACTT CAGATTTGAT TGAAGAGGAT AACCTACATC AAGACAAAAT 2520
   AATGGATGAG GATTTATACT CGGATTTTAG TGATGATGAC TTTATGGATT ATACAAAAAA 2580
   TCCAACTGAA AGTGAAAATG AAAGAGAAAG TGACGAAGAA GTTGAAGAAA GTTATGAAAG 2640
   TGATGAAGAT AAAAAAAGTT TATCTCCTAC TAAAAGCGAA GGAATTGAAG CGGCTGAAGC 2700
   GCTAAAGTTT TCTGTTGTTA AATCGTTAAC GCCTGGGTCA GAAGGAAGAG TTTTTATTGC 2760
   TCTTAAAAAA GATAAAGATA CAAGCTATAA GGTAATTTTA AAAATTGGAC AAAGGGGAAA 2820
   CACGCTTGTG GAATCGTTAA TTTTGAGAAA TATTAGTCAC CAATCTATAA TTAAACTTCA 2880
   AGACACTCTT TTTTATAAAG AGTTAACATG TTTGGTGTTA CCGTATTATA AATATGATCT 2940
   ATATAATTTT TTAATGGATC ATGGGAAATC TCTGTCTTTT GAATCTGTAA TTAAAATTGA 3000
   AAAACAAATA TTAACTGGAC TTCAATATAT TCATGGAAAA AAAATTATTC ATCGAGATAT 3060
   AAAAACTGAA AATATTTTCT TGGATAATGA CTCTAATGTT TGTATAGGTG ATTTTGGGGC 3120
   TTCTCAATTT CCTGTTTCCT CACCAGATTA TTTGGGAATT GCGGGGACTA TTGAAACTAA 3180
   TGCTCCTGAA GTTCTATCAA AGGATGCGTA CAACTGTAAA GCTGATATTT GGAGTGCTGG 3240
   TATAATTTTA TTTGAAATGC TTGCATATCC TAATGTTTTG TTTGAGGAGG AAGAAAGAGA 3300
   TAGTAGCGAT TTAATAAACA ATTGTAATCT TCATCTTATA AAAATTATAT CAACTCTGAA 3360
   GATTAACCCA AATGAATTTC CATCTGATTT GGAATCTAAT CTAGTAAAAC ATTTTATAAA 3420
   ATATGCTAAT AATGATAGAC CTCCATTTAC ACGATATAAT CGTCTAAATA ACCTTAAATT 3480
   ACATCTCGAT GGTGAATTTT TAATTCATAA AATGCTAACA TTTGATGCAT CTCTACGACC 3540
   AAGTGCGGAA GAACTATTAT CCTATCAGAT TTTTAGTAAA CAATAAATTT CATAAAAATG 3600
   GGCGTGGAAT TTTTTATTGT TTTATATAAA ACGGGTGTTT GAAAGCTCTT TTTTATTAAT 3660
   TTTATTTTTA CATCCTAGCT ACAATATTAT AGTTATCATG TTGTATACGC TGTTTTTTGT 3720
   TTTTTATTTT AAGGTAGTTT TATCTCGCAT AGCTCCGCTA GAGTTGTGTT ATGCGGATCC 3780
   TAAAGAAAAT ACAACTGAAC CTACACAACT TCCTACAGGG GAACAATCTA AGACTCTTAT 3840
   TCCCGTGGTA ACAAACGGAT ATGTTGAATA CTCTAAAGGA TGTGAACTAC GATTACTAGA 3900
   TACATATGTA AATGTATCTT CACGACCAGA AAAAAAGGTT AATGCTACAA TTGGATGGTC 3960
   ATTTGATCTT GGTTGTCAAA TTCCTTTAAT TTATAGAGAA TATTATAATT GTACTGGTAA 4020
   TATAATACCA TCACCAGAAA CTTGTGATGG TTATTCTTTA ACTTTGGTAA AATCTGAAAG 4080
   TATATCATCT TATGCACTTG TTAATGTTAG TTTGCTTATT CAACCAGGAA TTTTTGATTC 4140
   TGGTAGATAT TTATACTCAC TTGTTTTTGG AAACGATAGT TATAACGGAA GAATTGAAGT 4200
   TCGAGTGGAT AATGAGACAG ACTATCCATG TTTTATGATG CATGGATTGA CTGTAAAAAA 4260
   GGGTGATAAA CTTCATATTC CTTATAAACC ATCCACAAAT CCTAATCATA AACGATATAG 4320
   AGGTTGTTTT CCAATATCAA ATACTGAGCT ATGGAATAAT ATTAGTGATG AAAGTGTTGG 4380
   TAGATATTCA TATGATGAAG AATATGAAGA ATATGAAGAA GAAAACGAAG ATTTTGAAGA 4440
   TCTACAATCA AAAGATTGCC GCAAATCCAA TCTTTTTGAT ATGAAGAAGA CTTTTAATTT 4500
   GGCTGCAGGT TCTCAAAGTT TATTGATTGC TAGTTTGGGT AAATCAATTT CAGAACAACC 4560
   GTGGTCATTT AAAATTAATG AAAGTTATGA ACTTTTTAAT AATTTGTCTA TCACCCTTCA 4620
   ATCGGAAGAA GATTCTAATA TACTGAATCC TGAAATTGTA ACGTTTACCA CACCACCACC 4680
   TACTGAAAAT ACACATATGT TTATGTCAAA TAATGAAACT ATGTATGAAG AAGAAAGTGT 4740
   TTTAAGCATT ATTCAATTGT TTAACAATGG TTATAATAAT TGTAATACCC ATATAAAGGT 4800
   AATTGGATTT GGAACAATTA TCTTTATTAT TTTATTTTTT GTTGCTGTGT TTTTTTGTGG 4860
   ATATACTTGT GTATTAAACT CTCGTATTAA AATGATTAAC CATGCTTATA TACAACCCCA 4920
   GAAATTAAAT TTTTATGATA TTTAATAAAA CTATTATGAA ACTTCTTATA ACTTATTTGT 4980
   TTTTATTAAA TGGGTTGGGT TGGTTTTAAA ATTACATACG TGTATTAAGA ATTAACATCA 5040
   TAAAGGACAC ACCCATGAAA AACATTTAAA TTCTATTAAT TTGAACGGAT TAAACATTTT 5100
   CTCATTTTAA GAGTTGCTAC GACTTTTGAT AGTAAAATGA TTAAACTTCT ATTTATCTTA 5160
   TTTTATTTTA ACCCAATAAC TGGATATAAA TGGGTAGACC CTCCTCGTAG GTATAATTAC 5220
   ACCGTTTTAA GAATGATTCC AGATATTCCA AATCCAATGG ATCCTTCTAA AAACGCTGAA 5280
   GTTCGGTATG TAACTTCTAC TGACCCATGT GATATGGTTG CTTTGATTTC TAATCCAAAT 5340
   ATAGAATCTA CAATTAAAAC GATTCAATTT GTGCAAAAGA AAAAATTTTA CAATGCATCT 5400
   CTTAGTTGGT TTAAAGTTGG AGATGATTGT ACATATCCAA TATATTTAAT TCAATATTTT 5460
   GATTGTGATC CTCAAAGAGA ATTTGGCATA TGTTT 5495
(2) INFORMATION FOR SEQ ID NO:2:
   (i) SEQUENCE CHARACTERISTICS:
      (A) LENGTH: 5495 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   AAACATATGC CAAATTCTCT TTGAGGATCA CAATCAAAAT ATTGAATTAA ATATATTGGA 60
   TATGTACAAT CATCTCCAAC TTTAAACCAA CTAAGAGATG CATTGTAAAA TTTTTTCTTT 120
   TGCACAAATT GAATCGTTTT AATTGTAGAT TCTATATTTG GATTAGAAAT CAAAGCAACC 180
   ATATCACATG GGTCAGTAGA AGTTACATAC CGAACTTCAG CGTTTTTAGA AGGATCCATT 240
   GGATTTGGAA TATCTGGAAT CATTCTTAAA ACGGTGTAAT TATACCTACG AGGAGGGTCT 300
   ACCCATTTAT ATCCAGTTAT TGGGTTAAAA TAAAATAAGA TAAATAGAAG TTTAATCATT 360
   TTACTATCAA AAGTCGTAGC AACTCTTAAA ATGAGAAAAT GTTTAATCCG TTCAAATTAA 420
   TAGAATTTAA ATGTTTTTCA TGGGTGTGTC CTTTATGATG TTAATTCTTA ATACACGTAT 480
   GTAATTTTAA AACCAACCCA ACCCATTTAA TAAAAACAAA TAAGTTATAA GAAGTTTCAT 540
   AATAGTTTTA TTAAATATCA TAAAAATTTA ATTTCTGGGG TTGTATATAA GCATGGTTAA 600
   TCATTTTAAT ACGAGAGTTT AATACACAAG TATATCCACA AAAAAACACA GCAACAAAAA 660
   ATAAAATAAT AAAGATAATT GTTCCAAATC CAATTACCTT TATATGGGTA TTACAATTAT 720
   TATAACCATT GTTAAACAAT TGAATAATGC TTAAAACACT TTCTTCTTCA TACATAGTTT 780
   CATTATTTGA CATAAACATA TGTGTATTTT CAGTAGGTGG TGGTGTGGTA AACGTTACAA 840
   TTTCAGGATT CAGTATATTA GAATCTTCTT CCGATTGAAG GGTGATAGAC AAATTATTAA 900
   AAAGTTCATA ACTTTCATTA ATTTTAAATG ACCACGGTTG TTCTGAAATT GATTTACCCA 960
   AACTAGCAAT CAATAAACTT TGAGAACCTG CAGCCAAATT AAAAGTCTTC TTCATATCAA 1020
   AAAGATTGGA TTTGCGGCAA TCTTTTGATT GTAGATCTTC AAAATCTTCG TTTTCTTCTT 1080
   CATATTCTTC ATATTCTTCA TCATATGAAT ATCTACCAAC ACTTTCATCA CTAATATTAT 1140
   TCCATAGCTC AGTATTTGAT ATTGGAAAAC AACCTCTATA TCGTTTATGA TTAGGATTTG 1200
   TGGATGGTTT ATAAGGAATA TGAAGTTTAT CACCCTTTTT TACAGTCAAT CCATGCATCA 1260
   TAAAACATGG ATAGTCTGTC TCATTATCCA CTCGAACTTC AATTCTTCCG TTATAACTAT 1320
   CGTTTCCAAA AACAAGTGAG TATAAATATC TACCAGAATC AAAAATTCCT GGTTGAATAA 1380
   GCAAACTAAC ATTAACAAGT GCATAAGATG ATATACTTTC AGATTTTACC AAAGTTAAAG 1440
   AATAACCATC ACAAGTTTCT GGTGATGGTA TTATATTACC AGTACAATTA TAATATTCTC 1500
   TATAAATTAA AGGAATTTGA CAACCAAGAT CAAATGACCA TCCAATTGTA GCATTAACCT 1560
   TTTTTTCTGG TCGTGAAGAT ACATTTACAT ATGTATCTAG TAATCGTAGT TCACATCCTT 1620
   TAGAGTATTC AACATATCCG TTTGTTACCA CGGGAATAAG AGTCTTAGAT TGTTCCCCTG 1680
   TAGGAAGTTG TGTAGGTTCA GTTGTATTTT CTTTAGGATC CGCATAACAC AACTCTAGCG 1740
   GAGCTATGCG AGATAAAACT ACCTTAAAAT AAAAAACAAA AAACAGCGTA TACAACATGA 1800
   TAACTATAAT ATTGTAGCTA GGATGTAAAA ATAAAATTAA TAAAAAAGAG CTTTCAAACA 1860
   CCCGTTTTAT ATAAAACAAT AAAAAATTCC ACGCCCATTT TTATGAAATT TATTGTTTAC 1920
   TAAAAATCTG ATAGGATAAT AGTTCTTCCG CACTTGGTCG TAGAGATGCA TCAAATGTTA 1980
   GCATTTTATG AATTAAAAAT TCACCATCGA GATGTAATTT AAGGTTATTT AGACGATTAT 2040
   ATCGTGTAAA TGGAGGTCTA TCATTATTAG CATATTTTAT AAAATGTTTT ACTAGATTAG 2100
   ATTCCAAATC AGATGGAAAT TCATTTGGGT TAATCTTCAG AGTTGATATA ATTTTTATAA 2160
   GATGAAGATT ACAATTGTTT ATTAAATCGC TACTATCTCT TTCTTCCTCC TCAAACAAAA 2220
   CATTAGGATA TGCAAGCATT TCAAATAAAA TTATACCAGC ACTCCAAATA TCAGCTTTAC 2280
   AGTTGTACGC ATCCTTTGAT AGAACTTCAG GAGCATTAGT TTCAATAGTC CCCGCAATTC 2340
   CCAAATAATC TGGTGAGGAA ACAGGAAATT GAGAAGCCCC AAAATCACCT ATACAAACAT 2400
   TAGAGTCATT ATCCAAGAAA ATATTTTCAG TTTTTATATC TCGATGAATA ATTTTTTTTC 2460
   CATGAATATA TTGAAGTCCA GTTAATATTT GTTTTTCAAT TTTAATTACA GATTCAAAAG 2520
   ACAGAGATTT CCCATGATCC ATTAAAAAAT TATATAGATC ATATTTATAA TACGGTAACA 2580
   CCAAACATGT TAACTCTTTA TAAAAAAGAG TGTCTTGAAG TTTAATTATA GATTGGTGAC 2640
   TAATATTTCT CAAAATTAAC GATTCCACAA GCGTGTTTCC CCTTTGTCCA ATTTTTAAAA 2700
   TTACCTTATA GCTTGTATCT TTATCTTTTT TAAGAGCAAT AAAAACTCTT CCTTCTGACC 2760
   CAGGCGTTAA CGATTTAACA ACAGAAAACT TTAGCGCTTC AGCCGCTTCA ATTCCTTCGC 2820
   TTTTAGTAGG AGATAAACTT TTTTTATCTT CATCACTTTC ATAACTTTCT TCAACTTCTT 2880
   CGTCACTTTC TCTTTCATTT TCACTTTCAG TTGGATTTTT TGTATAATCC ATAAAGTCAT 2940
   CATCACTAAA ATCCGAGTAT AAATCCTCAT CCATTATTTT GTCTTGATGT AGGTTATCCT 3000
   CTTCAATCAA ATCTGAAGTT AGAGGAACCG GTTTATAGAA GTCTTCTGGA TCGACAGAAG 3060
   ATTCTCTATT CACGCTGATA CAACAAAACT TTTCGGTGGT ACACTTTGCC ATATCAGGTA 3120
   ATTGTGAAAT TGTTATGTTA ATATCAACTC ATCAACTGCA GCTTCTTCTA AAATTATAAC 3180
   ATAAATTAGG TGTGTATTAA CCTTTATTGG ATTCATACAT AAAATTTAAT TATGGGTGTG 3240
   GTTGAAGTTA ATATTGTTAC GTTTATTGAC AAAAATGGGG CGTTACCAGG AAACTCCCAA 3300
   GATGTTCATC CTGATCTTTG GTGTTTTATG GCTAGACAAT GCTATATCCT TTCTCTCACA 3360
   CGATTGGCTA TGCCTATAAT ATTACGATCT GCAAACTTAT GTTATTTTAT GGACTCTATA 3420
   AAACATCTAC CTAGAGTTTC AAGGCCTATA GTAAGAACAT CAACCTCTAA TAGTAAATTT 3480
   TTAAAACCAA CGGAGGGGGA GGAGGAGGAC TTTTTATTTT ATGAGGATAA ACCTGGGGCT 3540
   AGCATCGAAT GGAAATCGGC TGTTTCGGGG TATAACTATC TAAACTCTGG TATATTTGGA 3600
   AACTATCCTC TTAATCTATG GGTTTTCGGT GCAGCGGATT TATGTGAGCC AGTCATTTCT 3660
   AATATCCCAG GACCAAAGCG ACTAATTTAT GCATATGTAT CATGTGAATG GCCGGAACCA 3720
   TCCTGGAAAC CTGAGTGTCT TGAGATTTCC AAAAATAATG TAAAACATAT CGATGATTCT 3780
   GGGAGTGTTT ATTGCCCCTC GTTTATATGG GTACCTGTAT TAGACACCTT TCTGTCCTCC 3840
   TTTAAAGTAC CCCCTATGTT AAGTGACATT TTTTATGGTA CAACATTTAA TACTCCATTC 3900
   AACTTTGAAA GCTCTCCAAG ATGTCCCTCT ATATCCTCAT CTTCATCCTC CTCCTCCTCA 3960
   TCCTCTTCTT CATGCTCTGC ATCTCATTAT TCCTCAGAGA ATGATATTTG GAAATACGCT 4020
   AATGCGATGG TGGATAATGA ACTAGAAGAA ACTAATTGTG AAATTAAAAA TAGTTTTAAA 4080
   GAGGATTCTG ACAGTTCTAC ATGTTCTATA AGCGATGTAT GTGATTCTCT TTATATAAAT 4140
   GAGACACGTT CTGCCCCCCC GGTAATCGAA TCATTTATGT GTGCAATAAT ATCCCATAAT 4200
   GGTTATGAAG AAATTCCTGA ACCAGAAGAG GATGACTTGC ATGGGGTGAA ACCTATGAAA 4260
   AGGCTATCCA AACCAAATAT TTTTAAACGA TTATTTTCAA GGCGCGCCAA AACAATAACC 4320
   AAAAATGAAA ACGATGGTAT GTCTTCTACA CAACCTACCA ACGCTCAACG TTCATCTCTA 4380
   TTTAGTTCAT GTTTATGTGG AAACTAAGTT TGTTTATATT TGTTGTGAAT AAACAGTAGA 4440
   TGTAAACCTG AAATTCTGGT TTTTTATGTG AACCACTCCC ATATTCTTAC TGTATATAAA 4500
   CTGGCATTTT ATCCTACATT TTTAAATCTT CACTTGTTTT TAAAGCTTAA CTAAAAAGTT 4560
   TTAAAATGAG TGCTTCATTT GCTCTATTTG ATAACAACTC CGATCAAGCT AATATGCCAT 4620
   CATACTTCAG ACGCTGTCGT GGAGAACTTA ATGAAGGATT CTACGCACAA GTTCCGCCTG 4680
   GTTATTTTCC AGTTCGCCCT AAAACCACAC CAGCGCTTGC ACGTGTGAAA AACCAGGGTG 4740
   AACCGCACGC CTTCACTATT GTTCCTACAC CACCTACGGA TGTACGATTC TTCAAAAAGC 4800
   TTCGTGATGG AACCTTTGTG AAACTTCCAT TTTCATATCC TGATGAGAGG TATGAAGATG 4860
   ATATTGAACC TATGTATTGC AGACTATACG TTCTAAAGGA TTTTGAAACG CCAAGTTCTC 4920
   CCTACAATCA AGTAAACGCT TCTCAGCTTA TGGAAGTACC TTCTTCGCTT GTGGAATCGA 4980
   AGTGTTATTG GGATGGTCCA AAATTTATAA ATGTACCACC AATAAGATAT ATTCTAAAAA 5040
   GTGATTACTC TCTTAAAATA AATGTATCCG AAGATGCATT TGTGTTGGGT AGGATACCAG 5100
   AAAAACTAAA AACTTGAAAA ACTAAAAATG TTTACCCAAC CGCCTACTGT ACTTAACCTT 5160
   ATAAATTTAA AATATTTTTA AAATACTTGT CTCATAGATT TCATTCATAG ATTTCATTCA 5220
   TAGATTTCAT TCATAGATTT CATTCATAGA TTTCATTCAT AGATTTCATT CATAGATTTC 5280
   ATTCATAGAT TTCATTCATA GATTTCATTC ATAGATTTCA TTCATAGATT TCATTCATAG 5340
   ATTTCATTCA TAGATTTCAT TCATAGATTT CATTCATAGA TTTCATTCAT AGATTTCATT 5400
   CATAGATTTC ATTCATAGAT TTCATTCATA GATTTCATTC ATAGATTTCA TTCATAGATT 5460
   TCATTCATAG ATTTTATTTT TTAAATACAC TGCAG 5495
(2) INFORMATION FOR SEQ ID NO:3:
   (i) SEQUENCE CHARACTERISTICS:
      (A) LENGTH: 552 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..552
   (xi) SEQUENCE DESCRIPTION: SEQ ID NO:3:
   ATG AGT GCT TCA TTT GCT CTA TTT GAT AAC AAC TCC GAT CAA GCT AAT      48
   Met Ser Ala Ser Phe Ala Leu Phe Asp Asn Asn Ser Asp Gln Ala Asn
     1               5                  10                  15
   ATG CCA TCA TAC TTC AGA CGC TGT CGT GGA GAA CTT AAT GAA GGA TTC      96
   Met Pro Ser Tyr Phe Arg Arg Cys Arg Gly Glu Leu Asn Glu Gly Phe
                20                  25                  30
   TAC GCA CAA GTT CCG CCT GGT TAT TTT CCA GTT CGC CCT AAA ACC ACA     144
   Tyr Ala Gln Val Pro Pro Gly Tyr Phe Pro Val Arg Pro Lys Thr Thr
            35                  40                  45
   CCA GCG CTT GCA CGT GTG AAA AAC CAG GGT GAA CCG CAC GCC TTC ACT     192
   Pro Ala Leu Ala Arg Val Lys Asn Gln Gly Glu Pro His Ala Phe Thr
        50                  55                  60
   ATT GTT CCT ACA CCA CCT ACG GAT GTA CGA TTC TTC AAA AAG CTT CGT     240
   Ile Val Pro Thr Pro Pro Thr Asp Val Arg Phe Phe Lys Lys Leu Arg
    65                  70                  75                  80
   GAT GGA ACC TTT GTG AAA CTT CCA TTT TCA TAT CCT GAT GAG AGG TAT     288
   Asp Gly Thr Phe Val Lys Leu Pro Phe Ser Tyr Pro Asp Glu Arg Tyr
                    85                  90                  95
   GAA GAT GAT ATT GAA CCT ATG TAT TGC AGA CTA TAC GTT CTA AAG GAT     336
   Glu Asp Asp Ile Glu Pro Met Tyr Cys Arg Leu Tyr Val Leu Lys Asp
               100                 105                 110
   TTT GAA ACG CCA AGT TCT CCC TAC AAT CAA GTA AAC GCT TCT CAG CTT        384
   Phe Glu Thr Pro Ser Ser Pro Tyr Asn Gln Val Asn Ala Ser Gln Leu
           115                 120                 125
   ATG GAA GTA CCT TCT TCG CTT GTG GAA TCG AAG TGT TAT TGG GAT GGT        432
   Met Glu Val Pro Ser Ser Leu Val Glu Ser Lys Cys Tyr Trp Asp Gly
       130                 135                 140
   CCA AAA TTT ATA AAT GTA CCA CCA ATA AGA TAT ATT CTA AAA AGT GAT        480
   Pro Lys Phe Ile Asn Val Pro Pro Ile Arg Tyr Ile Leu Lys Ser Asp
   145                 150                 155                 160
   TAC TCT CTT AAA ATA AAT GTA TCC GAA GAT GCA TTT GTG TTG GGT AGG        528
   Tyr Ser Leu Lys Ile Asn Val Ser Glu Asp Ala Phe Val Leu Gly Arg
                   165                 170                 175
   ATA CCA GAA AAA CTA AAA ACT TGA                                        552
   Ile Pro Glu Lys Leu Lys Thr  *
               180
(2) INFORMATION FOR SEQ ID NO:4:
   (i) SEQUENCE CHARACTERISTICS:
      (A) LENGTH: 184 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Ser Ala Ser Phe Ala Leu Phe Asp Asn Asn Ser Asp Gln Ala Asn
    1               5                  10                  15
   Met Pro Ser Tyr Phe Arg Arg Cys Arg Gly Glu Leu Asn Glu Gly Phe
               20                  25                  30
   Tyr Ala Gln Val Pro Pro Gly Tyr Phe Pro Val Arg Pro Lys Thr Thr
           35                  40                  45
   Pro Ala Leu Ala Arg Val Lys Asn Gln Gly Glu Pro His Ala Phe Thr
       50                  55                  60
   Ile Val Pro Thr Pro Pro Thr Asp Val Arg Phe Phe Lys Lys Leu Arg
   65                  70                  75                  80
   Asp Gly Thr Phe Val Lys Leu Pro Phe Ser Tyr Pro Asp Glu Arg Tyr
                   85                  90                  95
   Glu Asp Asp Ile Glu Pro Met Tyr Cys Arg Leu Tyr Val Leu Lys Asp
               100                 105                 110
   Phe Glu Thr Pro Ser Ser Pro Tyr Asn Gln Val Asn Ala Ser Gln Leu
           115                 120                 125
   Met Glu Val Pro Ser Ser Leu Val Glu Ser Lys Cys Tyr Trp Asp Gly
       130                 135                 140
   Pro Lys Phe Ile Asn Val Pro Pro Ile Arg Tyr Ile Leu Lys Ser Asp
   145                 150                 155                 160
   Tyr Ser Leu Lys Ile Asn Val Ser Glu Asp Ala Phe Val Leu Gly Arg
                   165                     170                     175
   Ile Pro Glu Lys Leu Lys Thr  *
               180
(2) INFORMATION FOR SEQ ID NO:5:
   (i) SEQUENCE CHARACTERISTICS:
      (A) LENGTH: 1176 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1176
   (xi) SEQUENCE DESCRIPTION: SEQ ID NO:5:
   ATG GGT GTG GTT GAA GTT AAT ATT GTT ACG TTT ATT GAC AAA AAT GGG         48
   Met Gly Val Val Glu Val Asn Ile Val Thr Phe Ile Asp Lys Asn Gly
     1               5                  10                  15
   GCG TTA CCA GGA AAC TCC CAA GAT GTT CAT CCT GAT CTT TGG TGT TTT         96
   Ala Leu Pro Gly Asn Ser Gln Asp Val His Pro Asp Leu Trp Cys Phe
                20                  25                  30
   ATG GCT AGA CAA TGC TAT ATC CTT TCT CTC ACA CGA TTG GCT ATG CCT        144
   Met Ala Arg Gln Cys Tyr Ile Leu Ser Leu Thr Arg Leu Ala Met Pro
            35                  40                  45
   ATA ATA TTA CGA TCT GCA AAC TTA TGT TAT TTT ATG GAC TCT ATA AAA        192
   Ile Ile Leu Arg Ser Ala Asn Leu Cys Tyr Phe Met Asp Ser Ile Lys
        50                  55                  60
   CAT CTA CCT AGA GTT TCA AGG CCT ATA GTA AGA ACA TCA ACC TCT AAT        240
   His Leu Pro Arg Val Ser Arg Pro Ile Val Arg Thr Ser Thr Ser Asn
    65                  70                  75                  80
   AGT AAA TTT TTA AAA CCA ACG GAG GGG GAG GAG GAG GAC TTT TTA TTT        288
   Ser Lys Phe Leu Lys Pro Thr Glu Gly Glu Glu Glu Asp Phe Leu Phe
                    85                  90                  95
   TAT GAG GAT AAA CCT GGG GCT AGC ATC GAA TGG AAA TCG GCT GTT TCG        336
   Tyr Glu Asp Lys Pro Gly Ala Ser Ile Glu Trp Lys Ser Ala Val Ser
               100                 105                 110
   GGG TAT AAC TAT CTA AAC TCT GGT ATA TTT GGA AAC TAT CCT CTT AAT        384
   Gly Tyr Asn Tyr Leu Asn Ser Gly Ile Phe Gly Asn Tyr Pro Leu Asn
           115                 120                 125
   CTA TGG GTT TTC GGT GCA GCG GAT TTA TGT GAG CCA GTC ATT TCT AAT        432
   Leu Trp Val Phe Gly Ala Ala Asp Leu Cys Glu Pro Val Ile Ser Asn
       130                 135                 140
   ATC CCA GGA CCA AAG CGA CTA ATT TAT GCA TAT GTA TCA TGT GAA TGG      480
   Ile Pro Gly Pro Lys Arg Leu Ile Tyr Ala Tyr Val Ser Cys Glu Trp
   145                 150                 155                 160
   CCG GAA CCA TCC TGG AAA CCT GAG TGT CTT GAG ATT TCC AAA AAT AAT      528
   Pro Glu Pro Ser Trp Lys Pro Glu Cys Leu Glu Ile Ser Lys Asn Asn
                   165                 170                 175
   GTA AAA CAT ATC GAT GAT TCT GGG AGT GTT TAT TGC CCC TCG TTT ATA      576
   Val Lys His Ile Asp Asp Ser Gly Ser Val Tyr Cys Pro Ser Phe Ile
               180                 185                 190
   TGG GTA CCT GTA TTA GAC ACC TTT CTG TCC TCC TTT AAA GTA CCC CCT      624
   Trp Val Pro Val Leu Asp Thr Phe Leu Ser Ser Phe Lys Val Pro Pro
           195                 200                 205
   ATG TTA AGT GAC ATT TTT TAT GGT ACA ACA TTT AAT ACT CCA TTC AAC      672
   Met Leu Ser Asp Ile Phe Tyr Gly Thr Thr Phe Asn Thr Pro Phe Asn
       210                 215                 220
   TTT GAA AGC TCT CCA AGA TGT CCC TCT ATA TCC TCA TCT TCA TCC TCC      720
   Phe Glu Ser Ser Pro Arg Cys Pro Ser Ile Ser Ser Ser Ser Ser Ser
   225                 230                 235                 240
   TCC TCC TCA TCC TCT TCT TCA TGC TCT GCA TCT CAT TAT TCC TCA GAG      768
   Ser Ser Ser Ser Ser Ser Ser Cys Ser Ala Ser His Tyr Ser Ser Glu
                   245                 250                 255
   AAT GAT ATT TGG AAA TAC GCT AAT GCG ATG GTG GAT AAT GAA CTA GAA      816
   Asn Asp Ile Trp Lys Tyr Ala Asn Ala Met Val Asp Asn Glu Leu Glu
               260                 265                 270
   GAA ACT AAT TGT GAA ATT AAA AAT AGT TTT AAA GAG GAT TCT GAC AGT      864
   Glu Thr Asn Cys Glu Ile Lys Asn Ser Phe Lys Glu Asp Ser Asp Ser
           275                 280                 285
   TCT ACA TGT TCT ATA AGC GAT GTA TGT GAT TCT CTT TAT ATA AAT GAG      912
   Ser Thr Cys Ser Ile Ser Asp Val Cys Asp Ser Leu Tyr Ile Asn Glu
       290                 295                 300
   ACA CGT TCT GCC CCC CCG GTA ATC GAA TCA TTT ATG TGT GCA ATA ATA      960
   Thr Arg Ser Ala Pro Pro Val Ile Glu Ser Phe Met Cys Ala Ile Ile
   305                 310                 315                 320
   TCC CAT AAT GGT TAT GAA GAA ATT CCT GAA CCA GAA GAG GAT GAC TTG     1008
   Ser His Asn Gly Tyr Glu Glu Ile Pro Glu Pro Glu Glu Asp Asp Leu
                   325                 330                 335
   CAT GGG GTG AAA CCT ATG AAA AGG CTA TCC AAA CCA AAT ATT TTT AAA     1056
   His Gly Val Lys Pro Met Lys Arg Leu Ser Lys Pro Asn Ile Phe Lys
               340                 345                 350
   CGA TTA TTT TCA AGG CGC GCC AAA ACA ATA ACC AAA AAT GAA AAC GAT     1104
   Arg Leu Phe Ser Arg Arg Ala Lys Thr Ile Thr Lys Asn Glu Asn Asp
           355                 360                 365
   GGT ATG TCT TCT ACA CAA CCT ACC AAC GCT CAA CGT TCA TCT CTA TTT     1152
   Gly Met Ser Ser Thr Gln Pro Thr Asn Ala Gln Arg Ser Ser Leu Phe
       370                 375                 380
   AGT TCA TGT TTA TGT GGA AAC TAA                                     1176
   Ser Ser Cys Leu Cys Gly Asn  *
   385                 390
(2) INFORMATION FOR SEQ ID NO:6:
   (i) SEQUENCE CHARACTERISTICS:
      (A) LENGTH: 392 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Gly Val Val Glu Val Asn Ile Val Thr Phe Ile Asp Lys Asn Gly
    1               5                  10                  15
   Ala Leu Pro Gly Asn Ser Gln Asp Val His Pro Asp Leu Trp Cys Phe
               20                  25                  30
   Met Ala Arg Gln Cys Tyr Ile Leu Ser Leu Thr Arg Leu Ala Met Pro
           35                  40                  45
   Ile Ile Leu Arg Ser Ala Asn Leu Cys Tyr Phe Met Asp Ser Ile Lys
       50                  55                  60
   His Leu Pro Arg Val Ser Arg Pro Ile Val Arg Thr Ser Thr Ser Asn
   65                  70                  75                  80
   Ser Lys Phe Leu Lys Pro Thr Glu Gly Glu Glu Glu Asp Phe Leu Phe
                   85                  90                  95
   Tyr Glu Asp Lys Pro Gly Ala Ser Ile Glu Trp Lys Ser Ala Val Ser
               100                 105                 110
   Gly Tyr Asn Tyr Leu Asn Ser Gly Ile Phe Gly Asn Tyr Pro Leu Asn
           115                 120                 125
   Leu Trp Val Phe Gly Ala Ala Asp Leu Cys Glu Pro Val Ile Ser Asn
       130                 135                 140
   Ile Pro Gly Pro Lys Arg Leu Ile Tyr Ala Tyr Val Ser Cys Glu Trp
   145                 150                 155                 160
   Pro Glu Pro Ser Trp Lys Pro Glu Cys Leu Glu Ile Ser Lys Asn Asn
                   165                 170                 175
   Val Lys His Ile Asp Asp Ser Gly Ser Val Tyr Cys Pro Ser Phe Ile
               180                 185                 190
   Trp Val Pro Val Leu Asp Thr Phe Leu Ser Ser Phe Lys Val Pro Pro
           195                 200                 205
   Met Leu Ser Asp Ile Phe Tyr Gly Thr Thr Phe Asn Thr Pro Phe Asn
       210                 215                 220
   Phe Glu Ser Ser Pro Arg Cys Pro Ser Ile Ser Ser Ser Ser Ser Ser
   225                 230                 235                 240
   Ser Ser Ser Ser Ser Ser Ser Cys Ser Ala Ser His Tyr Ser Ser Glu
                   245                     250                 255
   Asn Asp Ile Trp Lys Tyr Ala Asn Ala Met Val Asp Asn Glu Leu Glu
               260                     265                 270
   Glu Thr Asn Cys Glu Ile Lys Asn Ser Phe Lys Glu Asp Ser Asp Ser
           275                     280                 285
   Ser Thr Cys Ser Ile Ser Asp Val Cys Asp Ser Leu Tyr Ile Asn Glu
       290                     295                 300
   Thr Arg Ser Ala Pro Pro Val Ile Glu Ser Phe Met Cys Ala Ile Ile
   305                     310                 315                 320
   Ser His Asn Gly Tyr Glu Glu Ile Pro Glu Pro Glu Glu Asp Asp Leu
                   325                     330                 335
   His Gly Val Lys Pro Met Lys Arg Leu Ser Lys Pro Asn Ile Phe Lys
               340                     345                 350
   Arg Leu Phe Ser Arg Arg Ala Lys Thr Ile Thr Lys Asn Glu Asn Asp
           355                     360                 365
   Gly Met Ser Ser Thr Gln Pro Thr Asn Ala Gln Arg Ser Ser Leu Phe
       370                     375                 380
   Ser Ser Cys Leu Cys Gly Asn  *
   385                     390
(2) INFORMATION FOR SEQ ID NO:7:
   (i) SEQUENCE CHARACTERISTICS:
      (A) LENGTH: 1203 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1203
   (xi) SEQUENCE DESCRIPTION: SEQ ID NO:7:
   ATG GCA AAG TGT ACC ACC GAA AAG TTT TGT TGT ATC AGC GTG AAT AGA       48
   Met Ala Lys Cys Thr Thr Glu Lys Phe Cys Cys Ile Ser Val Asn Arg
     1               5                  10                  15
   GAA TCT TCT GTC GAT CCA GAA GAC TTC TAT AAA CCG GTT CCT CTA ACT       96
   Glu Ser Ser Val Asp Pro Glu Asp Phe Tyr Lys Pro Val Pro Leu Thr
                20                  25                  30
   TCA GAT TTG ATT GAA GAG GAT AAC CTA CAT CAA GAC AAA ATA ATG GAT      144
   Ser Asp Leu Ile Glu Glu Asp Asn Leu His Gln Asp Lys Ile Met Asp
            35                  40                  45
   GAG GAT TTA TAC TCG GAT TTT AGT GAT GAT GAC TTT ATG GAT TAT ACA       192
   Glu Asp Leu Tyr Ser Asp Phe Ser Asp Asp Asp Phe Met Asp Tyr Thr
        50                  55                  60
   AAA AAT CCA ACT GAA AGT GAA AAT GAA AGA GAA AGT GAC GAA GAA GTT       240
   Lys Asn Pro Thr Glu Ser Glu Asn Glu Arg Glu Ser Asp Glu Glu Val
    65                  70                  75                  80
   GAA GAA AGT TAT GAA AGT GAT GAA GAT AAA AAA AGT TTA TCT CCT ACT       288
   Glu Glu Ser Tyr Glu Ser Asp Glu Asp Lys Lys Ser Leu Ser Pro Thr
                   85                  90                  95
   AAA AGC GAA GGA ATT GAA GCG GCT GAA GCG CTA AAG TTT TCT GTT GTT       336
   Lys Ser Glu Gly Ile Glu Ala Ala Glu Ala Leu Lys Phe Ser Val Val
               100                 105                 110
   AAA TCG TTA ACG CCT GGG TCA GAA GGA AGA GTT TTT ATT GCT CTT AAA       384
   Lys Ser Leu Thr Pro Gly Ser Glu Gly Arg Val Phe Ile Ala Leu Lys
           115                 120                 125
   AAA GAT AAA GAT ACA AGC TAT AAG GTA ATT TTA AAA ATT GGA CAA AGG       432
   Lys Asp Lys Asp Thr Ser Tyr Lys Val Ile Leu Lys Ile Gly Gln Arg
       130                 135                 140
   GGA AAC ACG CTT GTG GAA TCG TTA ATT TTG AGA AAT ATT AGT CAC CAA       480
   Gly Asn Thr Leu Val Glu Ser Leu Ile Leu Arg Asn Ile Ser His Gln
   145                 150                 155                 160
   TCT ATA ATT AAA CTT CAA GAC ACT CTT TTT TAT AAA GAG TTA ACA TGT       528
   Ser Ile Ile Lys Leu Gln Asp Thr Leu Phe Tyr Lys Glu Leu Thr Cys
                   165                 170                 175
   TTG GTG TTA CCG TAT TAT AAA TAT GAT CTA TAT AAT TTT TTA ATG GAT       576
   Leu Val Leu Pro Tyr Tyr Lys Tyr Asp Leu Tyr Asn Phe Leu Met Asp
               180                 185                 190
   CAT GGG AAA TCT CTG TCT TTT GAA TCT GTA ATT AAA ATT GAA AAA CAA       624
   His Gly Lys Ser Leu Ser Phe Glu Ser Val Ile Lys Ile Glu Lys Gln
           195                 200                 205
   ATA TTA ACT GGA CTT CAA TAT ATT CAT GGA AAA AAA ATT ATT CAT CGA       672
   Ile Leu Thr Gly Leu Gln Tyr Ile His Gly Lys Lys Ile Ile His Arg
       210                 215                 220
   GAT ATA AAA ACT GAA AAT ATT TTC TTG GAT AAT GAC TCT AAT GTT TGT       720
   Asp Ile Lys Thr Glu Asn Ile Phe Leu Asp Asn Asp Ser Asn Val Cys
   225                 230                 235                 240
   ATA GGT GAT TTT GGG GCT TCT CAA TTT CCT GTT TCC TCA CCA GAT TAT       768
   Ile Gly Asp Phe Gly Ala Ser Gln Phe Pro Val Ser Ser Pro Asp Tyr
                   245                 250                 255
   TTG GGA ATT GCG GGG ACT ATT GAA ACT AAT GCT CCT GAA GTT CTA TCA       816
   Leu Gly Ile Ala Gly Thr Ile Glu Thr Asn Ala Pro Glu Val Leu Ser
               260                 265                 270
   AAG GAT GCG TAC AAC TGT AAA GCT GAT ATT TGG AGT GCT GGT ATA ATT       864
   Lys Asp Ala Tyr Asn Cys Lys Ala Asp Ile Trp Ser Ala Gly Ile Ile
           275                 280                 285
   TTA TTT GAA ATG CTT GCA TAT CCT AAT GTT TTG TTT GAG GAG GAA GAA          912
   Leu Phe Glu Met Leu Ala Tyr Pro Asn Val Leu Phe Glu Glu Glu Glu
       290                 295                 300
   AGA GAT AGT AGC GAT TTA ATA AAC AAT TGT AAT CTT CAT CTT ATA AAA          960
   Arg Asp Ser Ser Asp Leu Ile Asn Asn Cys Asn Leu His Leu Ile Lys
   305                 310                 315                 320
   ATT ATA TCA ACT CTG AAG ATT AAC CCA AAT GAA TTT CCA TCT GAT TTG         1008
   Ile Ile Ser Thr Leu Lys Ile Asn Pro Asn Glu Phe Pro Ser Asp Leu
                   325                 330                 335
   GAA TCT AAT CTA GTA AAA CAT TTT ATA AAA TAT GCT AAT AAT GAT AGA         1056
   Glu Ser Asn Leu Val Lys His Phe Ile Lys Tyr Ala Asn Asn Asp Arg
               340                 345                 350
   CCT CCA TTT ACA CGA TAT AAT CGT CTA AAT AAC CTT AAA TTA CAT CTC         1104
   Pro Pro Phe Thr Arg Tyr Asn Arg Leu Asn Asn Leu Lys Leu His Leu
           355                 360                 365
   GAT GGT GAA TTT TTA ATT CAT AAA ATG CTA ACA TTT GAT GCA TCT CTA         1152
   Asp Gly Glu Phe Leu Ile His Lys Met Leu Thr Phe Asp Ala Ser Leu
       370                 375                 380
   CGA CCA AGT GCG GAA GAA CTA TTA TCC TAT CAG ATT TTT AGT AAA CAA         1200
   Arg Pro Ser Ala Glu Glu Leu Leu Ser Tyr Gln Ile Phe Ser Lys Gln
   385                 390                 395                 400
   TAA                                                                     1203
    *
(2) INFORMATION FOR SEQ ID NO:8:
   (i) SEQUENCE CHARACTERISTICS:
      (A) LENGTH: 401 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Ala Lys Cys Thr Thr Glu Lys Phe Cys Cys Ile Ser Val Asn Arg
    1               5                  10                  15
   Glu Ser Ser Val Asp Pro Glu Asp Phe Tyr Lys Pro Val Pro Leu Thr
               20                  25                  30
   Ser Asp Leu Ile Glu Glu Asp Asn Leu His Gln Asp Lys Ile Met Asp
           35                  40                  45
   Glu Asp Leu Tyr Ser Asp Phe Ser Asp Asp Asp Phe Met Asp Tyr Thr
       50                  55                  60
   Lys Asn Pro Thr Glu Ser Glu Asn Glu Arg Glu Ser Asp Glu Glu Val
   65                  70                  75                  80
   Glu Glu Ser Tyr Glu Ser Asp Glu Asp Lys Lys Ser Leu Ser Pro Thr
                   85                  90                  95
   Lys Ser Glu Gly Ile Glu Ala Ala Glu Ala Leu Lys Phe Ser Val Val
               100                 105                 110
   Lys Ser Leu Thr Pro Gly Ser Glu Gly Arg Val Phe Ile Ala Leu Lys
           115                 120                 125
   Lys Asp Lys Asp Thr Ser Tyr Lys Val Ile Leu Lys Ile Gly Gln Arg
       130                 135                 140
   Gly Asn Thr Leu Val Glu Ser Leu Ile Leu Arg Asn Ile Ser His Gln
   145                 150                 155                 160
   Ser Ile Ile Lys Leu Gln Asp Thr Leu Phe Tyr Lys Glu Leu Thr Cys
                   165                 170                 175
   Leu Val Leu Pro Tyr Tyr Lys Tyr Asp Leu Tyr Asn Phe Leu Met Asp
               180                 185                 190
   His Gly Lys Ser Leu Ser Phe Glu Ser Val Ile Lys Ile Glu Lys Gln
           195                 200                 205
   Ile Leu Thr Gly Leu Gln Tyr Ile His Gly Lys Lys Ile Ile His Arg
       210                 215                 220
   Asp Ile Lys Thr Glu Asn Ile Phe Leu Asp Asn Asp Ser Asn Val Cys
   225                 230                 235                 240
   Ile Gly Asp Phe Gly Ala Ser Gln Phe Pro Val Ser Ser Pro Asp Tyr
                   245                 250                 255
   Leu Gly Ile Ala Gly Thr Ile Glu Thr Asn Ala Pro Glu Val Leu Ser
               260                 265                 270
   Lys Asp Ala Tyr Asn Cys Lys Ala Asp Ile Trp Ser Ala Gly Ile Ile
           275                 280                 285
   Leu Phe Glu Met Leu Ala Tyr Pro Asn Val Leu Phe Glu Glu Glu Glu
       290                 295                 300
   Arg Asp Ser Ser Asp Leu Ile Asn Asn Cys Asn Leu His Leu Ile Lys
   305                 310                 315                 320
   Ile Ile Ser Thr Leu Lys Ile Asn Pro Asn Glu Phe Pro Ser Asp Leu
                   325                 330                 335
   Glu Ser Asn Leu Val Lys His Phe Ile Lys Tyr Ala Asn Asn Asp Arg
               340                 345                 350
   Pro Pro Phe Thr Arg Tyr Asn Arg Leu Asn Asn Leu Lys Leu His Leu
           355                 360                 365
   Asp Gly Glu Phe Leu Ile His Lys Met Leu Thr Phe Asp Ala Ser Leu
       370                 375                 380
   Arg Pro Ser Ala Glu Glu Leu Leu Ser Tyr Gln Ile Phe Ser Lys Gln
   385                 390                 395                 400
   *
(2) INFORMATION FOR SEQ ID NO:9:
   (i) SEQUENCE CHARACTERISTICS:
      (A) LENGTH: 1248 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1248
   (xi) SEQUENCE DESCRIPTION: SEQ ID NO:9:
   ATG TTG TAT ACG CTG TTT TTT GTT TTT TAT TTT AAG GTA GTT TTA TCT         48
   Met Leu Tyr Thr Leu Phe Phe Val Phe Tyr Phe Lys Val Val Leu Ser
    1               5                  10                  15
   CGC ATA GCT CCG CTA GAG TTG TGT TAT GCG GAT CCT AAA GAA AAT ACA         96
   Arg Ile Ala Pro Leu Glu Leu Cys Tyr Ala Asp Pro Lys Glu Asn Thr
               20                  25                  30
   ACT GAA CCT ACA CAA CTT CCT ACA GGG GAA CAA TCT AAG ACT CTT ATT        144
   Thr Glu Pro Thr Gln Leu Pro Thr Gly Glu Gln Ser Lys Thr Leu Ile
           35                  40                  45
   CCC GTG GTA ACA AAC GGA TAT GTT GAA TAC TCT AAA GGA TGT GAA CTA        192
   Pro Val Val Thr Asn Gly Tyr Val Glu Tyr Ser Lys Gly Cys Glu Leu
       50                  55                  60
   CGA TTA CTA GAT ACA TAT GTA AAT GTA TCT TCA CGA CCA GAA AAA AAG        240
   Arg Leu Leu Asp Thr Tyr Val Asn Val Ser Ser Arg Pro Glu Lys Lys
    65                  70                  75                  80
   GTT AAT GCT ACA ATT GGA TGG TCA TTT GAT CTT GGT TGT CAA ATT CCT        288
   Val Asn Ala Thr Ile Gly Trp Ser Phe Asp Leu Gly Cys Gln Ile Pro
                   85                  90                  95
   TTA ATT TAT AGA GAA TAT TAT AAT TGT ACT GGT AAT ATA ATA CCA TCA        336
   Leu Ile Tyr Arg Glu Tyr Tyr Asn Cys Thr Gly Asn Ile Ile Pro Ser
               100                 105                 110
   CCA GAA ACT TGT GAT GGT TAT TCT TTA ACT TTG GTA AAA TCT GAA AGT        384
   Pro Glu Thr Cys Asp Gly Tyr Ser Leu Thr Leu Val Lys Ser Glu Ser
           115                 120                 125
   ATA TCA TCT TAT GCA CTT GTT AAT GTT AGT TTG CTT ATT CAA CCA GGA        432
   Ile Ser Ser Tyr Ala Leu Val Asn Val Ser Leu Leu Ile Gln Pro Gly
       130                 135                 140
   ATT TTT GAT TCT GGT AGA TAT TTA TAC TCA CTT GTT TTT GGA AAC GAT        480
   Ile Phe Asp Ser Gly Arg Tyr Leu Tyr Ser Leu Val Phe Gly Asn Asp
   145                 150                 155                 160
   AGT TAT AAC GGA AGA ATT GAA GTT CGA GTG GAT AAT GAG ACA GAC TAT        528
   Ser Tyr Asn Gly Arg Ile Glu Val Arg Val Asp Asn Glu Thr Asp Tyr
                   165                 170                 175
   CCA TGT TTT ATG ATG CAT GGA TTG ACT GTA AAA AAG GGT GAT AAA CTT        576
   Pro Cys Phe Met Met His Gly Leu Thr Val Lys Lys Gly Asp Lys Leu
               180                 185                 190
   CAT ATT CCT TAT AAA CCA TCC ACA AAT CCT AAT CAT AAA CGA TAT AGA        624
   His Ile Pro Tyr Lys Pro Ser Thr Asn Pro Asn His Lys Arg Tyr Arg
           195                 200                 205
   GGT TGT TTT CCA ATA TCA AAT ACT GAG CTA TGG AAT AAT ATT AGT GAT        672
   Gly Cys Phe Pro Ile Ser Asn Thr Glu Leu Trp Asn Asn Ile Ser Asp
       210                 215                 220
   GAA AGT GTT GGT AGA TAT TCA TAT GAT GAA GAA TAT GAA GAA TAT GAA        720
   Glu Ser Val Gly Arg Tyr Ser Tyr Asp Glu Glu Tyr Glu Glu Tyr Glu
   225                 230                 235                 240
   GAA GAA AAC GAA GAT TTT GAA GAT CTA CAA TCA AAA GAT TGC CGC AAA        768
   Glu Glu Asn Glu Asp Phe Glu Asp Leu Gln Ser Lys Asp Cys Arg Lys
                   245                 250                 255
   TCC AAT CTT TTT GAT ATG AAG AAG ACT TTT AAT TTG GCT GCA GGT TCT        816
   Ser Asn Leu Phe Asp Met Lys Lys Thr Phe Asn Leu Ala Ala Gly Ser
               260                 265                 270
   CAA AGT TTA TTG ATT GCT AGT TTG GGT AAA TCA ATT TCA GAA CAA CCG        864
   Gln Ser Leu Leu Ile Ala Ser Leu Gly Lys Ser Ile Ser Glu Gln Pro
           275                 280                 285
   TGG TCA TTT AAA ATT AAT GAA AGT TAT GAA CTT TTT AAT AAT TTG TCT        912
   Trp Ser Phe Lys Ile Asn Glu Ser Tyr Glu Leu Phe Asn Asn Leu Ser
       290                 295                 300
   ATC ACC CTT CAA TCG GAA GAA GAT TCT AAT ATA CTG AAT CCT GAA ATT        960
   Ile Thr Leu Gln Ser Glu Glu Asp Ser Asn Ile Leu Asn Pro Glu Ile
   305                 310                 315                 320
   GTA ACG TTT ACC ACA CCA CCA CCT ACT GAA AAT ACA CAT ATG TTT ATG       1008
   Val Thr Phe Thr Thr Pro Pro Pro Thr Glu Asn Thr His Met Phe Met
                   325                 330                 335
   TCA AAT AAT GAA ACT ATG TAT GAA GAA GAA AGT GTT TTA AGC ATT ATT       1056
   Ser Asn Asn Glu Thr Met Tyr Glu Glu Glu Ser Val Leu Ser Ile Ile
               340                 345                 350
   CAA TTG TTT AAC AAT GGT TAT AAT AAT TGT AAT ACC CAT ATA AAG GTA       1104
   Gln Leu Phe Asn Asn Gly Tyr Asn Asn Cys Asn Thr His Ile Lys Val
           355                 360                 365
   ATT GGA TTT GGA ACA ATT ATC TTT ATT ATT TTA TTT TTT GTT GCT GTG       1152
   Ile Gly Phe Gly Thr Ile Ile Phe Ile Ile Leu Phe Phe Val Ala Val
       370                 375                 380
   TTT TTT TGT GGA TAT ACT TGT GTA TTA AAC TCT CGT ATT AAA ATG ATT       1200
   Phe Phe Cys Gly Tyr Thr Cys Val Leu Asn Ser Arg Ile Lys Met Ile
   385                 390                 395                 400
   AAC CAT GCT TAT ATA CAA CCC CAG AAA TTA AAT TTT TAT GAT ATT TAA       1248
   Asn His Ala Tyr Ile Gln Pro Gln Lys Leu Asn Phe Tyr Asp Ile  *
                   405                 410                 415
(2) INFORMATION FOR SEQ ID NO:10:
   (i) SEQUENCE CHARACTERISTICS:
      (A) LENGTH: 416 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Leu Tyr Thr Leu Phe Phe Val Phe Tyr Phe Lys Val Val Leu Ser
    1               5                  10                  15
   Arg Ile Ala Pro Leu Glu Leu Cys Tyr Ala Asp Pro Lys Glu Asn Thr
               20                  25                  30
   Thr Glu Pro Thr Gln Leu Pro Thr Gly Glu Gln Ser Lys Thr Leu Ile
           35                  40                  45
   Pro Val Val Thr Asn Gly Tyr Val Glu Tyr Ser Lys Gly Cys Glu Leu
       50                  55                  60
   Arg Leu Leu Asp Thr Tyr Val Asn Val Ser Ser Arg Pro Glu Lys Lys
    65                  70                  75                  80
   Val Asn Ala Thr Ile Gly Trp Ser Phe Asp Leu Gly Cys Gln Ile Pro
                   85                  90                  95
   Leu Ile Tyr Arg Glu Tyr Tyr Asn Cys Thr Gly Asn Ile Ile Pro Ser
               100                 105                 110
   Pro Glu Thr Cys Asp Gly Tyr Ser Leu Thr Leu Val Lys Ser Glu Ser
           115                 120                 125
   Ile Ser Ser Tyr Ala Leu Val Asn Val Ser Leu Leu Ile Gln Pro Gly
       130                 135                 140
   Ile Phe Asp Ser Gly Arg Tyr Leu Tyr Ser Leu Val Phe Gly Asn Asp
   145                 150                 155                 160
   Ser Tyr Asn Gly Arg Ile Glu Val Arg Val Asp Asn Glu Thr Asp Tyr
                   165                 170                 175
   Pro Cys Phe Met Met His Gly Leu Thr Val Lys Lys Gly Asp Lys Leu
               180                 185                 190
   His Ile Pro Tyr Lys Pro Ser Thr Asn Pro Asn His Lys Arg Tyr Arg
           195                 200                 205
   Gly Cys Phe Pro Ile Ser Asn Thr Glu Leu Trp Asn Asn Ile Ser Asp
       210                 215                 220
   Glu Ser Val Gly Arg Tyr Ser Tyr Asp Glu Glu Tyr Glu Glu Tyr Glu
   225                 230                 235                 240
   Glu Glu Asn Glu Asp Phe Glu Asp Leu Gln Ser Lys Asp Cys Arg Lys
                   245                 250                 255
   Ser Asn Leu Phe Asp Met Lys Lys Thr Phe Asn Leu Ala Ala Gly Ser
               260                 265                 270
   Gln Ser Leu Leu Ile Ala Ser Leu Gly Lys Ser Ile Ser Glu Gln Pro
           275                 280                 285
   Trp Ser Phe Lys Ile Asn Glu Ser Tyr Glu Leu Phe Asn Asn Leu Ser
       290                 295                 300
   Ile Thr Leu Gln Ser Glu Glu Asp Ser Asn Ile Leu Asn Pro Glu Ile
   305                 310                 315                 320
   Val Thr Phe Thr Thr Pro Pro Pro Thr Glu Asn Thr His Met Phe Met
                   325                 330                 335
   Ser Asn Asn Glu Thr Met Tyr Glu Glu Glu Ser Val Leu Ser Ile Ile
               340                 345                 350
   Gln Leu Phe Asn Asn Gly Tyr Asn Asn Cys Asn Thr His Ile Lys Val
           355                 360                 365
   Ile Gly Phe Gly Thr Ile Ile Phe Ile Ile Leu Phe Phe Val Ala Val
       370                 375                 380
   Phe Phe Cys Gly Tyr Thr Cys Val Leu Asn Ser Arg Ile Lys Met Ile
   385                 390                 395                 400
   Asn His Ala Tyr Ile Gln Pro Gln Lys Leu Asn Phe Tyr Asp Ile  *
                   405                 410                 415
(2) INFORMATION FOR SEQ ID NO:11:
   (i) SEQUENCE CHARACTERISTICS:
      (A) LENGTH: 357 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..357
   (xi) SEQUENCE DESCRIPTION: SEQ ID NO:11:
   ATG ATT AAA CTT CTA TTT ATC TTA TTT TAT TTT AAC CCA ATA ACT GGA        48
   Met Ile Lys Leu Leu Phe Ile Leu Phe Tyr Phe Asn Pro Ile Thr Gly
    1               5                  10                  15
   TAT AAA TGG GTA GAC CCT CCT CGT AGG TAT AAT TAC ACC GTT TTA AGA        96
   Tyr Lys Trp Val Asp Pro Pro Arg Arg Tyr Asn Tyr Thr Val Leu Arg
               20                  25                  30
   ATG ATT CCA GAT ATT CCA AAT CCA ATG GAT CCT TCT AAA AAC GCT GAA       144
   Met Ile Pro Asp Ile Pro Asn Pro Met Asp Pro Ser Lys Asn Ala Glu
           35                  40                  45
   GTT CGG TAT GTA ACT TCT ACT GAC CCA TGT GAT ATG GTT GCT TTG ATT       192
   Val Arg Tyr Val Thr Ser Thr Asp Pro Cys Asp Met Val Ala Leu Ile
       50                  55                  60
   TCT AAT CCA AAT ATA GAA TCT ACA ATT AAA ACG ATT CAA TTT GTG CAA        240
   Ser Asn Pro Asn Ile Glu Ser Thr Ile Lys Thr Ile Gln Phe Val Gln
    65                  70                  75                  80
   AAG AAA AAA TTT TAC AAT GCA TCT CTT AGT TGG TTT AAA GTT GGA GAT        288
   Lys Lys Lys Phe Tyr Asn Ala Ser Leu Ser Trp Phe Lys Val Gly Asp
                   85                  90                  95
   GAT TGT ACA TAT CCA ATA TAT TTA ATT CAA TAT TTT GAT TGT GAT CCT        336
   Asp Cys Thr Tyr Pro Ile Tyr Leu Ile Gln Tyr Phe Asp Cys Asp Pro
               100                 105                 110
   CAA AGA GAA TTT GGC ATA TGT                                            357
   Gln Arg Glu Phe Gly Ile Cys
           115
(2) INFORMATION FOR SEQ ID NO:12:
   (i) SEQUENCE CHARACTERISTICS:
      (A) LENGTH: 119 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Ile Lys Leu Leu Phe Ile Leu Phe Tyr Phe Asn Pro Ile Thr Gly
     1               5                  10                  15
   Tyr Lys Trp Val Asp Pro Pro Arg Arg Tyr Asn Tyr Thr Val Leu Arg
               20                  25                  30
   Met Ile Pro Asp Ile Pro Asn Pro Met Asp Pro Ser Lys Asn Ala Glu
           35                  40                  45
   Val Arg Tyr Val Thr Ser Thr Asp Pro Cys Asp Met Val Ala Leu Ile
       50                  55                  60
   Ser Asn Pro Asn Ile Glu Ser Thr Ile Lys Thr Ile Gln Phe Val Gln
    65                  70                  75                  80
   Lys Lys Lys Phe Tyr Asn Ala Ser Leu Ser Trp Phe Lys Val Gly Asp
                   85                  90                  95
   Asp Cys Thr Tyr Pro Ile Tyr Leu Ile Gln Tyr Phe Asp Cys Asp Pro
               100                 105                 110
   Gln Arg Glu Phe Gly Ile Cys
           115
(2) INFORMATION FOR SEQ ID NO:13:
   (i) SEQUENCE CHARACTERISTICS:
      (A) LENGTH: 743 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
   CGAAGCGGGA GGAGGATGCT GGTTATGATA TACCATCTCC AAATTTAGTT CAAATAAAAC    60
   CGGGATATAG TTACCTTTTT TGTCTTCCTA TTTTTCAATT AGAAATGAAA AACCCACCAA   120
   TCGCTTGTAT TTTTGGTAGA TCATCCTTAA ATTCAAGCGG AATAATTGTT CTTCCAACTA   180
   TATGGAAACC AAAAACAATT TGTCAATTTT TTATTAAAAA TATATCCTCT AAAACTGTAA   240
   CTATAGAAAA AGGTCAGAGA ATAGCTCAGT TAGTTCTTTT AAAAAACAAT CAACCACTAT   300
   GGTTACAACC ACAAATTAAT TGTCATTCTT TATTTCCAAA GTCAAACTAT TTAAGCTTAT   360
   CAAATCGAGA ATGTGATATG TGGAAGTTTA CAGAAGATCT GAATTTTGAA GCACCGAAAA   420
   GTTTACGAGG AATAAATGGA TTTGGATCCA CGGGATTGTA AAATTCGTTA ATAAAGTTAT   480
   ATTTAAAGTG CCAAACTTTC ACGTGTCATT TTTTTGGGAC CGTTTCTTTT TTGTTTAGTC   540
   GATAAAATAT TTTCAGTTTC CATAGAACTT ATTAGAGGTT CTGTATCTAG TATATCTGTA   600
   GAATTATTTT CATCATATTT AACGGTTTGA AGAGATAAGG GTTTTGTTGT ATTAGAATCT   660
   ATACCAAGGG TTTTTTCTAA AACCGCTACA TCTGCCATAA CAATATTATT TTCTGAAGTC   720
   ATTTTTATGG CTTGGGCACC ACC                                           743
(2) INFORMATION FOR SEQ ID NO:14:
   (i) SEQUENCE CHARACTERISTICS:
      (A) LENGTH: 743 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   GGTGGTGCCC AAGCCATAAA AATGACTTCA GAAAATAATA TTGTTATGGC AGATGTAGCG    60
   GTTTTAGAAA AAACCCTTGG TATAGATTCT AATACAACAA AACCCTTATC TCTTCAAACC   120
   GTTAAATATG ATGAAAATAA TTCTACAGAT ATACTAGATA CAGAACCTCT AATAAGTTCT   180
   ATGGAAACTG AAAATATTTT ATCGACTAAA CAAAAAAGAA ACGGTCCCAA AAAAATGACA   240
   CGTGAAAGTT TGGCACTTTA AATATAACTT TATTAACGAA TTTTACAATC CCGTGGATCC   300
   AAATCCATTT ATTCCTCGTA AACTTTTCGG TGCTTCAAAA TTCAGATCTT CTGTAAACTT   360
   CCACATATCA CATTCTCGAT TTGATAAGCT TAAATAGTTT GACTTTGGAA ATAAAGAATG   420
   ACAATTAATT TGTGGTTGTA ACCATAGTGG TTGATTGTTT TTTAAAAGAA CTAACTGAGC   480
   TATTCTCTGA CCTTTTTCTA TAGTTACAGT TTTAGAGGAT ATATTTTTAA TAAAAAATTG   540
   ACAAATTGTT TTTGGTTTCC ATATAGTTGG AAGAACAATT ATTCCGCTTG AATTTAAGGA     600
   TGATCTACCA AAAATACAAG CGATTGGTGG GTTTTTCATT TCTAATTGAA AAATAGGAAG     660
   ACAAAAAAGG TAACTATATC CCGGTTTTAT TTGAACTAAA TTTGGAGATG GTATATCATA     720
   ACCAGCATCC TCCTCCCGCT TCG                                            743
(2) INFORMATION FOR SEQ ID NO:15:
   (i) SEQUENCE CHARACTERISTICS:
      (A) LENGTH: 459 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..459
   (xi) SEQUENCE DESCRIPTION: SEQ ID NO:15:
   AAG CGG GAG GAG GAT GCT GGT TAT GAT ATA CCA TCT CCA AAT TTA GTT      48
   Lys Arg Glu Glu Asp Ala Gly Tyr Asp Ile Pro Ser Pro Asn Leu Val
     1               5                  10                  15
   CAA ATA AAA CCG GGA TAT AGT TAC CTT TTT TGT CTT CCT ATT TTT CAA      96
   Gln Ile Lys Pro Gly Tyr Ser Tyr Leu Phe Cys Leu Pro Ile Phe Gln
                20                  25                  30
   TTA GAA ATG AAA AAC CCA CCA ATC GCT TGT ATT TTT GGT AGA TCA TCC     144
   Leu Glu Met Lys Asn Pro Pro Ile Ala Cys Ile Phe Gly Arg Ser Ser
            35                  40                  45
   TTA AAT TCA AGC GGA ATA ATT GTT CTT CCA ACT ATA TGG AAA CCA AAA     192
   Leu Asn Ser Ser Gly Ile Ile Val Leu Pro Thr Ile Trp Lys Pro Lys
        50                  55                  60
   ACA ATT TGT CAA TTT TTT ATT AAA AAT ATA TCC TCT AAA ACT GTA ACT     240
   Thr Ile Cys Gln Phe Phe Ile Lys Asn Ile Ser Ser Lys Thr Val Thr
    65                  70                  75                  80
   ATA GAA AAA GGT CAG AGA ATA GCT CAG TTA GTT CTT TTA AAA AAC AAT     288
   Ile Glu Lys Gly Gln Arg Ile Ala Gln Leu Val Leu Leu Lys Asn Asn
                    85                  90                  95
   CAA CCA CTA TGG TTA CAA CCA CAA ATT AAT TGT CAT TCT TTA TTT CCA     336
   Gln Pro Leu Trp Leu Gln Pro Gln Ile Asn Cys His Ser Leu Phe Pro
               100                 105                 110
   AAG TCA AAC TAT TTA AGC TTA TCA AAT CGA GAA TGT GAT ATG TGG AAG     384
   Lys Ser Asn Tyr Leu Ser Leu Ser Asn Arg Glu Cys Asp Met Trp Lys
           115                 120                 125
   TTT ACA GAA GAT CTG AAT TTT GAA GCA CCG AAA AGT TTA CGA GGA ATA     432
   Phe Thr Glu Asp Leu Asn Phe Glu Ala Pro Lys Ser Leu Arg Gly Ile
       130                 135                 140
   AAT GGA TTT GGA TCC ACG GGA TTG TAA                                      459
   Asn Gly Phe Gly Ser Thr Gly Leu  *
   145                 150
(2) INFORMATION FOR SEQ ID NO:16:
   (i) SEQUENCE CHARACTERISTICS:
      (A) LENGTH: 153 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Lys Arg Glu Glu Asp Ala Gly Tyr Asp Ile Pro Ser Pro Asn Leu Val
     1               5                  10                  15
   Gln Ile Lys Pro Gly Tyr Ser Tyr Leu Phe Cys Leu Pro Ile Phe Gln
                20                  25                  30
   Leu Glu Met Lys Asn Pro Pro Ile Ala Cys Ile Phe Gly Arg Ser Ser
            35                  40                  45
   Leu Asn Ser Ser Gly Ile Ile Val Leu Pro Thr Ile Trp Lys Pro Lys
        50                  55                  60
   Thr Ile Cys Gln Phe Phe Ile Lys Asn Ile Ser Ser Lys Thr Val Thr
    65                  70                  75                  80
   Ile Glu Lys Gly Gln Arg Ile Ala Gln Leu Val Leu Leu Lys Asn Asn
                    85                  90                  95
   Gln Pro Leu Trp Leu Gln Pro Gln Ile Asn Cys His Ser Leu Phe Pro
               100                 105                 110
   Lys Ser Asn Tyr Leu Ser Leu Ser Asn Arg Glu Cys Asp Met Trp Lys
           115                 120                 125
   Phe Thr Glu Asp Leu Asn Phe Glu Ala Pro Lys Ser Leu Arg Gly Ile
       130                 135                 140
   Asn Gly Phe Gly Ser Thr Gly Leu  *
   145                 150
(2) INFORMATION FOR SEQ ID NO:17:
   (i) SEQUENCE CHARACTERISTICS:
      (A) LENGTH: 579 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 54..503
   (xi) SEQUENCE DESCRIPTION: SEQ ID NO:17:
   AATTATTACT CTAAATCTCA CTTCATTATA CTTATATAAT AATATAAAAC CTT ATG       56
                                                             Met
                                                               1
   TTT GTC ATT ATT AAC TTA ACA CTA GAT GGT ACT ATA AAG CTA ACT TAC     104
   Phe Val Ile Ile Asn Leu Thr Leu Asp Gly Thr Ile Lys Leu Thr Tyr
                     5                  10                  15
   AAT ATA AAT AGT AAG ATT AGT TTA TAT AAA TTA CAT TTA ATG GCT TTA     152
   Asn Ile Asn Ser Lys Ile Ser Leu Tyr Lys Leu His Leu Met Ala Leu
               20                  25                  30
   CCA GAT AAC GTT TTT AGT ATT ATT AAT GAA AAT TAT ATC GAT GGA ATT     200
   Pro Asp Asn Val Phe Ser Ile Ile Asn Glu Asn Tyr Ile Asp Gly Ile
           35                  40                  45
   TTA ACT ATG AAA ATG GGT GAA GAA ATA GAA AGC TCA TCA CCA TTA AAT     248
   Leu Thr Met Lys Met Gly Glu Glu Ile Glu Ser Ser Ser Pro Leu Asn
    50                  55                  60                  65
   GAA ACA AAT GTT AAT ATA GAT CAA CAT ACA ATA GAT ATT TTT GAT TAC     296
   Glu Thr Asn Val Asn Ile Asp Gln His Thr Ile Asp Ile Phe Asp Tyr
                    70                  75                  80
   GAT TCA GAT AAT GGA TGT TAT TAT AGT GAA AGA GAT AAT GAA ACC GCA     344
   Asp Ser Asp Asn Gly Cys Tyr Tyr Ser Glu Arg Asp Asn Glu Thr Ala
                85                  90                  95
   ACT CTT TTT TTA AAA CGT GTT GGT TAT AGA GAA ACC TCA AAA AAG CGT     392
   Thr Leu Phe Leu Lys Arg Val Gly Tyr Arg Glu Thr Ser Lys Lys Arg
           100                 105                 110
   AAA CGG ATT TGT GGA TTT ATT GTT TTA GCA ATT TTT ATG GTT ATT ATA     440
   Lys Arg Ile Cys Gly Phe Ile Val Leu Ala Ile Phe Met Val Ile Ile
       115                 120                 125
   TTA TGT TTT TTA TCA ATA ATT TTG GGA GTT TTT ATA GCG CCT CAT ATT     488
   Leu Cys Phe Leu Ser Ile Ile Leu Gly Val Phe Ile Ala Pro His Ile
   130                 135                 140                 145
   TAT AAA GGC CTA TAG TAAGAACATC AACCTCTAAT AGGTAAATTT TTAAAACCAA     543
   Tyr Lys Gly Leu  *
                   150
   CGGAGGGGGA GGAGGAGGAC TTTTTATTTT ATGAGA                             579
(2) INFORMATION FOR SEQ ID NO:18:
   (i) SEQUENCE CHARACTERISTICS:
      (A) LENGTH: 150 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Met Phe Val Ile Ile Asn Leu Thr Leu Asp Gly Thr Ile Lys Leu Thr
    1               5                  10                  15
   Tyr Asn Ile Asn Ser Lys Ile Ser Leu Tyr Lys Leu His Leu Met Ala
               20                  25                  30
   Leu Pro Asp Asn Val Phe Ser Ile Ile Asn Glu Asn Tyr Ile Asp Gly
           35                  40                  45
   Ile Leu Thr Met Lys Met Gly Glu Glu Ile Glu Ser Ser Ser Pro Leu
       50                  55                  60
   Asn Glu Thr Asn Val Asn Ile Asp Gln His Thr Ile Asp Ile Phe Asp
   65                  70                  75                  80
   Tyr Asp Ser Asp Asn Gly Cys Tyr Tyr Ser Glu Arg Asp Asn Glu Thr
                   85                  90                  95
   Ala Thr Leu Phe Leu Lys Arg Val Gly Tyr Arg Glu Thr Ser Lys Lys
               100                 105                 110
   Arg Lys Arg Ile Cys Gly Phe Ile Val Leu Ala Ile Phe Met Val Ile
           115                 120                 125
   Ile Leu Cys Phe Leu Ser Ile Ile Leu Gly Val Phe Ile Ala Pro His
       130                 135                 140
   Ile Tyr Lys Gly Leu  *
   145                 150
(2) INFORMATION FOR SEQ ID NO:19:
   (i) SEQUENCE CHARACTERISTICS:
      (A) LENGTH: 579 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:19:
   TCTCATAAAA TAAAAAGTCC TCCTCCTCCC CCTCCGTTGG TTTTAAAAAT TTACCTATTA      60
   GAGGTTGATG TTCTTACTAT AGGCCTTTAT AAATATGAGG CGCTATAAAA ACTCCCAAAA     120
   TTATTGATAA AAAACATAAT ATAATAACCA TAAAAATTGC TAAAACAATA AATCCACAAA     180
   TCCGTTTACG CTTTTTTGAG GTTTCTCTAT AACCAACACG TTTTAAAAAA AGAGTTGCGG     240
   TTTCATTATC TCTTTCACTA TAATAACATC CATTATCTGA ATCGTAATCA AAAATATCTA     300
   TTGTATGTTG ATCTATATTA ACATTTGTTT CATTTAATGG TGATGAGCTT TCTATTTCTT     360
   CACCCATTTT CATAGTTAAA ATTCCATCGA TATAATTTTC ATTAATAATA CTAAAAACGT     420
   TATCTGGTAA AGCCATTAAA TGTAATTTAT ATAAACTAAT CTTACTATTT ATATTGTAAG     480
   TTAGCTTTAT AGTACCATCT AGTGTTAAGT TAATAATGAC AAACATAAGG TTTTATATTA 540
   TTATATAAGT ATAATGAAGT GAGATTTAGA GTAATAATT 579
(2) INFORMATION FOR SEQ ID NO:20:
   (i) SEQUENCE CHARACTERISTICS:
      (A) LENGTH: 450 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:20:
   ATGTTTGTCA TTATTAACTT AACACTAGAT GGTACTATAA AGCTAACTTA CAATATAAAT 60
   AGTAAGATTA GTTTATATAA ATTACATTTA ATGGCTTTAC CAGATAACGT TTTTAGTATT 120
   ATTAATGAAA ATTATATCGA TGGAATTTTA ACTATGAAAA TGGGTGAAGA AATAGAAAGC 180
   TCATCACCAT TAAATGAAAC AAATGTTAAT ATAGATCAAC ATACAATAGA TATTTTTGAT 240
   TACGATTCAG ATAATGGATG TTATTATAGT GAAAGAGATA ATGAAACCGC AACTCTTTTT 300
   TTAAAACGTG TTGGTTATAG AGAAACCTCA AAAAAGCGTA AACGGATTTG TGGATTTATT 360
   GTTTTAGCAA TTTTTATGGT TATTATATTA TGTTTTTTAT CAATAATTTT GGGAGTTTTT 420
   ATAGCGCCTC ATATTTATAA AGGCCTATAG 450
(2) INFORMATION FOR SEQ ID NO:21:
   (i) SEQUENCE CHARACTERISTICS:
      (A) LENGTH: 294 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 3..293
   (ix) FEATURE:
      (A) NAME/KEY: R = A or G
      (B) LOCATION: 49
   (xi) SEQUENCE DESCRIPTION: SEQ ID NO:21:
   TC CAA AGT GTT TTT GTT TCA TTG TCT TAT TCT TGG AGC CAC CGA CGA          47
      Gln Ser Val Phe Val Ser Leu Ser Tyr Ser Trp Ser His Arg Arg
       1               5                  10                  15
   CRG TTT GAG TGT ATA TTT CAT CCA ATT TTA TTT AAT CAT GGT ATT GTG         95
   Xaa Phe Glu Cys Ile Phe His Pro Ile Leu Phe Asn His Gly Ile Val
                   20                  25                  30
   AAT TTG GAA AAT AAC CCT TTG ACA TTT AAG GAA CTA CAA AAA ATA AAT        143
   Asn Leu Glu Asn Asn Pro Leu Thr Phe Lys Glu Leu Gln Lys Ile Asn
               35                  40                  45
   TAT AGA CGT CAT ATT CTT GGT TTA CCA TTG ATT AGA GCT GGA TTG GTA        191
   Tyr Arg Arg His Ile Leu Gly Leu Pro Leu Ile Arg Ala Gly Leu Val
           50                  55                  60
   GAA GAA GAT AAT CAA CCT TTA ATG ATA CCT CCA GAG TTT TCC AGT AAA        239
   Glu Glu Asp Asn Gln Pro Leu Met Ile Pro Pro Glu Phe Ser Ser Lys
       65                  70                  75
   CTA CCT CGA ACA ATA GGA TTT TTA ACT CAA CAA ATT AGA GCC AAA ATG        287
   Leu Pro Arg Thr Ile Gly Phe Leu Thr Gln Gln Ile Arg Ala Lys Met
   80                  85                  90                  95
   GAA GCT T                                                              294
   Glu Ala
(2) INFORMATION FOR SEQ ID NO:22:
   (i) SEQUENCE CHARACTERISTICS:
      (A) LENGTH: 97 amino acids
      (B) TYPE: amino acid
      (D) TOPOLOGY: linear
   (ii) MOLECULE TYPE: protein
   (ix) NAME/KEY: Xaa = Arg or Gln LOCATION: 16
   (xi) SEQUENCE DESCRIPTION: SEQ ID NO:22:
   Gln Ser Val Phe Val Ser Leu Ser Tyr Ser Trp Ser His Arg Arg Xaa
    1               5                  10                  15
   Phe Glu Cys Ile Phe His Pro Ile Leu Phe Asn His Gly Ile Val Asn
               20                  25                  30
   Leu Glu Asn Asn Pro Leu Thr Phe Lys Glu Leu Gln Lys Ile Asn Tyr
           35                  40                  45
   Arg Arg His Ile Leu Gly Leu Pro Leu Ile Arg Ala Gly Leu Val Glu
       50                  55                  60
   Glu Asp Asn Gln Pro Leu Met Ile Pro Pro Glu Phe Ser Ser Lys Leu
   65                  70                  75                  80
   Pro Arg Thr Ile Gly Phe Leu Thr Gln Gln Ile Arg Ala Lys Met Glu
                   85                  90                  95
   Ala
(2) INFORMATION FOR SEQ ID NO:23:
   (i) SEQUENCE CHARACTERISTICS:
      (A) LENGTH: 294 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:23:
   AAGCTTCCAT TTTGGCTCTA ATTTGTTGAG TTAAAAATCC TATTGTTCGA GGTAGTTTAC    60
   TGGAAAACTC TGGAGGTATC ATTAAAGGTT GATTATCTTC TTCTACCAAT CCAGCTCTAA   120
   TCAATGGTAA ACCAAGAATA TGACGTCTAT AATTTATTTT TTGTAGTTCC TTAAATGTCA   180
   AAGGGTTATT TTCCAAATTC ACAATACCAT GATTAAATAA AATTGGATGA AATATACACT   240
   CAAACYGTCG TCGGTGGCTC CAAGAATAAG ACAATGAAAC AAAAACACTT TGGA         294
(2) INFORMATION FOR SEQ ID NO:24:
   (i) SEQUENCE CHARACTERISTICS:
      (A) LENGTH: 291 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
   CAAAGTGTTT TTGTTTCATT GTCTTATTCT TGGAGCCACC GACGACRGTT TGAGTGTATA      60
   TTTCATCCAA TTTTATTTAA TCATGGTATT GTGAATTTGG AAAATAACCC TTTGACATTT     120
   AAGGAACTAC AAAAAATAAA TTATAGACGT CATATTCTTG GTTTACCATT GATTAGAGCT     180
   GGATTGGTAG AAGAAGATAA TCAACCTTTA ATGATACCTC CAGAGTTTTC CAGTAAACTA     240
   CCTCGAACAA TAGGATTTTT AACTCAACAA ATTAGAGCCA AAATGGAAGC T              291
(2) INFORMATION FOR SEQ ID NO:25:
   (i) SEQUENCE CHARACTERISTICS:
      (A) LENGTH: 146 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..146
   (xi) SEQUENCE DESCRIPTION: SEQ ID NO:25:
   CTA GAA GAT TAT ATA ACA CAT CGA ATT AAT GCC GAT ATT TCA GAG GTT        48
   Leu Glu Asp Tyr Ile Thr His Arg Ile Asn Ala Asp Ile Ser Glu Val
     1               5                  10                  15
   GGT GTA TTG AGA AAT TAT ATT TCT GCT GAT AGA CAG AGT TTA AAA GTT        96
   Gly Val Leu Arg Asn Tyr Ile Ser Ala Asp Arg Gln Ser Leu Lys Val
                20                  25                  30
   TCT GAT AGA GAG TTT ATT AAT TAT ATT TAC TTG GCA CAT TTT GAA AGC  TT
   146
   Ser Asp Arg Glu Phe Ile Asn Tyr Ile Tyr Leu Ala His Phe Glu Ser
           35                  40                  45
(2) INFORMATION FOR SEQ ID NO:26:
   (i) SEQUENCE CHARACTERISTICS:
      (A) LENGTH: 48 amino acids
      (B) TYPE: amino acid
      (D) TOPOLOGY: linear
   (ii) MOLECULE TYPE: protein
   (xi) SEQUENCE DESCRIPTION: SEQ ID NO:26:
   Leu Glu Asp Tyr Ile Thr His Arg Ile Asn Ala Asp Ile Ser Glu Val
    1               5                  10                  15
   Gly Val Leu Arg Asn Tyr Ile Ser Ala Asp Arg Gln Ser Leu Lys Val
               20                  25                  30
   Ser Asp Arg Glu Phe Ile Asn Tyr Ile Tyr Leu Ala His Phe Glu Ser
           35                  40                  45
(2) INFORMATION FOR SEQ ID NO:27:
   (i) SEQUENCE CHARACTERISTICS:
      (A) LENGTH: 146 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:27:
   AAGCTTTCAA AATGTGCCAA GTAAATATAA TTAATAAACT CTCTATCAGA AACTTTTAAA     60
   CTCTGTCTAT CAGCAGAAAT ATAATTTCTC AATACACCAA CCTCTGAAAT ATCGGCATTA    120
   ATTCGATGTG TTATATAATC TTCTAG                                         146
(2) INFORMATION FOR SEQ ID NO:28:
   (i) SEQUENCE CHARACTERISTICS:
      (A) LENGTH: 144 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
   CTAGAAGATT ATATAACACA TCGAATTAAT GCCGATATTT CAGAGGTTGG TGTATTGAGA     60
   AATTATATTT CTGCTGATAG ACAGAGTTTA AAAGTTTCTG ATAGAGAGTT TATTAATTAT    120
   ATTTACTTGG CACATTTTGA AAGC                                           144
(2) INFORMATION FOR SEQ ID NO:29:
   (i) SEQUENCE CHARACTERISTICS:
      (A) LENGTH: 161 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 3..161
   (xi) SEQUENCE DESCRIPTION: SEQ ID NO:29:
   TT ATG TCA GTG GAC GTT ATA TTT CTC GAT GAC CAA CAT CTG TCA GTA          47
      Met Ser Val Asp Val Ile Phe Leu Asp Asp Gln His Leu Ser Val
       1               5                  10                  15
   AAT AAT TAT AGC GGA ACT ATT GAG TTT ATT CAT TTT AAT AAC TCT TGT         95
   Asn Asn Tyr Ser Gly Thr Ile Glu Phe Ile His Phe Asn Asn Ser Cys
                   20                  25                  30
   TAT ACC GTT TAT CAA ACT ATT GAA TAT TTT TCT TGT CCT CGC ATT TTT        143
   Tyr Thr Val Tyr Gln Thr Ile Glu Tyr Phe Ser Cys Pro Arg Ile Phe
               35                  40                  45
   AAT AAT GCT TTT AGA TCT                                                161
   Asn Asn Ala Phe Arg Ser
           50
(2) INFORMATION FOR SEQ ID NO:30:
   (i) SEQUENCE CHARACTERISTICS:
      (A) LENGTH: 53 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Met Ser Val Asp Val Ile Phe Leu Asp Asp Gln His Leu Ser Val Asn
    1               5                  10                  15
   Asn Tyr Ser Gly Thr Ile Glu Phe Ile His Phe Asn Asn Ser Cys Tyr
               20                  25                  30
   Thr Val Tyr Gln Thr Ile Glu Tyr Phe Ser Cys Pro Arg Ile Phe Asn
           35                  40                  45
   Asn Ala Phe Arg Ser
       50
(2) INFORMATION FOR SEQ ID NO:31:
   (i) SEQUENCE CHARACTERISTICS:
      (A) LENGTH: 161 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:31:
   AGATCTAAAA GCATTATTAA AAATGCGAGG ACAAGAAAAA TATTCAATAG TTTGATAAAC 60
   GGTATAACAA GAGTTATTAA AATGAATAAA CTCAATAGTT CCGCTATAAT TATTTACTGA 120
   CAGATGTTGG TCATCGAGAA ATATAACGTC CACTGACATA A 161
(2) INFORMATION FOR SEQ ID NO:32:
   (i) SEQUENCE CHARACTERISTICS:
      (A) LENGTH: 159 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:32:
   ATGTCAGTGG ACGTTATATT TCTCGATGAC CAACATCTGT CAGTAAATAA TTATAGCGGA 60
   ACTATTGAGT TTATTCATTT TAATAACTCT TGTTATACCG TTTATCAAAC TATTGAATAT 120
   TTTTCTTGTC CTCGCATTTT TAATAATGCT TTTAGATCT 159
(2) INFORMATION FOR SEQ ID NO:33:
   (i) SEQUENCE CHARACTERISTICS:
      (A) LENGTH: 261 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..261
   (xi) SEQUENCE DESCRIPTION: SEQ ID NO:33:
   GGT GGT GCC CAA GCC ATA AAA ATG ACT TCA GAA AAT AAT ATT GTT ATG          48
   Gly Gly Ala Gln Ala Ile Lys Met Thr Ser Glu Asn Asn Ile Val Met
    1               5                  10                  15
   GCA GAT GTA GCG GTT TTA GAA AAA ACC CTT GGT ATA GAT TCT AAT ACA          96
   Ala Asp Val Ala Val Leu Glu Lys Thr Leu Gly Ile Asp Ser Asn Thr
               20                  25                  30
   ACA AAA CCC TTA TCT CTT CAA ACC GTT AAA TAT GAT GAA AAT AAT TCT         144
   Thr Lys Pro Leu Ser Leu Gln Thr Val Lys Tyr Asp Glu Asn Asn Ser
           35                  40                  45
   ACA GAT ATA CTA GAT ACA GAA CCT CTA ATA AGT TCT ATG GAA ACT GAA         192
   Thr Asp Ile Leu Asp Thr Glu Pro Leu Ile Ser Ser Met Glu Thr Glu
       50                  55                  60
   AAT ATT TTA TCG ACT AAA CAA AAA AGA AAC GGT CCC AAA AAA ATG ACA         240
   Asn Ile Leu Ser Thr Lys Gln Lys Arg Asn Gly Pro Lys Lys Met Thr
    65                 70                  75                  80
   CGT GAA AGT TTG GCA CTT TAA                                             261
   Arg Glu Ser Leu Ala Leu  *
                   85
(2) INFORMATION FOR SEQ ID NO:34:
   (i) SEQUENCE CHARACTERISTICS:
      (A) LENGTH: 87 amino acids
      (B) TYPE: amino acid
      (D) TOPOLOGY: linear
   (ii) MOLECULE TYPE: protein
   (xi) SEQUENCE DESCRIPTION: SEQ ID NO:34:
   Gly Gly Ala Gln Ala Ile Lys Met Thr Ser Glu Asn Asn Ile Val Met
    1               5                  10                  15
   Ala Asp Val Ala Val Leu Glu Lys Thr Leu Gly Ile Asp Ser Asn Thr
               20                  25                  30
   Thr Lys Pro Leu Ser Leu Gln Thr Val Lys Tyr Asp Glu Asn Asn Ser
           35                  40                  45
   Thr Asp Ile Leu Asp Thr Glu Pro Leu Ile Ser Ser Met Glu Thr Glu
       50                  55                  60
   Asn Ile Leu Ser Thr Lys Gln Lys Arg Asn Gly Pro Lys Lys Met Thr
    65                 70                  75                  80
   Arg Glu Ser Leu Ala Leu  *
                   85
(2) INFORMATION FOR SEQ ID NO:35:
   (i) SEQUENCE CHARACTERISTICS:
      (A) LENGTH: 280 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 2..280
   (xi) SEQUENCE DESCRIPTION: SEQ ID NO:35:
   T GCA TTA AAT TTT ATT AAA TTA GAA AAA AAT AAT CCA GTA TAT TAT          46
     Ala Leu Asn Phe Ile Lys Leu Glu Lys Asn Asn Pro Val Tyr Tyr
       1               5                  10                  15
   TTT CCG GAA CCT ATG GCA TTC TGG CGT ATC ATC CTA GAA ACA GAT ATT        94
   Phe Pro Glu Pro Met Ala Phe Trp Arg Ile Ile Leu Glu Thr Asp Ile
                    20                  25                  30
   GTG CAA GGT ATA TAC TCA GTA CAA GAC CGG AAG CTG CGT GGT GAA TTA       142
   Val Gln Gly Ile Tyr Ser Val Gln Asp Arg Lys Leu Arg Gly Glu Leu
                35                  40                  45
   AGC CTA AAT GAT GCG TCA TTA ATT ACA GCT CAA CTT CAA ACT AAA TTT       190
   Ser Leu Asn Asp Ala Ser Leu Ile Thr Ala Gln Leu Gln Thr Lys Phe
            50                  55                  60
   TCT ACG CCA TAT ATT TTA CTT CAT TCC AAT GTA TCC AAA TTT TTT GGA       238
   Ser Thr Pro Tyr Ile Leu Leu His Ser Asn Val Ser Lys Phe Phe Gly
        65                  70                  75
   GAA AAT GTA ACA TTT GGA ATT CCG GAA GTA ATA TTT ATT TTT               280
   Glu Asn Val Thr Phe Gly Ile Pro Glu Val Ile Phe Ile Phe
    80                  85                  90
(2) INFORMATION FOR SEQ ID NO:36:
   (i) SEQUENCE CHARACTERISTICS:
      (A) LENGTH: 93 amino acids
      (B) TYPE: amino acid
      (D) TOPOLOGY: linear
   (ii) MOLECULE TYPE: protein
   (xi) SEQUENCE DESCRIPTION: SEQ ID NO:36:
   Ala Leu Asn Phe Ile Lys Leu Glu Lys Asn Asn Pro Val Tyr Tyr Phe
     1               5                  10                  15
   Pro Glu Pro Met Ala Phe Trp Arg Ile Ile Leu Glu Thr Asp Ile Val
                20                  25                  30
   Gln Gly Ile Tyr Ser Val Gln Asp Arg Lys Leu Arg Gly Glu Leu Ser
            35                  40                  45
   Leu Asn Asp Ala Ser Leu Ile Thr Ala Gln Leu Gln Thr Lys Phe Ser
        50                  55                  60
   Thr Pro Tyr Ile Leu Leu His Ser Asn Val Ser Lys Phe Phe Gly Glu
    65                 70                  75                  80
   Asn Val Thr Phe Gly Ile Pro Glu Val Ile Phe Ile Phe
                   85                  90
(2) INFORMATION FOR SEQ ID NO:37:
   (i) SEQUENCE CHARACTERISTICS:
      (A) LENGTH: 280 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:37:
   AAAAATAAAT ATTACTTCCG GAATTCCAAA TGTTACATTT TCTCCAAAAA ATTTGGATAC    60
   ATTGGAATGA AGTAAAATAT ATGGCGTAGA AAATTTAGTT TGAAGTTGAG CTGTAATTAA   120
   TGACGCATCA TTTAGGCTTA ATTCACCACG CAGCTTCCGG TCTTGTACTG AGTATATACC   180
   TTGCACAATA TCTGTTTCTA GGATGATACG CCAGAATGCC ATAGGTTCCG GAAAATAATA   240
   TACTGGATTA TTTTTTTCTA ATTTAATAAA ATTTAATGCA                         280
(2) INFORMATION FOR SEQ ID NO:38:
   (i) SEQUENCE CHARACTERISTICS:
      (A) LENGTH: 279 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:38:
   GCATTAAATT TTATTAAATT AGAAAAAAAT AATCCAGTAT ATTATTTTCC GGAACCTATG    60
   GCATTCTGGC GTATCATCCT AGAAACAGAT ATTGTGCAAG GTATATACTC AGTACAAGAC   120
   CGGAAGCTGC GTGGTGAATT AAGCCTAAAT GATGCGTCAT TAATTACAGC TCAACTTCAA   180
   ACTAAATTTT CTACGCCATA TATTTTACTT CATTCCAATG TATCCAAATT TTTTGGAGAA   240
   AATGTAACAT TTGGAATTCC GGAAGTAATA TTTATTTTT                          279
(2) INFORMATION FOR SEQ ID NO:39:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..27̅
      (D) OTHER INFORMATION: /label= primer
   (ix) FEATURE:
      (A) NAME/KEY: N = Inosine
      (B) LOCATION: 11
   (ix) FEATURE:
      (A) NAME/KEY: N = Inosine
      (B) LOCATION: 17
   (ix) FEATURE:
      (A) NAME/KEY: N = Inosine
      (B) LOCATION: 20
   (xi) SEQUENCE DESCRIPTION: SEQ ID NO:39:
      GGCGAATTCC NAARMGNGAN GARGAYG 27
(2) INFORMATION FOR SEQ ID NO:40:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..24̅
      (D) OTHER INFORMATION: /label= primer
   (ix) FEATURE:
      (A) NAME/KEY: N = Inosine
      (B) LOCATION: 11
   (ix) FEATURE:
      (A) NAME/KEY: N = Inosine
      (B) LOCATION: 16
   (ix) FEATURE:
      (A) NAME/KEY: N = Inosine
      (B) LOCATION: 22
   (xi) SEQUENCE DESCRIPTION: SEQ ID NO:40:
      CGCGGATCCG NTNSWNCCYA ANCC 24
(2) INFORMATION FOR SEQ ID NO:41:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..26̅
      (D) OTHER INFORMATION: /label= primer
   (ix) FEATURE:
      (A) NAME/KEY: N = Inosine
      (B) LOCATION: 18
   (ix) FEATURE:
      (A) NAME/KEY: N = Inosine
      (B) LOCATION: 24
   (xi) SEQUENCE DESCRIPTION: SEQ ID NO:41:
      GGCGAATTCT AYCAYWSNCA YGTNTA 26
(2) INFORMATION FOR SEQ ID NO:42:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..3O̅
      (D) OTHER INFORMATION: /label= primer
   (ix) FEATURE:
      (A) NAME/KEY: N = Inosine
      (B) LOCATION: 16
   (ix) FEATURE:
      (A) NAME/KEY: N = Inosine
      (B) LOCATION: 19
   (ix) FEATURE:
      (A) NAME/KEY: N = Inosine
      (B) LOCATION: 24
   (ix) FEATURE:
      (A) NAME/KEY: N = Inosine
      (B) LOCATION: 25
   (ix) FEATURE:
      (A) NAME/KEY: N = Inosine
      (B) LOCATION: 28
   (xi) SEQUENCE DESCRIPTION: SEQ ID NO:42:
      CGCGGATCCR TCRTTNSWNG GDANNSWNGT 30
(2) INFORMATION FOR SEQ ID NO:43:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..2O̅
      (D) OTHER INFORMATION: /label= primer
   (ix) FEATURE:
      (A) NAME/KEY: N = Inosine
      (B) LOCATION: 12
   (ix) FEATURE:
      (A) NAME/KEY: N = Inosine
      (B) LOCATION: 18
   (ix) FEATURE:
      (A) NAME/KEY: N = Inosine
      (B) LOCATION: 21
   (xi) SEQUENCE DESCRIPTION: SEQ ID NO:43:
      GGCGAATTCG GNAARWSNAC NRC 23
(2) INFORMATION FOR SEQ ID NO:44:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..2O̅
      (D) OTHER INFORMATION: /label= primer
   (ix) FEATURE:
      (A) NAME/KEY: N = Inosine
      (B) LOCATION: 15
   (xi) SEQUENCE DESCRIPTION: SEQ ID NO:44:
      GGCGGATCCG GTTGNCKRTC 20
(2) INFORMATION FOR SEQ ID NO:45:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..3O̅
      (D) OTHER INFORMATION: /label= label
   (xi) SEQUENCE DESCRIPTION: SEQ ID NO:45:
      CGCGGATCCA AGGTAATAAG TCAAAATGAG 30
(2) INFORMATION FOR SEQ ID NO:46:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..29̅
      (D) OTHER INFORMATION: /label= primer
   (xi) SEQUENCE DESCRIPTION: SEQ ID NO:46:
      CGCGGATCCG ACAAAAACAA AAAGTAATG 29
(2) INFORMATION FOR SEQ ID NO:47:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..26̅
      (D) OTHER INFORMATION: /label= primer
   (ix) FEATURE:
      (A) NAME/KEY: N = Inosine
      (B) LOCATION: 11
   (ix) FEATURE:
      (A) NAME/KEY: N = Inosine
      (B) LOCATION: 20
   (xi) SEQUENCE DESCRIPTION: SEQ ID NO:47:
      CCGAATTCYT NATGATHYTN ATHGARGG 28
(2) INFORMATION FOR SEQ ID NO:48:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..29̅
      (D) OTHER INFORMATION: /label= primer
   (ix) FEATURE:
      (A) NAME/KEY: N = Inosine
      (B) LOCATION: 21
   (xi) SEQUENCE DESCRIPTION: SEQ ID NO:48:
      CCGGATCCYT CRAARAARTT NGTRTGYTT 29
(2) INFORMATION FOR SEQ ID NO:49:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..35̅
      (D) OTHER INFORMATION: /label= primer
   (xi) SEQUENCE DESCRIPTION: SEQ ID NO:49:
      TCCCCCGGGG GCGCGCCTTG ACATTGATTA TTGAC 35
(2) INFORMATION FOR SEQ ID NO:50:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..35̅
      (D) OTHER INFORMATION: /label= primer
   (xi) SEQUENCE DESCRIPTION: SEQ ID NO:50:
      GCCCTTAAGG GGCGCGCCAA TGCGATGCAA TTTCC 35
(2) INFORMATION FOR SEQ ID NO:51:
   (i) SEQUENCE CHARACTERISTICS:
      (A) LENGTH: 10592 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:51:
   CTGCAGTGTA TTTAAAAAAT AAAAATCTAT GAATGAAATC TATGAATGAA ATCTATGAAT 60
   GAAATCTATG AATGAAATCT ATGAATGAAA TCTATGAATG AAATCTATGA ATGAAATCTA 120
   TGAATGAAAT CTATGAATGA AATCTATGAA TGAAATCTAT GAATGAAATC TATGAATGAA 180
   ATCTATGAAT GAAATCTATG AATGAAATCT ATGAATGAAA TCTATGAATG AAATCTATGA 240
   ATGAAATCTA TGAATGAAAT CTATGAATGA AATCTATGAA TGAAATCTAT GAGACAAAGT 300
   AATTTTTAAA AATATTTTAA ATTTTATTAA GAGTATAGGT TACAAGGTTT AATGCGTTGG 360
   GTAAACATTT TAGTTTTCAA GTTTTAGTTT TCTGGTATCT ACCAACACAA ATGCATCTTC 420
   GGATACATTA TTTAGAGAGT AATCACTTTT TAGAATATAT CTTATTGGTG GTACATTTAT 480
   AAATTTTGGA CCATCCCAAT AACACTTCGA TTCCACAAGC GAAGAAGGTA CTTCCATAAG 540
   CTGAGAAGCG TTTACTTGAT TGTAGGGAGA ACTTGGCGTT TCAAAATCCT TTAGAACGTA 600
   TAGTCTGCAA TACATAGGTT CAATATCATC TTCATACCTC TCATCAGGAT ATGAAAATGG 660
   AAGTTTCACA AAGGTTCCAT CACGAAGCTT TTTGAAGAAT CGTACATCCG TAGGTGGTGT 720
   AGGAACAATA GTGAAGGCGT GCGGTTCACC CTGGTTTTTC ACACGTGCAA GCGCTGGTGT 780
   GGTTTTAGGG CGAACTGGAA AATAACCAGG CGGAACTTGT GCGTAGAATC CTTCATTAAG 840
   TTCTCCACGA CAGCGTCTGA AGTATGATGG CATATTAGCT TGATCGGAGT TGTTATCAAA 900
   TAGAGCAAAT GAAGCACTCA TTTTAAAACT TTTTAGTTAA GCTTTAAAAA CAAGTGAAGA 960
   TTTAAAAATG TAGGATAAAA TGCCAGTTTA TATACAGTAA GAATATGGGA GTGGTTCACA 1020
   TAAAAAACCA GAATTTCAGG TTTACATCTA CTGTTTATTC ACAACAAATA TAAACAAACT 1080
   TAGTTTCCAC ATAAACATGA ACTAAATAGA GATGAACGTT GAGCGTTGGT AGGTTGTGTA 1140
   GAAGACATAC CATCGTTTTC ATTTTTGGTT ATTGTTTTGG CGCGCCTTGA AAATAATCGT 1200
   TTAAAAATAT TTGGTTTGGA TAGCCTTTTC ATAGGTTTCA CCCCATGCAA GTCATCCTCT 1260
   TCTGGTTCAG GAATTTCTTC ATAACCATTA TGGGATATTA TTGCACACAT AAATGATTCG 1320
   ATTACCGGGG GGGCAGAACG TGTCTCATTT ATATAAAGAG AATCACATAC ATCGCTTATA 1380
   GAACATGTAG AACTGTCAGA ATCCTCTTTA AAACTATTTT TAATTTCACA ATTAGTTTCT 1440
   TCTAGTTCAT TATCCACCAT CGCATTAGCG TATTTCCAAA TATCATTCTC TGAGGAATAA 1500
   TGAGATGCAG AGCATGAAGA AGAGGATGAG GAGGAGGAGG ATGAAGATGA GGATATAGAG 1560
   GGACATCTTG GAGAGCTTTC AAAGTTGAAT GGAGTATTAA ATGTTGTACC ATAAAAAATG 1620
   TCACTTAACA TAGGGGGTAC TTTAAAGGAG GACAGAAAGG TGTCTAATAC AGGTACCCAT 1680
   ATAAACGAGG GGCAATAAAC ACTCCCAGAA TCATCGATAT GTTTTACATT ATTTTTGGAA 1740
   ATCTCAAGAC ACTCAGGTTT CCAGGATGGT TCCGGCCATT CACATGATAC ATATGCATAA 1800
   ATTAGTCGCT TTGGTCCTGG GATATTAGAA ATGACTGGCT CACATAAATC CGCTGCACCG 1860
   AAAACCCATA GATTAAGAGG ATAGTTTCCA AATATACCAG AGTTTAGATA GTTATACCCC 1920
   GAAACAGCCG ATTTCCATTC GATGCTAGCC CCAGGTTTAT CCTCATAAAA TAAAAAGTCC 1980
   TCCTCCTCCC CCTCCGTTGG TTTTAAAAAT TTACTATTAG AGGTTGATGT TCTTACTATA 2040
   GGCCTTGAAA CTCTAGGTAG ATGTTTTATA GAGTCCATAA AATAACATAA GTTTGCAGAT 2100
   CGTAATATTA TAGGCATAGC CAATCGTGTG AGAGAAAGGA TATAGCATTG TCTAGCCATA 2160
   AAACACCAAA GATCAGGATG AACATCTTGG GAGTTTCCTG GTAACGCCCC ATTTTTGTCA 2220
   ATAAACGTAA CAATATTAAC TTCAACCACA CCCATAATTA AATTTTATGT ATGAATCCAA 2280
   TAAAGGTTAA TACACACCTA ATTTATGTTA TAATTTTAGA AGAAGCTGCA GTTGATGAGT 2340
   TGATATTAAC ATAACAATTT CACAATTACC TGATATGGCA AAGTGTACCA CCGAAAAGTT 2400
   TTGTTGTATC AGCGTGAATA GAGAATCTTC TGTCGATCCA GAAGACTTCT ATAAACCGGT 2460
   TCCTCTAACT TCAGATTTGA TTGAAGAGGA TAACCTACAT CAAGACAAAA TAATGGATGA 2520
   GGATTTATAC TCGGATTTTA GTGATGATGA CTTTATGGAT TATACAAAAA ATCCAACTGA 2580
   AAGTGAAAAT GAAAGAGAAA GTGACGAAGA AGTTGAAGAA AGTTATGAAA GTGATGAAGA 2640
   TAAAAAAAGT TTATCTCCTA CTAAAAGCGA AGGAATTGAA GCGGCTGAAG CGCTAAAGTT 2700
   TTCTGTTGTT AAATCGTTAA CGCCTGGGTC AGAAGGAAGA GTTTTTATTG CTCTTAAAAA 2760
   AGATAAAGAT ACAAGCTATA AGGTAATTTT AAAAATTGGA CAAAGGGGAA ACACGCTTGT 2820
   GGAATCGTTA ATTTTGAGAA ATATTAGTCA CCAATCTATA ATTAAACTTC AAGACACTCT 2880
   TTTTTATAAA GAGTTAACAT GTTTGGTGTT ACCGTATTAT AAATATGATC TATATAATTT 2940
   TTTAATGGAT CATGGGAAAT CTCTGTCTTT TGAATCTGTA ATTAAAATTG AAAAACAAAT 3000
   ATTAACTGGA CTTCAATATA TTCATGGAAA AAAAATTATT CATCGAGATA TAAAAACTGA 3060
   AAATATTTTC TTGGATAATG ACTCTAATGT TTGTATAGGT GATTTTGGGG CTTCTCAATT 3120
   TCCTGTTTCC TCACCAGATT ATTTGGGAAT TGCGGGGACT ATTGAAACTA ATGCTCCTGA 3180
   AGTTCTATCA AAGGATGCGT ACAACTGTAA AGCTGATATT TGGAGTGCTG GTATAATTTT 3240
   ATTTGAAATG CTTGCATATC CTAATGTTTT GTTTGAGGAG GAAGAAAGAG ATAGTAGCGA 3300
   TTTAATAAAC AATTGTAATC TTCATCTTAT AAAAATTATA TCAACTCTGA AGATTAACCC 3360
   AAATGAATTT CCATCTGATT TGGAATCTAA TCTAGTAAAA CATTTTATAA AATATGCTAA 3420
   TAATGATAGA CCTCCATTTA CACGATATAA TCGTCTAAAT AACCTTAAAT TACATCTCGA 3480
   TGGTGAATTT TTAATTCATA AAATGCTAAC ATTTGATGCA TCTCTACGAC CAAGTGCGGA 3540
   AGAACTATTA TCCTATCAGA TTTTTAGTAA ACAATAAATT TCATAAAAAT GGGCGTGGAA 3600
   TTTTTTATTG TTTTATATAA AACGGGTGTT TGAAAGCTCT TTTTTATTAA TTTTATTTTT 3660
   ACATCCTAGC TACAATATTA TAGTTATCAT GTTGTATACG CTGTTTTTTG TTTTTTATTT 3720
   TAAGGTAGTT TTATCTCGCA TAGCTCCGCT AGAGTTGTGT TATGCGGATC CTAAAGAAAA 3780
   TACAACTGAA CCTACACAAC TTCCTACAGG GGAACAATCT AAGACTCTTA TTCCCGTGGT 3840
   AACAAACGGA TATGTTGAAT ACTCTAAAGG ATGTGAACTA CGATTACTAG ATACATATGT 3900
   AAATGTATCT TCACGACCAG AAAAAAAGGT TAATGCTACA ATTGGATGGT CATTTGATCT 3960
   TGGTTGTCAA ATTCCTTTAA TTTATAGAGA ATATTATAAT TGTACTGGTA ATATAATACC 4020
   ATCACCAGAA ACTTGTGATG GTTATTCTTT AACTTTGGTA AAATCTGAAA GTATATCATC 4080
   TTATGCACTT GTTAATGTTA GTTTGCTTAT TCAACCAGGA ATTTTTGATT CTGGTAGATA 4140
   TTTATACTCA CTTGTTTTTG GAAACGATAG TTATAACGGA AGAATTGAAG TTCGAGTGGA 4200
   TAATGAGACA GACTATCCAT GTTTTATGAT GCATGGATTG ACTGTAAAAA AGGGTGATAA 4260
   ACTTCATATT CCTTATAAAC CATCCACAAA TCCTAATCAT AAACGATATA GAGGTTGTTT 4320
   TCCAATATCA AATACTGAGC TATGGAATAA TATTAGTGAT GAAAGTGTTG GTAGATATTC 4380
   ATATGATGAA GAATATGAAG AATATGAAGA AGAAAACGAA GATTTTGAAG ATCTACAATC 4440
   AAAAGATTGC CGCAAATCCA ATCTTTTTGA TATGAAGAAG ACTTTTAATT TGGCTGCAGG 4500
   TTCTCAAAGT TTATTGATTG CTAGTTTGGG TAAATCAATT TCAGAACAAC CGTGGTCATT 4560
   TAAAATTAAT GAAAGTTATG AACTTTTTAA TAATTTGTCT ATCACCCTTC AATCGGAAGA 4620
   AGATTCTAAT ATACTGAATC CTGAAATTGT AACGTTTACC ACACCACCAC CTACTGAAAA 4680
   TACACATATG TTTATGTCAA ATAATGAAAC TATGTATGAA GAAGAAAGTG TTTTAAGCAT 4740
   TATTCAATTG TTTAACAATG GTTATAATAA TTGTAATACC CATATAAAGG TAATTGGATT 4800
   TGGAACAATT ATCTTTATTA TTTTATTTTT TGTTGCTGTG TTTTTTTGTG GATATACTTG 4860
   TGTATTAAAC TCTCGTATTA AAATGATTAA CCATGCTTAT ATACAACCCC AGAAATTAAA 4920
   TTTTTATGAT ATTTAATAAA ACTATTATGA AACTTCTTAT AACTTATTTG TTTTTATTAA 4980
   ATGGGTTGGG TTGGTTTTAA AATTACATAC GTGTATTAAG AATTAACATC ATAAAGGACA 5040
   CACCCATGAA AAACATTTAA ATTCTATTAA TTTGAACGGA TTAAACATTT TCTCATTTTA 5100
   AGAGTTGCTA CGACTTTTGA TAGTAAAATG ATTAAACTTC TATTTATCTT ATTTTATTTT 5160
   AACCCAATAA CTGGATATAA ATGGGTAGAC CCTCCTCGTA GGTATAATTA CACCGTTTTA 5220
   AGAATGATTC CAGATATTCC AAATCCAATG GATCCTTCTA AAAACGCTGA AGTTCGGTAT 5280
   GTAACTTCTA CTGACCCATG TGATATGGTT GCTTTGATTT CTAATCCAAA TATAGAATCT 5340
   ACAATTAAAA CGATTCAATT TGTGCAAAAG AAAAAATTTT ACAATGCATC TCTTAGTTGG 5400
   TTTAAAGTTG GAGATGATTG TACATATCCA ATATATTTAA TTCAATATTT TGATTGTGAT 5460
   CCTCAAAGAG AATTTGGCAT ATGTTTAAAA AGATCTCCAG ATTTTTGGAA ACCATCGTTA 5520
   GTTGGTTACA CATTTTTAAC TGATGATGAA TTGGGATTAG TTTTAGCTGC CCCCGCTCCA 5580
   TTTAATCAAG GTCAATATAG ACGGGTTATT CAAATTGAAA ATGAAGTTTT TTATACTGAT 5640
   TTTATGGTTC AATTACCACG AGAAACTTGT TATTTTTCTA AAGAAGATAA ATTTGAACCA 5700
   ACTTTTATGG AATGGTGTAA GGAATCTAGA TCTGTAGGAG CATCAAAAGT TGACGATGAA 5760
   CTTTTTTATC TAAATAGAGC TGGTCCCCAA ACCCTGCTTA AATATTATGT TATTAAAGAT 5820
   TTTTATAGAC TTAACGGTAG AGAACCTCCA ATAAAATTTA AAGAAGCTCT TAGATACGAT 5880
   ATACCATATA AAGTGAATGA TAAATTTGAT GATGAATTAC CATCGAGGCC ACATATTAGT 5940
   AATACTATTA ATAAAACTAT TAAAGAAATT GTAAATCTTG AAGATTATTT TAAAAATACA 6000
   AATGTTATAG ATACTACTAC CCCAACACCA ATAAATAATA CCCCAAAAAA TATAACCGTG 6060
   GGAATTGTTA TAATTATATT AATAATACTA TTTATAATTG GATTTTTTGT TTATAAAAGA 6120
   CAAAAAATAT ATAATAATTA TAAAAAATTA ACAACAAATG TTTAGCCTTT ATAAATTAAT 6180
   TTACAGAATA AACAACTGGG CGGTCTTTTG TTTAATAAAA ATTCATGTAC CTACAACTTT 6240
   TATTCACTTG CAAGAGGGTT GAGACCAGAT TACTTATAAC TATGTTTCTA CCTATTTTAT 6300
   TTCTTTTTTT ATATGGTGTA AATGGATTTG TTTACAAAGG TACGTATATA AGTATGTTTT 6360
   TAAATACTAG TTCTGGCTTT TCTATTTTTC CCGATGATAA ATTTATTGTC AGTGGACGTT 6420
   TATTATTTCT CGATGACCAA CATCTGTCAG TAAATAATTA TAGCGGAACT ATTGAGTTTA 6480
   TTCATTTTAA TAACTCTTGT TATACCGTTT ATCAAACTAT TGAATATTTT TCTTGTCCTC 6540
   GCATTTTTAA TAATGCTTTT AGATCTTGTT TAAAAAAGGT ATCAAAACAT CATGAAAGTC 6600
   AACTTCGGAT AAATTCATCT ATAGAAAACG GTGTTTTGTT GGAAATTACA AATCCTAAAC 6660
   CAAATGATTC AGGTGTTTAT TTTATACGAG TTCAATTGGA AAATAATAAA ACAGATGTGT 6720
   TTGGAATACC TGCATTTATT TATTCCTTTA ATATGTCAAA CGAAGTAAAT AAATCAAACT 6780
   TCGATGATGT TACTACATCT TTATATACCT CATCACACCC TTCTTCCCAA ACTATTACAC 6840
   CTATCTATTT AAATGAAAAA CACGAACCGA TATGTCATAC TGTAAAAAAG GATGAAAATG 6900
   TGTATGAACT TTTACTAGGT TTGCATGGAA ATATAACTGA TGATATTTTT CTCGATGAGG 6960
   ATTCTGAATT GCTTAAAAGA GTAAATATAC CTACAACGAC AAATAATTAT ATATTTAAGC 7020
   CTTACCTAGA CCAACGTAAT AGAAAATTTT TAATTATTGT AATTTCGATT TCGATAATTT 7080
   TACTTATTCT TTTGGTATTA ATTGGATCAA TTATTAACAA TATTATTCGT AGACACTTTT 7140
   CTTCTTCTAG GCGTATTTAT CGTCCTAAAG GTAACTCGGA ATCTGAAAAT ATAGAACTGA 7200
   CATGTGGGGA AAACTCAGTA AACAAAAATA ATCCATTACC AAAAAAACCT AACCGCCAAA 7260
   AAAGATCTTC AACTATTCAA AGGGAGACAT CTCTTGAAAC TATTAAGGAA GAAGTATAAT 7320
   TTTAAAAATA TTTACCTACG TAGGTTGATG ACGACTTGTA TGACTAAAAA TTAGAATTTA 7380
   AATGATGAAA ATTTTTTAAA AATAATATAG TATTCCAAAG AGCCTTTTAG GAAAATCATC 7440
   AAGTCTCCAT TTCTCCAATC TTTACGATGT TTCGTTTATT TTTTTTAATC GCGTCTACAT 7500
   TATGTTCGGT AAGATTTGGT TTTTCAACAA TTCGTAATGT TATTGTTTCT GAAAAATCTG 7560
   GATTTGTAAT TGATGGTTAT AGTACTAACC CACCATTTAA TGAGACTAAA AAATTTACTA 7620
   GAGGATGGGT ATTTTTACAA ACCCCCCCTT CTTATTGTAA AGATGGGATA TCAATATCTA 7680
   ATATATGCAT TGAACGTAAT ATTTGTGAAG AAGATATTTT TTTGAATAAA CGATGTACAA 7740
   TTAAAACTAT TAATTATCCC TTAGCTGTAG CAGATTTTGA GATTAGTAAT AATACTATTA 7800
   AAAAAATAAA TGATGTTTAT TTTGTTAATG ATAGTGTTTT TCCAATAATA ACTACAAATA 7860
   AAAGTGGTAT CCATATCACA AATGTGACTA TAAATAATTC TGGAATTTAT ACATTGTATG 7920
   AAAATAATGA TAAGTGGAGT CATCAATCAA AAATCTTGGT AACTATAAAG AAAAAAGAAA 7980
   CAGTAATTAC TAAACCTAAA GTATATATAA AAAAACATGG TGGATTTTTT CATGTAAAAA 8040
   ATTATCACTC TCATGTATTT GTACCAAATG ATTCATTTAA AATTGAACTT AATCTTGAAT 8100
   CGGAAATTTA TGATTCTGAA TTTTCAGCAA GTATTGATTG GTATTATATG AAAACTAGCT 8160
   CGGAATGTTC AGTGTTTCAT ATATATGAAA CTTGTATATT TCACCCTCAT GCAAACTCTT 8220
   GTTTGAATCC AATAAACCCA TTGTGTAGTT TTACTTCCCC TTTGAGGGCA ACATCACTAA 8280
   TTAATAGATT TTATTTTAGA TGTAAACCTG AAGGTAAAAA CTGGACAACT GATTGTATAA 8340
   ACACCTTTTC TATTAATGCA GATAAACATA TTAAACAGCA TTCAAATAAT GTAGATTTGA 8400
   TTTTTTTAAA TACTCCAACT AATGCATCTG GTTTGTATGT TTTTATTCTT AAGTATAATG 8460
   GTCATCCAGA GGCTTGGACA TATACTTTGG TTTCAACGGT TAAAAATTTT ATGAATGTAA 8520
   TTAAGGATAT GACACGCCCC CTTTTGTCAA ATAATAAAAT GAAAAAACCT GAGCATTCTA 8580
   CTCAACCACC AACCATAACC AACATAACAC CTGGCTTTAA ATCTAAAAAT TGGGTAGATA 8640
   AATATATAAT TTCAGTAGCG GTGGTTTCTT GTATTACTAT TGTTATATTG ATTGTGGTAA 8700
   TAACCTTTTG TGTTCATCAA TGTATCGGTT TAAATCGTAA ACCATATGAA ATTATAAACC 8760
   CATTTAATAC AGCTTATAAA AGTATACCTA CAAATGAAAA AAATATTCTT CATTTTGCTG 8820
   AAGTAACAGA ATCTGATTAT TCCTCCGACG AATCCTTCGA CAGTGACTCA GAAGAGCTAA 8880
   ATCAACGAGG TGAAACAATA CAACAAGGGA AAAAGGAACA ATCTGGATAT ACTATTTGGT 8940
   TTAATGAAGA TTTAGAAGAA TCCGTCTCCA AAAAACTTAA CCAACCAAAC TATTCAAAAA 9000
   TAATTAATAG CTTAAAATCA ATCCAGAATG AATAAATCTA AACTCTCATT TAAAGAAAAA 9060
   AACGCTATAT ATGAATTTAA AAATATTTTA TCAAACACTT CATTGTCAAC TTTTCCTGTA 9120
   TTATCGTTTA ATGAGGAGCC AAAATCCAGA TTTTTTAAAA TGTTTAAAAA TATTTTACTG 9180
   GAAAAAATAA AAAAAACTTC AATGGATTAT TTAATTTATT GTACTCTAAA AATCTCACTT 9240
   TCATTTATAC TTTATAATAA ATAAAATTAT TAAAAAAACT TTATTGTTTT GTCATTATTA 9300
   ACTTTAACAC TAGATGGTAC TATAAAGCTA ACTTACAATA TAAATAGTAA GATTAGTTTA 9360
   TATAAATTAC ATTTAATGGC TTTACCAGAT AACGTTTTTA GTATTATTAA TGAAAATTAT 9420
   ATCGATGGAA TTTTAACTAT GAAAATGGGT GAAGAAATAG AAAGCTCATC ACCATTAAAT 9480
   GAAACAAATG TTAATATAGA TCAACATACA ATAGATATTT TTGATTACGA TTCAGATAAT 9540
   GGATGTTATT ATAGTGAAAG AGATAATGAA ACCGCAACTC TTTTTTTAAA ACGTGTTGGT 9600
   TATAGAGAAA CCTCAAAAAA GCGTAAACGG ATTTGTGGAT TTATTGTTTT AGCAATTTTT 9660
   ATGGTTATTA TATTATGTTT TTTATCAATA ATTTTGGGAG TTTTTATAGC GCCTCATATT 9720
   TATAAAGGCC TATAGTAAGA ACATCAACCT CTAATAGTAA ATTTTTAAAA CCAACGGAGG 9780
   GGGAGGAGGA GGACTTTTTA TTTTATGAGG ATAAACCTGG GGCTAGCATC GAATGGAAAT 9840
   CGGCTGTTTC GGGGTATAAC TATCTAAACT CTGGTATATT TGGAAACTAT CCTCTTAATC 9900
   TATGGGTTTT CGGTGCAGCG GATTTATGTG AGCCAGTCAT TTCTAATATC CCAGGACCAA 9960
   AGCGACTAAT TTATGCATAT GTATCATGTG AATGGCCGGA ACCATCCTGG AAACCTGAGT 10020
   GTCTTGAGAT TTCCAAAAAT AATGTAAAAC ATATCGATGA TTCTGGGAGT GTTTATTGCC 10080
   CCTCGTTTAT ATGGGTACCT GTATTAGACA CCTTTCTGTC CTCCTTTAAA GTACCCCCTA 10140
   TGTTAAGTGA CATTTTTTAT GGTACAACAT TTAATACTCC ATTCAACTTT GAAAGCTCTC 10200
   CAAGATGTCC CTCTATATCC TCATCTTCAT CCTCCTCCTC CTCATCCTCT TCTTCATGCT 10260
   CTGCATCTCA TTATTCCTCA GAGAATGATA TTTGGAAATA CGCTAATGCG ATGGTGGATA 10320
   ATGAACTAGA AGAAACTAAT TGTGAAATTA AAAATAGTTT TAAAGAGGAT TCTGACAGTT 10380
   CTACATGTTC TATAAGCGAT GTATGTGATT CTCTTTATAT AAATGAGACA CGTTCTGCCC 10440
   CCCCGGTAAT CGAATCATTT ATGTGTGCAA TAATATCCCA TAATGGTTAT GAAGAAATTC 10500
   CTGAACCAGA AGAGGATGAC TTGCATGGGG TGAAACCTAT GAAAAGGCTA TCCAAACCAA 10560
   ATATTTTTAA ACGATTATTT TCAAGGCGCG CC 10592
(2) INFORMATION FOR SEQ ID NO:52:
   (i) SEQUENCE CHARACTERISTICS:
      (A) LENGTH: 10592 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:52:
   GGCGCGCCTT GAAAATAATC GTTTAAAAAT ATTTGGTTTG GATAGCCTTT TCATAGGTTT 60
   CACCCCATGC AAGTCATCCT CTTCTGGTTC AGGAATTTCT TCATAACCAT TATGGGATAT 120
   TATTGCACAC ATAAATGATT CGATTACCGG GGGGGCAGAA CGTGTCTCAT TTATATAAAG 180
   AGAATCACAT ACATCGCTTA TAGAACATGT AGAACTGTCA GAATCCTCTT TAAAACTATT 240
   TTTAATTTCA CAATTAGTTT CTTCTAGTTC ATTATCCACC ATCGCATTAG CGTATTTCCA 300
   AATATCATTC TCTGAGGAAT AATGAGATGC AGAGCATGAA GAAGAGGATG AGGAGGAGGA 360
   GGATGAAGAT GAGGATATAG AGGGACATCT TGGAGAGCTT TCAAAGTTGA ATGGAGTATT 420
   AAATGTTGTA CCATAAAAAA TGTCACTTAA CATAGGGGGT ACTTTAAAGG AGGACAGAAA 480
   GGTGTCTAAT ACAGGTACCC ATATAAACGA GGGGCAATAA ACACTCCCAG AATCATCGAT 540
   ATGTTTTACA TTATTTTTGG AAATCTCAAG ACACTCAGGT TTCCAGGATG GTTCCGGCCA 600
   TTCACATGAT ACATATGCAT AAATTAGTCG CTTTGGTCCT GGGATATTAG AAATGACTGG 660
   CTCACATAAA TCCGCTGCAC CGAAAACCCA TAGATTAAGA GGATAGTTTC CAAATATACC 720
   AGAGTTTAGA TAGTTATACC CCGAAACAGC CGATTTCCAT TCGATGCTAG CCCCAGGTTT 780
   ATCCTCATAA AATAAAAAGT CCTCCTCCTC CCCCTCCGTT GGTTTTAAAA ATTTACTATT 840
   AGAGGTTGAT GTTCTTACTA TAGGCCTTTA TAAATATGAG GCGCTATAAA AACTCCCAAA 900
   ATTATTGATA AAAAACATAA TATAATAACC ATAAAAATTG CTAAAACAAT AAATCCACAA 960
   ATCCGTTTAC GCTTTTTTGA GGTTTCTCTA TAACCAACAC GTTTTAAAAA AAGAGTTGCG 1020
   GTTTCATTAT CTCTTTCACT ATAATAACAT CCATTATCTG AATCGTAATC AAAAATATCT 1080
   ATTGTATGTT GATCTATATT AACATTTGTT TCATTTAATG GTGATGAGCT TTCTATTTCT 1140
   TCACCCATTT TCATAGTTAA AATTCCATCG ATATAATTTT CATTAATAAT ACTAAAAACG 1200
   TTATCTGGTA AAGCCATTAA ATGTAATTTA TATAAACTAA TCTTACTATT TATATTGTAA 1260
   GTTAGCTTTA TAGTACCATC TAGTGTTAAA GTTAATAATG ACAAAACAAT AAAGTTTTTT 1320
   TAATAATTTT ATTTATTATA AAGTATAAAT GAAAGTGAGA TTTTTAGAGT ACAATAAATT 1380
   AAATAATCCA TTGAAGTTTT TTTTATTTTT TCCAGTAAAA TATTTTTAAA CATTTTAAAA 1440
   AATCTGGATT TTGGCTCCTC ATTAAACGAT AATACAGGAA AAGTTGACAA TGAAGTGTTT 1500
   GATAAAATAT TTTTAAATTC ATATATAGCG TTTTTTTCTT TAAATGAGAG TTTAGATTTA 1560
   TTCATTCTGG ATTGATTTTA AGCTATTAAT TATTTTTGAA TAGTTTGGTT GGTTAAGTTT 1620
   TTTGGAGACG GATTCTTCTA AATCTTCATT AAACCAAATA GTATATCCAG ATTGTTCCTT 1680
   TTTCCCTTGT TGTATTGTTT CACCTCGTTG ATTTAGCTCT TCTGAGTCAC TGTCGAAGGA 1740
   TTCGTCGGAG GAATAATCAG ATTCTGTTAC TTCAGCAAAA TGAAGAATAT TTTTTTCATT 1800
   TGTAGGTATA CTTTTATAAG CTGTATTAAA TGGGTTTATA ATTTCATATG GTTTACGATT 1860
   TAAACCGATA CATTGATGAA CACAAAAGGT TATTACCACA ATCAATATAA CAATAGTAAT 1920
   ACAAGAAACC ACCGCTACTG AAATTATATA TTTATCTACC CAATTTTTAG ATTTAAAGCC 1980
   AGGTGTTATG TTGGTTATGG TTGGTGGTTG AGTAGAATGC TCAGGTTTTT TCATTTTATT 2040
   ATTTGACAAA AGGGGGCGTG TCATATCCTT AATTACATTC ATAAAATTTT TAACCGTTGA 2100
   AACCAAAGTA TATGTCCAAG CCTCTGGATG ACCATTATAC TTAAGAATAA AAACATACAA 2160
   ACCAGATGCA TTAGTTGGAG TATTTAAAAA AATCAAATCT ACATTATTTG AATGCTGTTT 2220
   AATATGTTTA TCTGCATTAA TAGAAAAGGT GTTTATACAA TCAGTTGTCC AGTTTTTACC 2280
   TTCAGGTTTA CATCTAAAAT AAAATCTATT AATTAGTGAT GTTGCCCTCA AAGGGGAAGT 2340
   AAAACTACAC AATGGGTTTA TTGGATTCAA ACAAGAGTTT GCATGAGGGT GAAATATACA 2400
   AGTTTCATAT ATATGAAACA CTGAACATTC CGAGCTAGTT TTCATATAAT ACCAATCAAT 2460
   ACTTGCTGAA AATTCAGAAT CATAAATTTC CGATTCAAGA TTAAGTTCAA TTTTAAATGA 2520
   ATCATTTGGT ACAAATACAT GAGAGTGATA ATTTTTTACA TGAAAAAATC CACCATGTTT 2580
   TTTTATATAT ACTTTAGGTT TAGTAATTAC TGTTTCTTTT TTCTTTATAG TTACCAAGAT 2640
   TTTTGATTGA TGACTCCACT TATCATTATT TTCATACAAT GTATAAATTC CAGAATTATT 2700
   TATAGTCACA TTTGTGATAT GGATACCACT TTTATTTGTA GTTATTATTG GAAAAACACT 2760
   ATCATTAACA AAATAAACAT CATTTATTTT TTTAATAGTA TTATTACTAA TCTCAAAATC 2820
   TGCTACAGCT AAGGGATAAT TAATAGTTTT AATTGTACAT CGTTTATTCA AAAAAATATC 2880
   TTCTTCACAA ATATTACGTT CAATGCATAT ATTAGATATT GATATCCCAT CTTTACAATA 2940
   AGAAGGGGGG GTTTGTAAAA ATACCCATCC TCTAGTAAAT TTTTTAGTCT CATTAAATGG 3000
   TGGGTTAGTA CTATAACCAT CAATTACAAA TCCAGATTTT TCAGAAACAA TAACATTACG 3060
   AATTGTTGAA AAACCAAATC TTACCGAACA TAATGTAGAC GCGATTAAAA AAAATAAACG 3120
   AAACATCGTA AAGATTGGAG AAATGGAGAC TTGATGATTT TCCTAAAAGG CTCTTTGGAA 3180
   TACTATATTA TTTTTAAAAA ATTTTCATCA TTTAAATTCT AATTTTTAGT CATACAAGTC 3240
   GTCATCAACC TACGTAGGTA AATATTTTTA AAATTATACT TCTTCCTTAA TAGTTTCAAG 3300
   AGATGTCTCC CTTTGAATAG TTGAAGATCT TTTTTGGCGG TTAGGTTTTT TTGGTAATGG 3360
   ATTATTTTTG TTTACTGAGT TTTCCCCACA TGTCAGTTCT ATATTTTCAG ATTCCGAGTT 3420
   ACCTTTAGGA CGATAAATAC GCCTAGAAGA AGAAAAGTGT CTACGAATAA TATTGTTAAT 3480
   AATTGATCCA ATTAATACCA AAAGAATAAG TAAAATTATC GAAATCGAAA TTACAATAAT 3540
   TAAAAATTTT CTATTACGTT GGTCTAGGTA AGGCTTAAAT ATATAATTAT TTGTCGTTGT 3600
   AGGTATATTT ACTCTTTTAA GCAATTCAGA ATCCTCATCG AGAAAAATAT CATCAGTTAT 3660
   ATTTCCATGC AAACCTAGTA AAAGTTCATA CACATTTTCA TCCTTTTTTA CAGTATGACA 3720
   TATCGGTTCG TGTTTTTCAT TTAAATAGAT AGGTGTAATA GTTTGGGAAG AAGGGTGTGA 3780
   TGAGGTATAT AAAGATGTAG TAACATCATC GAAGTTTGAT TTATTTACTT CGTTTGACAT 3840
   ATTAAAGGAA TAAATAAATG CAGGTATTCC AAACACATCT GTTTTATTAT TTTCCAATTG 3900
   AACTCGTATA AAATAAACAC CTGAATCATT TGGTTTAGGA TTTGTAATTT CCAACAAAAC 3960
   ACCGTTTTCT ATAGATGAAT TTATCCGAAG TTGACTTTCA TGATGTTTTG ATACCTTTTT 4020
   TAAACAAGAT CTAAAAGCAT TATTAAAAAT GCGAGGACAA GAAAAATATT CAATAGTTTG 4080
   ATAAACGGTA TAACAAGAGT TATTAAAATG AATAAACTCA ATAGTTCCGC TATAATTATT 4140
   TACTGACAGA TGTTGGTCAT CGAGAAATAA TAAACGTCCA CTGACAATAA ATTTATCATC 4200
   GGGAAAAATA GAAAAGCCAG AACTAGTATT TAAAAACATA CTTATATACG TACCTTTGTA 4260
   AACAAATCCA TTTACACCAT ATAAAAAAAG AAATAAAATA GGTAGAAACA TAGTTATAAG 4320
   TAATCTGGTC TCAACCCTCT TGCAAGTGAA TAAAAGTTGT AGGTACATGA ATTTTTATTA 4380
   AACAAAAGAC CGCCCAGTTG TTTATTCTGT AAATTAATTT ATAAAGGCTA AACATTTGTT 4440
   GTTAATTTTT TATAATTATT ATATATTTTT TGTCTTTTAT AAACAAAAAA TCCAATTATA 4500
   AATAGTATTA TTAATATAAT TATAACAATT CCCACGGTTA TATTTTTTGG GGTATTATTT 4560
   ATTGGTGTTG GGGTAGTAGT ATCTATAACA TTTGTATTTT TAAAATAATC TTCAAGATTT 4620
   ACAATTTCTT TAATAGTTTT ATTAATAGTA TTACTAATAT GTGGCCTCGA TGGTAATTCA 4680
   TCATCAAATT TATCATTCAC TTTATATGGT ATATCGTATC TAAGAGCTTC TTTAAATTTT 4740
   ATTGGAGGTT CTCTACCGTT AAGTCTATAA AAATCTTTAA TAACATAATA TTTAAGCAGG 4800
   GTTTGGGGAC CAGCTCTATT TAGATAAAAA AGTTCATCGT CAACTTTTGA TGCTCCTACA 4860
   GATCTAGATT CCTTACACCA TTCCATAAAA GTTGGTTCAA ATTTATCTTC TTTAGAAAAA 4920
   TAACAAGTTT CTCGTGGTAA TTGAACCATA AAATCAGTAT AAAAAACTTC ATTTTCAATT 4980
   TGAATAACCC GTCTATATTG ACCTTGATTA AATGGAGCGG GGGCAGCTAA AACTAATCCC 5040
   AATTCATCAT CAGTTAAAAA TGTGTAACCA ACTAACGATG GTTTCCAAAA ATCTGGAGAT 5100
   CTTTTTAAAC ATATGCCAAA TTCTCTTTGA GGATCACAAT CAAAATATTG AATTAAATAT 5160
   ATTGGATATG TACAATCATC TCCAACTTTA AACCAACTAA GAGATGCATT GTAAAATTTT 5220
   TTCTTTTGCA CAAATTGAAT CGTTTTAATT GTAGATTCTA TATTTGGATT AGAAATCAAA 5280
   GCAACCATAT CACATGGGTC AGTAGAAGTT ACATACCGAA CTTCAGCGTT TTTAGAAGGA 5340
   TCCATTGGAT TTGGAATATC TGGAATCATT CTTAAAACGG TGTAATTATA CCTACGAGGA 5400
   GGGTCTACCC ATTTATATCC AGTTATTGGG TTAAAATAAA ATAAGATAAA TAGAAGTTTA 5460
   ATCATTTTAC TATCAAAAGT CGTAGCAACT CTTAAAATGA GAAAATGTTT AATCCGTTCA 5520
   AATTAATAGA ATTTAAATGT TTTTCATGGG TGTGTCCTTT ATGATGTTAA TTCTTAATAC 5580
   ACGTATGTAA TTTTAAAACC AACCCAACCC ATTTAATAAA AACAAATAAG TTATAAGAAG 5640
   TTTCATAATA GTTTTATTAA ATATCATAAA AATTTAATTT CTGGGGTTGT ATATAAGCAT 5700
   GGTTAATCAT TTTAATACGA GAGTTTAATA CACAAGTATA TCCACAAAAA AACACAGCAA 5760
   CAAAAAATAA AATAATAAAG ATAATTGTTC CAAATCCAAT TACCTTTATA TGGGTATTAC 5820
   AATTATTATA ACCATTGTTA AACAATTGAA TAATGCTTAA AACACTTTCT TCTTCATACA 5880
   TAGTTTCATT ATTTGACATA AACATATGTG TATTTTCAGT AGGTGGTGGT GTGGTAAACG 5940
   TTACAATTTC AGGATTCAGT ATATTAGAAT CTTCTTCCGA TTGAAGGGTG ATAGACAAAT 6000
   TATTAAAAAG TTCATAACTT TCATTAATTT TAAATGACCA CGGTTGTTCT GAAATTGATT 6060
   TACCCAAACT AGCAATCAAT AAACTTTGAG AACCTGCAGC CAAATTAAAA GTCTTCTTCA 6120
   TATCAAAAAG ATTGGATTTG CGGCAATCTT TTGATTGTAG ATCTTCAAAA TCTTCGTTTT 6180
   CTTCTTCATA TTCTTCATAT TCTTCATCAT ATGAATATCT ACCAACACTT TCATCACTAA 6240
   TATTATTCCA TAGCTCAGTA TTTGATATTG GAAAACAACC TCTATATCGT TTATGATTAG 6300
   GATTTGTGGA TGGTTTATAA GGAATATGAA GTTTATCACC CTTTTTTACA GTCAATCCAT 6360
   GCATCATAAA ACATGGATAG TCTGTCTCAT TATCCACTCG AACTTCAATT CTTCCGTTAT 6420
   AACTATCGTT TCCAAAAACA AGTGAGTATA AATATCTACC AGAATCAAAA ATTCCTGGTT 6480
   GAATAAGCAA ACTAACATTA ACAAGTGCAT AAGATGATAT ACTTTCAGAT TTTACCAAAG 6540
   TTAAAGAATA ACCATCACAA GTTTCTGGTG ATGGTATTAT ATTACCAGTA CAATTATAAT 6600
   ATTCTCTATA AATTAAAGGA ATTTGACAAC CAAGATCAAA TGACCATCCA ATTGTAGCAT 6660
   TAACCTTTTT TTCTGGTCGT GAAGATACAT TTACATATGT ATCTAGTAAT CGTAGTTCAC 6720
   ATCCTTTAGA GTATTCAACA TATCCGTTTG TTACCACGGG AATAAGAGTC TTAGATTGTT 6780
   CCCCTGTAGG AAGTTGTGTA GGTTCAGTTG TATTTTCTTT AGGATCCGCA TAACACAACT 6840
   CTAGCGGAGC TATGCGAGAT AAAACTACCT TAAAATAAAA AACAAAAAAC AGCGTATACA 6900
   ACATGATAAC TATAATATTG TAGCTAGGAT GTAAAAATAA AATTAATAAA AAAGAGCTTT 6960
   CAAACACCCG TTTTATATAA AACAATAAAA AATTCCACGC CCATTTTTAT GAAATTTATT 7020
   GTTTACTAAA AATCTGATAG GATAATAGTT CTTCCGCACT TGGTCGTAGA GATGCATCAA 7080
   ATGTTAGCAT TTTATGAATT AAAAATTCAC CATCGAGATG TAATTTAAGG TTATTTAGAC 7140
   GATTATATCG TGTAAATGGA GGTCTATCAT TATTAGCATA TTTTATAAAA TGTTTTACTA 7200
   GATTAGATTC CAAATCAGAT GGAAATTCAT TTGGGTTAAT CTTCAGAGTT GATATAATTT 7260
   TTATAAGATG AAGATTACAA TTGTTTATTA AATCGCTACT ATCTCTTTCT TCCTCCTCAA 7320
   ACAAAACATT AGGATATGCA AGCATTTCAA ATAAAATTAT ACCAGCACTC CAAATATCAG 7380
   CTTTACAGTT GTACGCATCC TTTGATAGAA CTTCAGGAGC ATTAGTTTCA ATAGTCCCCG 7440
   CAATTCCCAA ATAATCTGGT GAGGAAACAG GAAATTGAGA AGCCCCAAAA TCACCTATAC 7500
   AAACATTAGA GTCATTATCC AAGAAAATAT TTTCAGTTTT TATATCTCGA TGAATAATTT 7560
   TTTTTCCATG AATATATTGA AGTCCAGTTA ATATTTGTTT TTCAATTTTA ATTACAGATT 7620
   CAAAAGACAG AGATTTCCCA TGATCCATTA AAAAATTATA TAGATCATAT TTATAATACG 7680
   GTAACACCAA ACATGTTAAC TCTTTATAAA AAAGAGTGTC TTGAAGTTTA ATTATAGATT 7740
   GGTGACTAAT ATTTCTCAAA ATTAACGATT CCACAAGCGT GTTTCCCCTT TGTCCAATTT 7800
   TTAAAATTAC CTTATAGCTT GTATCTTTAT CTTTTTTAAG AGCAATAAAA ACTCTTCCTT 7860
   CTGACCCAGG CGTTAACGAT TTAACAACAG AAAACTTTAG CGCTTCAGCC GCTTCAATTC 7920
   CTTCGCTTTT AGTAGGAGAT AAACTTTTTT TATCTTCATC ACTTTCATAA CTTTCTTCAA 7980
   CTTCTTCGTC ACTTTCTCTT TCATTTTCAC TTTCAGTTGG ATTTTTTGTA TAATCCATAA 8040
   AGTCATCATC ACTAAAATCC GAGTATAAAT CCTCATCCAT TATTTTGTCT TGATGTAGGT 8100
   TATCCTCTTC AATCAAATCT GAAGTTAGAG GAACCGGTTT ATAGAAGTCT TCTGGATCGA 8160
   CAGAAGATTC TCTATTCACG CTGATACAAC AAAACTTTTC GGTGGTACAC TTTGCCATAT 8220
   CAGGTAATTG TGAAATTGTT ATGTTAATAT CAACTCATCA ACTGCAGCTT CTTCTAAAAT 8280
   TATAACATAA ATTAGGTGTG TATTAACCTT TATTGGATTC ATACATAAAA TTTAATTATG 8340
   GGTGTGGTTG AAGTTAATAT TGTTACGTTT ATTGACAAAA ATGGGGCGTT ACCAGGAAAC 8400
   TCCCAAGATG TTCATCCTGA TCTTTGGTGT TTTATGGCTA GACAATGCTA TATCCTTTCT 8460
   CTCACACGAT TGGCTATGCC TATAATATTA CGATCTGCAA ACTTATGTTA TTTTATGGAC 8520
   TCTATAAAAC ATCTACCTAG AGTTTCAAGG CCTATAGTAA GAACATCAAC CTCTAATAGT 8580
   AAATTTTTAA AACCAACGGA GGGGGAGGAG GAGGACTTTT TATTTTATGA GGATAAACCT 8640
   GGGGCTAGCA TCGAATGGAA ATCGGCTGTT TCGGGGTATA ACTATCTAAA CTCTGGTATA 8700
   TTTGGAAACT ATCCTCTTAA TCTATGGGTT TTCGGTGCAG CGGATTTATG TGAGCCAGTC 8760
   ATTTCTAATA TCCCAGGACC AAAGCGACTA ATTTATGCAT ATGTATCATG TGAATGGCCG 8820
   GAACCATCCT GGAAACCTGA GTGTCTTGAG ATTTCCAAAA ATAATGTAAA ACATATCGAT 8880
   GATTCTGGGA GTGTTTATTG CCCCTCGTTT ATATGGGTAC CTGTATTAGA CACCTTTCTG 8940
   TCCTCCTTTA AAGTACCCCC TATGTTAAGT GACATTTTTT ATGGTACAAC ATTTAATACT 9000
   CCATTCAACT TTGAAAGCTC TCCAAGATGT CCCTCTATAT CCTCATCTTC ATCCTCCTCC 9060
   TCCTCATCCT CTTCTTCATG CTCTGCATCT CATTATTCCT CAGAGAATGA TATTTGGAAA 9120
   TACGCTAATG CGATGGTGGA TAATGAACTA GAAGAAACTA ATTGTGAAAT TAAAAATAGT 9180
   TTTAAAGAGG ATTCTGACAG TTCTACATGT TCTATAAGCG ATGTATGTGA TTCTCTTTAT 9240
   ATAAATGAGA CACGTTCTGC CCCCCCGGTA ATCGAATCAT TTATGTGTGC AATAATATCC 9300
   CATAATGGTT ATGAAGAAAT TCCTGAACCA GAAGAGGATG ACTTGCATGG GGTGAAACCT 9360
   ATGAAAAGGC TATCCAAACC AAATATTTTT AAACGATTAT TTTCAAGGCG CGCCAAAACA 9420
   ATAACCAAAA ATGAAAACGA TGGTATGTCT TCTACACAAC CTACCAACGC TCAACGTTCA 9480
   TCTCTATTTA GTTCATGTTT ATGTGGAAAC TAAGTTTGTT TATATTTGTT GTGAATAAAC 9540
   AGTAGATGTA AACCTGAAAT TCTGGTTTTT TATGTGAACC ACTCCCATAT TCTTACTGTA 9600
   TATAAACTGG CATTTTATCC TACATTTTTA AATCTTCACT TGTTTTTAAA GCTTAACTAA 9660
   AAAGTTTTAA AATGAGTGCT TCATTTGCTC TATTTGATAA CAACTCCGAT CAAGCTAATA 9720
   TGCCATCATA CTTCAGACGC TGTCGTGGAG AACTTAATGA AGGATTCTAC GCACAAGTTC 9780
   CGCCTGGTTA TTTTCCAGTT CGCCCTAAAA CCACACCAGC GCTTGCACGT GTGAAAAACC 9840
   AGGGTGAACC GCACGCCTTC ACTATTGTTC CTACACCACC TACGGATGTA CGATTCTTCA 9900
   AAAAGCTTCG TGATGGAACC TTTGTGAAAC TTCCATTTTC ATATCCTGAT GAGAGGTATG 9960
   AAGATGATAT TGAACCTATG TATTGCAGAC TATACGTTCT AAAGGATTTT GAAACGCCAA 10020
   GTTCTCCCTA CAATCAAGTA AACGCTTCTC AGCTTATGGA AGTACCTTCT TCGCTTGTGG 10080
   AATCGAAGTG TTATTGGGAT GGTCCAAAAT TTATAAATGT ACCACCAATA AGATATATTC 10140
   TAAAAAGTGA TTACTCTCTA AATAATGTAT CCGAAGATGC ATTTGTGTTG GTAGATACCA 10200
   GAAAACTAAA ACTTGAAAAC TAAAATGTTT ACCCAACGCA TTAAACCTTG TAACCTATAC 10260
   TCTTAATAAA ATTTAAAATA TTTTTAAAAA TTACTTTGTC TCATAGATTT CATTCATAGA 10320
   TTTCATTCAT AGATTTCATT CATAGATTTC ATTCATAGAT TTCATTCATA GATTTCATTC 10380
   ATAGATTTCA TTCATAGATT TCATTCATAG ATTTCATTCA TAGATTTCAT TCATAGATTT 10440
   CATTCATAGA TTTCATTCAT AGATTTCATT CATAGATTTC ATTCATAGAT TTCATTCATA 10500
   GATTTCATTC ATAGATTTCA TTCATAGATT TCATTCATAG ATTTCATTCA TAGATTTCAT 10560
   TCATAGATTT TTATTTTTTA AATACACTGC AG 10592
(2) INFORMATION FOR SEQ ID NO:53:
   (i) SEQUENCE CHARACTERISTICS:
      (A) LENGTH: 1038 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1035
   (xi) SEQUENCE DESCRIPTION: SEQ ID NO:53:
   ATG ATT AAA CTT CTA TTT ATC TTA TTT TAT TTT AAC CCA ATA ACT GGA      48
   Met Ile Lys Leu Leu Phe Ile Leu Phe Tyr Phe Asn Pro Ile Thr Gly
     1               5                  10                  15
   TAT AAA TGG GTA GAC CCT CCT CGT AGG TAT AAT TAC ACC GTT TTA AGA      96
   Tyr Lys Trp Val Asp Pro Pro Arg Arg Tyr Asn Tyr Thr Val Leu Arg
                20                  25                  30
   ATG ATT CCA GAT ATT CCA AAT CCA ATG GAT CCT TCT AAA AAC GCT GAA     144
   Met Ile Pro Asp Ile Pro Asn Pro Met Asp Pro Ser Lys Asn Ala Glu
            35                  40                  45
   GTT CGG TAT GTA ACT TCT ACT GAC CCA TGT GAT ATG GTT GCT TTG ATT     192
   Val Arg Tyr Val Thr Ser Thr Asp Pro Cys Asp Met Val Ala Leu Ile
        50                  55                  60
   TCT AAT CCA AAT ATA GAA TCT ACA ATT AAA ACG ATT CAA TTT GTG CAA     240
   Ser Asn Pro Asn Ile Glu Ser Thr Ile Lys Thr Ile Gln Phe Val Gln
    65                  70                  75                  80
   AAG AAA AAA TTT TAC AAT GCA TCT CTT AGT TGG TTT AAA GTT GGA GAT     288
   Lys Lys Lys Phe Tyr Asn Ala Ser Leu Ser Trp Phe Lys Val Gly Asp
                    85                  90                  95
   GAT TGT ACA TAT CCA ATA TAT TTA ATT CAA TAT TTT GAT TGT GAT CCT     336
   Asp Cys Thr Tyr Pro Ile Tyr Leu Ile Gln Tyr Phe Asp Cys Asp Pro
               100                 105                 110
   CAA AGA GAA TTT GGC ATA TGT TTA AAA AGA TCT CCA GAT TTT TGG AAA     384
   Gln Arg Glu Phe Gly Ile Cys Leu Lys Arg Ser Pro Asp Phe Trp Lys
           115                 120                 125
   CCA TCG TTA GTT GGT TAC ACA TTT TTA ACT GAT GAT GAA TTG GGA TTA     432
   Pro Ser Leu Val Gly Tyr Thr Phe Leu Thr Asp Asp Glu Leu Gly Leu
       130                 135                 140
   GTT TTA GCT GCC CCC GCT CCA TTT AAT CAA GGT CAA TAT AGA CGG GTT     480
   Val Leu Ala Ala Pro Ala Pro Phe Asn Gln Gly Gln Tyr Arg Arg Val
   145                 150                 155                 160
   ATT CAA ATT GAA AAT GAA GTT TTT TAT ACT GAT TTT ATG GTT CAA TTA     528
   Ile Gln Ile Glu Asn Glu Val Phe Tyr Thr Asp Phe Met Val Gln Leu
                   165                 170                 175
   CCA CGA GAA ACT TGT TAT TTT TCT AAA GAA GAT AAA TTT GAA CCA ACT        576
   Pro Arg Glu Thr Cys Tyr Phe Ser Lys Glu Asp Lys Phe Glu Pro Thr
               180                 185                 190
   TTT ATG GAA TGG TGT AAG GAA TCT AGA TCT GTA GGA GCA TCA AAA GTT        624
   Phe Met Glu Trp Cys Lys Glu Ser Arg Ser Val Gly Ala Ser Lys Val
           195                 200                 205
   GAC GAT GAA CTT TTT TAT CTA AAT AGA GCT GGT CCC CAA ACC CTG CTT        672
   Asp Asp Glu Leu Phe Tyr Leu Asn Arg Ala Gly Pro Gln Thr Leu Leu
       210                 215                 220
   AAA TAT TAT GTT ATT AAA GAT TTT TAT AGA CTT AAC GGT AGA GAA CCT        720
   Lys Tyr Tyr Val Ile Lys Asp Phe Tyr Arg Leu Asn Gly Arg Glu Pro
   225                 230                 235                 240
   CCA ATA AAA TTT AAA GAA GCT CTT AGA TAC GAT ATA CCA TAT AAA GTG        768
   Pro Ile Lys Phe Lys Glu Ala Leu Arg Tyr Asp Ile Pro Tyr Lys Val
                   245                 250                 255
   AAT GAT AAA TTT GAT GAT GAA TTA CCA TCG AGG CCA CAT ATT AGT AAT        816
   Asn Asp Lys Phe Asp Asp Glu Leu Pro Ser Arg Pro His Ile Ser Asn
               260                 265                 270
   ACT ATT AAT AAA ACT ATT AAA GAA ATT GTA AAT CTT GAA GAT TAT TTT        864
   Thr Ile Asn Lys Thr Ile Lys Glu Ile Val Asn Leu Glu Asp Tyr Phe
           275                 280                 285
   AAA AAT ACA AAT GTT ATA GAT ACT ACT ACC CCA ACA CCA ATA AAT AAT        912
   Lys Asn Thr Asn Val Ile Asp Thr Thr Thr Pro Thr Pro Ile Asn Asn
       290                 295                 300
   ACC CCA AAA AAT ATA ACC GTG GGA ATT GTT ATA ATT ATA TTA ATA ATA        960
   Thr Pro Lys Asn Ile Thr Val Gly Ile Val Ile Ile Ile Leu Ile Ile
   305                 310                 315                 320
   CTA TTT ATA ATT GGA TTT TTT GTT TAT AAA AGA CAA AAA ATA TAT AAT       1008
   Leu Phe Ile Ile Gly Phe Phe Val Tyr Lys Arg Gln Lys Ile Tyr Asn
                   325                 330                 335
   AAT TAT AAA AAA TTA ACA ACA AAT GTT TAG                               1038
   Asn Tyr Lys Lys Leu Thr Thr Asn Val
               340                 345
(2) INFORMATION FOR SEQ ID NO:54:
   (i) SEQUENCE CHARACTERISTICS:
      (A) LENGTH: 345 amino acids
      (B) TYPE: amino acid
      (D) TOPOLOGY: linear
   (ii) MOLECULE TYPE: protein
   (xi) SEQUENCE DESCRIPTION: SEQ ID NO:54:
   Met Ile Lys Leu Leu Phe Ile Leu Phe Tyr Phe Asn Pro Ile Thr Gly
   1               5                   10                  15
   Tyr Lys Trp Val Asp Pro Pro Arg Arg Tyr Asn Tyr Thr Val Leu Arg
               20                  25                  30
   Met Ile Pro Asp Ile Pro Asn Pro Met Asp Pro Ser Lys Asn Ala Glu
           35                  40                  45
   Val Arg Tyr Val Thr Ser Thr Asp Pro Cys Asp Met Val Ala Leu Ile
       50                  55                  60
   Ser Asn Pro Asn Ile Glu Ser Thr Ile Lys Thr Ile Gln Phe Val Gln
   65                  70                  75                  80
   Lys Lys Lys Phe Tyr Asn Ala Ser Leu Ser Trp Phe Lys Val Gly Asp
                   85                  90                  95
   Asp Cys Thr Tyr Pro Ile Tyr Leu Ile Gln Tyr Phe Asp Cys Asp Pro
               100                 105                 110
   Gln Arg Glu Phe Gly Ile Cys Leu Lys Arg Ser Pro Asp Phe Trp Lys
           115                 120                 125
   Pro Ser Leu Val Gly Tyr Thr Phe Leu Thr Asp Asp Glu Leu Gly Leu
       130                 135                 140
   Val Leu Ala Ala Pro Ala Pro Phe Asn Gln Gly Gln Tyr Arg Arg Val
   145                 150                 155                 160
   Ile Gln Ile Glu Asn Glu Val Phe Tyr Thr Asp Phe Met Val Gln Leu
                   165                 170                 175
   Pro Arg Glu Thr Cys Tyr Phe Ser Lys Glu Asp Lys Phe Glu Pro Thr
               180                 185                 190
   Phe Met Glu Trp Cys Lys Glu Ser Arg Ser Val Gly Ala Ser Lys Val
           195                 200                 205
   Asp Asp Glu Leu Phe Tyr Leu Asn Arg Ala Gly Pro Gln Thr Leu Leu
       210                 215                 220
   Lys Tyr Tyr Val Ile Lys Asp Phe Tyr Arg Leu Asn Gly Arg Glu Pro
   225                 230                 235                 240
   Pro Ile Lys Phe Lys Glu Ala Leu Arg Tyr Asp Ile Pro Tyr Lys Val
                   245                 250                 255
   Asn Asp Lys Phe Asp Asp Glu Leu Pro Ser Arg Pro His Ile Ser Asn
               260                 265                 270
   Thr Ile Asn Lys Thr Ile Lys Glu Ile Val Asn Leu Glu Asp Tyr Phe
           275                 280                 285
   Lys Asn Thr Asn Val Ile Asp Thr Thr Thr Pro Thr Pro Ile Asn Asn
       290                 295                 300
   Thr Pro Lys Asn Ile Thr Val Gly Ile Val Ile Ile Ile Leu Ile Ile
   305                 310                 315                 320
   Leu Phe Ile Ile Gly Phe Phe Val Tyr Lys Arg Gln Lys Ile Tyr Asn
                   325                 330                 335
   Asn Tyr Lys Lys Leu Thr Thr Asn Val
               340                 345
(2) INFORMATION FOR SEQ ID NO:55:
   (i) SEQUENCE CHARACTERISTICS:
      (A) LENGTH: 1095 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1092
   (xi) SEQUENCE DESCRIPTION: SEQ ID NO:55:
   ATG TAC CTA CAA CTT TTA TTC ACT TGC AAG AGG GTT GAG ACC AGA TTA        48
   Met Tyr Leu Gln Leu Leu Phe Thr Cys Lys Arg Val Glu Thr Arg Leu
     1               5                  10                  15
   CTT ATA ACT ATG TTT CTA CCT ATT TTA TTT CTT TTT TTA TAT GGT GTA        96
   Leu Ile Thr Met Phe Leu Pro Ile Leu Phe Leu Phe Leu Tyr Gly Val
                20                  25                  30
   AAT GGA TTT GTT TAC AAA GGT ACG TAT ATA AGT ATG TTT TTA AAT ACT       144
   Asn Gly Phe Val Tyr Lys Gly Thr Tyr Ile Ser Met Phe Leu Asn Thr
            35                  40                  45
   AGT TCT GGC TTT TCT ATT TTT CCC GAT GAT AAA TTT ATT GTC AGT GGA       192
   Ser Ser Gly Phe Ser Ile Phe Pro Asp Asp Lys Phe Ile Val Ser Gly
        50                  55                  60
   CGT TTA TTA TTT CTC GAT GAC CAA CAT CTG TCA GTA AAT AAT TAT AGC       240
   Arg Leu Leu Phe Leu Asp Asp Gln His Leu Ser Val Asn Asn Tyr Ser
    65                  70                  75                  80
   GGA ACT ATT GAG TTT ATT CAT TTT AAT AAC TCT TGT TAT ACC GTT TAT       288
   Gly Thr Ile Glu Phe Ile His Phe Asn Asn Ser Cys Tyr Thr Val Tyr
                    85                  90                  95
   CAA ACT ATT GAA TAT TTT TCT TGT CCT CGC ATT TTT AAT AAT GCT TTT       336
   Gln Thr Ile Glu Tyr Phe Ser Cys Pro Arg Ile Phe Asn Asn Ala Phe
               100                 105                 110
   AGA TCT TGT TTA AAA AAG GTA TCA AAA CAT CAT GAA AGT CAA CTT CGG       384
   Arg Ser Cys Leu Lys Lys Val Ser Lys His His Glu Ser Gln Leu Arg
           115                 120                 125
   ATA AAT TCA TCT ATA GAA AAC GGT GTT TTG TTG GAA ATT ACA AAT CCT       432
   Ile Asn Ser Ser Ile Glu Asn Gly Val Leu Leu Glu Ile Thr Asn Pro
       130                 135                 140
   AAA CCA AAT GAT TCA GGT GTT TAT TTT ATA CGA GTT CAA TTG GAA AAT       480
   Lys Pro Asn Asp Ser Gly Val Tyr Phe Ile Arg Val Gln Leu Glu Asn
   145                 150                 155                 160
   AAT AAA ACA GAT GTG TTT GGA ATA CCT GCA TTT ATT TAT TCC TTT AAT       528
   Asn Lys Thr Asp Val Phe Gly Ile Pro Ala Phe Ile Tyr Ser Phe Asn
                   165                 170                 175
   ATG TCA AAC GAA GTA AAT AAA TCA AAC TTC GAT GAT GTT ACT ACA TCT      576
   Met Ser Asn Glu Val Asn Lys Ser Asn Phe Asp Asp Val Thr Thr Ser
               180                 185                 190
   TTA TAT ACC TCA TCA CAC CCT TCT TCC CAA ACT ATT ACA CCT ATC TAT      624
   Leu Tyr Thr Ser Ser His Pro Ser Ser Gln Thr Ile Thr Pro Ile Tyr
           195                 200                 205
   TTA AAT GAA AAA CAC GAA CCG ATA TGT CAT ACT GTA AAA AAG GAT GAA      672
   Leu Asn Glu Lys His Glu Pro Ile Cys His Thr Val Lys Lys Asp Glu
       210                 215                 220
   AAT GTG TAT GAA CTT TTA CTA GGT TTG CAT GGA AAT ATA ACT GAT GAT      720
   Asn Val Tyr Glu Leu Leu Leu Gly Leu His Gly Asn Ile Thr Asp Asp
   225                 230                 235                 240
   ATT TTT CTC GAT GAG GAT TCT GAA TTG CTT AAA AGA GTA AAT ATA CCT      768
   Ile Phe Leu Asp Glu Asp Ser Glu Leu Leu Lys Arg Val Asn Ile Pro
                   245                 250                 255
   ACA ACG ACA AAT AAT TAT ATA TTT AAG CCT TAC CTA GAC CAA CGT AAT      816
   Thr Thr Thr Asn Asn Tyr Ile Phe Lys Pro Tyr Leu Asp Gln Arg Asn
               260                 265                 270
   AGA AAA TTT TTA ATT ATT GTA ATT TCG ATT TCG ATA ATT TTA CTT ATT      864
   Arg Lys Phe Leu Ile Ile Val Ile Ser Ile Ser Ile Ile Leu Leu Ile
           275                 280                 285
   CTT TTG GTA TTA ATT GGA TCA ATT ATT AAC AAT ATT ATT CGT AGA CAC      912
   Leu Leu Val Leu Ile Gly Ser Ile Ile Asn Asn Ile Ile Arg Arg His
       290                 295                 300
   TTT TCT TCT TCT AGG CGT ATT TAT CGT CCT AAA GGT AAC TCG GAA TCT      960
   Phe Ser Ser Ser Arg Arg Ile Tyr Arg Pro Lys Gly Asn Ser Glu Ser
   305                 310                 315                 320
   GAA AAT ATA GAA CTG ACA TGT GGG GAA AAC TCA GTA AAC AAA AAT AAT     1008
   Glu Asn Ile Glu Leu Thr Cys Gly Glu Asn Ser Val Asn Lys Asn Asn
                   325                 330                 335
   CCA TTA CCA AAA AAA CCT AAC CGC CAA AAA AGA TCT TCA ACT ATT CAA     1056
   Pro Leu Pro Lys Lys Pro Asn Arg Gln Lys Arg Ser Ser Thr Ile Gln
               340                 345                 350
   AGG GAG ACA TCT CTT GAA ACT ATT AAG GAA GAA GTA TAA                 1095
   Arg Glu Thr Ser Leu Glu Thr Ile Lys Glu Glu Val
           355                 360
(2) INFORMATION FOR SEQ ID NO:56:
   (i) SEQUENCE CHARACTERISTICS:
      (A) LENGTH: 364 amino acids
      (B) TYPE: amino acid
      (D) TOPOLOGY: linear
   (ii) MOLECULE TYPE: protein
   (xi) SEQUENCE DESCRIPTION: SEQ ID NO:56:
   Met Tyr Leu Gln Leu Leu Phe Thr Cys Lys Arg Val Glu Thr Arg Leu
    1               5                  10                  15
   Leu Ile Thr Met Phe Leu Pro Ile Leu Phe Leu Phe Leu Tyr Gly Val
               20                  25                  30
   Asn Gly Phe Val Tyr Lys Gly Thr Tyr Ile Ser Met Phe Leu Asn Thr
           35                  40                  45
   Ser Ser Gly Phe Ser Ile Phe Pro Asp Asp Lys Phe Ile Val Ser Gly
       50                  55                  60
   Arg Leu Leu Phe Leu Asp Asp Gln His Leu Ser Val Asn Asn Tyr Ser
   65                  70                  75                  80
   Gly Thr Ile Glu Phe Ile His Phe Asn Asn Ser Cys Tyr Thr Val Tyr
                   85                  90                  95
   Gln Thr Ile Glu Tyr Phe Ser Cys Pro Arg Ile Phe Asn Asn Ala Phe
               100                 105                 110
   Arg Ser Cys Leu Lys Lys Val Ser Lys His His Glu Ser Gln Leu Arg
           115                 120                 125
   Ile Asn Ser Ser Ile Glu Asn Gly Val Leu Leu Glu Ile Thr Asn Pro
       130                 135                 140
   Lys Pro Asn Asp Ser Gly Val Tyr Phe Ile Arg Val Gln Leu Glu Asn
   145                 150                 155                 160
   Asn Lys Thr Asp Val Phe Gly Ile Pro Ala Phe Ile Tyr Ser Phe Asn
                   165                 170                 175
   Met Ser Asn Glu Val Asn Lys Ser Asn Phe Asp Asp Val Thr Thr Ser
               180                 185                 190
   Leu Tyr Thr Ser Ser His Pro Ser Ser Gln Thr Ile Thr Pro Ile Tyr
           195                 200                 205
   Leu Asn Glu Lys His Glu Pro Ile Cys His Thr Val Lys Lys Asp Glu
       210                 215                 220
   Asn Val Tyr Glu Leu Leu Leu Gly Leu His Gly Asn Ile Thr Asp Asp
   225                 230                 235                 240
   Ile Phe Leu Asp Glu Asp Ser Glu Leu Leu Lys Arg Val Asn Ile Pro
                   245                 250                 255
   Thr Thr Thr Asn Asn Tyr Ile Phe Lys Pro Tyr Leu Asp Gln Arg Asn
               260                 265                 270
   Arg Lys Phe Leu Ile Ile Val Ile Ser Ile Ser Ile Ile Leu Leu Ile
           275                 280                 285
   Leu Leu Val Leu Ile Gly Ser Ile Ile Asn Asn Ile Ile Arg Arg His
       290                 295                 300
   Phe Ser Ser Ser Arg Arg Ile Tyr Arg Pro Lys Gly Asn Ser Glu Ser
   305                 310                 315                 320
   Glu Asn Ile Glu Leu Thr Cys Gly Glu Asn Ser Val Asn Lys Asn Asn
   325 330 335
   Pro Leu Pro Lys Lys Pro Asn Arg Gln Lys Arg Ser Ser Thr Ile Gln
   340 345 350
   Arg Glu Thr Ser Leu Glu Thr Ile Lys Glu Glu Val
   355 360
(2) INFORMATION FOR SEQ ID NO:57:
   (i) SEQUENCE CHARACTERISTICS:
      (A) LENGTH: 1569 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1566
   (xi) SEQUENCE DESCRIPTION: SEQ ID NO:57:
   ATG TTT CGT TTA TTT TTT TTA ATC GCG TCT ACA TTA TGT TCG GTA AGA        48
   Met Phe Arg Leu Phe Phe Leu Ile Ala Ser Thr Leu Cys Ser Val Arg
     1               5                  10                  15
   TTT GGT TTT TCA ACA ATT CGT AAT GTT ATT GTT TCT GAA AAA TCT GGA        96
   Phe Gly Phe Ser Thr Ile Arg Asn Val Ile Val Ser Glu Lys Ser Gly
                20                  25                  30
   TTT GTA ATT GAT GGT TAT AGT ACT AAC CCA CCA TTT AAT GAG ACT AAA       144
   Phe Val Ile Asp Gly Tyr Ser Thr Asn Pro Pro Phe Asn Glu Thr Lys
            35                  40                  45
   AAA TTT ACT AGA GGA TGG GTA TTT TTA CAA ACC CCC CCT TCT TAT TGT       192
   Lys Phe Thr Arg Gly Trp Val Phe Leu Gln Thr Pro Pro Ser Tyr Cys
        50                  55                  60
   AAA GAT GGG ATA TCA ATA TCT AAT ATA TGC ATT GAA CGT AAT ATT TGT       240
   Lys Asp Gly Ile Ser Ile Ser Asn Ile Cys Ile Glu Arg Asn Ile Cys
    65                  70                  75                  80
   GAA GAA GAT ATT TTT TTG AAT AAA CGA TGT ACA ATT AAA ACT ATT AAT       288
   Glu Glu Asp Ile Phe Leu Asn Lys Arg Cys Thr Ile Lys Thr Ile Asn
                    85                  90                  95
   TAT CCC TTA GCT GTA GCA GAT TTT GAG ATT AGT AAT AAT ACT ATT AAA       336
   Tyr Pro Leu Ala Val Ala Asp Phe Glu Ile Ser Asn Asn Thr Ile Lys
               100                 105                 110
   AAA ATA AAT GAT GTT TAT TTT GTT AAT GAT AGT GTT TTT CCA ATA ATA       384
   Lys Ile Asn Asp Val Tyr Phe Val Asn Asp Ser Val Phe Pro Ile Ile
           115                 120                 125
   ACT ACA AAT AAA AGT GGT ATC CAT ATC ACA AAT GTG ACT ATA AAT AAT       432
   Thr Thr Asn Lys Ser Gly Ile His Ile Thr Asn Val Thr Ile Asn Asn
       130                 135                 140
   TCT GGA ATT TAT ACA TTG TAT GAA AAT AAT GAT AAG TGG AGT CAT CAA         480
   Ser Gly Ile Tyr Thr Leu Tyr Glu Asn Asn Asp Lys Trp Ser His Gln
   145                 150                 155                 160
   TCA AAA ATC TTG GTA ACT ATA AAG AAA AAA GAA ACA GTA ATT ACT AAA         528
   Ser Lys Ile Leu Val Thr Ile Lys Lys Lys Glu Thr Val Ile Thr Lys
                   165                 170                 175
   CCT AAA GTA TAT ATA AAA AAA CAT GGT GGA TTT TTT CAT GTA AAA AAT         576
   Pro Lys Val Tyr Ile Lys Lys His Gly Gly Phe Phe His Val Lys Asn
               180                 185                 190
   TAT CAC TCT CAT GTA TTT GTA CCA AAT GAT TCA TTT AAA ATT GAA CTT         624
   Tyr His Ser His Val Phe Val Pro Asn Asp Ser Phe Lys Ile Glu Leu
           195                 200                 205
   AAT CTT GAA TCG GAA ATT TAT GAT TCT GAA TTT TCA GCA AGT ATT GAT         672
   Asn Leu Glu Ser Glu Ile Tyr Asp Ser Glu Phe Ser Ala Ser Ile Asp
       210                 215                 220
   TGG TAT TAT ATG AAA ACT AGC TCG GAA TGT TCA GTG TTT CAT ATA TAT         720
   Trp Tyr Tyr Met Lys Thr Ser Ser Glu Cys Ser Val Phe His Ile Tyr
   225                 230                 235                 240
   GAA ACT TGT ATA TTT CAC CCT CAT GCA AAC TCT TGT TTG AAT CCA ATA         768
   Glu Thr Cys Ile Phe His Pro His Ala Asn Ser Cys Leu Asn Pro Ile
                   245                 250                 255
   AAC CCA TTG TGT AGT TTT ACT TCC CCT TTG AGG GCA ACA TCA CTA ATT         816
   Asn Pro Leu Cys Ser Phe Thr Ser Pro Leu Arg Ala Thr Ser Leu Ile
               260                 265                 270
   AAT AGA TTT TAT TTT AGA TGT AAA CCT GAA GGT AAA AAC TGG ACA ACT         864
   Asn Arg Phe Tyr Phe Arg Cys Lys Pro Glu Gly Lys Asn Trp Thr Thr
           275                 280                 285
   GAT TGT ATA AAC ACC TTT TCT ATT AAT GCA GAT AAA CAT ATT AAA CAG         912
   Asp Cys Ile Asn Thr Phe Ser Ile Asn Ala Asp Lys His Ile Lys Gln
       290                 295                 300
   CAT TCA AAT AAT GTA GAT TTG ATT TTT TTA AAT ACT CCA ACT AAT GCA         960
   His Ser Asn Asn Val Asp Leu Ile Phe Leu Asn Thr Pro Thr Asn Ala
   305                 310                 315                 320
   TCT GGT TTG TAT GTT TTT ATT CTT AAG TAT AAT GGT CAT CCA GAG GCT        1008
   Ser Gly Leu Tyr Val Phe Ile Leu Lys Tyr Asn Gly His Pro Glu Ala
                   325                 330                 335
   TGG ACA TAT ACT TTG GTT TCA ACG GTT AAA AAT TTT ATG AAT GTA ATT        1056
   Trp Thr Tyr Thr Leu Val Ser Thr Val Lys Asn Phe Met Asn Val Ile
               340                 345                 350
   AAG GAT ATG ACA CGC CCC CTT TTG TCA AAT AAT AAA ATG AAA AAA CCT        1104
   Lys Asp Met Thr Arg Pro Leu Leu Ser Asn Asn Lys Met Lys Lys Pro
           355                 360                 365
   GAG CAT TCT ACT CAA CCA CCA ACC ATA ACC AAC ATA ACA CCT GGC TTT        1152
   Glu His Ser Thr Gln Pro Pro Thr Ile Thr Asn Ile Thr Pro Gly Phe
       370                 375                 380
   AAA TCT AAA AAT TGG GTA GAT AAA TAT ATA ATT TCA GTA GCG GTG GTT        1200
   Lys Ser Lys Asn Trp Val Asp Lys Tyr Ile Ile Ser Val Ala Val Val
   385                 390                 395                 400
   TCT TGT ATT ACT ATT GTT ATA TTG ATT GTG GTA ATA ACC TTT TGT GTT        1248
   Ser Cys Ile Thr Ile Val Ile Leu Ile Val Val Ile Thr Phe Cys Val
                   405                 410                 415
   CAT CAA TGT ATC GGT TTA AAT CGT AAA CCA TAT GAA ATT ATA AAC CCA        1296
   His Gln Cys Ile Gly Leu Asn Arg Lys Pro Tyr Glu Ile Ile Asn Pro
               420                 425                 430
   TTT AAT ACA GCT TAT AAA AGT ATA CCT ACA AAT GAA AAA AAT ATT CTT        1344
   Phe Asn Thr Ala Tyr Lys Ser Ile Pro Thr Asn Glu Lys Asn Ile Leu
           435                 440                 445
   CAT TTT GCT GAA GTA ACA GAA TCT GAT TAT TCC TCC GAC GAA TCC TTC        1392
   His Phe Ala Glu Val Thr Glu Ser Asp Tyr Ser Ser Asp Glu Ser Phe
       450                 455                 460
   GAC AGT GAC TCA GAA GAG CTA AAT CAA CGA GGT GAA ACA ATA CAA CAA        1440
   Asp Ser Asp Ser Glu Glu Leu Asn Gln Arg Gly Glu Thr Ile Gln Gln
   465                 470                 475                 480
   GGG AAA AAG GAA CAA TCT GGA TAT ACT ATT TGG TTT AAT GAA GAT TTA        1488
   Gly Lys Lys Glu Gln Ser Gly Tyr Thr Ile Trp Phe Asn Glu Asp Leu
                   485                 490                 495
   GAA GAA TCC GTC TCC AAA AAA CTT AAC CAA CCA AAC TAT TCA AAA ATA        1536
   Glu Glu Ser Val Ser Lys Lys Leu Asn Gln Pro Asn Tyr Ser Lys Ile
               500                 505                 510
   ATT AAT AGC TTA AAA TCA ATC CAG AAT GAA TAA                            1569
   Ile Asn Ser Leu Lys Ser Ile Gln Asn Glu
           515                 520
(2) INFORMATION FOR SEQ ID NO:58:
   (i) SEQUENCE CHARACTERISTICS:
      (A) LENGTH: 522 amino acids
      (B) TYPE: amino acid
      (D) TOPOLOGY: linear
   (ii) MOLECULE TYPE: protein
   (xi) SEQUENCE DESCRIPTION: SEQ ID NO:58:
   Met Phe Arg Leu Phe Phe Leu Ile Ala Ser Thr Leu Cys Ser Val Arg
    1               5                  10                  15
   Phe Gly Phe Ser Thr Ile Arg Asn Val Ile Val Ser Glu Lys Ser Gly
               20                  25                  30
   Phe Val Ile Asp Gly Tyr Ser Thr Asn Pro Pro Phe Asn Glu Thr Lys
           35                  40                  45
   Lys Phe Thr Arg Gly Trp Val Phe Leu Gln Thr Pro Pro Ser Tyr Cys
       50                  55                  60
   Lys Asp Gly Ile Ser Ile Ser Asn Ile Cys Ile Glu Arg Asn Ile Cys
   65 70 75 80
   Glu Glu Asp Ile Phe Leu Asn Lys Arg Cys Thr Ile Lys Thr Ile Asn
   85 90 95
   Tyr Pro Leu Ala Val Ala Asp Phe Glu Ile Ser Asn Asn Thr Ile Lys
   100 105 110
   Lys Ile Asn Asp Val Tyr Phe Val Asn Asp Ser Val Phe Pro Ile Ile
   115 120 125
   Thr Thr Asn Lys Ser Gly Ile His Ile Thr Asn Val Thr Ile Asn Asn
   130 135 140
   Ser Gly Ile Tyr Thr Leu Tyr Glu Asn Asn Asp Lys Trp Ser His Gln
   145 150 155 160
   Ser Lys Ile Leu Val Thr Ile Lys Lys Lys Glu Thr Val Ile Thr Lys
   165 170 175
   Pro Lys Val Tyr Ile Lys Lys His Gly Gly Phe Phe His Val Lys Asn
   180 185 190
   Tyr His Ser His Val Phe Val Pro Asn Asp Ser Phe Lys Ile Glu Leu
   195 200 205
   Asn Leu Glu Ser Glu Ile Tyr Asp Ser Glu Phe Ser Ala Ser Ile Asp
   210 215 220
   Trp Tyr Tyr Met Lys Thr Ser Ser Glu Cys Ser Val Phe His Ile Tyr
   225 230 235 240
   Glu Thr Cys Ile Phe His Pro His Ala Asn Ser Cys Leu Asn Pro Ile
   245 250 255
   Asn Pro Leu Cys Ser Phe Thr Ser Pro Leu Arg Ala Thr Ser Leu Ile
   260 265 270
   Asn Arg Phe Tyr Phe Arg Cys Lys Pro Glu Gly Lys Asn Trp Thr Thr
   275 280 285
   Asp Cys Ile Asn Thr Phe Ser Ile Asn Ala Asp Lys His Ile Lys Gln
   290 295 300
   His Ser Asn Asn Val Asp Leu Ile Phe Leu Asn Thr Pro Thr Asn Ala
   305 310 315 320
   Ser Gly Leu Tyr Val Phe Ile Leu Lys Tyr Asn Gly His Pro Glu Ala
   325 330 335
   Trp Thr Tyr Thr Leu Val Ser Thr Val Lys Asn Phe Met Asn Val Ile
   340 345 350
   Lys Asp Met Thr Arg Pro Leu Leu Ser Asn Asn Lys Met Lys Lys Pro
   355 360 365
   Glu His Ser Thr Gln Pro Pro Thr Ile Thr Asn Ile Thr Pro Gly Phe
   370 375 380
   Lys Ser Lys Asn Trp Val Asp Lys Tyr Ile Ile Ser Val Ala Val Val
   385                 390                 395                 400
   Ser Cys Ile Thr Ile Val Ile Leu Ile Val Val Ile Thr Phe Cys Val
                   405                 410                 415
   His Gln Cys Ile Gly Leu Asn Arg Lys Pro Tyr Glu Ile Ile Asn Pro
               420                 425                 430
   Phe Asn Thr Ala Tyr Lys Ser Ile Pro Thr Asn Glu Lys Asn Ile Leu
           435                 440                 445
   His Phe Ala Glu Val Thr Glu Ser Asp Tyr Ser Ser Asp Glu Ser Phe
       450                 455                 460
   Asp Ser Asp Ser Glu Glu Leu Asn Gln Arg Gly Glu Thr Ile Gln Gln
   465                 470                 475                 480
   Gly Lys Lys Glu Gln Ser Gly Tyr Thr Ile Trp Phe Asn Glu Asp Leu
                   485                 490                 495
   Glu Glu Ser Val Ser Lys Lys Leu Asn Gln Pro Asn Tyr Ser Lys Ile
               500                 505                 510
   Ile Asn Ser Leu Lys Ser Ile Gln Asn Glu
           515                 520
(2) INFORMATION FOR SEQ ID NO:59:
   (i) SEQUENCE CHARACTERISTICS:
      (A) LENGTH: 237 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..234
   (xi) SEQUENCE DESCRIPTION: SEQ ID NO:59:
   ATG AAT AAA TCT AAA CTC TCA TTT AAA GAA AAA AAC GCT ATA TAT GAA          48
   Met Asn Lys Ser Lys Leu Ser Phe Lys Glu Lys Asn Ala Ile Tyr Glu
     1               5                  10                  15
   TTT AAA AAT ATT TTA TCA AAC ACT TCA TTG TCA ACT TTT CCT GTA TTA          96
   Phe Lys Asn Ile Leu Ser Asn Thr Ser Leu Ser Thr Phe Pro Val Leu
               20                  25                  30
   TCG TTT AAT GAG GAG CCA AAA TCC AGA TTT TTT AAA ATG TTT AAA AAT         144
   Ser Phe Asn Glu Glu Pro Lys Ser Arg Phe Phe Lys Met Phe Lys Asn
           35                  40                  45
   ATT TTA CTG GAA AAA ATA AAA AAA ACT TCA ATG GAT TAT TTA ATT TAT         192
   Ile Leu Leu Glu Lys Ile Lys Lys Thr Ser Met Asp Tyr Leu Ile Tyr
       50                  55                  60
   TGT ACT CTA AAA ATC TCA CTT TCA TTT ATA CTT TAT AAT AAA                    234
   Cys Thr Leu Lys Ile Ser Leu Ser Phe Ile Leu Tyr Asn Lys
    65                 70                  75
   TAA                                                                        237
(2) INFORMATION FOR SEQ ID NO:60:
   (i) SEQUENCE CHARACTERISTICS:
      (A) LENGTH: 78 amino acids
      (B) TYPE: amino acid
      (D) TOPOLOGY: linear
   (ii) MOLECULE TYPE: protein
   (xi) SEQUENCE DESCRIPTION: SEQ ID NO:60:
   Met Asn Lys Ser Lys Leu Ser Phe Lys Glu Lys Asn Ala Ile Tyr Glu
     1               5                  10                  15
   Phe Lys Asn Ile Leu Ser Asn Thr Ser Leu Ser Thr Phe Pro Val Leu
                20                  25                  30
   Ser Phe Asn Glu Glu Pro Lys Ser Arg Phe Phe Lys Met Phe Lys Asn
            35                  40                  45
   Ile Leu Leu Glu Lys Ile Lys Lys Thr Ser Met Asp Tyr Leu Ile Tyr
        50                  55                  60
   Cys Thr Leu Lys Ile Ser Leu Ser Phe Ile Leu Tyr Asn Lys
    65                  70                  75
(2) INFORMATION FOR SEQ ID NO:61:
   (i) SEQUENCE CHARACTERISTICS:
      (A) LENGTH: 360 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..357
   (xi) SEQUENCE DESCRIPTION: SEQ ID NO:61:
   ATG GCT TTA CCA GAT AAC GTT TTT AGT ATT ATT AAT GAA AAT TAT ATC      48
   Met Ala Leu Pro Asp Asn Val Phe Ser Ile Ile Asn Glu Asn Tyr Ile
    1               5                  10                  15
   GAT GGA ATT TTA ACT ATG AAA ATG GGT GAA GAA ATA GAA AGC TCA TCA      96
   Asp Gly Ile Leu Thr Met Lys Met Gly Glu Glu Ile Glu Ser Ser Ser
               20                  25                  30
   CCA TTA AAT GAA ACA AAT GTT AAT ATA GAT CAA CAT ACA ATA GAT ATT     144
   Pro Leu Asn Glu Thr Asn Val Asn Ile Asp Gln His Thr Ile Asp Ile
           35                  40                  45
   TTT GAT TAC GAT TCA GAT AAT GGA TGT TAT TAT AGT GAA AGA GAT AAT     192
   Phe Asp Tyr Asp Ser Asp Asn Gly Cys Tyr Tyr Ser Glu Arg Asp Asn
       50                  55                  60
   GAA ACC GCA ACT CTT TTT TTA AAA CGT GTT GGT TAT AGA GAA ACC TCA     240
   Glu Thr Ala Thr Leu Phe Leu Lys Arg Val Gly Tyr Arg Glu Thr Ser
   65                  70                  75                  80
   AAA AAG CGT AAA CGG ATT TGT GGA TTT ATT GTT TTA GCA ATT TTT ATG     288
   Lys Lys Arg Lys Arg Ile Cys Gly Phe Ile Val Leu Ala Ile Phe Met
                   85                  90                  95
   GTT ATT ATA TTA TGT TTT TTA TCA ATA ATT TTG GGA GTT TTT ATA GCG     336
   Val Ile Ile Leu Cys Phe Leu Ser Ile Ile Leu Gly Val Phe Ile Ala
               100                 105                 110
   CCT CAT ATT TAT AAA GGC CTA TAG                                     360
   Pro His Ile Tyr Lys Gly Leu
           115
(2) INFORMATION FOR SEQ ID NO:62:
   (i) SEQUENCE CHARACTERISTICS:
      (A) LENGTH: 119 amino acids
      (B) TYPE: amino acid
      (D) TOPOLOGY: linear
   (ii) MOLECULE TYPE: protein
   (xi) SEQUENCE DESCRIPTION: SEQ ID NO:62:
   Met Ala Leu Pro Asp Asn Val Phe Ser Ile Ile Asn Glu Asn Tyr Ile
    1               5                  10                  15
   Asp Gly Ile Leu Thr Met Lys Met Gly Glu Glu Ile Glu Ser Ser Ser
               20                  25                  30
   Pro Leu Asn Glu Thr Asn Val Asn Ile Asp Gln His Thr Ile Asp Ile
           35                  40                  45
   Phe Asp Tyr Asp Ser Asp Asn Gly Cys Tyr Tyr Ser Glu Arg Asp Asn
       50                  55                  60
   Glu Thr Ala Thr Leu Phe Leu Lys Arg Val Gly Tyr Arg Glu Thr Ser
   65                  70                  75                  80
   Lys Lys Arg Lys Arg Ile Cys Gly Phe Ile Val Leu Ala Ile Phe Met
                   85                  90                  95
   Val Ile Ile Leu Cys Phe Leu Ser Ile Ile Leu Gly Val Phe Ile Ala
               100                 105                 110
   Pro His Ile Tyr Lys Gly Leu
           115
(2) INFORMATION FOR SEQ ID NO:63:
   (i) SEQUENCE CHARACTERISTICS:
      (A) LENGTH: 2044 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:63:
   AAATTTACTA GAACAGAACC TGGATCTGGT GTTATTTTTT CCAGTAAGCC TATATCCCTT 60
   CAGTAATACC CCAAAAACAC CAAAATTTCC ATGGTATGGA GCCACTCATT TGTATAACAA 120
   AAATGTTTTT TGTGAAGCTG TACGTCGATG TGCTTCTAAA CATGCTATAG AAGCCGCATC 180
   ATCTATTTGG GATTTAAATC CACCACAGTC AAATGAAGAA TTGGAAAAGT TTCTAACTAA 240
   AGCGGTTATT CGCATAACCA TATCTGAGGG ACTAGGTATT TTAAAAACTG CAAATACCCC 300
   ATTTAGCTGT GGTCAAAAAA CTGCTGATGA TGTTAAATTT AAGTCTCACT CCTCACGTAG 360
   GAGTAAGAGT CAGTCAAGAA GTAGACACAG TCGGGGTGAT TTCGACGACA GTAGTGATTA 420
   AAGTTTGTTA CACCCACTAA TTTAAATAAA TAAAAAATTT ATATTTAAAG CTATTTGTCT 480
   GTCTTTTTTT GTTATATATA TTCTTGCTTA GTGAGAGTAT AAACTATTTT GTTTTTAAAA 540
   ATGGAATTTA ACATAGAAGA CTTTGATGAA TCGTTGCTAG GGGCTGTTGG ATACTCTAAT 600
   AATTTTAAAG GTAAGCAAAG CCTTCCGATT AAGGCTTCTA GTCCATCATC GTTAATTAAA 660
   AATCTTTTAG ATGAATTAAA TTTTCCGGAA GGTCCTAGTT TATTATCTTC TATGGAAAAA 720
   TGGAATGAGG ATTTATTTTC CTGCATCCCA AGATTTTTGG AAATCTACAT TGAAAATTCT 780
   ATTTTATCAA CATCTGTCGA TGAGGTTATT AAAAATTTGG ATAATTCTTT AAATTATGAT 840
   GATGTAATCG ATTTTCAGGT CCATGGACCT GAAACATTTC CAAAAACCCC ATTATTGGAA 900
   GAGGAATTGG AAAATTATGT AACATCTGTT CAAAAGTATT TTTTATCTGA ACTTAAAGCT 960
   AGAGAAGTTA CATATTCATT TCTACTCACT AAATATTGTA AAGCGCTTTT GTTATATCTT 1020
   CGCTATAATA CAAAATCATC GATTAAGGGT AATAAGGACA TAAATGCATT TCACCAAAAA 1080
   TTTAAACAAA ATGTGCGGGA ACGTTATTAT AGAGAGGTTG CAAATATAGC ACGATTGTTA 1140
   TATTTACATC TGTATTTATC AGTAACTAGG GAAGTGTCTT GGAAACTACA TGCCGATCAA 1200
   GTATTACTCC AAAGTGTTTT TGTTTCATTG TCTTATTCTT GGAGCCACCG ACGACAGTTT 1260
   GAGTGTATAT TTCATCCAAT TTTATTTAAT CATGGTATTG TGAATTTGGA AAATAACCCT 1320
   TTGACATTTA AGGAACTACA AAAAATAAAT TATAGACGTC ATATTCTTGG TTTACCATTG 1380
   ATTAGAGCTG GATTGGTAGA AGAAGATAAT CAACCTTTAA TGATACCTCC AGAGTTTTCC 1440
   AGTAAACTAC CTCGAACAAT AGGATTTTTA ACTCAACAAA TTAGAGCCAA AATGGAAGCT 1500
   TATTCAGACA ACCATCCTGT AACACCAAAA TTTCCTCGTA TTGAACATTC ATATGCTAAA 1560
   CCTATAGATC CTATTAACTA TGGAACTACA ATAGAAGCTA TGATGGACCC ACCATCACCA 1620
   AGCGCTATTT TACCAGGAGA TCCAAATCCT GAAATTAATG TTAAGGTTAA AAGCACTGTT 1680
   TCATCCTTTC AAATTCCACC TAATATTACC TTGGAAGAAC TGGAGTCAGG TGAATATAAT 1740
   TTATTTACAG ATGGTGTTAC CTACAATGAT ATACCTGAAA ATGAGTTAAA TAAAATGTTT 1800
   CAATTATAAT TTTTTTTTAA TTTTTTCCAT TTAAAACGTT AGTATATAAT ATGAGGTTAT 1860
   ATTAATCAAT AACACCAATA TATTGGGGAA TTGCCACTAA GATACACGTG AGTGGTACTT 1920
   TTGTAGTTAG TGGGTATAAA TAAGGTGGGG TAAGGTGGGG TTCAAATCAT TTTTTATTAC 1980
   TCAGTGTTTG CTTAAGAAAT TATATATTTA ATATATTTAC TATGGAAAGA GACCATGGTT 2040
   TTGC 2044
(2) INFORMATION FOR SEQ ID NO:64:
   (i) SEQUENCE CHARACTERISTICS:
      (A) LENGTH: 2044 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:64:
   GCAAAACCAT GGTCTCTTTC CATAGTAAAT ATATTAAATA TATAATTTCT TAAGCAAACA 60
   CTGAGTAATA AAAAATGATT TGAACCCCAC CTTACCCCAC CTTATTTATA CCCACTAACT 120
   ACAAAAGTAC CACTCACGTG TATCTTAGTG GCAATTCCCC AATATATTGG TGTTATTGAT 180
   TAATATAACC TCATATTATA TACTAACGTT TTAAATGGAA AAAATTAAAA AAAAATTATA 240
   ATTGAAACAT TTTATTTAAC TCATTTTCAG GTATATCATT GTAGGTAACA CCATCTGTAA 300
   ATAAATTATA TTCACCTGAC TCCAGTTCTT CCAAGGTAAT ATTAGGTGGA ATTTGAAAGG 360
   ATGAAACAGT GCTTTTAACC TTAACATTAA TTTCAGGATT TGGATCTCCT GGTAAAATAG 420
   CGCTTGGTGA TGGTGGGTCC ATCATAGCTT CTATTGTAGT TCCATAGTTA ATAGGATCTA 480
   TAGGTTTAGC ATATGAATGT TCAATACGAG GAAATTTTGG TGTTACAGGA TGGTTGTCTG 540
   AATAAGCTTC CATTTTGGCT CTAATTTGTT GAGTTAAAAA TCCTATTGTT CGAGGTAGTT 600
   TACTGGAAAA CTCTGGAGGT ATCATTAAAG GTTGATTATC TTCTTCTACC AATCCAGCTC 660
   TAATCAATGG TAAACCAAGA ATATGACGTC TATAATTTAT TTTTTGTAGT TCCTTAAATG 720
   TCAAAGGGTT ATTTTCCAAA TTCACAATAC CATGATTAAA TAAAATTGGA TGAAATATAC 780
   ACTCAAACTG TCGTCGGTGG CTCCAAGAAT AAGACAATGA AACAAAAACA CTTTGGAGTA 840
   ATACTTGATC GGCATGTAGT TTCCAAGACA CTTCCCTAGT TACTGATAAA TACAGATGTA 900
   AATATAACAA TCGTGCTATA TTTGCAACCT CTCTATAATA ACGTTCCCGC ACATTTTGTT 960
   TAAATTTTTG GTGAAATGCA TTTATGTCCT TATTACCCTT AATCGATGAT TTTGTATTAT    1020
   AGCGAAGATA TAACAAAAGC GCTTTACAAT ATTTAGTGAG TAGAAATGAA TATGTAACTT    1080
   CTCTAGCTTT AAGTTCAGAT AAAAAATACT TTTGAACAGA TGTTACATAA TTTTCCAATT    1140
   CCTCTTCCAA TAATGGGGTT TTTGGAAATG TTTCAGGTCC ATGGACCTGA AAATCGATTA    1200
   CATCATCATA ATTTAAAGAA TTATCCAAAT TTTTAATAAC CTCATCGACA GATGTTGATA    1260
   AAATAGAATT TTCAATGTAG ATTTCCAAAA ATCTTGGGAT GCAGGAAAAT AAATCCTCAT    1320
   TCCATTTTTC CATAGAAGAT AATAAACTAG GACCTTCCGG AAAATTTAAT TCATCTAAAA    1380
   GATTTTTAAT TAACGATGAT GGACTAGAAG CCTTAATCGG AAGGCTTTGC TTACCTTTAA    1440
   AATTATTAGA GTATCCAACA GCCCCTAGCA ACGATTCATC AAAGTCTTCT ATGTTAAATT    1500
   CCATTTTTAA AAACAAAATA GTTTATACTC TCACTAAGCA AGAATATATA TAACAAAAAA    1560
   AGACAGACAA ATAGCTTTAA ATATAAATTT TTTATTTATT TAAATTAGTG GGTGTAACAA    1620
   ACTTTAATCA CTACTGTCGT CGAAATCACC CCGACTGTGT CTACTTCTTG ACTGACTCTT    1680
   ACTCCTACGT GAGGAGTGAG ACTTAAATTT AACATCATCA GCAGTTTTTT GACCACAGCT    1740
   AAATGGGGTA TTTGCAGTTT TTAAAATACC TAGTCCCTCA GATATGGTTA TGCGAATAAC    1800
   CGCTTTAGTT AGAAACTTTT CCAATTCTTC ATTTGACTGT GGTGGATTTA AATCCCAAAT    1860
   AGATGATGCG GCTTCTATAG CATGTTTAGA AGCACATCGA CGTACAGCTT CACAAAAAAC    1920
   ATTTTTGTTA TACAAATGAG TGGCTCCATA CCATGGAAAT TTTGGTGTTT TTGGGGTATT    1980
   ACTGAAGGGA TATAGGCTTA CTGGAAAAAA TAACACCAGA TCCAGGTTCT GTTCTAGTAA    2040
   ATTT                                                                2044
(2) INFORMATION FOR SEQ ID NO:65:
   (i) SEQUENCE CHARACTERISTICS:
      (A) LENGTH: 420 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..417
   (xi) SEQUENCE DESCRIPTION: SEQ ID NO:65:
   AAT TTA CTA GAA CAG AAC CTG GAT CTG GTG TTA TTT TTT CCA GTA AGC      48
   Asn Leu Leu Glu Gln Asn Leu Asp Leu Val Leu Phe Phe Pro Val Ser
     1               5                  10                  15
   CTA TAT CCC TTC AGT AAT ACC CCA AAA ACA CCA AAA TTT CCA TGG TAT          96
   Leu Tyr Pro Phe Ser Asn Thr Pro Lys Thr Pro Lys Phe Pro Trp Tyr
               20                  25                  30
   GGA GCC ACT CAT TTG TAT AAC AAA AAT GTT TTT TGT GAA GCT GTA CGT         144
   Gly Ala Thr His Leu Tyr Asn Lys Asn Val Phe Cys Glu Ala Val Arg
           35                  40                  45
   CGA TGT GCT TCT AAA CAT GCT ATA GAA GCC GCA TCA TCT ATT TGG GAT         192
   Arg Cys Ala Ser Lys His Ala Ile Glu Ala Ala Ser Ser Ile Trp Asp
       50                  55                  60
   TTA AAT CCA CCA CAG TCA AAT GAA GAA TTG GAA AAG TTT CTA ACT AAA         240
   Leu Asn Pro Pro Gln Ser Asn Glu Glu Leu Glu Lys Phe Leu Thr Lys
   65                  70                  75                  80
   GCG GTT ATT CGC ATA ACC ATA TCT GAG GGA CTA GGT ATT TTA AAA ACT         288
   Ala Val Ile Arg Ile Thr Ile Ser Glu Gly Leu Gly Ile Leu Lys Thr
                   85                  90                  95
   GCA AAT ACC CCA TTT AGC TGT GGT CAA AAA ACT GCT GAT GAT GTT AAA         336
   Ala Asn Thr Pro Phe Ser Cys Gly Gln Lys Thr Ala Asp Asp Val Lys
               100                 105                 110
   TTT AAG TCT CAC TCC TCA CGT AGG AGT AAG AGT CAG TCA AGA AGT AGA         384
   Phe Lys Ser His Ser Ser Arg Arg Ser Lys Ser Gln Ser Arg Ser Arg
           115                 120                 125
   CAC AGT CGG GGT GAT TTC GAC GAC AGT AGT GAT TAA                         420
   His Ser Arg Gly Asp Phe Asp Asp Ser Ser Asp
       130                 135
(2) INFORMATION FOR SEQ ID NO:66:
   (i) SEQUENCE CHARACTERISTICS:
      (A) LENGTH: 139 amino acids
      (B) TYPE: amino acid
      (D) TOPOLOGY: linear
   (ii) MOLECULE TYPE: protein
   (xi) SEQUENCE DESCRIPTION: SEQ ID NO:66:
   Asn Leu Leu Glu Gln Asn Leu Asp Leu Val Leu Phe Phe Pro Val Ser
   1               5                   10                  15
   Leu Tyr Pro Phe Ser Asn Thr Pro Lys Thr Pro Lys Phe Pro Trp Tyr
               20                  25                  30
   Gly Ala Thr His Leu Tyr Asn Lys Asn Val Phe Cys Glu Ala Val Arg
           35                  40                  45
   Arg Cys Ala Ser Lys His Ala Ile Glu Ala Ala Ser Ser Ile Trp Asp
       50                  55                  60
   Leu Asn Pro Pro Gln Ser Asn Glu Glu Leu Glu Lys Phe Leu Thr Lys
   65                  70                  75                  80
   Ala Val Ile Arg Ile Thr Ile Ser Glu Gly Leu Gly Ile Leu Lys Thr
                   85                  90                  95
   Ala Asn Thr Pro Phe Ser Cys Gly Gln Lys Thr Ala Asp Asp Val Lys
               100                 105                 110
   Phe Lys Ser His Ser Ser Arg Arg Ser Lys Ser Gln Ser Arg Ser Arg
           115                 120                 125
   His Ser Arg Gly Asp Phe Asp Asp Ser Ser Asp
       130                 135
(2) INFORMATION FOR SEQ ID NO:67:
   (i) SEQUENCE CHARACTERISTICS:
      (A) LENGTH: 1269 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1266
   (xi) SEQUENCE DESCRIPTION: SEQ ID NO:67:
   ATG GAA TTT AAC ATA GAA GAC TTT GAT GAA TCG TTG CTA GGG GCT GTT       48
   Met Glu Phe Asn Ile Glu Asp Phe Asp Glu Ser Leu Leu Gly Ala Val
     1               5                  10                  15
   GGA TAC TCT AAT AAT TTT AAA GGT AAG CAA AGC CTT CCG ATT AAG GCT       96
   Gly Tyr Ser Asn Asn Phe Lys Gly Lys Gln Ser Leu Pro Ile Lys Ala
                20                  25                  30
   TCT AGT CCA TCA TCG TTA ATT AAA AAT CTT TTA GAT GAA TTA AAT TTT      144
   Ser Ser Pro Ser Ser Leu Ile Lys Asn Leu Leu Asp Glu Leu Asn Phe
            35                  40                  45
   CCG GAA GGT CCT AGT TTA TTA TCT TCT ATG GAA AAA TGG AAT GAG GAT      192
   Pro Glu Gly Pro Ser Leu Leu Ser Ser Met Glu Lys Trp Asn Glu Asp
        50                  55                  60
   TTA TTT TCC TGC ATC CCA AGA TTT TTG GAA ATC TAC ATT GAA AAT TCT      240
   Leu Phe Ser Cys Ile Pro Arg Phe Leu Glu Ile Tyr Ile Glu Asn Ser
    65                  70                  75                  80
   ATT TTA TCA ACA TCT GTC GAT GAG GTT ATT AAA AAT TTG GAT AAT TCT      288
   Ile Leu Ser Thr Ser Val Asp Glu Val Ile Lys Asn Leu Asp Asn Ser
                    85                  90                  95
   TTA AAT TAT GAT GAT GTA ATC GAT TTT CAG GTC CAT GGA CCT GAA ACA      336
   Leu Asn Tyr Asp Asp Val Ile Asp Phe Gln Val His Gly Pro Glu Thr
               100                 105                 110
   TTT CCA AAA ACC CCA TTA TTG GAA GAG GAA TTG GAA AAT TAT GTA ACA      384
   Phe Pro Lys Thr Pro Leu Leu Glu Glu Glu Leu Glu Asn Tyr Val Thr
           115                 120                 125
   TCT GTT CAA AAG TAT TTT TTA TCT GAA CTT AAA GCT AGA GAA GTT ACA      432
   Ser Val Gln Lys Tyr Phe Leu Ser Glu Leu Lys Ala Arg Glu Val Thr
       130                 135                 140
   TAT TCA TTT CTA CTC ACT AAA TAT TGT AAA GCG CTT TTG TTA TAT CTT      480
   Tyr Ser Phe Leu Leu Thr Lys Tyr Cys Lys Ala Leu Leu Leu Tyr Leu
   145                 150                 155                 160
   CGC TAT AAT ACA AAA TCA TCG ATT AAG GGT AAT AAG GAC ATA AAT GCA      528
   Arg Tyr Asn Thr Lys Ser Ser Ile Lys Gly Asn Lys Asp Ile Asn Ala
                   165                 170                 175
   TTT CAC CAA AAA TTT AAA CAA AAT GTG CGG GAA CGT TAT TAT AGA GAG      576
   Phe His Gln Lys Phe Lys Gln Asn Val Arg Glu Arg Tyr Tyr Arg Glu
               180                 185                 190
   GTT GCA AAT ATA GCA CGA TTG TTA TAT TTA CAT CTG TAT TTA TCA GTA      624
   Val Ala Asn Ile Ala Arg Leu Leu Tyr Leu His Leu Tyr Leu Ser Val
           195                 200                 205
   ACT AGG GAA GTG TCT TGG AAA CTA CAT GCC GAT CAA GTA TTA CTC CAA      672
   Thr Arg Glu Val Ser Trp Lys Leu His Ala Asp Gln Val Leu Leu Gln
       210                 215                 220
   AGT GTT TTT GTT TCA TTG TCT TAT TCT TGG AGC CAC CGA CGA CAG TTT      720
   Ser Val Phe Val Ser Leu Ser Tyr Ser Trp Ser His Arg Arg Gln Phe
   225                 230                 235                 240
   GAG TGT ATA TTT CAT CCA ATT TTA TTT AAT CAT GGT ATT GTG AAT TTG      768
   Glu Cys Ile Phe His Pro Ile Leu Phe Asn His Gly Ile Val Asn Leu
                   245                 250                 255
   GAA AAT AAC CCT TTG ACA TTT AAG GAA CTA CAA AAA ATA AAT TAT AGA      816
   Glu Asn Asn Pro Leu Thr Phe Lys Glu Leu Gln Lys Ile Asn Tyr Arg
               260                 265                 270
   CGT CAT ATT CTT GGT TTA CCA TTG ATT AGA GCT GGA TTG GTA GAA GAA      864
   Arg His Ile Leu Gly Leu Pro Leu Ile Arg Ala Gly Leu Val Glu Glu
           275                 280                 285
   GAT AAT CAA CCT TTA ATG ATA CCT CCA GAG TTT TCC AGT AAA CTA CCT      912
   Asp Asn Gln Pro Leu Met Ile Pro Pro Glu Phe Ser Ser Lys Leu Pro
       290                 295                 300
   CGA ACA ATA GGA TTT TTA ACT CAA CAA ATT AGA GCC AAA ATG GAA GCT      960
   Arg Thr Ile Gly Phe Leu Thr Gln Gln Ile Arg Ala Lys Met Glu Ala
   305                 310                 315                 320
   TAT TCA GAC AAC CAT CCT GTA ACA CCA AAA TTT CCT CGT ATT GAA CAT     1008
   Tyr Ser Asp Asn His Pro Val Thr Pro Lys Phe Pro Arg Ile Glu His
                   325                 330                 335
   TCA TAT GCT AAA CCT ATA GAT CCT ATT AAC TAT GGA ACT ACA ATA GAA     1056
   Ser Tyr Ala Lys Pro Ile Asp Pro Ile Asn Tyr Gly Thr Thr Ile Glu
               340                 345                 350
   GCT ATG ATG GAC CCA CCA TCA CCA AGC GCT ATT TTA CCA GGA GAT CCA     1104
   Ala Met Met Asp Pro Pro Ser Pro Ser Ala Ile Leu Pro Gly Asp Pro
           355                 360                 365
   AAT CCT GAA ATT AAT GTT AAG GTT AAA AGC ACT GTT TCA TCC TTT CAA       1152
   Asn Pro Glu Ile Asn Val Lys Val Lys Ser Thr Val Ser Ser Phe Gln
       370                 375                 380
   ATT CCA CCT AAT ATT ACC TTG GAA GAA CTG GAG TCA GGT GAA TAT AAT       1200
   Ile Pro Pro Asn Ile Thr Leu Glu Glu Leu Glu Ser Gly Glu Tyr Asn
   385                 390                 395                 400
   TTA TTT ACA GAT GGT GTT ACC TAC AAT GAT ATA CCT GAA AAT GAG TTA       1248
   Leu Phe Thr Asp Gly Val Thr Tyr Asn Asp Ile Pro Glu Asn Glu Leu
                   405                 410                 415
   AAT AAA ATG TTT CAA TTA TAA                                           1269
   Asn Lys Met Phe Gln Leu
               420
(2) INFORMATION FOR SEQ ID NO:68:
   (i) SEQUENCE CHARACTERISTICS:
      (A) LENGTH: 422 amino acids
      (B) TYPE: amino acid
      (D) TOPOLOGY: linear
   (ii) MOLECULE TYPE: protein
   (xi) SEQUENCE DESCRIPTION: SEQ ID NO:68:
   Met Glu Phe Asn Ile Glu Asp Phe Asp Glu Ser Leu Leu Gly Ala Val
    1               5                  10                  15
   Gly Tyr Ser Asn Asn Phe Lys Gly Lys Gln Ser Leu Pro Ile Lys Ala
               20                  25                  30
   Ser Ser Pro Ser Ser Leu Ile Lys Asn Leu Leu Asp Glu Leu Asn Phe
           35                  40                  45
   Pro Glu Gly Pro Ser Leu Leu Ser Ser Met Glu Lys Trp Asn Glu Asp
       50                  55                  60
   Leu Phe Ser Cys Ile Pro Arg Phe Leu Glu Ile Tyr Ile Glu Asn Ser
   65                  70                  75                  80
   Ile Leu Ser Thr Ser Val Asp Glu Val Ile Lys Asn Leu Asp Asn Ser
                   85                  90                  95
   Leu Asn Tyr Asp Asp Val Ile Asp Phe Gln Val His Gly Pro Glu Thr
               100                 105                 110
   Phe Pro Lys Thr Pro Leu Leu Glu Glu Glu Leu Glu Asn Tyr Val Thr
           115                 120                 125
   Ser Val Gln Lys Tyr Phe Leu Ser Glu Leu Lys Ala Arg Glu Val Thr
       130                 135                 140
   Tyr Ser Phe Leu Leu Thr Lys Tyr Cys Lys Ala Leu Leu Leu Tyr Leu
   145                 150                 155                 160
   Arg Tyr Asn Thr Lys Ser Ser Ile Lys Gly Asn Lys Asp Ile Asn Ala
                   165                 170                 175
   Phe His Gln Lys Phe Lys Gln Asn Val Arg Glu Arg Tyr Tyr Arg Glu
               180                 185                 190
   Val Ala Asn Ile Ala Arg Leu Leu Tyr Leu His Leu Tyr Leu Ser Val
           195                 200                 205
   Thr Arg Glu Val Ser Trp Lys Leu His Ala Asp Gln Val Leu Leu Gln
       210                 215                 220
   Ser Val Phe Val Ser Leu Ser Tyr Ser Trp Ser His Arg Arg Gln Phe
   225                 230                 235                 240
   Glu Cys Ile Phe His Pro Ile Leu Phe Asn His Gly Ile Val Asn Leu
                   245                 250                 255
   Glu Asn Asn Pro Leu Thr Phe Lys Glu Leu Gln Lys Ile Asn Tyr Arg
               260                 265                 270
   Arg His Ile Leu Gly Leu Pro Leu Ile Arg Ala Gly Leu Val Glu Glu
           275                 280                 285
   Asp Asn Gln Pro Leu Met Ile Pro Pro Glu Phe Ser Ser Lys Leu Pro
       290                 295                 300
   Arg Thr Ile Gly Phe Leu Thr Gln Gln Ile Arg Ala Lys Met Glu Ala
   305                 310                 315                 320
   Tyr Ser Asp Asn His Pro Val Thr Pro Lys Phe Pro Arg Ile Glu His
                   325                 330                 335
   Ser Tyr Ala Lys Pro Ile Asp Pro Ile Asn Tyr Gly Thr Thr Ile Glu
               340                 345                 350
   Ala Met Met Asp Pro Pro Ser Pro Ser Ala Ile Leu Pro Gly Asp Pro
           355                 360                 365
   Asn Pro Glu Ile Asn Val Lys Val Lys Ser Thr Val Ser Ser Phe Gln
       370                 375                 380
   Ile Pro Pro Asn Ile Thr Leu Glu Glu Leu Glu Ser Gly Glu Tyr Asn
   385                 390                 395                 400
   Leu Phe Thr Asp Gly Val Thr Tyr Asn Asp Ile Pro Glu Asn Glu Leu
                   405                 410                 415
   Asn Lys Met Phe Gln Leu
               420
(2) INFORMATION FOR SEQ ID NO:69:
   (i) SEQUENCE CHARACTERISTICS:
      (A) LENGTH: 626 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:69:
   AAGCTTATTG TCCGTGCTTT GCTGATGGTT TCTGGAACTG TCTCCCCTAT TTTAAGATAC    60
   AATATAGAAG GAGTAAAATG TAAACTAAGT TCATGGAACC TTACAACCGC TGCCTTTTCT   120
   ACCCCATCTA CTGTTAATGA AAAAATTAAT GATGTGGTTG AAACAACTTC AAACCCTTTG   180
   AGTAAACTAA AAAAGAATTG TAATAGAGAA AAGGAACTTT CAAAATCAAA AAGTATAGTT   240
   TCAGGAGGTG TTAGTGTTCA TGGATTAGAA CAAAGCTGTA GCTCTCATAC CTCCAATTTT   300
   CAGAAATGCC CAGATAAAAC CAAGTCATCA AATAAAAATG ATGCAAACAA ACGTGAGTCA   360
   AGGGGAAAAA GAAAGTCTGA ACCAATAGTA AATAGTTTTG GAGTCGCAAA AGTTTCATCC   420
   AACCCACCGC CATCAAAAAA GAGAGCATCA TCACAATCTA CCGGACCACT TGGACCAATG   480
   CCAGAAGAAG GACCGACCCC CAAGGGTGGT TTTAGAAGAA TACCTTCTGG GGATTGTCAT   540
   ACCCCAGTTC CAAGGGACAT TGTAAAATCT ATCTACTGTT CACCAGAGAC TGTGAAAGAA   600
   TTAACAGATC ATCCATTGTT TCCTGA                                         626
(2) INFORMATION FOR SEQ ID NO:70:
   (i) SEQUENCE CHARACTERISTICS:
      (A) LENGTH: 626 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:70:
   TCAGGAAACA ATGGATGATC TGTTAATTCT TTCACAGTCT CTGGTGAACA GTAGATAGAT    60
   TTTACAATGT CCCTTGGAAC TGGGGTATGA CAATCCCCAG AAGGTATTCT TCTAAAACCA   120
   CCCTTGGGGG TCGGTCCTTC TTCTGGCATT GGTCCAAGTG GTCCGGTAGA TTGTGATGAT   180
   GCTCTCTTTT TTGATGGCGG TGGGTTGGAT GAAACTTTTG CGACTCCAAA ACTATTTACT   240
   ATTGGTTCAG ACTTTCTTTT TCCCCTTGAC TCACGTTTGT TTGCATCATT TTTATTTGAT   300
   GACTTGGTTT TATCTGGGCA TTTCTGAAAA TTGGAGGTAT GAGAGCTACA GCTTTGTTCT   360
   AATCCATGAA CACTAACACC TCCTGAAACT ATACTTTTTG ATTTTGAAAG TTCCTTTTCT   420
   CTATTACAAT TCTTTTTTAG TTTACTCAAA GGGTTTGAAG TTGTTTCAAC CACATCATTA   480
   ATTTTTTCAT TAACAGTAGA TGGGGTAGAA AAGGCAGCGG TTGTAAGGTT CCATGAACTT   540
   AGTTTACATT TTACTCCTTC TATATTGTAT CTTAAAATAG GGGAGACAGT TCCAGAAACC   600
   ATCAGCAAAG CACGGACAAT AAGCTT                                         626
(2) INFORMATION FOR SEQ ID NO:71:
   (i) SEQUENCE CHARACTERISTICS:
      (A) LENGTH: 624 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..624
   (xi) SEQUENCE DESCRIPTION: SEQ ID NO:71:
   AAG CTT ATT GTC CGT GCT TTG CTG ATG GTT TCT GGA ACT GTC TCC CCT       48
   Lys Leu Ile Val Arg Ala Leu Leu Met Val Ser Gly Thr Val Ser Pro
     1               5                  10                  15
   ATT TTA AGA TAC AAT ATA GAA GGA GTA AAA TGT AAA CTA AGT TCA TGG       96
   Ile Leu Arg Tyr Asn Ile Glu Gly Val Lys Cys Lys Leu Ser Ser Trp
                20                  25                  30
   AAC CTT ACA ACC GCT GCC TTT TCT ACC CCA TCT ACT GTT AAT GAA AAA      144
   Asn Leu Thr Thr Ala Ala Phe Ser Thr Pro Ser Thr Val Asn Glu Lys
            35                  40                  45
   ATT AAT GAT GTG GTT GAA ACA ACT TCA AAC CCT TTG AGT AAA CTA AAA      192
   Ile Asn Asp Val Val Glu Thr Thr Ser Asn Pro Leu Ser Lys Leu Lys
        50                  55                  60
   AAG AAT TGT AAT AGA GAA AAG GAA CTT TCA AAA TCA AAA AGT ATA GTT      240
   Lys Asn Cys Asn Arg Glu Lys Glu Leu Ser Lys Ser Lys Ser Ile Val
    65                  70                  75                  80
   TCA GGA GGT GTT AGT GTT CAT GGA TTA GAA CAA AGC TGT AGC TCT CAT      288
   Ser Gly Gly Val Ser Val His Gly Leu Glu Gln Ser Cys Ser Ser His
                    85                  90                  95
   ACC TCC AAT TTT CAG AAA TGC CCA GAT AAA ACC AAG TCA TCA AAT AAA      336
   Thr Ser Asn Phe Gln Lys Cys Pro Asp Lys Thr Lys Ser Ser Asn Lys
               100                 105                 110
   AAT GAT GCA AAC AAA CGT GAG TCA AGG GGA AAA AGA AAG TCT GAA CCA      384
   Asn Asp Ala Asn Lys Arg Glu Ser Arg Gly Lys Arg Lys Ser Glu Pro
           115                 120                 125
   ATA GTA AAT AGT TTT GGA GTC GCA AAA GTT TCA TCC AAC CCA CCG CCA      432
   Ile Val Asn Ser Phe Gly Val Ala Lys Val Ser Ser Asn Pro Pro Pro
       130                 135                 140
   TCA AAA AAG AGA GCA TCA TCA CAA TCT ACC GGA CCA CTT GGA CCA ATG      480
   Ser Lys Lys Arg Ala Ser Ser Gln Ser Thr Gly Pro Leu Gly Pro Met
   145                 150                 155                 160
   CCA GAA GAA GGA CCG ACC CCC AAG GGT GGT TTT AGA AGA ATA CCT TCT      528
   Pro Glu Glu Gly Pro Thr Pro Lys Gly Gly Phe Arg Arg Ile Pro Ser
                   165                 170                 175
   GGG GAT TGT CAT ACC CCA GTT CCA AGG GAC ATT GTA AAA TCT ATC TAC        576
   Gly Asp Cys His Thr Pro Val Pro Arg Asp Ile Val Lys Ser Ile Tyr
               180                 185                 190
   TGT TCA CCA GAG ACT GTG AAA GAA TTA ACA GAT CAT CCA TTG TTT CCT        624
   Cys Ser Pro Glu Thr Val Lys Glu Leu Thr Asp His Pro Leu Phe Pro
           195                 200                 205
(2) INFORMATION FOR SEQ ID NO:72:
   (i) SEQUENCE CHARACTERISTICS:
      (A) LENGTH: 208 amino acids
      (B) TYPE: amino acid
      (D) TOPOLOGY: linear
   (ii) MOLECULE TYPE: protein
   (xi) SEQUENCE DESCRIPTION: SEQ ID NO:72:
   Lys Leu Ile Val Arg Ala Leu Leu Met Val Ser Gly Thr Val Ser Pro
    1               5                  10                  15
   Ile Leu Arg Tyr Asn Ile Glu Gly Val Lys Cys Lys Leu Ser Ser Trp
               20                  25                  30
   Asn Leu Thr Thr Ala Ala Phe Ser Thr Pro Ser Thr Val Asn Glu Lys
           35                  40                  45
   Ile Asn Asp Val Val Glu Thr Thr Ser Asn Pro Leu Ser Lys Leu Lys
       50                  55                  60
   Lys Asn Cys Asn Arg Glu Lys Glu Leu Ser Lys Ser Lys Ser Ile Val
   65                  70                  75                  80
   Ser Gly Gly Val Ser Val His Gly Leu Glu Gln Ser Cys Ser Ser His
                   85                  90                  95
   Thr Ser Asn Phe Gln Lys Cys Pro Asp Lys Thr Lys Ser Ser Asn Lys
               100                 105                 110
   Asn Asp Ala Asn Lys Arg Glu Ser Arg Gly Lys Arg Lys Ser Glu Pro
           115                 120                 125
   Ile Val Asn Ser Phe Gly Val Ala Lys Val Ser Ser Asn Pro Pro Pro
       130                 135                 140
   Ser Lys Lys Arg Ala Ser Ser Gln Ser Thr Gly Pro Leu Gly Pro Met
   145                 150                 155                 160
   Pro Glu Glu Gly Pro Thr Pro Lys Gly Gly Phe Arg Arg Ile Pro Ser
                   165                 170                 175
   Gly Asp Cys His Thr Pro Val Pro Arg Asp Ile Val Lys Ser Ile Tyr
               180                 185                 190
   Cys Ser Pro Glu Thr Val Lys Glu Leu Thr Asp His Pro Leu Phe Pro
           195                 200                 205
(2) INFORMATION FOR SEQ ID NO:73:
   (i) SEQUENCE CHARACTERISTICS:
      (A) LENGTH: 655 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:73:
   AAGCTTGACT TGTTGATTAA AGTTAAAGAA TTGTTTAACA AATTAACATT CTTAGGTCTT     60
   CCTCTAGGTC GTTTAATAAC AGGCTCATTT TTGTTGTCTG TAGAGTCATA CCTGTTTTCG    120
   AGTTTGTGTT TAGAAGTCAT CATAAACAAG AAGTAGTTTC AGTCAAACCG GTTTTTTGAG    180
   ATATACAACC AAGTGGTGGT GGAGGTAATA TAGGAGCTTC TGGTGAAAGC TGTGATGGAT    240
   AAAATAATCT GTCTATTATA TCAAAAAATT TGGTTTTTAA ACCTTTGGGT GTATTTATTT    300
   TCATAATATC ATCAGCCAAT CCCCGGAGAG CTATAAATGG ATTAACCCAA AATACATCAT    360
   TCGTTGATTT GAACCATAAA ATGATTTCTA TTGGGTTACA ATCCGTTCTT ATTATAATTC    420
   CAGAAAGGTT TTTTATATCT TTATTTATTT TTTTATATAT ATTTTTTTCT ATTTGATGAT    480
   TACGGCATGG TCCTTGAAGT AGTATATTAA TGTTGTTATA TTGATTTTTA CTCGACGGAA    540
   GCATGGTTAA AATATCTTTT ATATACGAAG AAACAATTAG AATTATTAAT GAATTTATTA    600
   AACCCCATCT TCTAAAATTG TGGAGAATAT GAAAAATATT CCGTTTTATA TACAA          655
(2) INFORMATION FOR SEQ ID NO:74:
   (i) SEQUENCE CHARACTERISTICS:
      (A) LENGTH: 655 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:74:
   TTGTATATAA AACGGAATAT TTTTCATATT CTCCACAATT TTAGAAGATG GGGTTTAATA     60
   AATTCATTAA TAATTCTAAT TGTTTCTTCG TATATAAAAG ATATTTTAAC CATGCTTCCG    120
   TCGAGTAAAA ATCAATATAA CAACATTAAT ATACTACTTC AAGGACCATG CCGTAATCAT    180
   CAAATAGAAA AAAATATATA TAAAAAAATA AATAAAGATA TAAAAAACCT TTCTGGAATT    240
   ATAATAAGAA CGGATTGTAA CCCAATAGAA ATCATTTTAT GGTTCAAATC AACGAATGAT    300
   GTATTTTGGG TTAATCCATT TATAGCTCTC CGGGGATTGG CTGATGATAT TATGAAAATA    360
   AATACACCCA AAGGTTTAAA AACCAAATTT TTTGATATAA TAGACAGATT ATTTTATCCA    420
   TCACAGCTTT CACCAGAAGC TCCTATATTA CCTCCACCAC CACTTGGTTG TATATCTCAA      480
   AAAACCGGTT TGACTGAAAC TACTTCTTGT TTATGATGAC TTCTAAACAC AAACTCGAAA      540
   ACAGGTATGA CTCTACAGAC AACAAAAATG AGCCTGTTAT TAAACGACCT AGAGGAAGAC      600
   CTAAGAATGT TAATTTGTTA AACAATTCTT TAACTTTAAT CAACAAGTCA AGCTT           655
(2) INFORMATION FOR SEQ ID NO:75:
   (i) SEQUENCE CHARACTERISTICS:
      (A) LENGTH: 516 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..513
   (xi) SEQUENCE DESCRIPTION: SEQ ID NO:75:
   TTG TAT ATA AAA CGG AAT ATT TTT CAT ATT CTC CAC AAT TTT AGA AGA         48
   Leu Tyr Ile Lys Arg Asn Ile Phe His Ile Leu His Asn Phe Arg Arg
     1               5                  10                  15
   TGG GGT TTA ATA AAT TCA TTA ATA ATT CTA ATT GTT TCT TCG TAT ATA         96
   Trp Gly Leu Ile Asn Ser Leu Ile Ile Leu Ile Val Ser Ser Tyr Ile
                20                  25                  30
   AAA GAT ATT TTA ACC ATG CTT CCG TCG AGT AAA AAT CAA TAT AAC AAC        144
   Lys Asp Ile Leu Thr Met Leu Pro Ser Ser Lys Asn Gln Tyr Asn Asn
            35                  40                  45
   ATT AAT ATA CTA CTT CAA GGA CCA TGC CGT AAT CAT CAA ATA GAA AAA        192
   Ile Asn Ile Leu Leu Gln Gly Pro Cys Arg Asn His Gln Ile Glu Lys
        50                  55                  60
   AAT ATA TAT AAA AAA ATA AAT AAA GAT ATA AAA AAC CTT TCT GGA ATT        240
   Asn Ile Tyr Lys Lys Ile Asn Lys Asp Ile Lys Asn Leu Ser Gly Ile
    65                  70                  75                  80
   ATA ATA AGA ACG GAT TGT AAC CCA ATA GAA ATC ATT TTA TGG TTC AAA        288
   Ile Ile Arg Thr Asp Cys Asn Pro Ile Glu Ile Ile Leu Trp Phe Lys
                    85                  90                  95
   TCA ACG AAT GAT GTA TTT TGG GTT AAT CCA TTT ATA GCT CTC CGG GGA        336
   Ser Thr Asn Asp Val Phe Trp Val Asn Pro Phe Ile Ala Leu Arg Gly
               100                 105                 110
   TTG GCT GAT GAT ATT ATG AAA ATA AAT ACA CCC AAA GGT TTA AAA ACC        384
   Leu Ala Asp Asp Ile Met Lys Ile Asn Thr Pro Lys Gly Leu Lys Thr
           115                 120                 125
   AAA TTT TTT GAT ATA ATA GAC AGA TTA TTT TAT CCA TCA CAG CTT TCA        432
   Lys Phe Phe Asp Ile Ile Asp Arg Leu Phe Tyr Pro Ser Gln Leu Ser
       130                 135                 140
   CCA GAA GCT CCT ATA TTA CCT CCA CCA CCA CTT GGT TGT ATA TCT CAA        480
   Pro Glu Ala Pro Ile Leu Pro Pro Pro Pro Leu Gly Cys Ile Ser Gln
   145                 150                 155                 160
   AAA ACC GGT TTG ACT GAA ACT ACT TCT TGT TTA TGA                        516
   Lys Thr Gly Leu Thr Glu Thr Thr Ser Cys Leu
                   165                 170
(2) INFORMATION FOR SEQ ID NO:76:
   (i) SEQUENCE CHARACTERISTICS:
      (A) LENGTH: 171 amino acids
      (B) TYPE: amino acid
      (D) TOPOLOGY: linear
   (ii) MOLECULE TYPE: protein
   (xi) SEQUENCE DESCRIPTION: SEQ ID NO:76:
   Leu Tyr Ile Lys Arg Asn Ile Phe His Ile Leu His Asn Phe Arg Arg
    1               5                  10                  15
   Trp Gly Leu Ile Asn Ser Leu Ile Ile Leu Ile Val Ser Ser Tyr Ile
               20                  25                  30
   Lys Asp Ile Leu Thr Met Leu Pro Ser Ser Lys Asn Gln Tyr Asn Asn
           35                  40                  45
   Ile Asn Ile Leu Leu Gln Gly Pro Cys Arg Asn His Gln Ile Glu Lys
       50                  55                  60
   Asn Ile Tyr Lys Lys Ile Asn Lys Asp Ile Lys Asn Leu Ser Gly Ile
   65                  70                  75                  80
   Ile Ile Arg Thr Asp Cys Asn Pro Ile Glu Ile Ile Leu Trp Phe Lys
                   85                  90                  95
   Ser Thr Asn Asp Val Phe Trp Val Asn Pro Phe Ile Ala Leu Arg Gly
               100                 105                 110
   Leu Ala Asp Asp Ile Met Lys Ile Asn Thr Pro Lys Gly Leu Lys Thr
           115                 120                 125
   Lys Phe Phe Asp Ile Ile Asp Arg Leu Phe Tyr Pro Ser Gln Leu Ser
       130                 135                 140
   Pro Glu Ala Pro Ile Leu Pro Pro Pro Pro Leu Gly Cys Ile Ser Gln
   145                 150                 155                 160
   Lys Thr Gly Leu Thr Glu Thr Thr Ser Cys Leu
                   165                 170
(2) INFORMATION FOR SEQ ID NO:77:
   (i) SEQUENCE CHARACTERISTICS:
      (A) LENGTH: 1823 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:77:
   GCTTTGGACG AGATTTAGGC TGGGAAATTG TTAGTTGAGC ATATTGATTT TCGCATAGTT     60
   CATAAATATG GTCTTCGTCA TCATCATCAC AAACTTGATC ATACACATTT TTATACTCAA    120
   AAGTTGGAAT AGTTCGTTCG TAAATAGGAT TTTCCCTTTC AATTCGTTGA GATGACTTTC    180
   TTCTTACACC GTTTTGTCTC TTAATAGTAG CATATATCGG TTCATCTTCA TATGATGAAA    240
   TACGACGGCT GGCCATAATT CTATAAATAA GAGAAGGTTA AAAATAAGTT GCTTAACTGG    300
   TTTAGGCAAC TAATAAAATA TCTCTAAAAC ATACCCCCTT TTATATAAAT CTGTGGATTA    360
   GTTTCGGATT TCGAAACCCA CCTTCTTATT AGTGTTGCAT CTGACGAAGT TCCCGACTAA    420
   CCATTCTCAA ACAGGTTCTA TAAGAATAAC AAAATATCGC AACCCCTACT GTTACCAGGG    480
   ATATATAAAA CAAAATAGCG GAAGTTGAAG GCGTATTAAT ATTGATACCC CTAGCTGAAC    540
   AACTAGAGCT CCAAAACTGT CTCCTAGCTT CAGCTCTAGA ATAATCTAAA CGATCATCGT    600
   CGAAGGCATA AACTGAAAAA ATAATGGATA GTAAAAATAC TATCTCCATT GCTTTGATTG    660
   AAAAACAAAA ACCTTTTAAT TGGACTATCA ACAAAATAAA TGAAAGCCTT CTGATGACCA    720
   ACAACGAAGA AATAAACTTG ACTGAAAATT TTAAAAATAC GGGCACCTTT TATAGCAAGA    780
   TAATTGACCT TGAAATCCGA ACTGCTACAT CTAGTCAATA TGCAGTCTTT GTTACACAAA    840
   TGTGTTCTGA TGACGAAAAT ATGAATAATA CAAATATTTT TGTTATTAAT GGTGTTATTG    900
   ATTCTGGATA TAGAGGAATA GTTAAAGCGT TAGTTTATTA CCATCCAACT GTAGAAAAAT    960
   TAAATCCATA CGATCTTAAA ATTAAACTTC CACTAATAGA ACTTAGTAAA GATTTAATAC   1020
   CACTATCACC TAGTTTACAT AGTTATAGTG AATTATATAA TTTTTTTAAT GTCTTTAATA   1080
   AAAAACGTGA TGAAGATGCT GGTTATGATA TACCATCTCC AAATTTAGTT CAAATAAAAC   1140
   CGGGATATAG TTACCTTTTT TGTCTTCCTA TTTTTCAATT AGAAATGAAA AACCCACCAA   1200
   TCGCTTGTAT TTTTGGTAGA TCATCCTTAA ATTCAAGCGG AATAATTGTT CTTCCAACTA   1260
   TATGGAAACC AAAAACAATT TGTCAATTTT TTATTAAAAA TATATCCTCT AAAACTGTAA   1320
   CTATAGAAAA AGGTCAGAGA ATAGCTCAGT TAGTTCTTTT AAAAAACAAT CAACCACTAT   1380
   GGTTACAACC ACAAATTAAT TGTCATTCTT TATTTCCAAA GTCAAACTAT TTAAGCTTAT   1440
   CAAATCGAGA ATGTGATATG TGGAAGTTTA CAGAAGATCT GAATTTTGAA GCACCGAAAA   1500
   GTTTACGAGG AATAAATGGA TTTGGATCCA CGGGATTGTA AAATTCGTTA ATAAAGTTAT   1560
   ATTTAAAGTG CCAAACTTTC ACGTGTCATT TTTTTGGGAC CGTTTCTTTT TTGTTTAGTC   1620
   GATAAAATAT TTTCAGTTTC CATAGAACTT ATTAGAGGTT CTGTATCTAG TATATCTGTA   1680
   GAATTATTTT CATCATATTT AACGGTTTGA AGAGATAAGG GTTTTGTTGT ATTAGAATCT   1740
   ATACCAAGGG TTTTTTCTAA AACCGCTACA TCTGCCATAA CAATATTATT TTCTGAAGTC 1800
   ATTTTTATGG CTTGGGCACC ACC 1823
(2) INFORMATION FOR SEQ ID NO:78:
   (i) SEQUENCE CHARACTERISTICS:
      (A) LENGTH: 1823 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:78:
   GGTGGTGCCC AAGCCATAAA AATGACTTCA GAAAATAATA TTGTTATGGC AGATGTAGCG 60
   GTTTTAGAAA AAACCCTTGG TATAGATTCT AATACAACAA AACCCTTATC TCTTCAAACC 120
   GTTAAATATG ATGAAAATAA TTCTACAGAT ATACTAGATA CAGAACCTCT AATAAGTTCT 180
   ATGGAAACTG AAAATATTTT ATCGACTAAA CAAAAAAGAA ACGGTCCCAA AAAAATGACA 240
   CGTGAAAGTT TGGCACTTTA AATATAACTT TATTAACGAA TTTTACAATC CCGTGGATCC 300
   AAATCCATTT ATTCCTCGTA AACTTTTCGG TGCTTCAAAA TTCAGATCTT CTGTAAACTT 360
   CCACATATCA CATTCTCGAT TTGATAAGCT TAAATAGTTT GACTTTGGAA ATAAAGAATG 420
   ACAATTAATT TGTGGTTGTA ACCATAGTGG TTGATTGTTT TTTAAAAGAA CTAACTGAGC 480
   TATTCTCTGA CCTTTTTCTA TAGTTACAGT TTTAGAGGAT ATATTTTTAA TAAAAAATTG 540
   ACAAATTGTT TTTGGTTTCC ATATAGTTGG AAGAACAATT ATTCCGCTTG AATTTAAGGA 600
   TGATCTACCA AAAATACAAG CGATTGGTGG GTTTTTCATT TCTAATTGAA AAATAGGAAG 660
   ACAAAAAAGG TAACTATATC CCGGTTTTAT TTGAACTAAA TTTGGAGATG GTATATCATA 720
   ACCAGCATCT TCATCACGTT TTTTATTAAA GACATTAAAA AAATTATATA ATTCACTATA 780
   ACTATGTAAA CTAGGTGATA GTGGTATTAA ATCTTTACTA AGTTCTATTA GTGGAAGTTT 840
   AATTTTAAGA TCGTATGGAT TTAATTTTTC TACAGTTGGA TGGTAATAAA CTAACGCTTT 900
   AACTATTCCT CTATATCCAG AATCAATAAC ACCATTAATA ACAAAAATAT TTGTATTATT 960
   CATATTTTCG TCATCAGAAC ACATTTGTGT AACAAAGACT GCATATTGAC TAGATGTAGC 1020
   AGTTCGGATT TCAAGGTCAA TTATCTTGCT ATAAAAGGTG CCCGTATTTT TAAAATTTTC 1080
   AGTCAAGTTT ATTTCTTCGT TGTTGGTCAT CAGAAGGCTT TCATTTATTT TGTTGATAGT 1140
   CCAATTAAAA GGTTTTTGTT TTTCAATCAA AGCAATGGAG ATAGTATTTT TACTATCCAT 1200
   TATTTTTTCA GTTTATGCCT TCGACGATGA TCGTTTAGAT TATTCTAGAG CTGAAGCTAG 1260
   GAGACAGTTT TGGAGCTCTA GTTGTTCAGC TAGGGGTATC AATATTAATA CGCCTTCAAC 1320
   TTCCGCTATT TTGTTTTATA TATCCCTGGT AACAGTAGGG GTTGCGATAT TTTGTTATTC    1380
   TTATAGAACC TGTTTGAGAA TGGTTAGTCG GGAACTTCGT CAGATGCAAC ACTAATAAGA    1440
   AGGTGGGTTT CGAAATCCGA AACTAATCCA CAGATTTATA TAAAAGGGGG TATGTTTTAG    1500
   AGATATTTTA TTAGTTGCCT AAACCAGTTA AGCAACTTAT TTTTAACCTT CTCTTATTTA    1560
   TAGAATTATG GCCAGCCGTC GTATTTCATC ATATGAAGAT GAACCGATAT ATGCTACTAT    1620
   TAAGAGACAA AACGGTGTAA GAAGAAAGTC ATCTCAACGA ATTGAAAGGG AAAATCCTAT    1680
   TTACGAACGA ACTATTCCAA CTTTTGAGTA TAAAAATGTG TATGATCAAG TTTGTGATGA    1740
   TGATGACGAA GACCATATTT ATGAACTATG CGAAAATCAA TATGCTCAAC TAACAATTTC    1800
   CCAGCCTAAA TCTCGTCCAA AGC                                            1823
(2) INFORMATION FOR SEQ ID NO:79:
   (i) SEQUENCE CHARACTERISTICS:
      (A) LENGTH: 918 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..915
   (xi) SEQUENCE DESCRIPTION: SEQ ID NO:79:
   ATG GAT AGT AAA AAT ACT ATC TCC ATT GCT TTG ATT GAA AAA CAA AAA       48
   Met Asp Ser Lys Asn Thr Ile Ser Ile Ala Leu Ile Glu Lys Gln Lys
     1               5                  10                  15
   CCT TTT AAT TGG ACT ATC AAC AAA ATA AAT GAA AGC CTT CTG ATG ACC       96
   Pro Phe Asn Trp Thr Ile Asn Lys Ile Asn Glu Ser Leu Leu Met Thr
                20                  25                  30
   AAC AAC GAA GAA ATA AAC TTG ACT GAA AAT TTT AAA AAT ACG GGC ACC      144
   Asn Asn Glu Glu Ile Asn Leu Thr Glu Asn Phe Lys Asn Thr Gly Thr
            35                  40                  45
   TTT TAT AGC AAG ATA ATT GAC CTT GAA ATC CGA ACT GCT ACA TCT AGT      192
   Phe Tyr Ser Lys Ile Ile Asp Leu Glu Ile Arg Thr Ala Thr Ser Ser
        50                  55                  60
   CAA TAT GCA GTC TTT GTT ACA CAA ATG TGT TCT GAT GAC GAA AAT ATG      240
   Gln Tyr Ala Val Phe Val Thr Gln Met Cys Ser Asp Asp Glu Asn Met
    65                  70                  75                  80
   AAT AAT ACA AAT ATT TTT GTT ATT AAT GGT GTT ATT GAT TCT GGA TAT      288
   Asn Asn Thr Asn Ile Phe Val Ile Asn Gly Val Ile Asp Ser Gly Tyr
                    85                  90                  95
   AGA GGA ATA GTT AAA GCG TTA GTT TAT TAC CAT CCA ACT GTA GAA AAA         336
   Arg Gly Ile Val Lys Ala Leu Val Tyr Tyr His Pro Thr Val Glu Lys
               100                 105                 110
   TTA AAT CCA TAC GAT CTT AAA ATT AAA CTT CCA CTA ATA GAA CTT AGT         384
   Leu Asn Pro Tyr Asp Leu Lys Ile Lys Leu Pro Leu Ile Glu Leu Ser
           115                 120                 125
   AAA GAT TTA ATA CCA CTA TCA CCT AGT TTA CAT AGT TAT AGT GAA TTA         432
   Lys Asp Leu Ile Pro Leu Ser Pro Ser Leu His Ser Tyr Ser Glu Leu
       130                 135                 140
   TAT AAT TTT TTT AAT GTC TTT AAT AAA AAA CGT GAT GAA GAT GCT GGT         480
   Tyr Asn Phe Phe Asn Val Phe Asn Lys Lys Arg Asp Glu Asp Ala Gly
   145                 150                 155                 160
   TAT GAT ATA CCA TCT CCA AAT TTA GTT CAA ATA AAA CCG GGA TAT AGT         528
   Tyr Asp Ile Pro Ser Pro Asn Leu Val Gln Ile Lys Pro Gly Tyr Ser
                   165                 170                 175
   TAC CTT TTT TGT CTT CCT ATT TTT CAA TTA GAA ATG AAA AAC CCA CCA         576
   Tyr Leu Phe Cys Leu Pro Ile Phe Gln Leu Glu Met Lys Asn Pro Pro
               180                 185                 190
   ATC GCT TGT ATT TTT GGT AGA TCA TCC TTA AAT TCA AGC GGA ATA ATT         624
   Ile Ala Cys Ile Phe Gly Arg Ser Ser Leu Asn Ser Ser Gly Ile Ile
           195                 200                 205
   GTT CTT CCA ACT ATA TGG AAA CCA AAA ACA ATT TGT CAA TTT TTT ATT         672
   Val Leu Pro Thr Ile Trp Lys Pro Lys Thr Ile Cys Gln Phe Phe Ile
       210                 215                 220
   AAA AAT ATA TCC TCT AAA ACT GTA ACT ATA GAA AAA GGT CAG AGA ATA         720
   Lys Asn Ile Ser Ser Lys Thr Val Thr Ile Glu Lys Gly Gln Arg Ile
   225                 230                 235                 240
   GCT CAG TTA GTT CTT TTA AAA AAC AAT CAA CCA CTA TGG TTA CAA CCA         768
   Ala Gln Leu Val Leu Leu Lys Asn Asn Gln Pro Leu Trp Leu Gln Pro
                   245                 250                 255
   CAA ATT AAT TGT CAT TCT TTA TTT CCA AAG TCA AAC TAT TTA AGC TTA         816
   Gln Ile Asn Cys His Ser Leu Phe Pro Lys Ser Asn Tyr Leu Ser Leu
               260                 265                 270
   TCA AAT CGA GAA TGT GAT ATG TGG AAG TTT ACA GAA GAT CTG AAT TTT         864
   Ser Asn Arg Glu Cys Asp Met Trp Lys Phe Thr Glu Asp Leu Asn Phe
           275                 280                 285
   GAA GCA CCG AAA AGT TTA CGA GGA ATA AAT GGA TTT GGA TCC ACG GGA         912
   Glu Ala Pro Lys Ser Leu Arg Gly Ile Asn Gly Phe Gly Ser Thr Gly
       290                 295                 300
   TTG TAA                                                                 918
   Leu
   305
(2) INFORMATION FOR SEQ ID NO:80:
   (i) SEQUENCE CHARACTERISTICS:
      (A) LENGTH: 305 amino acids
      (B) TYPE: amino acid
      (D) TOPOLOGY: linear
   (ii) MOLECULE TYPE: protein
   (xi) SEQUENCE DESCRIPTION: SEQ ID NO:80:
   Met Asp Ser Lys Asn Thr Ile Ser Ile Ala Leu Ile Glu Lys Gln Lys
    1               5                  10                  15
   Pro Phe Asn Trp Thr Ile Asn Lys Ile Asn Glu Ser Leu Leu Met Thr
               20                  25                  30
   Asn Asn Glu Glu Ile Asn Leu Thr Glu Asn Phe Lys Asn Thr Gly Thr
           35                  40                  45
   Phe Tyr Ser Lys Ile Ile Asp Leu Glu Ile Arg Thr Ala Thr Ser Ser
       50                  55                  60
   Gln Tyr Ala Val Phe Val Thr Gln Met Cys Ser Asp Asp Glu Asn Met
   65                  70                  75                  80
   Asn Asn Thr Asn Ile Phe Val Ile Asn Gly Val Ile Asp Ser Gly Tyr
                   85                  90                  95
   Arg Gly Ile Val Lys Ala Leu Val Tyr Tyr His Pro Thr Val Glu Lys
               100                 105                 110
   Leu Asn Pro Tyr Asp Leu Lys Ile Lys Leu Pro Leu Ile Glu Leu Ser
           115                 120                 125
   Lys Asp Leu Ile Pro Leu Ser Pro Ser Leu His Ser Tyr Ser Glu Leu
       130                 135                 140
   Tyr Asn Phe Phe Asn Val Phe Asn Lys Lys Arg Asp Glu Asp Ala Gly
   145                 150                 155                 160
   Tyr Asp Ile Pro Ser Pro Asn Leu Val Gln Ile Lys Pro Gly Tyr Ser
                   165                 170                 175
   Tyr Leu Phe Cys Leu Pro Ile Phe Gln Leu Glu Met Lys Asn Pro Pro
               180                 185                 190
   Ile Ala Cys Ile Phe Gly Arg Ser Ser Leu Asn Ser Ser Gly Ile Ile
           195                 200                 205
   Val Leu Pro Thr Ile Trp Lys Pro Lys Thr Ile Cys Gln Phe Phe Ile
       210                 215                 220
   Lys Asn Ile Ser Ser Lys Thr Val Thr Ile Glu Lys Gly Gln Arg Ile
   225                 230                 235                 240
   Ala Gln Leu Val Leu Leu Lys Asn Asn Gln Pro Leu Trp Leu Gln Pro
                   245                 250                 255
   Gln Ile Asn Cys His Ser Leu Phe Pro Lys Ser Asn Tyr Leu Ser Leu
               260                 265                 270
   Ser Asn Arg Glu Cys Asp Met Trp Lys Phe Thr Glu Asp Leu Asn Phe
           275                 280                 285
   Glu Ala Pro Lys Ser Leu Arg Gly Ile Asn Gly Phe Gly Ser Thr Gly
       290                 295                 300
   Leu
   305
(2) INFORMATION FOR SEQ ID NO:81:
   (i) SEQUENCE CHARACTERISTICS:
      (A) LENGTH: 261 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..258
   (xi) SEQUENCE DESCRIPTION: SEQ ID NO:81:
   ATG GAG ATA GTA TTT TTA CTA TCC ATT ATT TTT TCA GTT TAT GCC TTC          48
   Met Glu Ile Val Phe Leu Leu Ser Ile Ile Phe Ser Val Tyr Ala Phe
     1               5                  10                  15
   GAC GAT GAT CGT TTA GAT TAT TCT AGA GCT GAA GCT AGG AGA CAG TTT          96
   Asp Asp Asp Arg Leu Asp Tyr Ser Arg Ala Glu Ala Arg Arg Gln Phe
                20                  25                  30
   TGG AGC TCT AGT TGT TCA GCT AGG GGT ATC AAT ATT AAT ACG CCT TCA         144
   Trp Ser Ser Ser Cys Ser Ala Arg Gly Ile Asn Ile Asn Thr Pro Ser
            35                  40                  45
   ACT TCC GCT ATT TTG TTT TAT ATA TCC CTG GTA ACA GTA GGG GTT GCG         192
   Thr Ser Ala Ile Leu Phe Tyr Ile Ser Leu Val Thr Val Gly Val Ala
        50                  55                  60
   ATA TTT TGT TAT TCT TAT AGA ACC TGT TTG AGA ATG GTT AGT CGG GAA         240
   Ile Phe Cys Tyr Ser Tyr Arg Thr Cys Leu Arg Met Val Ser Arg Glu
    65                  70                  75                  80
   CTT CGT CAG ATG CAA CAC TAA                                             261
   Leu Arg Gln Met Gln His
                    85
(2) INFORMATION FOR SEQ ID NO:82:
   (i) SEQUENCE CHARACTERISTICS:
      (A) LENGTH: 86 amino acids
      (B) TYPE: amino acid
      (D) TOPOLOGY: linear
   (ii) MOLECULE TYPE: protein
   (xi) SEQUENCE DESCRIPTION: SEQ ID NO:82:
   Met Glu Ile Val Phe Leu Leu Ser Ile Ile Phe Ser Val Tyr Ala Phe
    1               5                  10                  15
   Asp Asp Asp Arg Leu Asp Tyr Ser Arg Ala Glu Ala Arg Arg Gln Phe
               20                  25                  30
   Trp Ser Ser Ser Cys Ser Ala Arg Gly Ile Asn Ile Asn Thr Pro Ser
           35                  40                  45
   Thr Ser Ala Ile Leu Phe Tyr Ile Ser Leu Val Thr Val Gly Val Ala
       50                  55                  60
   Ile Phe Cys Tyr Ser Tyr Arg Thr Cys Leu Arg Met Val Ser Arg Glu
   65                  70                  75                  80
   Leu Arg Gln Met Gln His
                   85
(2) INFORMATION FOR SEQ ID NO:83:
   (i) SEQUENCE CHARACTERISTICS:
      (A) LENGTH: 255 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..255
   (xi) SEQUENCE DESCRIPTION: SEQ ID NO:83:
   ATG GCC AGC CGT CGT ATT TCA TCA TAT GAA GAT GAA CCG ATA TAT GCT      48
   Met Ala Ser Arg Arg Ile Ser Ser Tyr Glu Asp Glu Pro Ile Tyr Ala
    1               5                  10                  15
   ACT ATT AAG AGA CAA AAC GGT GTA AGA AGA AAG TCA TCT CAA CGA ATT      96
   Thr Ile Lys Arg Gln Asn Gly Val Arg Arg Lys Ser Ser Gln Arg Ile
               20                  25                  30
   GAA AGG GAA AAT CCT ATT TAC GAA CGA ACT ATT CCA ACT TTT GAG TAT     144
   Glu Arg Glu Asn Pro Ile Tyr Glu Arg Thr Ile Pro Thr Phe Glu Tyr
           35                  40                  45
   AAA AAT GTG TAT GAT CAA GTT TGT GAT GAT GAT GAC GAA GAC CAT ATT     192
   Lys Asn Val Tyr Asp Gln Val Cys Asp Asp Asp Asp Glu Asp His Ile
       50                  55                  60
   TAT GAA CTA TGC GAA AAT CAA TAT GCT CAA CTA ACA ATT TCC CAG CCT     240
   Tyr Glu Leu Cys Glu Asn Gln Tyr Ala Gln Leu Thr Ile Ser Gln Pro
   65                  70                  75                  80
   AAA TCT CGT CCA AAG                                                 255
   Lys Ser Arg Pro Lys
                   85
(2) INFORMATION FOR SEQ 10 NO:84:
   (i) SEQUENCE CHARACTERISTICS:
      (A) LENGTH: 85 amino acids
      (B) TYPE: amino acid
      (D) TOPOLOGY: linear
   (ii) MOLECULE TYPE: protein
   (xi) SEQUENCE DESCRIPTION: SEQ ID NO:84:
(2) INFORMATION FOR SEQ ID NO:85:
   (i) SEQUENCE CHARACTERISTICS:
      (A) LENGTH: 749 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:85:
   AAGCTTTCAA AATGTGCCAA GTAAATATAA TTAATAAACT CTCTATCAGA AACTTTTAAA     60
   CTCTGTCTAT CAGCAGAAAT ATAATTTCTC AATACACCAA CCTCTGAAAT ATCGGCATTA    120
   ATTCGATGTG TTATATAATC TTCTAGTTTA ACAGCAATCT TTCCTGTAGC ATACCCACTT    180
   TGACAACAAA ATTTTGATAA CAAAGAAAAT GATATTAAAT CTATACATTT AAGATTAGTT    240
   TTGTTTTGTT GTACAGGTAT TTTATATGTT TCGATAAAAT CTTTTATAGC TTGTAGGTCA    300
   TAGGTATGAA AAGGCTTTAA ACTGTTTGTA GCTTGAAATA GATAAAATCT TGTTGCTAAA    360
   ACTAAAGTTT TTTCTTCAGG ACCAAATTTT GAAGTAAACC AAAACGGTGT AGGATTTGTT    420
   CCATATATTC GTCTAAAGGC TGCAAGTATT TGTTGTTCGT GATGAATATA TAATAATGTT    480
   AACCCATGGC GTCCTTTATT ACATTTCGAT AAGCATGTTT TTATAGATAA TGTAGGGTCA    540
   TATTTAGCAG ATTCTAAAGT TCTTCCAGAT TTAGGAGTTA GACGCTCTGT CGTTATAGAT      600
   AATATAGTTA TTAAATCATC ATGAATATTA AACGTATGCT GATCATCAAT ACAAGAAAGT      660
   ATTAATTTTG TAGAGATTGG GTTTCCATAT AATAAAGATT TAGCTATAAC AGACGCTTCA      720
   TAATTATTTT TAATTGAACA TATAAACAT                                        749
(2) INFORMATION FOR SEQ ID NO:86:
   (i) SEQUENCE CHARACTERISTICS:
      (A) LENGTH: 749 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:86:
   ATGTTTATAT GTTCAATTAA AAATAATTAT GAAGCGTCTG TTATAGCTAA ATCTTTATTA       60
   TATGGAAACC CAATCTCTAC AAAATTAATA CTTTCTTGTA TTGATGATCA GCATACGTTT      120
   AATATTCATG ATGATTTAAT AACTATATTA TCTATAACGA CAGAGCGTCT AACTCCTAAA      180
   TCTGGAAGAA CTTTAGAATC TGCTAAATAT GACCCTACAT TATCTATAAA AACATGCTTA      240
   TCGAAATGTA ATAAAGGACG CCATGGGTTA ACATTATTAT ATATTCATCA CGAACAACAA      300
   ATACTTGCAG CCTTTAGACG AATATATGGA ACAAATCCTA CACCGTTTTG GTTTACTTCA      360
   AAATTTGGTC CTGAAGAAAA AACTTTAGTT TTAGCAACAA GATTTTATCT ATTTCAAGCT      420
   ACAAACAGTT TAAAGCCTTT TCATACCTAT GACCTACAAG CTATAAAAGA TTTTATCGAA      480
   ACATATAAAA TACCTGTACA ACAAAACAAA ACTAATCTTA AATGTATAGA TTTAATATCA      540
   TTTTCTTTGT TATCAAAATT TTGTTGTCAA AGTGGGTATG CTACAGGAAA GATTGCTGTT      600
   AAACTAGAAG ATTATATAAC ACATCGAATT AATGCCGATA TTTCAGAGGT TGGTGTATTG      660
   AGAAATTATA TTTCTGCTGA TAGACAGAGT TTAAAAGTTT CTGATAGAGA GTTTATTAAT      720
   TATATTTACT TGGCACATTT TGAAAGCTT                                        749
(2) INFORMATION FOR SEQ ID NO:87:
   (i) SEQUENCE CHARACTERISTICS:
      (A) LENGTH: 747 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..747
   (xi) SEQUENCE DESCRIPTION: SEQ ID NO:87:
   ATG TTT ATA TGT TCA ATT AAA AAT AAT TAT GAA GCG TCT GTT ATA GCT      48
   Met Phe Ile Cys Ser Ile Lys Asn Asn Tyr Glu Ala Ser Val Ile Ala
     1               5                  10                  15
   AAA TCT TTA TTA TAT GGA AAC CCA ATC TCT ACA AAA TTA ATA CTT TCT      96
   Lys Ser Leu Leu Tyr Gly Asn Pro Ile Ser Thr Lys Leu Ile Leu Ser
                20                  25                  30
   TGT ATT GAT GAT CAG CAT ACG TTT AAT ATT CAT GAT GAT TTA ATA ACT     144
   Cys Ile Asp Asp Gln His Thr Phe Asn Ile His Asp Asp Leu Ile Thr
            35                  40                  45
   ATA TTA TCT ATA ACG ACA GAG CGT CTA ACT CCT AAA TCT GGA AGA ACT     192
   Ile Leu Ser Ile Thr Thr Glu Arg Leu Thr Pro Lys Ser Gly Arg Thr
        50                  55                  60
   TTA GAA TCT GCT AAA TAT GAC CCT ACA TTA TCT ATA AAA ACA TGC TTA     240
   Leu Glu Ser Ala Lys Tyr Asp Pro Thr Leu Ser Ile Lys Thr Cys Leu
    65                  70                  75                  80
   TCG AAA TGT AAT AAA GGA CGC CAT GGG TTA ACA TTA TTA TAT ATT CAT     288
   Ser Lys Cys Asn Lys Gly Arg His Gly Leu Thr Leu Leu Tyr Ile His
                    85                  90                  95
   CAC GAA CAA CAA ATA CTT GCA GCC TTT AGA CGA ATA TAT GGA ACA AAT     336
   His Glu Gln Gln Ile Leu Ala Ala Phe Arg Arg Ile Tyr Gly Thr Asn
               100                 105                 110
   CCT ACA CCG TTT TGG TTT ACT TCA AAA TTT GGT CCT GAA GAA AAA ACT     384
   Pro Thr Pro Phe Trp Phe Thr Ser Lys Phe Gly Pro Glu Glu Lys Thr
           115                 120                 125
   TTA GTT TTA GCA ACA AGA TTT TAT CTA TTT CAA GCT ACA AAC AGT TTA     432
   Leu Val Leu Ala Thr Arg Phe Tyr Leu Phe Gln Ala Thr Asn Ser Leu
       130                 135                 140
   AAG CCT TTT CAT ACC TAT GAC CTA CAA GCT ATA AAA GAT TTT ATC GAA     480
   Lys Pro Phe His Thr Tyr Asp Leu Gln Ala Ile Lys Asp Phe Ile Glu
   145                 150                 155                 160
   ACA TAT AAA ATA CCT GTA CAA CAA AAC AAA ACT AAT CTT AAA TGT ATA     528
   Thr Tyr Lys Ile Pro Val Gln Gln Asn Lys Thr Asn Leu Lys Cys Ile
                   165                 170                 175
   GAT TTA ATA TCA TTT TCT TTG TTA TCA AAA TTT TGT TGT CAA AGT GGG     576
   Asp Leu Ile Ser Phe Ser Leu Leu Ser Lys Phe Cys Cys Gln Ser Gly
               180                 185                 190
   TAT GCT ACA GGA AAG ATT GCT GTT AAA CTA GAA GAT TAT ATA ACA CAT     624
   Tyr Ala Thr Gly Lys Ile Ala Val Lys Leu Glu Asp Tyr Ile Thr His
           195                 200                 205
   CGA ATT AAT GCC GAT ATT TCA GAG GTT GGT GTA TTG AGA AAT TAT ATT     672
   Arg Ile Asn Ala Asp Ile Ser Glu Val Gly Val Leu Arg Asn Tyr Ile
       210                 215                 220
   TCT GCT GAT AGA CAG AGT TTA AAA GTT TCT GAT AGA GAG TTT ATT AAT     720
   Ser Ala Asp Arg Gln Ser Leu Lys Val Ser Asp Arg Glu Phe Ile Asn
   225                 230                 235                 240
   TAT ATT TAC TTG GCA CAT TTT GAA AGC                                   747
   Tyr Ile Tyr Leu Ala His Phe Glu Ser
                   245
(2) INFORMATION FOR SEQ ID NO:88:
   (i) SEQUENCE CHARACTERISTICS:
      (A) LENGTH: 249 amino acids
      (B) TYPE: amino acid
      (D) TOPOLOGY: linear
   (ii) MOLECULE TYPE: protein
   (xi) SEQUENCE DESCRIPTION: SEQ ID NO:88:
   Met Phe Ile Cys Ser Ile Lys Asn Asn Tyr Glu Ala Ser Val Ile Ala
    1               5                  10                  15
   Lys Ser Leu Leu Tyr Gly Asn Pro Ile Ser Thr Lys Leu Ile Leu Ser
               20                  25                  30
   Cys Ile Asp Asp Gln His Thr Phe Asn Ile His Asp Asp Leu Ile Thr
           35                  40                  45
   Ile Leu Ser Ile Thr Thr Glu Arg Leu Thr Pro Lys Ser Gly Arg Thr
       50                  55                  60
   Leu Glu Ser Ala Lys Tyr Asp Pro Thr Leu Ser Ile Lys Thr Cys Leu
   65                  70                  75                  80
   Ser Lys Cys Asn Lys Gly Arg His Gly Leu Thr Leu Leu Tyr Ile His
                   85                  90                  95
   His Glu Gln Gln Ile Leu Ala Ala Phe Arg Arg Ile Tyr Gly Thr Asn
               100                 105                 110
   Pro Thr Pro Phe Trp Phe Thr Ser Lys Phe Gly Pro Glu Glu Lys Thr
           115                 120                 125
   Leu Val Leu Ala Thr Arg Phe Tyr Leu Phe Gln Ala Thr Asn Ser Leu
       130                 135                 140
   Lys Pro Phe His Thr Tyr Asp Leu Gln Ala Ile Lys Asp Phe Ile Glu
   145                 150                 155                 160
   Thr Tyr Lys Ile Pro Val Gln Gln Asn Lys Thr Asn Leu Lys Cys Ile
                   165                 170                 175
   Asp Leu Ile Ser Phe Ser Leu Leu Ser Lys Phe Cys Cys Gln Ser Gly
               180                 185                 190
   Tyr Ala Thr Gly Lys Ile Ala Val Lys Leu Glu Asp Tyr Ile Thr His
           195                 200                 205
   Arg Ile Asn Ala Asp Ile Ser Glu Val Gly Val Leu Arg Asn Tyr Ile
       210                 215                 220
   Ser Ala Asp Arg Gln Ser Leu Lys Val Ser Asp Arg Glu Phe Ile Asn
   225 230 235 240
   Tyr Ile Tyr Leu Ala His Phe Glu Ser
   245
(2) INFORMATION FOR SEQ ID NO:89:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..30̅
      (D) OTHER INFORMATION: /label= primer
   (xi) SEQUENCE DESCRIPTION: SEQ ID NO:89:
      GCCGGTACCA GGCTTTGGAC GAGATTTAGG 30
(2) INFORMATION FOR SEQ ID NO:90:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..29̅
      (D) OTHER INFORMATION: /label= primer
   (xi) SEQUENCE DESCRIPTION: SEQ ID NO:90:
      GCCGAATTCA ATATAATTAA TAAACTCTC 29
(2) INFORMATION FOR SEQ ID NO:91:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..28̅
      (D) OTHER INFORMATION: /label= primer
   (xi) SEQUENCE DESCRIPTION: SEQ ID NO:91:
      CCGGAATTCG CTTAGTGAGA GTATAAAC 28
(2) INFORMATION FOR SEQ ID NO:92:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 1..28̅
      (D) OTHER INFORMATION: /label= primer
   (xi) SEQUENCE DESCRIPTION: SEQ ID NO:92:
      CCGGAATTCC CTCATATTAT ATACTAAC 28

While various embodiments of the present invention have been described in detail, it is apparent that modifications and adaptations of those embodiments will occur to those skilled in the art. It is to be expressly understood, however, that such modifications and adaptations are within the scope of the present invention, as set forth in the following claims.

## Claims

1. An isolated canine herpesvirus (CHV) nucleic acid molecule consisting of:
(a) a nucleic acid sequence that is any of:
| | |
|---|---|
| a CdUTPase gene | SEQ ID NO: 13, 15, 77 or 79, |
| a CgE gene | SEQ ID NO: 51 or 57, |
| a CgG gene | SEQ ID NO: 1, 9 or 51, |
| a CgI gene | SEQ ID NO: 29, 32, 51 or 55, |
| a CPK gene | SEQ ID NO: 1, 7 or 51, |
| a CUS2 gene | SEQ ID NO: 2, 5 or 52, |
| a CUS9 gene | SEQ ID NO: 17, 20, 51 or 61, |
| a CUL49 gene | SEQ ID NO: 65, 78 or 83, |
| a CUL51 gene | SEQ ID NO: 14, 33 or 78, |
| a CgL gene | SEQ ID NO: 74 or 75, |
| a CUL48.5 gene | SEQ ID NO: 78 or 81, |
| a CUS8.5 open reading frame | SEQ ID NO: 51 or 59, |
SEQ ID NO:3, SEQ ID NO:19, SEQ ID NO:31 and SEQ ID NO:73; or
(b) a nucleic acid sequence that is fully complementary to a nucleic acid sequence of (a).

2. An isolated CHV nucleic acid molecule consisting of a nucleic acid sequence that is at least 80% identical to a nucleic acid sequence of any of SEQ ID NOs: 1 to 3, 51, 52, 63, 64, 67, 69 to 71 and 85 to 87, and complements of such nucleic acid sequences.

3. An isolated CHV nucleic acid molecule consisting of a nucleic acid sequence that is at least 80% identical to a nucleic acid sequence of any of SEQ ID NOs: 5, 7, 9, 13 to 15, 17, 19, 20, 29, 31 to 33, 55, 57, 59, 61, 65, 73 to 75, 77 to 79, 81 and 83, and complements of such nucleic acid sequences.

4. An isolated nucleic acid molecule either:
(a) consisting of a nucleic acid sequence of any of SEQ ID NOs: 1 to 3, 5, 7, 9, 13 to 15, 17, 19, 20, 29, 31 to 33, 51, 52, 55, 57, 59, 61, 65, 73 to 75, 77 to 79, 81 and 83, and complements of such nucleic acid sequences; or
(b) that is an allelic variant of a CHV nucleic acid molecule consisting of any such nucleic acid sequence.

5. An isolated CHV nucleic acid molecule, which encodes either:
(a) a protein consisting of an amino acid sequence of any of SEQ ID NOs: 4, 6, 8, 10, 16, 18, 30, 34, 56, 58, 60, 62, 66, 76, 80, 82 and 84; or
(b) a protein encoded by an allelic variant of a CHV nucleic acid molecule encoding a protein according to (a).

6. An isolated CHV nucleic acid molecule comprising a recombinant CHV genome in which one or more of the following CHV genes have been inactivated:
| | |
|---|---|
| a CdUTPase gene | SEQ ID NO:_13, 15, 77 or 79; |
| a CgC gene; | |
| a CgE gene | SEQ ID NO:_51 or 57; |
| a CgG gene | SEQ ID NO: 1, 9 or 51; |
| a CgI gene | SEQ ID NO: 29, 32, 51 or 55; |
| a CPK gene | SEQ ID NO:_1, 7 or 51; |
| a CTK gene | SEQ ID NO:_35 or 38; |
| a CIR6 gene | SEQ ID NO: 2, 3 or 52; |
| a CUS2 gene | SEQ ID NO: 2, 5 or 52; |
| a CUS9 gene | SEQ ID NO:_17, 20, 51 or 61; |
| a CUL49 gene | SEQ ID NO: 63, 65, 78 or 83; |
| a CUL 51 gene | SEQ ID NO:14, 33 or 78; |
| a CUL45 gene; | |
| a CgD gene | SEQ ID NO: 1, 11, 51 or 53; |
| a CgB gene; | |
| a CUL48 gene | SEQ ID NO: 21, 24; 63 or 67; |
| a CUL52 gene | SEQ ID NO: 25, 28, 85 or 87; |
| a CgL gene | SEQ ID NO: 74 or 75; |
| a CUL49.5 gene | SEQ ID NO: 78 or 81; |
| a CICP0 gene | SEQ ID NO: 69 or 73; |
| a CICP4 gene | SEQ ID NO: 69, 71 or 73; |
| a CUS8.5 gene | SEQ ID NO: 51 or 59. |

7. An isolated CHV nucleic acid molecule as claimed in Claim 6, further comprising a heterologous nucleic acid molecule operatively linked to a transcription control region.

8. An isolated CHV nucleic acid molecule as claimed in claim 7, wherein the heterologous nucleic acid molecule encodes a protective compound that is a protective protein or protective RNA species, said protective compound being capable of protecting a canid from an infectious, metabolic or genetic disease.

9. An isolated CHV nucleic acid molecule comprising a recombinant CHV genome having a heterologous nucleic acid molecule in a region of the genome that is:
(a) a CHV gene that is any of:
| | |
|---|---|
| a CdUTPase gene | SEQ ID NO: 13, 15, 77 or 79; |
| a CgC gene; | |
| a CgE gene | SEQ ID NO: 51 or 57; |
| a CgG gene | SEQ ID NO: 1, 9 or 51; |
| a CgI gene | SEQ ID NO: 29, 32, 51 or 55; |
| a CPK gene | SEQ ID NO: 1, 7 or 51; |
| a CTK gene | SEQ ID NO: 35 or 38; |
| a CIR6 gene | SEQ ID NO: 2, 3 or 52; |
| a CUS2 gene | SEQ ID NO: 2, 5 or 52; |
| a CUS9 gene | SEQ ID NO: 17, 20, 51 or 61; |
| a CUL49 gene | SEQ ID NO: 63, 65, 78 or 83; |
| a CUL51 gene | SEQ ID NO: 14, 33 or 78; |
| a CUL45 gene; | |
| a CgD gene | SEQ ID NO: 1,11,51 or 53; |
| a CgB gene; | |
| a CUL48 gene | SEQ ID NO: 21, 24; 63 or 67; |
| a CUL52 gene | SEQ ID NO: 25, 28, 85 or 87; |
| a CgL gene | SEQ ID NO: 74 or 75; |
| a CUL49.5 gene | SEQ ID NO: 78 or 81; |
| a CICP0 gene | SEQ ID NO: 69 or 73; |
| a CICP4 gene | SEQ ID NO: 69, 71 or 73; |
| a CUS8.5 open reading frame | SEQ ID NO: 51 or 59; |
(b) a naturally-occurring Asc I restriction endonuclease site within the genome;
(c) a US region of the genome that is SEQ ID NO: 1, 2, 17, 19, 29 or 31; or
(d) an internal inverted repeat region of the genome that is any of:
| | |
|---|---|
| nCAsc₉₃₀₀ | SEQ ID NO: 1, 2, 7, 19, 29 or 31, |
| nCAsc₁₀₀₀₀ | SEQ ID NO: 1, 2, 7, 19, 29 or 31, |
| nCHin₃₀₀₀ | SEQ ID NO: 1, 2, 7, 19, 29 or 31, |
| nCHin₁₉₀₀ | SEQ ID NO: 63, 64, 77, 78, 85 or 86, |
| nCHin₅₅₀₀ | SEQ ID NO: 63, 64, 77, 78, 85 or 86, and |
| nCHin₈₅₀₀ | SEQ ID NO: 63, 64, 77, 78, 85 or 86. |

10. An isolated CHV nucleic acid molecule as claimed in claim 9, further comprising a heterologous nucleic acid molecule in a region of said CHV genome that is any of:
| | |
|---|---|
| a CHV US region comprising nCUS₅₄₉₅ | SEQ ID NO: 1 or 2, |
| a CgE gene comprising nCgE₇₅₀ | SEQ ID NO: 51 or 52, |
| a CgI gene comprising nCgI₁₆₁ | SEQ ID NO: 29 or 31, |
| CUS9 gene comprising nCUS9₅₇₉ | SEQ ID NO: 17 or 19, |
| CHV UL region comprising nCdUTP/CUL51₇₄₃ | SEQ ID NO: 13 or 14, |
| CTK gene comprising nCTK₂₈₀ | SEQ ID NO: 35 or 37, |
| CUL48 gene comprising nCUL48₂₉₄ | SEQ ID NO: 21 or 23, |
| CUL49 gene included in nCHin₃₀₀₀ | SEQ ID NO: 63, 64 77 or 78, |
| CUL52 gene comprising CUL52₁₄₆ | SEQ ID NO: 25 or 27, |
| CHV region comprising nCUS₁₀₅₉₂ | SEQ ID NO: 51 or 52, |
| CHV UL region comprising nCUL₁₈₂₃ | SEQ ID NO: 77 or 78, |
| CHV UL region comprising nCUL49/CUL48₂₀₄₄ | SEQ ID NO: 63 or 64, |
| CHV IR region comprising nCICP4₆₂₆ | SEQ ID NO: 69 or 70, |
| CHV UL region comprising nCgL₆₅₅ | SEQ ID NO: 73 or 74, |
| CHV UL region comprising nCUL52₇₄₉ | SEQ ID NO: 85 or 86, |
| CHV UL region comprising CdUTP₃₂₀₀ | SEQ ID NO: 13 or 14, or |
an allelic variant of any such region.

11. An isolated CHV nucleic acid molecule as claimed in any one of claims 9 or 10, further comprising a heterologous nucleic acid molecule in a region of the CHV genome that is:
(a) a nucleic acid molecule comprising a nucleic acid sequence of any of SEQ ID NOs: 1 to 3, 5, 7, 9, 11, 13 to 15, 17, 19 to 21, 23 to 25, 27 to 29, 31 to 33, 35, 51 to 53, 55, 57, 59, 61, 63 to 65, 69 to 71, 73 to 75, 77 to 79, 81, 83 and 85 to 87, and complements of said nucleic acid sequences; or
(b) an allelic variant of a nucleic acid molecule of (a).

12. A therapeutic composition for use as a delivery vehicle, comprising an isolated CHV nucleic acid molecule as claimed in any one of claims 1 to 11.

13. A transfected cell comprising an isolated CHV nucleic acid molecule as claimed in any one of claims 1 to 11.

14. A composition that is a recombinant molecule, a recombinant virus or a recombinant cell, which composition comprises a molecule as claimed in any one of claims 1 to 5 operatively linked to a transcription control sequence.

15. A recombinant vector comprising a CHV nucleic acid molecule as claimed in any one of claims 1 to 5, which CHV nucleic acid molecule comprises a heterologous nucleic acid molecule being operatively linked to a transcription control sequence.

16. A canine cell line that complements a CHV nucleic acid molecule as claimed in claim 6, having a defect in a gene essential to CHV reproduction *in vitro.*

17. A cell line as claimed in claim 16, wherein the essential gene is any of:
| | |
|---|---|
| a CgD gene | SEQ ID NO: 1, 11, 51 or 53, |
| a CgB gene, | |
| a CUL48 gene | SEQ ID NO: 21, 24, 63 or 67, |
| a CUL52 gene | SEQ ID NO: 25, 28, 85 or 87, |
| a CgL gene | SE ID NO: 74 or 75, and |
| a CICP4 gene | SEQ ID NO: 69, 71 or 73. |

18. An isolated CHV protein consisting of an amino acid sequence selected from:
| | |
|---|---|
| a CdUTPase protein | SEQ ID NO: 16 or 80, |
| a CgE protein | SEQ ID NO: 58, |
| a CgG protein | SEQ ID NO: 10, |
| a CgI protein | SEQ ID NO: 30 or 56, |
| a CPK protein | SEQ ID NO: 8, |
| a CUS2 protein | SEQ ID NO: 6, |
| a CUS 9 protein | SEQ ID NO: 18 or 62, |
| a CUL49 protein | SEQ ID NO: 66 or 84, |
| a CUL51 protein | SEQ ID NO: 34, |
| a CgL protein | SEQ ID NO: 76, and |
| a CUL49.5 protein | SEQ ID NO: 82. |

19. An isolated CHV protein that is at least 80 % identical to a CHV protein having an amino acid sequence of any of SEQ ID NO: 4,68, 72 and 88.

20. An isolated CHV protein comprising an amino acid sequence that is at least 80 % identical to an amino acid sequence of any of SEQ ID NO: 4, 6, 8, 10, 16, 18, 30, 34, 56, 58, 60, 62, 66, 68, 72, 76, 80, 82, 84 and 88.

21. A protein as claimed in claim 20, either:
(a) comprising an amino acid sequence of any of SEQ ID NO: 4, 6, 8, 10, 16, 18, 30, 34, 56, 58, 60, 62, 66, 68, 72, 76, 80, 82, 84 and 88; or
(b) that is coded by an allelic variant of a CHV nucleic acid molecule encoding a CHV protein of (a).

22. An isolated antibody that selectively binds to a CHV protein as claimed in any of claims 18 to 21.

23. A method for increasing recombinant CHV plaque forming efficiency, which method comprises either:
(a) introducing a recombinant CHV genome into a canine cell expressing CHV alpha trans-inducing factor and culturing the cell to produce a recombinant CHV; or
(b) co-introducing a recombinant CHV genome and a CHV alpha trans-inducing factor gene into a canine cell and culturing the cell to produce recombinant CHV.

24. A canine cell line comprising a CHV alpha trans-inducing factor gene.

25. A cell line as claimed in claim 24, wherein the gene comprises nucleic acid molecule nCUL48₁₂₆₉ SEQ ID NO:_67.

26. The use of a cell line. antibody, protein, vector, cell or nucleic acid molecule according to any preceding claim in the preparation of a therapeutic composition for protecting an animal from CHV infection.

## Patentansprüche

1. Ein isoliertes Hunde-Herpesvirus (CHV)-Nukleinsäuremolekül, bestehend aus:
(a) einer Nukleinsäuresequenz, die irgendeine der folgenden ist:
| | |
|---|---|
| ein CdUTPase-Gen | SEQ ID NR: 13, 15, 77 oder 79, |
| ein CgE-Gen | SEQ ID NR: 51 oder 57, |
| ein CgG-Gen | SEQ ID NR: 1, 9 oder 51, |
| ein CgI-Gen | SEQ ID NR: 29, 32, 51 oder 55, |
| ein CPK-Gen | SEQ ID NR: 1, 7 oder 51, |
| ein CUS2-Gen | SEQ ID NR: 2, 5 oder 52 |
| ein CUS9-Gen | SEQ ID NR: 17, 20, 51 oder 61, |
| ein CUL49-Gen | SEQ ID NR: 65, 78 oder 83, |
| ein CUL51-Gen | SEQ ID NR: 14, 33 oder 78, |
| ein CgL-Gen | SEQ ID NR: 74 oder 75, |
| ein CUL48,5-Gen | SEQ ID NR: 78 oder 81, |
| ein CUS8,5 "offenes Leseraster" | SEQ ID NR: 51 oder 59, |
SEQ ID NR: 3, SEQ ID NR: 19, SEQ ID NR: 31 und SEQ ID NR: 73; oder
(b) einer Nukleinsäuresequenz, die voll komplementär zu einer Nukleinsäuresequenz von (a) ist.

2. Ein isoliertes CHV-Nukleinsäuremolekül, bestehend aus einer Nukleinsäuresequenz, die mindestens zu 80 % identisch ist mit irgendeiner der Nukleinsäuresequenzen der SEQ ID NRn: 1 bis 3, 51, 52, 63, 64, 67, 69 bis 71 und 85 bis 87, und Komplementäre solcher Nukleinsäuresequenzen.

3. Ein isoliertes CHV-Nukleinsäuremolekül, bestehend aus einer Nukleinsäuresequenz, die mindestens zu 80 % identisch ist mit irgendeiner der Nukleinsäuresequenzen der SEQ ID NRn: 5, 7, 9, 13 bis 15, 17 19, 20, 29, 31 bis 33, 55, 57, 59, 61, 65, 73 bis 75, 77 bis 79, 81 und 83 und Komplementären solcher Nukleinsäuresequenzen.

4. Ein isoliertes Nukleinsäuremolekül, welches entweder:
(a) aus irgendeiner der Nukleinsäuresequenzen SEQ ID NR: 1 bis 3, 5, 7, 9, 13 bis 15, 17, 19, 20, 29, 31 bis 33, 51, 52, 55, 57, 59, 61, 65, 73 bis 75, 77 bis 79, 81 und 83, und Komplementären solcher Nukleinsäuresequenzen besteht; oder
(b) eine Allelvariante eines CHV-Nukleinsäuremoleküls ist, bestehend aus irgendeiner dieser Nukleinsäuresequenzen.

5. Ein isoliertes CHV-Nukleinsäuremolekül, welches entweder:
(a) ein Protein kodiert, bestehend aus irgendeiner der Aminosäuresequenzen SEQ ID NR: 4, 6, 8, 10,16, 18, 30, 34, 56, 58, 60, 62, 66, 76, 80, 82 und 84 oder
(b) ein Protein kodiert, das durch eine Allelvariante eines CHV-Nukleinsäuremoleküls kodiert wird, welches ein Protein gemäß (a) kodiert.

6. Ein isoliertes CHV-Nukleinsäuremolekül, beinhaltend ein rekombinantes CHV-Genom, in welchem eines oder mehrere der nachfolgenden CHV-Gene inaktiviert wurde(n):
| | |
|---|---|
| ein CdUTPase-Gen | SEQ ID NR: 13, 15, 77 oder 79; |
| ein CgC-Gen | |
| ein CgE-Gen | SEQ ID NR: 51 oder 57; |
| ein CgG-Gen | SEQ ID NR: 1, 9 oder 51; |
| ein CgI-Gen | SEQ ID NR: 29, 32, 51 oder 55; |
| ein CPK-Gen | SEQ ID NR: 1, 7 oder 51; |
| ein CTK-Gen | SEQ ID NR: 35 oder 38; |
| ein CIR6-Gen | SEQ ID NR: 2, 3 oder 52; |
| ein CUS2-Gen | SEQ ID NR: 2, 5 oder 52; |
| ein CUS9-Gen | SEQ ID NR: 17, 20, 51 oder 61; |
| ein CUL49-Gen | SEQ ID NR: 63, 65, 78 oder 83; |
| ein CUL51-Gen | SEQ ID NR: 14, 33 oder 78; |
| ein CUL45-Gen | |
| ein CgD-Gen | SEQ ID NR: 1, 11, 51 oder 53; |
| ein CgB-Gen | |
| ein CUL48-Gen | SEQ ID NR: 21, 24; 63 oder 67; |
| ein CUL52-Gen | SEQ ID NR: 25, 28, 85 oder 87; |
| ein CgL-Gen | SEQ ID NR: 74 oder 75; |
| ein CUL49,5-Gen | SEQ ID NR: 78 oder 81; |
| ein CICP0-Gen | SEQ ID NR: 69 oder 73; |
| ein CICP4-Gen | SEQ ID NR: 69, 71 oder 73; |
| ein CUS8,5-Gen | SEQ ID NR: 51 oder 59. |

7. Ein isoliertes CHV-Nukleinsäuremolekül nach Anspruch 6, weiterhin beinhaltend ein heterologes Nukleinsäuremolekül, das operativ mit einem Transkriptionssteuerbereich verbunden ist.

8. Ein isoliertes CHV-Nukleinsäuremolekül nach Anspruch 7, wobei das heterologe Nukleinsäuremolekül eine schützende Verbindung kodiert, welche ein schützendes Protein oder eine schützende RNS-Spezies ist, wobei die schützende Verbindung dazu geeignet ist, einen Hund vor einer infektiösen, metabolischen oder genetischen Krankheit zu schützen.

9. Ein isoliertes CHV-Nukleinsäuremolekül, beinhaltend ein rekombinantes CHV-Genom, das ein heterologes Nukleinsäuremolekül in einem Bereich des Genoms aufweist, welches
(a) ein CHV-Gen ist, das irgendeines der folgenden ist:
| | |
|---|---|
| ein CdUTPase-Gen | SEQ ID NR: 13, 15, 77 oder 79; |
| ein CgC-Gen; | |
| ein CgE-Gen | SEQ ID NR: 51 oder 57; |
| ein CgG-Gen | SEQ ID NR: 1, 9 oder 51; |
| ein CgI-Gen | SEQ ID NR: 29, 32, 51 oder 55; |
| ein CPK-Gen | SEQ ID NR: 1, 7 oder 51; |
| ein CTK-Gen | SEQ ID NR: 35 oder 38; |
| ein CIR6-Gen | SEQ ID NR: 2, 3 oder 52; |
| ein CUS2-Gen | SEQ ID NR: 2, 5 oder 52; |
| ein CUS9-Gen | SEQ ID NR: 17, 20, 51 oder 61; |
| ein CUL49-Gen | SEQ ID NR: 63, 65, 78 oder 83; |
| ein CUL51-Gen | SEQ ID NR: 14, 33 oder 78; |
| ein CUL 45-Gen; | |
| ein CgD-Gen | SEQ ID NR: 1, 11, 51 oder 53; |
| ein CgB-Gen; | |
| ein CUL48-Gen | SEQ ID NR: 21, 24; 63 oder 67; |
| ein CUL52-Gen | SEQ ID NR: 25, 28, 85 oder 87; |
| ein CgL-Gen | SEQ ID NR: 74 oder 75; |
| ein CUL49,5-Gen | SEQ ID NR: 78 oder 81; |
| ein CICP0-Gen | SEQ ID NR: 69 oder 73; |
| ein CICP4-Gen | SEQ ID NR: 69, 71 oder 73; |
| ein CUS8,5 "offenes Leseraster" | SEQ ID NR: 51 oder 59 |
ist;
(b) eine natürlich vorkommende Asc I-Endonuclease-Spaltstelle in dem Genom ist;
(c) ein US-Bereich des Genoms ist, das der SEQ ID NR: 1, 2, 17, 19, 29 oder 31 entspricht; oder
(d) ein innerer invertierter Wiederholungsbereich des Genoms ist, welcher irgendeiner der folgenden ist:
| | |
|---|---|
| nCAsc₉₃₀₀ | SEQ ID NR: 1, 2, 7, 19, 29 oder 31, |
| nCAsc₁₀₀₀₀ | SEQ ID NR: 1, 2, 7, 19, 29 oder 31, |
| nCHin₃₀₀₀ | SEQ ID NR: 1, 2, 7, 19, 29 oder 31, |
| nCHin₁₉₀₀ | SEQ ID NR: 63, 64, 77, 78, 85 oder 86, |
| nCHin₅₅₀₀ | SEQ ID NR: 63, 64, 77, 78, 85 oder 86 |
| und | |
| nCHin₈₅₀₀ | SEQ ID NR: 63, 64, 77, 78, 85 oder 86. |

10. Ein isoliertes CHV-Nukleinsäuremolekül nach Anspruch 9, weiterhin beinhaltend ein heterologes Nukleinsäuremolekül in einem Bereich des CHV-Genoms, welches irgendeins der folgenden ist:
| | |
|---|---|
| ein CCHV US-Bereich, beinhaltend nCUS₅₄₉₅ | SEQ ID NR: 1 oder 2, |
| ein CgE-Gen, beinhaltend nCgE₇₅₀ | SEQ ID NR: 51 oder 52, |
| ein CgI-Gen, beinhaltend nCgI₁₆₁ | SEQ ID NR: 29 oder 31, |
| ein CUS9-Gen, beinhaltend nCUS9₅₇₉ | SEQ ID NR: 17 oder 19, |
| ein CHV UL-Bereich, | |
| beinhaltend nCdUTP/CUL51₇₄₃ | SEQ ID NR: 13 oder 14, |
| CTK-Gen, beinhaltend nCTK₂₈₀ | SEQ ID NR: 35 oder 37, |
| CUL48-Gen, beinhaltend nCUL48₂₉₄ | SEQ ID NR: 21 oder 23, |
| CUL49-Gen, eingeschl. in nCHin₃₀₀₀ | SEQ ID NR: 63, 64, 77 oder 78 |
| CUL52-Gen, beinhaltend CUL52₁₄₆ | SEQ ID NR: 25 oder 27, |
| CHV-Bereich, beinhaltend nCUS₁₀₅₉₂ | SEQ ID NR: 51 oder 52 |
| CHV UL-Bereich, beinhaltend nCUL₁₈₂₃ | SEQ ID NR: 77 oder 78 |
| CHV UL-Bereich, beinhaltend | |
| nCUL49/CUL48₂₀₄₄ | SEQ ID NR: 63 oder 64 |
| CHV IR-Bereich, beinhaltend nCICP4₆₂₆ | SEQ ID NR: 69 oder 70 |
| CHV UL-Bereich, beinhaltend nCgL₆₅₅ | SEQ ID NR: 73 oder 74 |
| CHV UL-Bereich, beinhaltend nCUL52₇₄₉ | SEQ ID NR: 85 oder 86, |
| CHV UL-Bereich, beinhaltend CdUTP₃₂₀₀ | SEQ ID NR: 13 oder 14 oder |
eine Allelvariante von einem solchen Bereich.

11. Ein isoliertes CHV-Nukleinsäuremolekül nach irgendeinem der Ansprüche 9 oder 10, weiterhin beinhaltend ein heterologes Nukleinsäuremolekül im Bereich des CHV-Genoms, welches:
(a) ein Nukleinsäuremolekül ist, beinhaltend irgendeine der Nukleinsäuresequenzen SEQ ID NR: 1 bis 3, 5, 7, 9, 11, 13 bis 15, 17, 19 bis 21, 23 bis 25, 27 bis 29, 31 bis 33, 35, 51 bis 53, 55, 57, 59, 61, 63 bis 65, 69 bis 71, 73 bis 75, 77 bis 79, 81, 83 und 85 bis 87 und Komplementäre dieser Nukleinsäuresequenzen; oder
(b) eine Allelvariante eines Nukleinsäuremoleküls von (a) ist.

12. Eine therapeutische Zusammensetzung zur Verwendung als ein Zufuhrvehikel, beinhaltend ein isoliertes CHV-Nukleinsäuremolekül nach irgendeinem der Ansprüche 1 bis 11.

13. Eine transfizierte Zelle, die ein isoliertes CHV-Nukleinsäuremolekül nach irgendeinem der Ansprüche 1 bis 11 beinhaltet.

14. Eine Zusammensetzung, die ein rekombinantes Molekül, ein rekombinantes Virus oder eine rekombinante Zelle ist, wobei die Zusammensetzung ein Molekül nach irgendeinem der Ansprüche 1 bis 5 beinhaltet, das operativ mit einer Transkriptionssteuersequenz verbunden ist.

15. Ein rekombinanter Vektor, beinhaltend ein CHV-Nukleinsäuremolekül nach irgendeinem der Ansprüche 1 bis 5, wobei das CHV-Nukleinsäuremolekül ein heterologes Nukleinsäuremolekül beinhaltet, das operativ mit einer Transkriptionssteuersequenz verbunden ist.

16. Eine Hunde-Zelllinie, die komplementär zu einem CHV-Nukleinsäuremolekül nach Anspruch 6 ist, das einen Defekt in einem Gen aufweist, das wesentlich für die CHV-Reproduktion in vitro ist.

17. Eine Zelllinie nach Anspruch 16, wobei das essentielle Gen irgendeins der folgenden ist:
| | |
|---|---|
| ein CgD-Gen | SEQ ID NR: 1, 11, 51 oder 53 |
| ein CgB-Gen | |
| ein CUL48-Gen | SEQ ID NR: 21, 24, 63 oder 67, |
| ein CUL52-Gen | SEQ ID NR: 25, 28, 85 oder 87, |
| ein CgL-Gen | SE ID NR: 74 oder 75, und |
| ein CICP4-Gen | SEQ ID NR: 69, 71 oder 73. |

18. Ein isoliertes CHV-Protein, bestehend aus einer Aminosäuresequenz, ausgewählt aus:
| | |
|---|---|
| einem CdUTPase-Protein | SEQ ID NR: 16 oder 80, |
| ein CgE-Protein | SEQ ID NR: 58, |
| ein CgG-Protein | SEQ ID NR: 10, |
| ein CgI-Protein | SEQ ID NR: 30 oder 56, |
| ein CPK-Protein | SEQ ID NR: 8, |
| ein CUS2-Protein | SEQ ID NR: 6, |
| ein CUS9-Protein | SEQ ID NR: 18 oder 62, |
| ein CUL49-Protein | SEQ ID NR: 66 oder 84, |
| ein CUL51-Protein | SEQ ID NR: 34, |
| ein CgL-Protein | SEQ ID NR: 76, und |
| ein CUL49,5-Protein | SEQ ID NR: 82. |

19. Ein isoliertes CHV-Protein, das mindestens zu 80 % identisch mit einem CHV-Protein ist, das irgendeine der Aminosäuresequenzen SEQ ID NR: 4, 68, 72 und 88 aufweist.

20. Ein isoliertes CHV-Protein, beinhaltend eine Aminosäuresequenz, die mindestens zu 80 % identisch mit irgendeiner der Aminosäuresequenzen SEQ ID NR: 4, 6, 8, 10, 16, 18, 30, 34, 56, 58, 60, 62, 66, 68, 72, 80, 82, 84 und 88, ist.

21. Ein Protein nach Anspruch 20, welches entweder:
(a) irgendeine der Aminosäuresequenzen SEQ ID NR: 4, 6, 8, 10, 16, 18, 30, 34, 56, 58, 60, 62, 66, 68, 72, 76, 80, 82, 84 und 88 beinhaltet; oder
(b) das durch eine Allelvariante eines CHV-Nukleinsäuremoleküls kodiert ist, welches ein CHV-Protein aus (a) kodiert.

22. Ein isolierter Antikörper, der selektiv an ein CHV-Protein nach irgendeinem der Ansprüche 18 bis 21 anbindet.

23. Ein Verfahren zur Erhöhung der Effizienz der Ausbildung rekombinanter CHVplaque, wobei das Verfahren entweder beinhaltet:
(a) Einführen eines rekombinanten CHV-Genoms in eine Hundezelle, die den CHV-Alpha-Transinduktionsfaktor exprimiert und Kultivieren der Zelle, um ein rekombinantes CHV herzustellen; oder
(b) gemeinsames Einführen eines rekombinanten CHV-Genoms und eines CHV-Alpha-Transinduktionsfaktor-Gens in eine Hundezelle und Kultivieren der Zelle, um ein rekombinantes CHV herzustellen.

24. Eine Hundezelllinie, beinhaltend ein CHV-Alpha-Transinduktionsfaktor-Gen.

25. Eine Zelllinie nach Anspruch 24, wobei das Gen das Nukleinsäuremolekül nCUL48₁₂₆₉ SEQ ID NR: 67 beinhaltet.

26. Die Verwendung einer Zelllinie, von Antikörpern, Proteinen, Vektoren, Zellen oder Nukleinsäuremolekülen, nach irgendeinem der vorhergehenden Ansprüche bei der Herstellung einer therapeutischen Zusammensetzung zum Schutz eines Tieres vor einer CHV-Infektion.

## Revendications

1. Molécule d'acide nucléique de l'herpèsvirus canin (CHV) isolée consistant en :
(a) une séquence d'acide nucléique qui est l'une quelconque parmi :
| | |
|---|---|
| un gène CdUTPase | SEQ ID NO: 13, 15, 77 ou 79, |
| un gène CgE | SEQ ID NO: 51 ou 57, |
| un gène CgG | SEQ ID NO: 1, 9 ou 51, |
| un gène CgI | SEQ ID NO: 29, 32, 51 ou 55, |
| un gène CPK | SEQ ID NO: 1, 7 ou 51, |
| un gène CUS2 | SEQ ID NO: 2, 5 ou 52, |
| un gène CUS9 | SEQ ID NO: 17, 20, 51 ou 61, |
| un gène CUL49 | SEQ ID NO: 65, 78 ou 83, |
| un gène CUL51 | SEQ ID NO: 14, 33 ou 78, |
| un gène CgL | SEQ ID NO: 74 ou 75, |
| un gène CUL48.5 | SEQ ID NO: 78 ou 81, |
| un cadre ouvert de lecture CUS8.5 | SEQ ID NO: 51 ou 59, |
SEQ ID NO:3, SEQ ID NO:19, SEQ ID NO:31 et SEQ ID NO:73 ou
(b) une séquence d'acide nucléique qui est entièrement complémentaire d'une séquence d'acide nucléique du (a).

2. Molécule d'acide nucléique du CHV isolée consistant en une séquence d'acide nucléique qui est au moins à 80% identique à une séquence d'acide nucléique de l'une quelconque des SEQ ID NO: 1 à 3, 51, 52, 63, 64, 67, 69 à 71 et 85 à 87 et des compléments de telles séquences d'acide nucléique.

3. Molécule d'acide nucléique du CHV isolée consistant en une séquence d'acide nucléique qui est au moins à 80% identique à une séquence d'acide nucléique de l'une quelconque des SEQ ID NO: 5, 7, 9, 13 à 15, 17, 19, 20, 29, 31 à 33, 55, 57, 59, 61, 65, 73 à 75, 77 à 79, 81 et 83 et des compléments de telles séquences d'acide nucléique.

4. Molécule d'acide nucléique isolée :
(a) soit consistant en une séquence d'acide nucléique de l'une quelconque des SEQ ID NO: 1 à 3, 5, 7, 9, 13 à 15, 17, 19, 20, 29, 31 à 33, 51, 52, 55, 57, 59, 61, 65, 73 à 75, 77 à 79, 81 et 83 et des compléments de telles séquences d'acide nucléique, ou
(b) soit qui est une variante allélique d'une molécule d'acide nucléique du CHV consistant en une quelconque séquence d'acide nucléique.

5. Molécule d'acide nucléique du CHV isolée, qui code pour :
(a) soit une protéine consistant en une séquence d'acides aminés de l'une quelconque des SEQ ID NO: 4, 6, 8, 10, 16, 18, 30, 34, 56, 58, 60, 62, 66, 76, 80, 82 et 84, ou
(b) soit une protéine codée par une variante allélique de la molécule d'acide nucléique du CHV codant pour la protéine selon (a).

6. Molécule d'acide nucléique du CHV isolée comprenant un génome du CHV recombinant dans laquelle un ou plusieurs des gènes du CHV suivants ont été inactivés :
| | |
|---|---|
| un gène CdUTPase | SEQ ID NO: 13, 15, 77 ou 79 ; |
| un gène CgC ; | |
| un gène CgE | SEQ ID NO: 51 ou 57 ; |
| un gène CgG | SEQ ID NO: 1, 9 ou 51 ; |
| un gène CgI | SEQ ID NO: 29, 32, 51 ou 55 ; |
| un gène CPK | SEQ ID NO: 1, 7 ou 51 ; |
| un gène CTK | SEQ ID NO: 35 ou 38 ; |
| un gène CIR6 | SEQ ID NO: 2, 3 ou 52 ; |
| un gène CUS2 | SEQ ID NO: 2, 5 ou 52 ; |
| un gène CUS9 | SEQ ID NO: 17, 20, 51 ou 61 ; |
| un gène CUL49 | SEQ ID NO: 63, 65, 78 ou 83 ; |
| un gène CUL51 | SEQ ID NO: 14, 33 ou 78 ; |
| un gène CUL45 ; | |
| un gène CgD | SEQ ID NO: 1, 11, 51 ou 53 : |
| un gène CgB ; | |
| un gène CUL48 | SEQ ID NO: 21, 24, 63 ou 67 ; |
| un gène CUL52 | SEQ ID NO: 25, 28, 85 ou 87 ; |
| un gène CgL | SEQ ID NO: 74 ou 75 ; |
| un gène CUL49.5 | SEQ ID NO: 78 ou 81 ; |
| un gène CICP0 | SEQ ID NO: 69 ou 73 ; |
| un gène CICP4 | SEQ ID NO: 69, 71 ou 73 ; |
| un gène CUS8.5 | SEQ ID NO: 51 ou 59. |

7. Molécule d'acide nucléique du CHV isolée selon la revendication 6, comprenant en outre une molécule d'acide nucléique hétérologue opérativement liée à une région de contrôle de la transcription.

8. Molécule d'acide nucléique du CHV isolée selon la revendication 7, dans laquelle la molécule d'acide nucléique hétérologue code pour un composé protecteur qui est une protéine protectrice ou une espèce d'ARN protecteur, ledit composé protecteur étant capable de protéger un canidé contre une maladie infectieuse, métabolique ou génétique.

9. Molécule d'acide nucléique du CHV isolée comprenant un génome du CHV recombinant possédant une molécule d'acide nucléique hétérologue dans une région du génome qui est :
(a) un gène du CHV qui est l'un quelconque parmi :
| | |
|---|---|
| un gène CdUTPase | SEQ ID NO: 13, 15, 77 ou 79 ; |
| un gène CgC ; | |
| un gène CgE | SEQ ID NO: 51 ou 57 ; |
| un gène CgG | SEQ ID NO: 1, 9 ou 51; |
| un gène CgI | SEQ ID NO: 29, 32, 51 ou 55 ; |
| un gène CPK | SEQ ID NO: 1, 7 ou 51 ; |
| un gène CTK | SEQ ID NO: 35 ou 38 ; |
| un gène CIR6 | SEQ ID NO: 2, 3 ou 52 ; |
| un gène CUS2 | SEQ ID NO: 2, 5 ou 52 ; |
| un gène CUS9 | SEQ ID NO: 17, 20, 51 ou 61 ; |
| un gène CUL49 | SEQ ID NO: 63, 65, 78 ou 83 ; |
| un gène CUL51 | SEQ ID NO: 14, 33 ou 78 ; |
| un gène CUL45 ; | |
| un gène CgD | SEQ ID NO: 1, 11, 51 ou 53 : |
| un gène CgB ; | |
| un gène CUL48 | SEQ ID NO:21, 24, 63 ou 67 ; |
| un gène CUL52 | SEQ ID NO: 25, 28, 85 ou 87 ; |
| un gène CgL | SEQ ID NO: 74 ou 75 ; |
| un gène CUL49.5 | SEQ ID NO: 78 ou 81 ; |
| un gène CICP0 | SEQ ID NO: 69 ou 73 ; |
| un gène CICP4 | SEQ ID NO:69, 71 ou 73 ; |
| un cadre ouvert de lecture CUS8.5 | SEQ ID NO: 51 ou 59 ; |
(b) un site de l'endonucléase de restriction Asc I se présentant naturellement dans le génome ;
(c) une région US du génome qui est SEQ ID NO: 1, 2, 17, 19, 29 ou 31 ou
(d) une région répétée inversée interne du génome qui est l'une quelconque parmi :
| | |
|---|---|
| nCAsc₉₃₀₀ | SEQ ID NO: 1, 2, 7, 19, 29 ou 31, |
| nCAsc₁₀₀₀₀ | SEQ ID NO: 1, 2, 7, 19, 29 ou 31, |
| nCHin₃₀₀₀ | SEQ ID NO: 1, 2, 7, 19, 29 ou 31, |
| nCHin₁₉₀₀ | SEQ ID NO: 63, 64, 77, 78, 85 ou 86, |
| nCHin₅₅₀₀ | SEQ ID NO: 63, 64, 77, 78, 85 ou 86 et |
| nCHin₈₅₀₀ | SEQ ID NO: 63, 64, 77, 78, 85 ou 86. |

10. Molécule d'acide nucléique du CHV isolée selon la revendication 9, comprenant en outre une molécule d'acide nucléique hétérologue dans une région dudit génome du CHV qui est l'une quelconque parmi :
| | |
|---|---|
| une région US du CHV comprenant nCUS₅₄₉₅ | SEQ ID NO: 1 ou 2, |
| un gène CgE comprenant nCgE₇₅₀ | SEQ ID NO: 51 ou 52, |
| un gène CgI comprenant nCgI₁₆₁ | SEQ ID NO: 29 ou 31, |
| gène CUS9 comprenant nCUS9₅₇₉ | SEQ ID NO: 17 ou 19, |
| région UL du CHV comprenant nCdUTP/CUL51₇₄₃ | SEQ ID NO: 13 ou 14, |
| gène CTK comprenant nCTK₂₈₀ | SEQ ID NO: 35 ou 37, |
| gène CUL48 comprenant nCUL48₂₉₄ | SEQ ID NO: 21 ou 23, |
| gène CUL49 inclus dans nCHin₃₀₀₀ | SEQ ID NO: 63, 64, 77 ou 78, |
| gène CUL52 comprenant CUL52₁₄₆ | SEQ ID NO: 25 ou 27, |
| région du CHV comprenant nCUS₁₀₅₉₂ | SEQ ID NO: 51 ou 52, |
| région UL du CHV comprenant nCUL₁₈₂₃ | SEQ ID NO: 77 ou 78, |
| région UL du CHV comprenant nCUL49/CUL48₂₀₄₄ | SEQ ID NO: 63 ou 64, |
| région IR du CHV comprenant nCICP4₆₂₆ | SEQ ID NO: 69 ou 70, |
| région UL du CHV comprenant nCgL₆₅₅ | SEQ ID NO: 73 ou 74, |
| région UL du CHV comprenant nCUL52₇₄₉ | SEQ ID NO: 85 ou 86, |
| région UL du CHV comprenant CdUTP₃₂₀₀ | SEQ ID NO: 13 ou 14 ou |
| une variante allélique d'une telle région. | |

11. Molécule d'acide nucléique du CHV isolée selon l'une quelconque des revendications 9 ou 10, comprenant en outre une molécule d'acide nucléique hétérologue dans une région du génome du CHV qui est :
(a) une molécule d'acide nucléique comprenant une séquence d'acide nucléique de l'une quelconque de SEQ ID NO: 1 à 3, 5, 7, 9, 11, 13 à 15, 17, 19 à 21, 23 à 25, 27 à 29, 31 à 33, 35, 51 à 53, 55, 57, 59, 61, 63 à 65, 69 à 71, 73 à 75, 77 à 79, 81, 83 et 85 à 87 et des compléments desdites séquences d'acide nucléique, ou
(b) une variante allélique d'une molécule d'acide nucléique de (a).

12. Composition thérapeutique pour l'utilisation en tant que véhicule d'administration, comprenant une molécule d'acide nucléique du CHV isolée selon l'une quelconque des revendications 1 à 11.

13. Cellule transfectée comprenant une molécule d'acide nucléique du CHV isolée selon l'une quelconque des revendications 1 à 11.

14. Composition qui est une molécule recombinante, un virus recombinant ou une cellule recombinante, laquelle composition comprend une molécule selon l'une quelconque des revendications 1 à 5 opérativement liée à une séquence de contrôle de la transcription.

15. Vecteur recombinant comprenant une molécule d'acide nucléique du CHV selon l'une quelconque des revendications 1 à 5, laquelle molécule d'acide nucléique du CHV comprend une molécule d'acide nucléique hétérologue étant opérativement liée à une séquence de contrôle de la transcription.

16. Lignée cellulaire canine qui complète une molécule d'acide nucléique du CHV selon la revendication 6, possédant une anomalie dans un gène essentiel à la reproduction du CHV *in vitro.*

17. Lignée cellulaire selon la revendication 16, dans laquelle le gène essentiel est l'un quelconque parmi :
| | |
|---|---|
| un gène CgD | SEQ ID NO: 1, 11, 51 ou 53, |
| un gène CgB, | |
| un gène CUL48 | SEQ ID NO: 21, 24, 63 ou 67, |
| un gène CUL52 | SEQ ID NO: 25, 28, 85 ou 87, |
| un gène CgL | SEQ ID NO: 74 ou 75 et |
| un gène CICP4 | SEQ ID NO: 69, 71 ou 73. |

18. Protéine du CHV isolée consistant en une séquence d'acides aminés choisie parmi:
| | |
|---|---|
| une protéine CdUTPase | SEQ ID NO: 16 ou 80, |
| une protéine CgE | SEQ ID NO: 58, |
| une protéine CgG | SEQ ID NO: 10, |
| une protéine CgI | SEQ ID NO: 30 ou 56, |
| une protéine CPK | SEQ ID NO: 8, |
| une protéine CUS2 | SEQ ID NO: 6, |
| une protéine CUS 9 | SEQ ID NO: 18 ou 62, |
| une protéine CUL49 | SEQ ID NO: 66 ou 84, |
| une protéine CUL51 | SEQ ID NO: 34, |
| une protéine CgL | SEQ ID NO: 76 et |
| une protéine CUL49.5 | SEQ ID NO: 82. |

19. Protéine du CHV isolée qui est au moins à 80% identique à une protéine du CHV possédant une séquence d'acides aminés de l'une quelconque des SEQ ID NO: 4, 68, 72 et 88.

20. Protéine du CHV isolée comprenant une séquence d'acides aminés qui est au moins à 80% identique à une séquence d'acides aminés de l'une quelconque des SEQ ID NO: 4, 6, 8, 10, 16, 18, 30, 34, 56, 58, 60, 62, 66, 68, 72, 76, 80, 82, 84 et 88.

21. Protéine selon la revendication 20 :
(a) soit comprenant une séquence d'acides aminés de l'une quelconque des SEQ ID NO: 4, 6, 8, 10, 16, 18, 30, 34, 56, 58, 60, 62, 66, 68, 72, 76, 80, 82, 84 et 88,
(b) soit qui est codée par une variante allélique d'une molécule d'acide nucléique du CHV codant pour une protéine du CHV de (a).

22. Anticorps isolé qui se lie sélectivement à une protéine du CHV selon l'une quelconque des revendications 18 à 21.

23. Procédé pour accroître l'efficacité à former des plages du CHV recombinant, lequel procédé comprend :
(a) soit l'introduction d'un génome recombinant du CHV dans une cellule canine exprimant le facteur alpha d'induction trans du CHV et la mise en culture de la cellule pour produire un CHV recombinant,
(b) soit la co-introduction d'un génome recombinant du CHV et d'un gène du facteur alpha d'induction trans du CHV dans une cellule canine et la mise en culture de la cellule pour produire le CHV recombinant.

24. Lignée cellulaire canine comprenant un gène du facteur alpha d'induction trans du CHV.

25. Lignée cellulaire selon la revendication 24, dans laquelle le gène comprend la molécule d'acide nucléique nCUL48₁₂₆₉ SEQ ID NO: 67.

26. Utilisation d'une lignée cellulaire, d'un anticorps, d'une protéine, d'un vecteur, d'une cellule ou d'une molécule d'acide nucléique selon une revendication précédente quelconque dans la préparation d'une composition thérapeutique pour la protection d'un animal contre l'infection par le CHV.
